# EUROPEAN PATENT APPLICATION

(11) **EP 2 706 118 A1**
(43) Date of publication of application: **12.03.2014**
(21) Application number: 12183949.2
(22) Date of filing: 11.09.2012
(51) Int. Cl.: C12P 13/00, C12N 9/10, C12N 9/04, C12N 9/06

(54) **Method for the production of primary amines**

(71) Applicant: BASF SE, 67056 Ludwigshafen (DE); Rijksuniversiteit Groningen, 9712 Groningen (NL)
(72) Inventor: Baldenius, Kai-Uwe Dr., 69115 Heidelberg (DE); Ditrich, Klaus Prof. Dr., 67161 Gönnheim (DE); Breuer, Michael Dr., 64285 Darmstadt (DE); Navickas, Vaidotas Dr., 68199 Mannheim (DE); Jansen, Dirk Prof., 9301 KP Roden (DE); Bartsch, Sebastian Dr., 04103 Leipzig (DE); Dr. Crismaru, Ciprian Gica, 9982 JG Uithuizermeeden (NL)
(74) Representative: Reitstötter - Kinzebach

(57) **Abstract**

The present invention relates to a novel enzymatically catalyzed method for the production of aliphatic primary amines, which method comprises the enzymatic oxidation of a primary aliphatic alcohol catalyzed by an alcohol dehydrogenase, amination of the resulting oxocompound catalyzed by a transaminase and optionally coupled with an enzymatically catalyzed regeneration of the consumed cofactor; recombinant expression systems and microorganisms procuring the required enzyme activities; and bioreactors for performing such methods; as well as the use of the obtained amines in organic synthesis, in particular for preparing organic polymers.

SEQUENCE LISTING

<110>  BASF SE

<120>  Method for the production of primary amines

<130>  M/53272

<160>  70

<170>  PatentIn version 3.3

<210>  1
<211>  1362
<212>  DNA
<213>  Vibrio fluvialis

<400>  1
atgaacaaac cgcaaagctg ggaagcccgg gccgagacct attcgctcta tggtttcacc      60

gacatgcctt cgctgcatca gcgcggcacg gtcgtcgtga cccatggcga gggaccctat     120

atcgtcgatg tgaatggccg gcgttatctg gacgccaact cgggcctgtg gaacatggtc     180

gcgggctttg accacaaggg gctgatcgac gccgccaagg cccaatacga gcgttttccc     240

ggttatcacg cctttttcgg ccgcatgtcc gatcagacgg taatgctgtc ggaaaagctg     300

gtcgaggtgt cgccctttga ttcgggccgg gtgttctata caaactcggg gtccgaggcg     360

aatgacacca tggtcaagat gctatggttc ctgcatgcag ccgagggcaa accgcaaaag     420

cgcaagatcc tgacccgctg gaacgcctat cacggcgtga ccgccgtttc ggccagcatg     480

accggcaagc cctataattc ggtctttggc ctgccgctgc cgggctttgt gcatctgacc     540

tgcccgcatt actggcgcta tggcgaagag ggcgaaaccg aagagcagtt cgtcgcccgc     600

ctcgcccgcg agctggagga aacgatccag cgcgagggcg ccgacaccat cgccggtttc     660

tttgccgaac cggtgatggg cgcgggcggc gtgattcccc cggccaaggg gtatttccag     720

gcgatcctgc caatcctgcg caaatatgac atcccggtca tctcggacga ggtgatctgc     780

ggtttcggac gcaccggtaa cacctggggc tgcgtgacct atgactttac acccgatgca     840

atcatctcgt ccaagaatct tacagcgggc tttttcccca tgggggcggt gatccttggc     900

ccggaacttt ccaaacggct ggaaaccgca atcgaggcga tcgaggaatt cccccatggc     960

tttaccgcct cgggccatcc ggtcggctgt gctattgcgc tgaaagcaat cgacgtggtg    1020

atgaatgaag ggctggctga gaacgtccgc cgccttgccc cccgtttcga ggaaaggctg    1080

aaacatatcg ccgagcgccc gaacatcggt gaatatcgcg gcatcggctt catgtgggcg    1140

ctggaggctg tcaaggacaa ggcaagcaag acgccgttcg acggcaacct gtcggtcagc    1200

gagcgtatcg ccaatacctg caccgatctg gggctgattt gccggccgct tggtcagtcc    1260

gtcgtccttt gtccgccctt tatcctgacc gaggcgcaga tggatgagat gttcgataaa    1320

ctcgaaaaag cccttgataa ggtctttgcc gaggttgcct ga                       1362

<210>  2
<211>  1362
<212>  DNA
<213>  Vibrio fluvialis

<220>
<221>  CDS
<222>  (1)..(1362)

<400>  2
atg aat aaa ccg caa agc tgg gaa gcg cgt gcg gaa acg tac agc ttg        48
Met Asn Lys Pro Gln Ser Trp Glu Ala Arg Ala Glu Thr Tyr Ser Leu
1               5                   10                  15

tac ggc ttt acc gat atg ccg agc ctg cac cag cgc ggt acc gtt gtc        96
Tyr Gly Phe Thr Asp Met Pro Ser Leu His Gln Arg Gly Thr Val Val
            20                  25                  30

gtc acg cat ggt gag ggc ccg tat att gtc gat gtg aat ggc cgt cgt       144
Val Thr His Gly Glu Gly Pro Tyr Ile Val Asp Val Asn Gly Arg Arg
        35                  40                  45

tac ttg gat gcg aat tct ggt ctg tgg aac atg gtt gct ggt ttt gat       192
Tyr Leu Asp Ala Asn Ser Gly Leu Trp Asn Met Val Ala Gly Phe Asp
    50                  55                  60

cac aag ggc ctg att gac gcg gcg aag gcc cag tat gag cgt ttt ccg       240
His Lys Gly Leu Ile Asp Ala Ala Lys Ala Gln Tyr Glu Arg Phe Pro
65                  70                  75                  80

ggt tat cac gcg ttc ttc ggc cgt atg agc gac cag acc gtc atg ttg       288
Gly Tyr His Ala Phe Phe Gly Arg Met Ser Asp Gln Thr Val Met Leu
                85                  90                  95

agc gaa aaa ctg gtc gag gtt tcg ccg ttc gac agc ggt cgc gtt ttc       336
Ser Glu Lys Leu Val Glu Val Ser Pro Phe Asp Ser Gly Arg Val Phe
            100                 105                 110

tat acc aac agc ggt agc gag gcg aac gat acc atg gtt aag atg ctg       384
Tyr Thr Asn Ser Gly Ser Glu Ala Asn Asp Thr Met Val Lys Met Leu
        115                 120                 125

tgg ttc ctg cat gcc gcg gaa ggt aaa cct cag aag cgc aag att ttg       432
Trp Phe Leu His Ala Ala Glu Gly Lys Pro Gln Lys Arg Lys Ile Leu
    130                 135                 140

acg cgc tgg aac gcg tat cac ggt gtc acg gcg gtg agc gcg agc atg       480
Thr Arg Trp Asn Ala Tyr His Gly Val Thr Ala Val Ser Ala Ser Met
145                 150                 155                 160

acg ggt aaa ccg tac aat tcc gtg ttt ggt ctg cct ctg ccg ggt ttt       528
Thr Gly Lys Pro Tyr Asn Ser Val Phe Gly Leu Pro Leu Pro Gly Phe
                165                 170                 175

gtt cac ctg acg tgc ccg cac tac tgg cgc tac ggc gaa gag ggt gag       576
Val His Leu Thr Cys Pro His Tyr Trp Arg Tyr Gly Glu Glu Gly Glu
            180                 185                 190

act gaa gag caa ttt gtc gcc cgt ctg gcg cgt gaa ctg gaa gaa acc       624
Thr Glu Glu Gln Phe Val Ala Arg Leu Ala Arg Glu Leu Glu Glu Thr
        195                 200                 205

atc cag cgc gaa ggc gcg gac act atc gcc ggt ttc ttt gcc gag ccg       672
Ile Gln Arg Glu Gly Ala Asp Thr Ile Ala Gly Phe Phe Ala Glu Pro

210                 215                 220

gtt atg ggt gct ggt ggc gtg atc ccg cca gcg aaa ggt tat ttc caa       720
Val Met Gly Ala Gly Gly Val Ile Pro Pro Ala Lys Gly Tyr Phe Gln
225                 230                 235                 240

gca atc ctg ccg att ctg cgt aag tac gac att ccg gtg att agc gat       768
Ala Ile Leu Pro Ile Leu Arg Lys Tyr Asp Ile Pro Val Ile Ser Asp
                245                 250                 255

gag gtg atc tgt ggc ttc ggt cgt acc ggt aat acc tgg ggt tgt gtg       816
Glu Val Ile Cys Gly Phe Gly Arg Thr Gly Asn Thr Trp Gly Cys Val
            260                 265                 270

acc tac gat ttc acc cca gac gca atc atc tct agc aaa aac ctg act       864
Thr Tyr Asp Phe Thr Pro Asp Ala Ile Ile Ser Ser Lys Asn Leu Thr
        275                 280                 285

gca ggt ttc ttt cca atg ggc gct gtc atc ctg ggt ccg gag ctg agc       912
Ala Gly Phe Phe Pro Met Gly Ala Val Ile Leu Gly Pro Glu Leu Ser
    290                 295                 300

aag cgt ctg gaa acg gcc atc gag gcc att gaa gag ttc ccg cac ggt       960
Lys Arg Leu Glu Thr Ala Ile Glu Ala Ile Glu Glu Phe Pro His Gly
305                 310                 315                 320

ttc acc gcg agc ggc cat ccg gtg ggc tgc gcg att gcg ctg aaa gcg      1008
Phe Thr Ala Ser Gly His Pro Val Gly Cys Ala Ile Ala Leu Lys Ala
                325                 330                 335

att gac gtg gtt atg aac gaa ggt ctg gca gaa aac gtc cgt cgt ttg      1056
Ile Asp Val Val Met Asn Glu Gly Leu Ala Glu Asn Val Arg Arg Leu
            340                 345                 350

gca ccg cgc ttt gaa gag cgc ctg aaa cat att gca gag cgt ccg aac      1104
Ala Pro Arg Phe Glu Glu Arg Leu Lys His Ile Ala Glu Arg Pro Asn
        355                 360                 365

atc ggt gag tac cgt ggc att ggt ttt atg tgg gcg ctg gaa gca gtg      1152
Ile Gly Glu Tyr Arg Gly Ile Gly Phe Met Trp Ala Leu Glu Ala Val
    370                 375                 380

aag gac aaa gct tcc aag acc ccg ttc gac ggc aat ctg agc gtg tct      1200
Lys Asp Lys Ala Ser Lys Thr Pro Phe Asp Gly Asn Leu Ser Val Ser
385                 390                 395                 400

gag cgc att gcg aat acg tgc acc gat ctg ggc ctg atc tgc cgt ccg      1248
Glu Arg Ile Ala Asn Thr Cys Thr Asp Leu Gly Leu Ile Cys Arg Pro
                405                 410                 415

ttg ggc caa tcc gtt gtt ctg tgt ccg ccg ttt atc ctg acc gag gca      1296
Leu Gly Gln Ser Val Val Leu Cys Pro Pro Phe Ile Leu Thr Glu Ala
            420                 425                 430

cag atg gac gag atg ttt gat aaa ctg gag aaa gca ctg gac aag gtg      1344
Gln Met Asp Glu Met Phe Asp Lys Leu Glu Lys Ala Leu Asp Lys Val
        435                 440                 445

ttc gct gag gtt gcc taa                                              1362
Phe Ala Glu Val Ala
    450

<210>  3
<211>  453

<212>  PRT
<213>  Vibrio fluvialis

<400>  3

Met Asn Lys Pro Gln Ser Trp Glu Ala Arg Ala Glu Thr Tyr Ser Leu
1               5                   10                  15

Tyr Gly Phe Thr Asp Met Pro Ser Leu His Gln Arg Gly Thr Val Val
            20                  25                  30

Val Thr His Gly Glu Gly Pro Tyr Ile Val Asp Val Asn Gly Arg Arg
        35                  40                  45

Tyr Leu Asp Ala Asn Ser Gly Leu Trp Asn Met Val Ala Gly Phe Asp
    50                  55                  60

His Lys Gly Leu Ile Asp Ala Ala Lys Ala Gln Tyr Glu Arg Phe Pro
65                  70                  75                  80

Gly Tyr His Ala Phe Phe Gly Arg Met Ser Asp Gln Thr Val Met Leu
                85                  90                  95

Ser Glu Lys Leu Val Glu Val Ser Pro Phe Asp Ser Gly Arg Val Phe
            100                 105                 110

Tyr Thr Asn Ser Gly Ser Glu Ala Asn Asp Thr Met Val Lys Met Leu
        115                 120                 125

Trp Phe Leu His Ala Ala Glu Gly Lys Pro Gln Lys Arg Lys Ile Leu
    130                 135                 140

Thr Arg Trp Asn Ala Tyr His Gly Val Thr Ala Val Ser Ala Ser Met
145                 150                 155                 160

Thr Gly Lys Pro Tyr Asn Ser Val Phe Gly Leu Pro Leu Pro Gly Phe
                165                 170                 175

Val His Leu Thr Cys Pro His Tyr Trp Arg Tyr Gly Glu Glu Gly Glu
            180                 185                 190

Thr Glu Glu Gln Phe Val Ala Arg Leu Ala Arg Glu Leu Glu Glu Thr
        195                 200                 205

Ile Gln Arg Glu Gly Ala Asp Thr Ile Ala Gly Phe Phe Ala Glu Pro
    210                 215                 220

Val Met Gly Ala Gly Gly Val Ile Pro Pro Ala Lys Gly Tyr Phe Gln
225                 230                 235                 240

Ala Ile Leu Pro Ile Leu Arg Lys Tyr Asp Ile Pro Val Ile Ser Asp
                245                 250                 255

Glu Val Ile Cys Gly Phe Gly Arg Thr Gly Asn Thr Trp Gly Cys Val
            260                 265                 270

Thr Tyr Asp Phe Thr Pro Asp Ala Ile Ile Ser Ser Lys Asn Leu Thr
        275                 280                 285

Ala Gly Phe Phe Pro Met Gly Ala Val Ile Leu Gly Pro Glu Leu Ser
    290                 295                 300

Lys Arg Leu Glu Thr Ala Ile Glu Ala Ile Glu Glu Phe Pro His Gly
305                 310                 315                 320

Phe Thr Ala Ser Gly His Pro Val Gly Cys Ala Ile Ala Leu Lys Ala
                325                 330                 335

Ile Asp Val Val Met Asn Glu Gly Leu Ala Glu Asn Val Arg Arg Leu
            340                 345                 350

Ala Pro Arg Phe Glu Glu Arg Leu Lys His Ile Ala Glu Arg Pro Asn
        355                 360                 365

Ile Gly Glu Tyr Arg Gly Ile Gly Phe Met Trp Ala Leu Glu Ala Val
    370                 375                 380

Lys Asp Lys Ala Ser Lys Thr Pro Phe Asp Gly Asn Leu Ser Val Ser
385                 390                 395                 400

Glu Arg Ile Ala Asn Thr Cys Thr Asp Leu Gly Leu Ile Cys Arg Pro
                405                 410                 415

Leu Gly Gln Ser Val Val Leu Cys Pro Pro Phe Ile Leu Thr Glu Ala
            420                 425                 430

Gln Met Asp Glu Met Phe Asp Lys Leu Glu Lys Ala Leu Asp Lys Val
        435                 440                 445

Phe Ala Glu Val Ala
    450

<210>  4
<211>  473
<212>  PRT
<213>  Artificial

<220>
<223>  Vf-omegaTA with Hs Tag

<400>  4

Met Gly Ser Ser His His His His His His Ser Ser Gly Leu Val Pro
1               5                   10                  15

Arg Gly Ser His Met Asn Lys Pro Gln Ser Trp Glu Ala Arg Ala Glu
            20                  25                  30

Thr Tyr Ser Leu Tyr Gly Phe Thr Asp Met Pro Ser Leu His Gln Arg
        35                  40                  45

Gly Thr Val Val Val Thr His Gly Glu Gly Pro Tyr Ile Val Asp Val
    50                  55                  60

Asn Gly Arg Arg Tyr Leu Asp Ala Asn Ser Gly Leu Trp Asn Met Val
65                  70                  75                  80

Ala Gly Phe Asp His Lys Gly Leu Ile Asp Ala Ala Lys Ala Gln Tyr
                85                  90                  95

Glu Arg Phe Pro Gly Tyr His Ala Phe Phe Gly Arg Met Ser Asp Gln
            100                 105                 110

Thr Val Met Leu Ser Glu Lys Leu Val Glu Val Ser Pro Phe Asp Ser
        115                 120                 125

Gly Arg Val Phe Tyr Thr Asn Ser Gly Ser Glu Ala Asn Asp Thr Met
    130                 135                 140

Val Lys Met Leu Trp Phe Leu His Ala Ala Glu Gly Lys Pro Gln Lys
145                 150                 155                 160

Arg Lys Ile Leu Thr Arg Trp Asn Ala Tyr His Gly Val Thr Ala Val
                165                 170                 175

Ser Ala Ser Met Thr Gly Lys Pro Tyr Asn Ser Val Phe Gly Leu Pro
            180                 185                 190

Leu Pro Gly Phe Val His Leu Thr Cys Pro His Tyr Trp Arg Tyr Gly
        195                 200                 205

Glu Glu Gly Glu Thr Glu Glu Gln Phe Val Ala Arg Leu Ala Arg Glu
    210                 215                 220

Leu Glu Glu Thr Ile Gln Arg Glu Gly Ala Asp Thr Ile Ala Gly Phe
225                 230                 235                 240

Phe Ala Glu Pro Val Met Gly Ala Gly Gly Val Ile Pro Pro Ala Lys
                245                 250                 255

Gly Tyr Phe Gln Ala Ile Leu Pro Ile Leu Arg Lys Tyr Asp Ile Pro
            260                 265                 270

Val Ile Ser Asp Glu Val Ile Cys Gly Phe Gly Arg Thr Gly Asn Thr
        275                 280                 285

Trp Gly Cys Val Thr Tyr Asp Phe Thr Pro Asp Ala Ile Ile Ser Ser
    290                 295                 300

Lys Asn Leu Thr Ala Gly Phe Phe Pro Met Gly Ala Val Ile Leu Gly
305                 310                 315                 320

Pro Glu Leu Ser Lys Arg Leu Glu Thr Ala Ile Glu Ala Ile Glu Glu
                325                 330                 335

Phe Pro His Gly Phe Thr Ala Ser Gly His Pro Val Gly Cys Ala Ile
            340                 345                 350

Ala Leu Lys Ala Ile Asp Val Val Met Asn Glu Gly Leu Ala Glu Asn
        355                 360                 365

Val Arg Arg Leu Ala Pro Arg Phe Glu Glu Arg Leu Lys His Ile Ala
    370                 375                 380

Glu Arg Pro Asn Ile Gly Glu Tyr Arg Gly Ile Gly Phe Met Trp Ala
385                 390                 395                 400

Leu Glu Ala Val Lys Asp Lys Ala Ser Lys Thr Pro Phe Asp Gly Asn
                405                 410                 415

Leu Ser Val Ser Glu Arg Ile Ala Asn Thr Cys Thr Asp Leu Gly Leu
            420                 425                 430

Ile Cys Arg Pro Leu Gly Gln Ser Val Val Leu Cys Pro Pro Phe Ile
        435                 440                 445

Leu Thr Glu Ala Gln Met Asp Glu Met Phe Asp Lys Leu Glu Lys Ala
    450                 455                 460

Leu Asp Lys Val Phe Ala Glu Val Ala
465                 470

<210>  5
<211>  1380
<212>  DNA
<213>  Chromobacterium violaceum

<220>
<221>  CDS
<222>  (1)..(1380)

<400> 5
atg cag aag caa cgt acg acc agc caa tgg cgc gaa ctg gat gcc gcc      48
Met Gln Lys Gln Arg Thr Thr Ser Gln Trp Arg Glu Leu Asp Ala Ala
1               5                   10                  15

cat cac ctg cat ccg ttc acc gat acc gca tcg ctg aac cag gcg ggc      96
His His Leu His Pro Phe Thr Asp Thr Ala Ser Leu Asn Gln Ala Gly
            20                  25                  30

gcg cgc gtg atg acg cgc gga gag ggc gtc tac ctg tgg gat tcg gaa      144
Ala Arg Val Met Thr Arg Gly Glu Gly Val Tyr Leu Trp Asp Ser Glu
        35                  40                  45

ggc aac aag atc atc gac ggc atg gcc gga ctg tgg tgc gtg aac gtc      192
Gly Asn Lys Ile Ile Asp Gly Met Ala Gly Leu Trp Cys Val Asn Val
    50                  55                  60

ggc tac ggc cgc aag gac ttt gcc gaa gcg gcg cgc cgg cag atg gaa      240
Gly Tyr Gly Arg Lys Asp Phe Ala Glu Ala Ala Arg Arg Gln Met Glu
65                  70                  75                  80

gag ctg ccg ttc tac aac acc ttc ttc aag acc acc cat ccg gcg gtg      288
Glu Leu Pro Phe Tyr Asn Thr Phe Phe Lys Thr Thr His Pro Ala Val
                85                  90                  95

gtc gag ctg tcc agc ctg ctg gct gaa gtg acg ccg gcc ggt ttc gac      336
Val Glu Leu Ser Ser Leu Leu Ala Glu Val Thr Pro Ala Gly Phe Asp
            100                 105                 110

cgc gtg ttc tat acc aat tcc ggt tcc gaa tcg gtg gac acc atg atc      384
Arg Val Phe Tyr Thr Asn Ser Gly Ser Glu Ser Val Asp Thr Met Ile
        115                 120                 125

cgc atg gtg cgc cgc tac tgg gac gtg cag ggc aag ccg gag aag aag      432
Arg Met Val Arg Arg Tyr Trp Asp Val Gln Gly Lys Pro Glu Lys Lys
    130                 135                 140

acg ctg atc ggc cgc tgg aac ggc tat cac ggc tcc acc atc ggc ggc      480
Thr Leu Ile Gly Arg Trp Asn Gly Tyr His Gly Ser Thr Ile Gly Gly
145                 150                 155                 160

gcc agc ctg ggc ggc atg aag tac atg cac gag cag ggc gac ttg ccg      528
Ala Ser Leu Gly Gly Met Lys Tyr Met His Glu Gln Gly Asp Leu Pro
                165                 170                 175

att ccg ggc atg gcc cac atc gag cag cct tgg tgg tac aag cac ggc      576
Ile Pro Gly Met Ala His Ile Glu Gln Pro Trp Trp Tyr Lys His Gly
            180                 185                 190

aag gac atg acg ccg gac gag ttc ggc gtg gtg gcc gcg cgc tgg ctg      624
Lys Asp Met Thr Pro Asp Glu Phe Gly Val Val Ala Ala Arg Trp Leu
        195                 200                 205

gaa gag aag att ctg gaa atc ggc gcc gac aag gtg gcc gcc ttc gtc      672
Glu Glu Lys Ile Leu Glu Ile Gly Ala Asp Lys Val Ala Ala Phe Val
    210                 215                 220

ggc gaa ccc atc cag ggc gcc ggc ggc gtg atc gtc ccg ccg gcc acc      720
Gly Glu Pro Ile Gln Gly Ala Gly Gly Val Ile Val Pro Pro Ala Thr
225                 230                 235                 240

tac tgg ccg gaa atc gag cgc att tgc cgc aag tac gac gtg ctg ctg      768
Tyr Trp Pro Glu Ile Glu Arg Ile Cys Arg Lys Tyr Asp Val Leu Leu
                245                 250                 255

gtg gcc gac gaa gtg atc tgc ggc ttc ggg cgt acc ggc gaa tgg ttc     816
Val Ala Asp Glu Val Ile Cys Gly Phe Gly Arg Thr Gly Glu Trp Phe
            260                 265                 270

ggc cat cag cat ttc ggc ttc cag ccc gac ctg ttc acc gcc gcc aag     864
Gly His Gln His Phe Gly Phe Gln Pro Asp Leu Phe Thr Ala Ala Lys
        275                 280                 285

ggc ctg tcc tcc ggc tat ctg ccg ata ggc gcg gtc ttt gtc ggc aag     912
Gly Leu Ser Ser Gly Tyr Leu Pro Ile Gly Ala Val Phe Val Gly Lys
    290                 295                 300

cgc gtg gcc gaa ggc ctg atc gcc ggc ggc gac ttc aac cac ggc ttc     960
Arg Val Ala Glu Gly Leu Ile Ala Gly Gly Asp Phe Asn His Gly Phe
305                 310                 315                 320

acc tac tcc ggc cac ccg gtc tgc gcc gcc gtc gcc cac gcc aac gtg     1008
Thr Tyr Ser Gly His Pro Val Cys Ala Ala Val Ala His Ala Asn Val
                325                 330                 335

gcg gcg ctg cgc gac gag ggc atc gtc cag cgc gtc aag gac gac atc     1056
Ala Ala Leu Arg Asp Glu Gly Ile Val Gln Arg Val Lys Asp Asp Ile
            340                 345                 350

ggc ccg tac atg caa aag cgc tgg cgt gaa acc ttc agc cgt ttc gag     1104
Gly Pro Tyr Met Gln Lys Arg Trp Arg Glu Thr Phe Ser Arg Phe Glu
        355                 360                 365

cat gtg gac gac gtg cgc ggc gtc ggc atg gtg cag gcg ttc acc ctg     1152
His Val Asp Asp Val Arg Gly Val Gly Met Val Gln Ala Phe Thr Leu
    370                 375                 380

gtg aag aac aag gcg aag cgc gag ctg ttc ccc gat ttc ggc gag atc     1200
Val Lys Asn Lys Ala Lys Arg Glu Leu Phe Pro Asp Phe Gly Glu Ile
385                 390                 395                 400

ggc acg ctg tgc cgc gac atc ttc ttc cgc aac aac ctg atc atg cgg     1248
Gly Thr Leu Cys Arg Asp Ile Phe Phe Arg Asn Asn Leu Ile Met Arg
                405                 410                 415

gca tgc ggc gac cac atc gtg tcg gcg ccg ccg ctg gtg atg acg cgg     1296
Ala Cys Gly Asp His Ile Val Ser Ala Pro Pro Leu Val Met Thr Arg
            420                 425                 430

gcg gaa gtg gac gag atg ctg gcg gtg gcg gaa cgc tgt ctg gag gaa     1344
Ala Glu Val Asp Glu Met Leu Ala Val Ala Glu Arg Cys Leu Glu Glu
        435                 440                 445

ttc gag cag acg ctg aag gcg cgc ggg ctg gct tag                     1380
Phe Glu Gln Thr Leu Lys Ala Arg Gly Leu Ala
    450                 455

<210>  6
<211>  459
<212>  PRT
<213>  Chromobacterium violaceum

<400>  6

Met Gln Lys Gln Arg Thr Thr Ser Gln Trp Arg Glu Leu Asp Ala Ala
1               5                   10                  15

His His Leu His Pro Phe Thr Asp Thr Ala Ser Leu Asn Gln Ala Gly
20                  25                  30

Ala Arg Val Met Thr Arg Gly Glu Gly Val Tyr Leu Trp Asp Ser Glu
35                  40                  45

Gly Asn Lys Ile Ile Asp Gly Met Ala Gly Leu Trp Cys Val Asn Val
50                  55                  60

Gly Tyr Gly Arg Lys Asp Phe Ala Glu Ala Ala Arg Arg Gln Met Glu
65                  70                  75                  80

Glu Leu Pro Phe Tyr Asn Thr Phe Phe Lys Thr Thr His Pro Ala Val
                85                  90                  95

Val Glu Leu Ser Ser Leu Leu Ala Glu Val Thr Pro Ala Gly Phe Asp
            100                 105                 110

Arg Val Phe Tyr Thr Asn Ser Gly Ser Glu Ser Val Asp Thr Met Ile
        115                 120                 125

Arg Met Val Arg Arg Tyr Trp Asp Val Gln Gly Lys Pro Glu Lys Lys
    130                 135                 140

Thr Leu Ile Gly Arg Trp Asn Gly Tyr His Gly Ser Thr Ile Gly Gly
145                 150                 155                 160

Ala Ser Leu Gly Gly Met Lys Tyr Met His Glu Gln Gly Asp Leu Pro
                165                 170                 175

Ile Pro Gly Met Ala His Ile Glu Gln Pro Trp Trp Tyr Lys His Gly
            180                 185                 190

Lys Asp Met Thr Pro Asp Glu Phe Gly Val Val Ala Ala Arg Trp Leu
        195                 200                 205

Glu Glu Lys Ile Leu Glu Ile Gly Ala Asp Lys Val Ala Ala Phe Val
    210                 215                 220

Gly Glu Pro Ile Gln Gly Ala Gly Gly Val Ile Val Pro Pro Ala Thr
225                 230                 235                 240

Tyr Trp Pro Glu Ile Glu Arg Ile Cys Arg Lys Tyr Asp Val Leu Leu
                245                 250                 255

Val Ala Asp Glu Val Ile Cys Gly Phe Gly Arg Thr Gly Glu Trp Phe
            260                 265                 270

Gly His Gln His Phe Gly Phe Gln Pro Asp Leu Phe Thr Ala Ala Lys

275                 280                 285

Gly Leu Ser Ser Gly Tyr Leu Pro Ile Gly Ala Val Phe Val Gly Lys
    290                 295                 300

Arg Val Ala Glu Gly Leu Ile Ala Gly Gly Asp Phe Asn His Gly Phe
305                 310                 315                 320

Thr Tyr Ser Gly His Pro Val Cys Ala Ala Val Ala His Ala Asn Val
                325                 330                 335

Ala Ala Leu Arg Asp Glu Gly Ile Val Gln Arg Val Lys Asp Asp Ile
            340                 345                 350

Gly Pro Tyr Met Gln Lys Arg Trp Arg Glu Thr Phe Ser Arg Phe Glu
        355                 360                 365

His Val Asp Asp Val Arg Gly Val Gly Met Val Gln Ala Phe Thr Leu
    370                 375                 380

Val Lys Asn Lys Ala Lys Arg Glu Leu Phe Pro Asp Phe Gly Glu Ile
385                 390                 395                 400

Gly Thr Leu Cys Arg Asp Ile Phe Phe Arg Asn Asn Leu Ile Met Arg
                405                 410                 415

Ala Cys Gly Asp His Ile Val Ser Ala Pro Pro Leu Val Met Thr Arg
            420                 425                 430

Ala Glu Val Asp Glu Met Leu Ala Val Ala Glu Arg Cys Leu Glu Glu
        435                 440                 445

Phe Glu Gln Thr Leu Lys Ala Arg Gly Leu Ala
    450                 455

<210>  7
<211>  479
<212>  PRT
<213>  Artificial

<220>
<223>  Cv-omega TA + His Tag

<400>  7

Met Gly Ser Ser His His His His His His Ser Ser Gly Leu Val Pro
1               5                   10                  15

Arg Gly Ser His Met Gln Lys Gln Arg Thr Thr Ser Gln Trp Arg Glu
            20                  25                  30

Leu Asp Ala Ala His His Leu His Pro Phe Thr Asp Thr Ala Ser Leu
        35                  40                  45

Asn Gln Ala Gly Ala Arg Val Met Thr Arg Gly Glu Gly Val Tyr Leu
    50                  55                  60

Trp Asp Ser Glu Gly Asn Lys Ile Ile Asp Gly Met Ala Gly Leu Trp
65                  70                  75                  80

Cys Val Asn Val Gly Tyr Gly Arg Lys Asp Phe Ala Glu Ala Ala Arg
                85                  90                  95

Arg Gln Met Glu Glu Leu Pro Phe Tyr Asn Thr Phe Phe Lys Thr Thr
            100                 105                 110

His Pro Ala Val Val Glu Leu Ser Ser Leu Leu Ala Glu Val Thr Pro
        115                 120                 125

Ala Gly Phe Asp Arg Val Phe Tyr Thr Asn Ser Gly Ser Glu Ser Val
    130                 135                 140

Asp Thr Met Ile Arg Met Val Arg Arg Tyr Trp Asp Val Gln Gly Lys
145                 150                 155                 160

Pro Glu Lys Lys Thr Leu Ile Gly Arg Trp Asn Gly Tyr His Gly Ser
                165                 170                 175

Thr Ile Gly Gly Ala Ser Leu Gly Gly Met Lys Tyr Met His Glu Gln
            180                 185                 190

Gly Asp Leu Pro Ile Pro Gly Met Ala His Ile Glu Gln Pro Trp Trp
        195                 200                 205

Tyr Lys His Gly Lys Asp Met Thr Pro Asp Glu Phe Gly Val Val Ala
    210                 215                 220

Ala Arg Trp Leu Glu Glu Lys Ile Leu Glu Ile Gly Ala Asp Lys Val
225                 230                 235                 240

Ala Ala Phe Val Gly Glu Pro Ile Gln Gly Ala Gly Gly Val Ile Val
                245                 250                 255

Pro Pro Ala Thr Tyr Trp Pro Glu Ile Glu Arg Ile Cys Arg Lys Tyr
            260                 265                 270

Asp Val Leu Leu Val Ala Asp Glu Val Ile Cys Gly Phe Gly Arg Thr
        275                 280                 285

Gly Glu Trp Phe Gly His Gln His Phe Gly Phe Gln Pro Asp Leu Phe

290                 295                 300

Thr Ala Ala Lys Gly Leu Ser Ser Gly Tyr Leu Pro Ile Gly Ala Val
305                 310                 315                 320

Phe Val Gly Lys Arg Val Ala Glu Gly Leu Ile Ala Gly Gly Asp Phe
                325                 330                 335

Asn His Gly Phe Thr Tyr Ser Gly His Pro Val Cys Ala Ala Val Ala
            340                 345                 350

His Ala Asn Val Ala Ala Leu Arg Asp Glu Gly Ile Val Gln Arg Val
        355                 360                 365

Lys Asp Asp Ile Gly Pro Tyr Met Gln Lys Arg Trp Arg Glu Thr Phe
    370                 375                 380

Ser Arg Phe Glu His Val Asp Asp Val Arg Gly Val Gly Met Val Gln
385                 390                 395                 400

Ala Phe Thr Leu Val Lys Asn Lys Ala Lys Arg Glu Leu Phe Pro Asp
                405                 410                 415

Phe Gly Glu Ile Gly Thr Leu Cys Arg Asp Ile Phe Phe Arg Asn Asn
            420                 425                 430

Leu Ile Met Arg Ala Cys Gly Asp His Ile Val Ser Ala Pro Pro Leu
        435                 440                 445

Val Met Thr Arg Ala Glu Val Asp Glu Met Leu Ala Val Ala Glu Arg
    450                 455                 460

Cys Leu Glu Glu Phe Glu Gln Thr Leu Lys Ala Arg Gly Leu Ala
465                 470                 475

<210>  8
<211>  36
<212>  DNA
<213>  Artificial

<220>
<223>  Forward Primer

<400>  8
gcgcgcatat gcagaagcaa cgtacgacca gccaat                                36

<210>  9
<211>  37
<212>  DNA
<213>  Artificial

<220>

<223>  Reverse Primer

<400>  9
gatattgtac tcgagctaag ccagcccgcg cgccttc                              37


<210>  10
<211>  1113
<212>  DNA
<213>  Rhodococcus jostii


<220>
<221>  CDS
<222>  (1)..(1113)

<400>  10
atg aag acc aaa gca gcg gtt ctg ttc gaa acg cac aag ccg ttc gaa       48
Met Lys Thr Lys Ala Ala Val Leu Phe Glu Thr His Lys Pro Phe Glu
1               5                   10                  15

atc gtg gaa ctc gag ctc gac ggt ccg ggc cct ggc gaa gtg ctc atc       96
Ile Val Glu Leu Glu Leu Asp Gly Pro Gly Pro Gly Glu Val Leu Ile
            20                  25                  30

aag tat gtc gcg gca ggc ctg tgc cac tcg gac ctg cac ctc acc gac      144
Lys Tyr Val Ala Ala Gly Leu Cys His Ser Asp Leu His Leu Thr Asp
        35                  40                  45

ggc gat ctg ccg ccg cgt ttc ccg atc gtc ggc ggg cac gag ggt tcc      192
Gly Asp Leu Pro Pro Arg Phe Pro Ile Val Gly Gly His Glu Gly Ser
    50                  55                  60

ggc atc atc gag gaa gtc ggc ccc ggt gtc acc aag gtg aag ccc ggc      240
Gly Ile Ile Glu Glu Val Gly Pro Gly Val Thr Lys Val Lys Pro Gly
65                  70                  75                  80

gac cac gtg gtc tgc agc ttc atc ccg aac tgc ggc acg tgt cgg tac      288
Asp His Val Val Cys Ser Phe Ile Pro Asn Cys Gly Thr Cys Arg Tyr
                85                  90                  95

tgt gcc acc ggc cgc cag aac ctg tgt gac atg ggc gcc acc atc ctc      336
Cys Ala Thr Gly Arg Gln Asn Leu Cys Asp Met Gly Ala Thr Ile Leu
            100                 105                 110

gag ggg tcg atg ccc gac ggc tcg ttc cgg ttc cac tcg ggc aag cag      384
Glu Gly Ser Met Pro Asp Gly Ser Phe Arg Phe His Ser Gly Lys Gln
        115                 120                 125

gat ttc ggt gcc atg tgc atg ctc ggt acg ttc gcc gag cgc gcc acc      432
Asp Phe Gly Ala Met Cys Met Leu Gly Thr Phe Ala Glu Arg Ala Thr
    130                 135                 140

atc tcg cag cac tcg gtg gtg aag gtg gac gac tgg ctc ccc ctg gag      480
Ile Ser Gln His Ser Val Val Lys Val Asp Asp Trp Leu Pro Leu Glu
145                 150                 155                 160

acc gcc gtc ctc gtc ggc tgc ggt gtg ccg tcc ggt tgg ggc acc gcg      528
Thr Ala Val Leu Val Gly Cys Gly Val Pro Ser Gly Trp Gly Thr Ala
                165                 170                 175

gtg tac gcc ggc gga gtt cgc gcc ggt gac acg gtc gtc atc tac ggc      576
Val Tyr Ala Gly Gly Val Arg Ala Gly Asp Thr Val Val Ile Tyr Gly
            180                 185                 190

atc ggc ggt ctc ggc atc aac gcc gtg cag ggc gcg gtc agc tcc ggc     624
Ile Gly Gly Leu Gly Ile Asn Ala Val Gln Gly Ala Val Ser Ser Gly
        195                 200                 205

gcc aag tac gtc gtg gtg gtg gat ccg gtg gcg atg aag cgg gag gcc     672
Ala Lys Tyr Val Val Val Val Asp Pro Val Ala Met Lys Arg Glu Ala
    210                 215                 220

gcg ctg aag ttc ggc gcc acc cac gcg ttc gcg gac gcc gcg acc gcg     720
Ala Leu Lys Phe Gly Ala Thr His Ala Phe Ala Asp Ala Ala Thr Ala
225                 230                 235                 240

gcg gag aag gtc aac gaa ctg acc tgg ggt cag ggc gcc gat cag gcc     768
Ala Glu Lys Val Asn Glu Leu Thr Trp Gly Gln Gly Ala Asp Gln Ala
                245                 250                 255

ctc atc ctg gtg ggc acc gtc gac gaa gag gtg gtg cag aac gcg acc     816
Leu Ile Leu Val Gly Thr Val Asp Glu Glu Val Val Gln Asn Ala Thr
            260                 265                 270

gcc gtc atc ggc aag ggc ggc acc gtc gtc atc acc ggc ctc gcc gac     864
Ala Val Ile Gly Lys Gly Gly Thr Val Val Ile Thr Gly Leu Ala Asp
        275                 280                 285

ccc gcc aag ctc acc gtg cac atc tcg gga acc gac ctg acc ctg aac     912
Pro Ala Lys Leu Thr Val His Ile Ser Gly Thr Asp Leu Thr Leu Asn
    290                 295                 300

cag aag acg atc aag ggt tcg ctg ttc ggt tcg gcc aac ccg cag tac     960
Gln Lys Thr Ile Lys Gly Ser Leu Phe Gly Ser Ala Asn Pro Gln Tyr
305                 310                 315                 320

gac atc gtc cgc ctg ctg cgc ctg tac gac gcg ggt cag ctc aag ctc     1008
Asp Ile Val Arg Leu Leu Arg Leu Tyr Asp Ala Gly Gln Leu Lys Leu
                325                 330                 335

gac gag ctg atc acc acc aag tac acg ctc gag cag gtg aac gag ggt     1056
Asp Glu Leu Ile Thr Thr Lys Tyr Thr Leu Glu Gln Val Asn Glu Gly
            340                 345                 350

tac cag gat ctg cgg gac gga aag aac ctg cgt ggc gtg atc gtc cac     1104
Tyr Gln Asp Leu Arg Asp Gly Lys Asn Leu Arg Gly Val Ile Val His
        355                 360                 365

cag cag tag                                                         1113
Gln Gln
    370


<210>  11
<211>  370
<212>  PRT
<213>  Rhodococcus jostii

<400>  11

Met Lys Thr Lys Ala Ala Val Leu Phe Glu Thr His Lys Pro Phe Glu
1               5                   10                  15


Ile Val Glu Leu Glu Leu Asp Gly Pro Gly Pro Gly Glu Val Leu Ile
            20                  25                  30


Lys Tyr Val Ala Ala Gly Leu Cys His Ser Asp Leu His Leu Thr Asp

35                  40                  45

Gly Asp Leu Pro Pro Arg Phe Pro Ile Val Gly Gly His Glu Gly Ser
    50                  55                  60

Gly Ile Ile Glu Glu Val Gly Pro Gly Val Thr Lys Val Lys Pro Gly
65                  70                  75                  80

Asp His Val Val Cys Ser Phe Ile Pro Asn Cys Gly Thr Cys Arg Tyr
                85                  90                  95

Cys Ala Thr Gly Arg Gln Asn Leu Cys Asp Met Gly Ala Thr Ile Leu
            100                 105                 110

Glu Gly Ser Met Pro Asp Gly Ser Phe Arg Phe His Ser Gly Lys Gln
        115                 120                 125

Asp Phe Gly Ala Met Cys Met Leu Gly Thr Phe Ala Glu Arg Ala Thr
    130                 135                 140

Ile Ser Gln His Ser Val Val Lys Val Asp Asp Trp Leu Pro Leu Glu
145                 150                 155                 160

Thr Ala Val Leu Val Gly Cys Gly Val Pro Ser Gly Trp Gly Thr Ala
                165                 170                 175

Val Tyr Ala Gly Gly Val Arg Ala Gly Asp Thr Val Val Ile Tyr Gly
            180                 185                 190

Ile Gly Gly Leu Gly Ile Asn Ala Val Gln Gly Ala Val Ser Ser Gly
        195                 200                 205

Ala Lys Tyr Val Val Val Val Asp Pro Val Ala Met Lys Arg Glu Ala
    210                 215                 220

Ala Leu Lys Phe Gly Ala Thr His Ala Phe Ala Asp Ala Ala Thr Ala
225                 230                 235                 240

Ala Glu Lys Val Asn Glu Leu Thr Trp Gly Gln Gly Ala Asp Gln Ala
                245                 250                 255

Leu Ile Leu Val Gly Thr Val Asp Glu Glu Val Val Gln Asn Ala Thr
            260                 265                 270

Ala Val Ile Gly Lys Gly Gly Thr Val Val Ile Thr Gly Leu Ala Asp
        275                 280                 285

Pro Ala Lys Leu Thr Val His Ile Ser Gly Thr Asp Leu Thr Leu Asn
    290                 295                 300

Gln Lys Thr Ile Lys Gly Ser Leu Phe Gly Ser Ala Asn Pro Gln Tyr
305                 310                 315                 320

Asp Ile Val Arg Leu Leu Arg Leu Tyr Asp Ala Gly Gln Leu Lys Leu
                325                 330                 335

Asp Glu Leu Ile Thr Thr Lys Tyr Thr Leu Glu Gln Val Asn Glu Gly
            340                 345                 350

Tyr Gln Asp Leu Arg Asp Gly Lys Asn Leu Arg Gly Val Ile Val His
        355                 360                 365

Gln Gln
    370

<210>  12
<211>  390
<212>  PRT
<213>  Artificial

<220>
<223>  Rj_ADH with His Tag

<400>  12

Met Gly Ser Ser His His His His His His Ser Ser Gly Leu Val Pro
1               5                   10                  15

Arg Gly Ser His Met Lys Thr Lys Ala Ala Val Leu Phe Glu Thr His
            20                  25                  30

Lys Pro Phe Glu Ile Val Glu Leu Glu Leu Asp Gly Pro Gly Pro Gly
        35                  40                  45

Glu Val Leu Ile Lys Tyr Val Ala Ala Gly Leu Cys His Ser Asp Leu
    50                  55                  60

His Leu Thr Asp Gly Asp Leu Pro Pro Arg Phe Pro Ile Val Gly Gly
65                  70                  75                  80

His Glu Gly Ser Gly Ile Ile Glu Glu Val Gly Pro Gly Val Thr Lys
                85                  90                  95

Val Lys Pro Gly Asp His Val Val Cys Ser Phe Ile Pro Asn Cys Gly
            100                 105                 110

Thr Cys Arg Tyr Cys Ala Thr Gly Arg Gln Asn Leu Cys Asp Met Gly
        115                 120                 125

Ala Thr Ile Leu Glu Gly Ser Met Pro Asp Gly Ser Phe Arg Phe His

130                 135                 140

Ser Gly Lys Gln Asp Phe Gly Ala Met Cys Met Leu Gly Thr Phe Ala
145                 150                 155                 160

Glu Arg Ala Thr Ile Ser Gln His Ser Val Val Lys Val Asp Asp Trp
                165                 170                 175

Leu Pro Leu Glu Thr Ala Val Leu Val Gly Cys Gly Val Pro Ser Gly
            180                 185                 190

Trp Gly Thr Ala Val Tyr Ala Gly Gly Val Arg Ala Gly Asp Thr Val
        195                 200                 205

Val Ile Tyr Gly Ile Gly Gly Leu Gly Ile Asn Ala Val Gln Gly Ala
    210                 215                 220

Val Ser Ser Gly Ala Lys Tyr Val Val Val Val Asp Pro Val Ala Met
225                 230                 235                 240

Lys Arg Glu Ala Ala Leu Lys Phe Gly Ala Thr His Ala Phe Ala Asp
                245                 250                 255

Ala Ala Thr Ala Ala Glu Lys Val Asn Glu Leu Thr Trp Gly Gln Gly
            260                 265                 270

Ala Asp Gln Ala Leu Ile Leu Val Gly Thr Val Asp Glu Glu Val Val
        275                 280                 285

Gln Asn Ala Thr Ala Val Ile Gly Lys Gly Gly Thr Val Val Ile Thr
    290                 295                 300

Gly Leu Ala Asp Pro Ala Lys Leu Thr Val His Ile Ser Gly Thr Asp
305                 310                 315                 320

Leu Thr Leu Asn Gln Lys Thr Ile Lys Gly Ser Leu Phe Gly Ser Ala
                325                 330                 335

Asn Pro Gln Tyr Asp Ile Val Arg Leu Leu Arg Leu Tyr Asp Ala Gly
            340                 345                 350

Gln Leu Lys Leu Asp Glu Leu Ile Thr Thr Lys Tyr Thr Leu Glu Gln
        355                 360                 365

Val Asn Glu Gly Tyr Gln Asp Leu Arg Asp Gly Lys Asn Leu Arg Gly
    370                 375                 380

Val Ile Val His Gln Gln
385                 390

<210> 13
<211> 32
<212> DNA
<213> Artificial

<220>
<223> PCR Forward Primer

<400> 13
gcgcgcatat gaagaccaaa gcagcggttc tg 32


<210> 14
<211> 33
<212> DNA
<213> Artificial

<220>
<223> PCR Reverse Primer

<400> 14
gagctaagct tctactgctg gtggacgatc acg 33


<210> 15
<211> 1014
<212> DNA
<213> Geobacillus stearothermophilus


<220>
<221> CDS
<222> (1)..(1014)

<400> 15
atg aaa gct gca gtt gtg gaa caa ttt aaa aag ccg tta caa gtg aaa 48
Met Lys Ala Ala Val Val Glu Gln Phe Lys Lys Pro Leu Gln Val Lys
1               5                   10                  15

gaa gtg gaa aaa cct aag atc tca tac ggg gaa gta tta gtg cgc atc 96
Glu Val Glu Lys Pro Lys Ile Ser Tyr Gly Glu Val Leu Val Arg Ile
            20                  25                  30

aaa gcg tgt ggg gta tgc cat aca gac ttg cat gcc gca cat ggc gac 144
Lys Ala Cys Gly Val Cys His Thr Asp Leu His Ala Ala His Gly Asp
        35                  40                  45

tgg cct gta aag cct aaa ctg cct ctc att cct ggc cat gaa ggc gtc 192
Trp Pro Val Lys Pro Lys Leu Pro Leu Ile Pro Gly His Glu Gly Val
    50                  55                  60

ggt gta att gaa gaa gta ggt cct ggg gta aca cat tta aaa gtt gga 240
Gly Val Ile Glu Glu Val Gly Pro Gly Val Thr His Leu Lys Val Gly
65                  70                  75                  80

gat cgc gta ggt atc cct tgg ctt tat tcg gcg tgc ggt cat tgt gac 288
Asp Arg Val Gly Ile Pro Trp Leu Tyr Ser Ala Cys Gly His Cys Asp
                85                  90                  95

tat tgc tta agc gga caa gaa aca tta tgc gaa cgt caa caa aac gct 336
Tyr Cys Leu Ser Gly Gln Glu Thr Leu Cys Glu Arg Gln Gln Asn Ala
            100                 105                 110

ggc tat tcc gtc gat ggt ggt tat gct gaa tat tgc cgt gct gca gcc     384
Gly Tyr Ser Val Asp Gly Gly Tyr Ala Glu Tyr Cys Arg Ala Ala Ala
        115                 120                 125

gat tat gtc gta aaa att cct gat aac tta tcg ttt gaa gaa gcc gct     432
Asp Tyr Val Val Lys Ile Pro Asp Asn Leu Ser Phe Glu Glu Ala Ala
    130                 135                 140

cca atc ttt tgc gct ggt gta aca aca tat aaa gcg ctc aaa gta aca     480
Pro Ile Phe Cys Ala Gly Val Thr Thr Tyr Lys Ala Leu Lys Val Thr
145                 150                 155                 160

ggc gca aaa cca ggt gaa tgg gta gcc att tac ggt atc ggc ggg ctt     528
Gly Ala Lys Pro Gly Glu Trp Val Ala Ile Tyr Gly Ile Gly Gly Leu
                165                 170                 175

gga cat gtc gca gtc caa tac gca aag gcg atg ggg tta aac gtc gtt     576
Gly His Val Ala Val Gln Tyr Ala Lys Ala Met Gly Leu Asn Val Val
            180                 185                 190

gct gtc gat tta ggt gat gaa aaa ctt gag ctt gct aaa caa ctt ggt     624
Ala Val Asp Leu Gly Asp Glu Lys Leu Glu Leu Ala Lys Gln Leu Gly
        195                 200                 205

gca gat ctt gtc gtc aat ccg aaa cat gat gat gca gca caa tgg ata     672
Ala Asp Leu Val Val Asn Pro Lys His Asp Asp Ala Ala Gln Trp Ile
    210                 215                 220

aaa gaa aaa gtg ggc ggt gtg cat gcg act gtc gtc aca gct gtt tca     720
Lys Glu Lys Val Gly Gly Val His Ala Thr Val Val Thr Ala Val Ser
225                 230                 235                 240

aaa gcc gcg ttc gaa tca gcc tac aaa tcc att cgt cgc ggt ggt gct     768
Lys Ala Ala Phe Glu Ser Ala Tyr Lys Ser Ile Arg Arg Gly Gly Ala
                245                 250                 255

tgc gta ctc gtc gga tta ccg ccg gaa gaa ata cct att cca att ttc     816
Cys Val Leu Val Gly Leu Pro Pro Glu Glu Ile Pro Ile Pro Ile Phe
            260                 265                 270

gat aca gta tta aat gga gta aaa att att ggt tct atc gtt ggt acg     864
Asp Thr Val Leu Asn Gly Val Lys Ile Ile Gly Ser Ile Val Gly Thr
        275                 280                 285

cgc aaa gac tta caa gag gca ctt caa ttt gca gca gaa gga aaa gta     912
Arg Lys Asp Leu Gln Glu Ala Leu Gln Phe Ala Ala Glu Gly Lys Val
    290                 295                 300

aaa aca att gtc gaa gtg caa ccg ctt gaa aac att aac gac gta ttc     960
Lys Thr Ile Val Glu Val Gln Pro Leu Glu Asn Ile Asn Asp Val Phe
305                 310                 315                 320

gat cgt atg tta aaa ggg caa att aac ggc cgc gtc gtg tta aaa gta    1008
Asp Arg Met Leu Lys Gly Gln Ile Asn Gly Arg Val Val Leu Lys Val
                325                 330                 335

gat taa                                                            1014
Asp

<210>  16
<211>  337
<212>  PRT
<213>  Geobacillus stearothermophilus

<400>  16

Met Lys Ala Ala Val Val Glu Gln Phe Lys Lys Pro Leu Gln Val Lys
1               5                   10                  15

Glu Val Glu Lys Pro Lys Ile Ser Tyr Gly Glu Val Leu Val Arg Ile
            20                  25                  30

Lys Ala Cys Gly Val Cys His Thr Asp Leu His Ala Ala His Gly Asp
        35                  40                  45

Trp Pro Val Lys Pro Lys Leu Pro Leu Ile Pro Gly His Glu Gly Val
    50                  55                  60

Gly Val Ile Glu Glu Val Gly Pro Gly Val Thr His Leu Lys Val Gly
65                  70                  75                  80

Asp Arg Val Gly Ile Pro Trp Leu Tyr Ser Ala Cys Gly His Cys Asp
                85                  90                  95

Tyr Cys Leu Ser Gly Gln Glu Thr Leu Cys Glu Arg Gln Gln Asn Ala
            100                 105                 110

Gly Tyr Ser Val Asp Gly Gly Tyr Ala Glu Tyr Cys Arg Ala Ala Ala
        115                 120                 125

Asp Tyr Val Val Lys Ile Pro Asp Asn Leu Ser Phe Glu Glu Ala Ala
    130                 135                 140

Pro Ile Phe Cys Ala Gly Val Thr Thr Tyr Lys Ala Leu Lys Val Thr
145                 150                 155                 160

Gly Ala Lys Pro Gly Glu Trp Val Ala Ile Tyr Gly Ile Gly Gly Leu
                165                 170                 175

Gly His Val Ala Val Gln Tyr Ala Lys Ala Met Gly Leu Asn Val Val
            180                 185                 190

Ala Val Asp Leu Gly Asp Glu Lys Leu Glu Leu Ala Lys Gln Leu Gly
        195                 200                 205

Ala Asp Leu Val Val Asn Pro Lys His Asp Asp Ala Ala Gln Trp Ile
    210                 215                 220

Lys Glu Lys Val Gly Gly Val His Ala Thr Val Val Thr Ala Val Ser
225                 230                 235                 240

Lys Ala Ala Phe Glu Ser Ala Tyr Lys Ser Ile Arg Arg Gly Gly Ala
                245                 250                 255

Cys Val Leu Val Gly Leu Pro Pro Glu Glu Ile Pro Ile Pro Ile Phe
                260                 265                 270

Asp Thr Val Leu Asn Gly Val Lys Ile Ile Gly Ser Ile Val Gly Thr
        275                 280                 285

Arg Lys Asp Leu Gln Glu Ala Leu Gln Phe Ala Ala Glu Gly Lys Val
    290                 295                 300

Lys Thr Ile Val Glu Val Gln Pro Leu Glu Asn Ile Asn Asp Val Phe
305                 310                 315                 320

Asp Arg Met Leu Lys Gly Gln Ile Asn Gly Arg Val Val Leu Lys Val
                325                 330                 335

Asp

<210>  17
<211>  357
<212>  PRT
<213>  Artificial

<220>
<223>  Gs_ADH1 with His Tag

<400>  17

Met Gly Ser Ser His His His His His His Ser Ser Gly Leu Val Pro
1               5                   10                  15

Arg Gly Ser His Met Lys Ala Ala Val Val Glu Gln Phe Lys Lys Pro
                20                  25                  30

Leu Gln Val Lys Glu Val Glu Lys Pro Lys Ile Ser Tyr Gly Glu Val
        35                  40                  45

Leu Val Arg Ile Lys Ala Cys Gly Val Cys His Thr Asp Leu His Ala
    50                  55                  60

Ala His Gly Asp Trp Pro Val Lys Pro Lys Leu Pro Leu Ile Pro Gly
65                  70                  75                  80

His Glu Gly Val Gly Val Ile Glu Glu Val Gly Pro Gly Val Thr His
                85                  90                  95

Leu Lys Val Gly Asp Arg Val Gly Ile Pro Trp Leu Tyr Ser Ala Cys
                100                 105                 110

Gly His Cys Asp Tyr Cys Leu Ser Gly Gln Glu Thr Leu Cys Glu Arg

115                 120                 125

Gln Gln Asn Ala Gly Tyr Ser Val Asp Gly Gly Tyr Ala Glu Tyr Cys
    130                 135                 140

Arg Ala Ala Ala Asp Tyr Val Val Lys Ile Pro Asp Asn Leu Ser Phe
145                 150                 155                 160

Glu Glu Ala Ala Pro Ile Phe Cys Ala Gly Val Thr Thr Tyr Lys Ala
                165                 170                 175

Leu Lys Val Thr Gly Ala Lys Pro Gly Glu Trp Val Ala Ile Tyr Gly
            180                 185                 190

Ile Gly Gly Leu Gly His Val Ala Val Gln Tyr Ala Lys Ala Met Gly
        195                 200                 205

Leu Asn Val Val Ala Val Asp Leu Gly Asp Glu Lys Leu Glu Leu Ala
    210                 215                 220

Lys Gln Leu Gly Ala Asp Leu Val Val Asn Pro Lys His Asp Asp Ala
225                 230                 235                 240

Ala Gln Trp Ile Lys Glu Lys Val Gly Gly Val His Ala Thr Val Val
                245                 250                 255

Thr Ala Val Ser Lys Ala Ala Phe Glu Ser Ala Tyr Lys Ser Ile Arg
            260                 265                 270

Arg Gly Gly Ala Cys Val Leu Val Gly Leu Pro Pro Glu Glu Ile Pro
        275                 280                 285

Ile Pro Ile Phe Asp Thr Val Leu Asn Gly Val Lys Ile Ile Gly Ser
    290                 295                 300

Ile Val Gly Thr Arg Lys Asp Leu Gln Glu Ala Leu Gln Phe Ala Ala
305                 310                 315                 320

Glu Gly Lys Val Lys Thr Ile Val Glu Val Gln Pro Leu Glu Asn Ile
                325                 330                 335

Asn Asp Val Phe Asp Arg Met Leu Lys Gly Gln Ile Asn Gly Arg Val
            340                 345                 350

Val Leu Lys Val Asp
        355

<210>  18
<211>  32

<212>  DNA
<213>  Artificial

<220>
<223>  PCR Forward Primer

<400>  18
gcttgaccat atgaaagctg cagttgtgga ac                                    32


<210>  19
<211>  33
<212>  DNA
<213>  Artificial

<220>
<223>  PCR Reverse Primer

<400>  19
ggatcgctcg agttaatcta cttttaacac gac                                   33


<210>  20
<211>  1020
<212>  DNA
<213>  Geobacillus stearothermophilus


<220>
<221>  CDS
<222>  (1)..(1020)

<400>  20
atg aaa gca gca gta gtt aac gaa ttt aaa aaa gca ctt gaa att aaa        48
Met Lys Ala Ala Val Val Asn Glu Phe Lys Lys Ala Leu Glu Ile Lys
1               5                   10                  15

gag gtg gaa aga ccc aaa cta gag gaa ggt gaa gtt tta gtt aag atc        96
Glu Val Glu Arg Pro Lys Leu Glu Glu Gly Glu Val Leu Val Lys Ile
            20                  25                  30

gaa gca tgc ggt gtg tgc cat acg gat tta cat gca gcc cat ggt gac       144
Glu Ala Cys Gly Val Cys His Thr Asp Leu His Ala Ala His Gly Asp
        35                  40                  45

tgg ccg att aaa ccg aaa ttg ccg tta att ccg ggg cat gaa gga gtc       192
Trp Pro Ile Lys Pro Lys Leu Pro Leu Ile Pro Gly His Glu Gly Val
    50                  55                  60

gga att gtt gta gaa gtc gca aag gga gtt aaa tca ata aaa gtg gga       240
Gly Ile Val Val Glu Val Ala Lys Gly Val Lys Ser Ile Lys Val Gly
65                  70                  75                  80

gat cgc gtc ggc atc cca tgg tta tat tct gca tgt ggt gaa tgt gaa       288
Asp Arg Val Gly Ile Pro Trp Leu Tyr Ser Ala Cys Gly Glu Cys Glu
                85                  90                  95

tat tgt tta act ggt caa gaa aca ctt tgc ccg cat caa ttg aat ggc       336
Tyr Cys Leu Thr Gly Gln Glu Thr Leu Cys Pro His Gln Leu Asn Gly
            100                 105                 110

gga tat tcc gtt gat ggc gga tat gcg gaa tac tgt aaa gct ccc gca       384
Gly Tyr Ser Val Asp Gly Gly Tyr Ala Glu Tyr Cys Lys Ala Pro Ala
        115                 120                 125

gat tat gtg gcg aaa att cct gat aac tta gat cca gtt gaa gta gcg 432
Asp Tyr Val Ala Lys Ile Pro Asp Asn Leu Asp Pro Val Glu Val Ala
    130                 135                 140

cca att tta tgt gca ggc gta acg aca tat aaa gcg ttg aaa gta tca 480
Pro Ile Leu Cys Ala Gly Val Thr Thr Tyr Lys Ala Leu Lys Val Ser
145                 150                 155                 160

ggt gct cgg cca ggt gaa tgg gtg gcc att tac ggt att gga gga ctt 528
Gly Ala Arg Pro Gly Glu Trp Val Ala Ile Tyr Gly Ile Gly Gly Leu
                165                 170                 175

ggt cat att gca tta caa tac gcc aaa gcg atg gga ctt aat gtg gta 576
Gly His Ile Ala Leu Gln Tyr Ala Lys Ala Met Gly Leu Asn Val Val
            180                 185                 190

gct gtt gat atc agt gat gaa aaa tca aaa tta gcc aaa gat tta ggt 624
Ala Val Asp Ile Ser Asp Glu Lys Ser Lys Leu Ala Lys Asp Leu Gly
        195                 200                 205

gca gat att gca atc aac ggt tta aaa gaa gat cct gtc aaa gcg ata 672
Ala Asp Ile Ala Ile Asn Gly Leu Lys Glu Asp Pro Val Lys Ala Ile
    210                 215                 220

cat gat caa gta ggc gga gtt cat gct gct att agt gta gcg gtg aat 720
His Asp Gln Val Gly Gly Val His Ala Ala Ile Ser Val Ala Val Asn
225                 230                 235                 240

aaa aaa gca ttt gaa cag gcc tat cag tca gtg aaa cgg gga ggc aca 768
Lys Lys Ala Phe Glu Gln Ala Tyr Gln Ser Val Lys Arg Gly Gly Thr
                245                 250                 255

tta gtt gtt gtc ggt ctg cca aat gct gat ctg cct att ccg atc ttc 816
Leu Val Val Val Gly Leu Pro Asn Ala Asp Leu Pro Ile Pro Ile Phe
            260                 265                 270

gat aca gta tta aac ggc gtt tcc gtt aaa ggt tcc atc gtc ggt aca 864
Asp Thr Val Leu Asn Gly Val Ser Val Lys Gly Ser Ile Val Gly Thr
        275                 280                 285

aga aag gac atg cag gaa gca tta gat ttt gct gct cgc gga aaa gtc 912
Arg Lys Asp Met Gln Glu Ala Leu Asp Phe Ala Ala Arg Gly Lys Val
    290                 295                 300

cgt cca att gtt gaa acc gct gaa tta gaa gaa ata aat gaa gta ttt 960
Arg Pro Ile Val Glu Thr Ala Glu Leu Glu Glu Ile Asn Glu Val Phe
305                 310                 315                 320

gaa aga atg gaa aaa gga aaa atc aac ggt cgt atc gta cta aaa ttg 1008
Glu Arg Met Glu Lys Gly Lys Ile Asn Gly Arg Ile Val Leu Lys Leu
                325                 330                 335

aag gag gat tga 1020
Lys Glu Asp

<210> 21
<211> 339
<212> PRT
<213> Geobacillus stearothermophilus

<400> 21

Met Lys Ala Ala Val Val Asn Glu Phe Lys Lys Ala Leu Glu Ile Lys

1               5                   10                  15

Glu Val Glu Arg Pro Lys Leu Glu Glu Gly Glu Val Leu Val Lys Ile
            20                  25                  30

Glu Ala Cys Gly Val Cys His Thr Asp Leu His Ala Ala His Gly Asp
        35                  40                  45

Trp Pro Ile Lys Pro Lys Leu Pro Leu Ile Pro Gly His Glu Gly Val
    50                  55                  60

Gly Ile Val Val Glu Val Ala Lys Gly Val Lys Ser Ile Lys Val Gly
65                  70                  75                  80

Asp Arg Val Gly Ile Pro Trp Leu Tyr Ser Ala Cys Gly Glu Cys Glu
                85                  90                  95

Tyr Cys Leu Thr Gly Gln Glu Thr Leu Cys Pro His Gln Leu Asn Gly
            100                 105                 110

Gly Tyr Ser Val Asp Gly Gly Tyr Ala Glu Tyr Cys Lys Ala Pro Ala
        115                 120                 125

Asp Tyr Val Ala Lys Ile Pro Asp Asn Leu Asp Pro Val Glu Val Ala
    130                 135                 140

Pro Ile Leu Cys Ala Gly Val Thr Thr Tyr Lys Ala Leu Lys Val Ser
145                 150                 155                 160

Gly Ala Arg Pro Gly Glu Trp Val Ala Ile Tyr Gly Ile Gly Gly Leu
                165                 170                 175

Gly His Ile Ala Leu Gln Tyr Ala Lys Ala Met Gly Leu Asn Val Val
            180                 185                 190

Ala Val Asp Ile Ser Asp Glu Lys Ser Lys Leu Ala Lys Asp Leu Gly
        195                 200                 205

Ala Asp Ile Ala Ile Asn Gly Leu Lys Glu Asp Pro Val Lys Ala Ile
    210                 215                 220

His Asp Gln Val Gly Gly Val His Ala Ala Ile Ser Val Ala Val Asn
225                 230                 235                 240

Lys Lys Ala Phe Glu Gln Ala Tyr Gln Ser Val Lys Arg Gly Gly Thr
                245                 250                 255

Leu Val Val Val Gly Leu Pro Asn Ala Asp Leu Pro Ile Pro Ile Phe
            260                 265                 270

Asp Thr Val Leu Asn Gly Val Ser Val Lys Gly Ser Ile Val Gly Thr
        275                 280                 285

Arg Lys Asp Met Gln Glu Ala Leu Asp Phe Ala Ala Arg Gly Lys Val
    290                 295                 300

Arg Pro Ile Val Glu Thr Ala Glu Leu Glu Glu Ile Asn Glu Val Phe
305                 310                 315                 320

Glu Arg Met Glu Lys Gly Lys Ile Asn Gly Arg Ile Val Leu Lys Leu
                325                 330                 335

Lys Glu Asp

<210>  22
<211>  359
<212>  PRT
<213>  Artificial

<220>
<223>  Gs_ADH2 with His Tag

<400>  22

Met Gly Ser Ser His His His His His His Ser Ser Gly Leu Val Pro
1               5                   10                  15

Arg Gly Ser His Met Lys Ala Ala Val Val Asn Glu Phe Lys Lys Ala
            20                  25                  30

Leu Glu Ile Lys Glu Val Glu Arg Pro Lys Leu Glu Glu Gly Glu Val
        35                  40                  45

Leu Val Lys Ile Glu Ala Cys Gly Val Cys His Thr Asp Leu His Ala
    50                  55                  60

Ala His Gly Asp Trp Pro Ile Lys Pro Lys Leu Pro Leu Ile Pro Gly
65                  70                  75                  80

His Glu Gly Val Gly Ile Val Val Glu Val Ala Lys Gly Val Lys Ser
                85                  90                  95

Ile Lys Val Gly Asp Arg Val Gly Ile Pro Trp Leu Tyr Ser Ala Cys
            100                 105                 110

Gly Glu Cys Glu Tyr Cys Leu Thr Gly Gln Glu Thr Leu Cys Pro His
        115                 120                 125

Gln Leu Asn Gly Gly Tyr Ser Val Asp Gly Gly Tyr Ala Glu Tyr Cys

130                 135                 140

Lys Ala Pro Ala Asp Tyr Val Ala Lys Ile Pro Asp Asn Leu Asp Pro
145                 150                 155                 160

Val Glu Val Ala Pro Ile Leu Cys Ala Gly Val Thr Thr Tyr Lys Ala
                165                 170                 175

Leu Lys Val Ser Gly Ala Arg Pro Gly Glu Trp Val Ala Ile Tyr Gly
            180                 185                 190

Ile Gly Gly Leu Gly His Ile Ala Leu Gln Tyr Ala Lys Ala Met Gly
        195                 200                 205

Leu Asn Val Val Ala Val Asp Ile Ser Asp Glu Lys Ser Lys Leu Ala
    210                 215                 220

Lys Asp Leu Gly Ala Asp Ile Ala Ile Asn Gly Leu Lys Glu Asp Pro
225                 230                 235                 240

Val Lys Ala Ile His Asp Gln Val Gly Gly Val His Ala Ala Ile Ser
                245                 250                 255

Val Ala Val Asn Lys Lys Ala Phe Glu Gln Ala Tyr Gln Ser Val Lys
            260                 265                 270

Arg Gly Gly Thr Leu Val Val Val Gly Leu Pro Asn Ala Asp Leu Pro
        275                 280                 285

Ile Pro Ile Phe Asp Thr Val Leu Asn Gly Val Ser Val Lys Gly Ser
    290                 295                 300

Ile Val Gly Thr Arg Lys Asp Met Gln Glu Ala Leu Asp Phe Ala Ala
305                 310                 315                 320

Arg Gly Lys Val Arg Pro Ile Val Glu Thr Ala Glu Leu Glu Glu Ile
                325                 330                 335

Asn Glu Val Phe Glu Arg Met Glu Lys Gly Lys Ile Asn Gly Arg Ile
            340                 345                 350

Val Leu Lys Leu Lys Glu Asp
        355

<210>  23
<211>  32
<212>  DNA
<213>  Artificial

<220>

<223>  PCR Forward Primer

<400>  23
gcttgaccat atgaaagcag cagtagttaa cg                                    32


<210>  24
<211>  37
<212>  DNA
<213>  Artificial

<220>
<223>  PCR Reverse Primer

<400>  24
ggatcgctcg agtcaatcct ccttcaattt tagtacg                               37


<210>  25
<211>  1011
<212>  DNA
<213>  Pseudomonas putida

<400>  25
atgaaagctg ctgtcgttgc accaggccgt cgcgtggacg tggtagagaa aaccctgcgc      60

cctctcgaac acggcgaagc gctgctgaag atgcagtgct gcggtgtgtg ccacaccgac     120

ctgcacgtga aaaatggcga ctttggtgac aagaccggtg tagtactggg ccacgaaggt     180

attggcgtgg tccaggaagt tggcccgggc gtcacctcgc tcaagccagg cgaccgtgcc     240

agcgttgcct ggttctacca gggttgcgga cattgcgaat attgcaacag cggcaacgag     300

accctgtgcc gcgacgtgaa gaattccggc tacaccgtgg acggcggcat ggccgaggcc     360

tgcatcgtca aggccgacta ctcggttaaa gtgcccgacg gcctcgactc cgctgccgcc     420

agcagcatca cctgcgccgg cgtcacgacc tacaaggcag tgaaaatctc caacgtccgc     480

cccggccaat ggatcgccat ctacgggctc ggcggcctgg gcaacctggc cctgcaatat     540

gccaagaatg tgttcaacgc caaagtgatc gccatcgacg tcaacgaaga gcaactgcgc     600

ttcgccagcg agatgggcgc ggatctggtc gtcaatccgc tcaacgaaga cgccgctaag     660

ttcatccagg ccaagaccgg tggcgcacat gctgccgtcg ttactgccgt ggccaaaggc     720

gcgttcaatt ctgccgtcga tgccttgcgc gctggcgggc gactggtggc cgtcgggctt     780

ccgtcggagt ccatggacct gaatattcct cgcctggtgc ttgatgggat cgaagtggtg     840

ggctcgctgg taggtacacg ccaggacctg caggaagcct tccagtttgc cgctgaaggc     900

aaagtggtgc ccaaggtaac gctgcgaccg atcgagcata tcaaccagat ctttgacgag     960

atgctggagg gcaagatcaa aggccggatg gtgatacagt tcgaaggctg a              1011


<210>  26
<211>  1017
<212>  DNA
<213>  Artificial

<220>

<223>  Pp_ADH NS codon optimized


<220>
<221>  CDS
<222>  (1)..(1017)

<400>  26
atg aaa gca gcg gtg gtc gca cct ggt cgt cgt gtg gac gta gtc gag        48
Met Lys Ala Ala Val Val Ala Pro Gly Arg Arg Val Asp Val Val Glu
1               5                   10                  15

aaa acc ctg cgt ccg ctg gaa cat ggt gag gcg ctg ctg aag atg caa        96
Lys Thr Leu Arg Pro Leu Glu His Gly Glu Ala Leu Leu Lys Met Gln
            20                  25                  30

tgt tgt ggt gtc tgc cac acg gat ttg cac gtg aag aac ggc gac ttt       144
Cys Cys Gly Val Cys His Thr Asp Leu His Val Lys Asn Gly Asp Phe
        35                  40                  45

ggt gat aag acc ggt gtt gtc ctg ggc cat gaa ggc atc ggc gtg gtt       192
Gly Asp Lys Thr Gly Val Val Leu Gly His Glu Gly Ile Gly Val Val
    50                  55                  60

caa gag gtc ggt ccg ggt gtt acc agc ttg aaa ccg ggc gac cgt gca       240
Gln Glu Val Gly Pro Gly Val Thr Ser Leu Lys Pro Gly Asp Arg Ala
65                  70                  75                  80

agc gtc gcg tgg ttc tat cag ggc tgc ggc cac tgt gag tat tgc aac       288
Ser Val Ala Trp Phe Tyr Gln Gly Cys Gly His Cys Glu Tyr Cys Asn
                85                  90                  95

tcg ggt aat gaa act ttg tgc cgc gat gtg aaa aac agc ggt tac acg       336
Ser Gly Asn Glu Thr Leu Cys Arg Asp Val Lys Asn Ser Gly Tyr Thr
            100                 105                 110

gtt gac ggt ggc atg gcc gaa gcg tgc att gtc aag gcg gac tac agc       384
Val Asp Gly Gly Met Ala Glu Ala Cys Ile Val Lys Ala Asp Tyr Ser
        115                 120                 125

gtt aaa gtg cca gat ggt ctg gac agc gca gct gcc tcc agc att acc       432
Val Lys Val Pro Asp Gly Leu Asp Ser Ala Ala Ala Ser Ser Ile Thr
    130                 135                 140

tgt gcg ggc gtg acc acc tac aaa gcg gtg aag atc agc aat gtt cgt       480
Cys Ala Gly Val Thr Thr Tyr Lys Ala Val Lys Ile Ser Asn Val Arg
145                 150                 155                 160

cga tcg ccg ggt cag tgg att gct att tac ggt ctg ggt ggt ctg ggc       528
Arg Ser Pro Gly Gln Trp Ile Ala Ile Tyr Gly Leu Gly Gly Leu Gly
                165                 170                 175

aac ctg gca ctg caa tat gcg aag aat gtg ttc aat gcg aag gtt atc       576
Asn Leu Ala Leu Gln Tyr Ala Lys Asn Val Phe Asn Ala Lys Val Ile
            180                 185                 190

gcg att gat gtc aat gaa gag cag ttg cgt ttc gcc agc gaa atg ggt       624
Ala Ile Asp Val Asn Glu Glu Gln Leu Arg Phe Ala Ser Glu Met Gly
        195                 200                 205

gcg gat ctg gtt gtt aac ccg ctg aac gag gac gcc gca aag ttt atc       672
Ala Asp Leu Val Val Asn Pro Leu Asn Glu Asp Ala Ala Lys Phe Ile
    210                 215                 220

cag gca aaa acg ggt ggc gcg cat gcc gcg gta gtg acc gcg gtt gcg       720

Gln Ala Lys Thr Gly Gly Ala His Ala Ala Val Val Thr Ala Val Ala
225                 230                 235                 240

aaa ggc gca ttc aat tcc gcc gtg gac gcc ttg cgc gct ggc ggt cgc       768
Lys Gly Ala Phe Asn Ser Ala Val Asp Ala Leu Arg Ala Gly Gly Arg
                245                 250                 255

ctg gtt gca gtt ggc ctg ccg agc gag tct atg gat ctg aat atc ccg       816
Leu Val Ala Val Gly Leu Pro Ser Glu Ser Met Asp Leu Asn Ile Pro
            260                 265                 270

cgt ctg gtt ctg gac ggt att gag gtc gtc ggc agc ctg gtg ggt acc       864
Arg Leu Val Leu Asp Gly Ile Glu Val Val Gly Ser Leu Val Gly Thr
        275                 280                 285

cgc cag gat ctg cag gaa gcg ttt cag ttt gcg gct gaa ggt aaa gtc       912
Arg Gln Asp Leu Gln Glu Ala Phe Gln Phe Ala Ala Glu Gly Lys Val
    290                 295                 300

gtg ccg aag gtt acg ctg cgt ccg att gag cac att aac caa atc ttt       960
Val Pro Lys Val Thr Leu Arg Pro Ile Glu His Ile Asn Gln Ile Phe
305                 310                 315                 320

gac gag atg ctg gaa ggt aag atc aaa ggt cgc atg gtg atc caa ttc      1008
Asp Glu Met Leu Glu Gly Lys Ile Lys Gly Arg Met Val Ile Gln Phe
                325                 330                 335

gag ggt taa                                                          1017
Glu Gly

<210>  27
<211>  338
<212>  PRT
<213>  Artificial

<220>
<223>  Synthetic Construct

<400>  27

Met Lys Ala Ala Val Val Ala Pro Gly Arg Arg Val Asp Val Val Glu
1               5                   10                  15

Lys Thr Leu Arg Pro Leu Glu His Gly Glu Ala Leu Leu Lys Met Gln
            20                  25                  30

Cys Cys Gly Val Cys His Thr Asp Leu His Val Lys Asn Gly Asp Phe
        35                  40                  45

Gly Asp Lys Thr Gly Val Val Leu Gly His Glu Gly Ile Gly Val Val
    50                  55                  60

Gln Glu Val Gly Pro Gly Val Thr Ser Leu Lys Pro Gly Asp Arg Ala
65                  70                  75                  80

Ser Val Ala Trp Phe Tyr Gln Gly Cys Gly His Cys Glu Tyr Cys Asn
                85                  90                  95

Ser Gly Asn Glu Thr Leu Cys Arg Asp Val Lys Asn Ser Gly Tyr Thr
        100                 105                 110

Val Asp Gly Gly Met Ala Glu Ala Cys Ile Val Lys Ala Asp Tyr Ser
    115                 120                 125

Val Lys Val Pro Asp Gly Leu Asp Ser Ala Ala Ala Ser Ser Ile Thr
    130                 135                 140

Cys Ala Gly Val Thr Thr Tyr Lys Ala Val Lys Ile Ser Asn Val Arg
145                 150                 155                 160

Arg Ser Pro Gly Gln Trp Ile Ala Ile Tyr Gly Leu Gly Gly Leu Gly
                165                 170                 175

Asn Leu Ala Leu Gln Tyr Ala Lys Asn Val Phe Asn Ala Lys Val Ile
        180                 185                 190

Ala Ile Asp Val Asn Glu Glu Gln Leu Arg Phe Ala Ser Glu Met Gly
        195                 200                 205

Ala Asp Leu Val Val Asn Pro Leu Asn Glu Asp Ala Ala Lys Phe Ile
    210                 215                 220

Gln Ala Lys Thr Gly Gly Ala His Ala Ala Val Val Thr Ala Val Ala
225                 230                 235                 240

Lys Gly Ala Phe Asn Ser Ala Val Asp Ala Leu Arg Ala Gly Gly Arg
                245                 250                 255

Leu Val Ala Val Gly Leu Pro Ser Glu Ser Met Asp Leu Asn Ile Pro
        260                 265                 270

Arg Leu Val Leu Asp Gly Ile Glu Val Val Gly Ser Leu Val Gly Thr
        275                 280                 285

Arg Gln Asp Leu Gln Glu Ala Phe Gln Phe Ala Ala Glu Gly Lys Val
    290                 295                 300

Val Pro Lys Val Thr Leu Arg Pro Ile Glu His Ile Asn Gln Ile Phe
305                 310                 315                 320

Asp Glu Met Leu Glu Gly Lys Ile Lys Gly Arg Met Val Ile Gln Phe
                325                 330                 335

Glu Gly

<210>  28
<211>  356
<212>  PRT
<213>  Pseudomonas putida

<400>  28

Met Gly Ser Ser His His His His His His Ser Ser Gly Leu Val Pro
1               5                   10                  15

Arg Gly Ser His Met Lys Ala Ala Val Val Ala Pro Gly Arg Arg Val
            20                  25                  30

Asp Val Val Glu Lys Thr Leu Arg Pro Leu Glu His Gly Glu Ala Leu
        35                  40                  45

Leu Lys Met Gln Cys Cys Gly Val Cys His Thr Asp Leu His Val Lys
    50                  55                  60

Asn Gly Asp Phe Gly Asp Lys Thr Gly Val Val Leu Gly His Glu Gly
65                  70                  75                  80

Ile Gly Val Val Gln Glu Val Gly Pro Gly Val Thr Ser Leu Lys Pro
                85                  90                  95

Gly Asp Arg Ala Ser Val Ala Trp Phe Tyr Gln Gly Cys Gly His Cys
            100                 105                 110

Glu Tyr Cys Asn Ser Gly Asn Glu Thr Leu Cys Arg Asp Val Lys Asn
        115                 120                 125

Ser Gly Tyr Thr Val Asp Gly Gly Met Ala Glu Ala Cys Ile Val Lys
    130                 135                 140

Ala Asp Tyr Ser Val Lys Val Pro Asp Gly Leu Asp Ser Ala Ala Ala
145                 150                 155                 160

Ser Ser Ile Thr Cys Ala Gly Val Thr Thr Tyr Lys Ala Val Lys Ile
                165                 170                 175

Ser Asn Val Arg Pro Gly Gln Trp Ile Ala Ile Tyr Gly Leu Gly Gly
            180                 185                 190

Leu Gly Asn Leu Ala Leu Gln Tyr Ala Lys Asn Val Phe Asn Ala Lys
        195                 200                 205

Val Ile Ala Ile Asp Val Asn Glu Glu Gln Leu Arg Phe Ala Ser Glu
    210                 215                 220

Met Gly Ala Asp Leu Val Val Asn Pro Leu Asn Glu Asp Ala Ala Lys
225                 230                 235                 240

Phe Ile Gln Ala Lys Thr Gly Gly Ala His Ala Ala Val Val Thr Ala
                245                 250                 255

Val Ala Lys Gly Ala Phe Asn Ser Ala Val Asp Ala Leu Arg Ala Gly
            260                 265                 270

Gly Arg Leu Val Ala Val Gly Leu Pro Ser Glu Ser Met Asp Leu Asn
        275                 280                 285

Ile Pro Arg Leu Val Leu Asp Gly Ile Glu Val Val Gly Ser Leu Val
    290                 295                 300

Gly Thr Arg Gln Asp Leu Gln Glu Ala Phe Gln Phe Ala Ala Glu Gly
305                 310                 315                 320

Lys Val Val Pro Lys Val Thr Leu Arg Pro Ile Glu His Ile Asn Gln
                325                 330                 335

Ile Phe Asp Glu Met Leu Glu Gly Lys Ile Lys Gly Arg Met Val Ile
            340                 345                 350

Gln Phe Glu Gly
        355

<210>  29
<211>  1164
<212>  DNA
<213>  Geobacillus thermodentrificans

<400>  29
atgcaaaatt ttacgtttcg caatccgacc aaactcattt ttgggagagg acaaattgag      60

cagctcaaag aagaagtgcc gaaatatggc aaaaaagtgc tgcttgtcta tgggggcggc     120

agcattaaac gaaacggact atatgatgaa gtcatgagcc tattgacgga cattggcgcc     180

gaagtcgttg aactgccagg cgtcgaaccg aacccgcgcc tttcgaccgt caaaaaaggg     240

gtagacattt gcagacggga aggaattgaa tttttgcttg ccgttggcgg cggcagtgtg     300

atcgactgta cgaaagcgat tgcagccggc gcgaagtttg atggcgatcc gtgggagttc     360

attacgaaaa aagcgactgt cactgaggcg ttgccgtttg ggacggtcct gacgctggca     420

gcgaccggct cggaaatgaa cgccggatcg gtgatcacca attgggagac gaaagaaaaa     480

tacggctggg gcagcccggt gacattcccg caattttcga ttttggatcc gacgtacacg     540

atgacggtgc cgaaagacca taccgtttac ggcatcgtcg atatgatgtc ccatgtgttt     600

gagcaatatt tccatcatac gccgaacacg ccgctgcaag accggatgtg cgaggcagtg     660

ttaaaaacgg tcattgaggc ggcgccaaaa ttggttgacg acttagagaa ctacgagctg     720

cgcgagacga tcatgtactc gggcacgatc gccttaaacg gctttttgca aatgggcgtg     780

cgcggtgatt gggcgacgca tgatatcgag catgcggtct ctgctgtata cgatatcccg 840

cacgccgggg gattggccat tttgttcccg aattggatga aacatgtgct tgatgaaaat 900

gtcagccgtt tcgcccaact ggcggtgcgc gtctttgacg tcgatccgac gggcaaaacg 960

gagcgcgacg tggcgcttga gggcatcgag cggttgcgcg cgttttggtc gagcctcggg 1020

gcgccgtctc gattggctga ttatggcatc ggcgaggaaa atctcgagct gatggcggat 1080

aaagcgatgg cgtttggtga gtttggccgt ttcaaaacgt taaatcgtga tgatgtgctc 1140

gccattttgc gtgcgtcttt ataa 1164

<210> 30
<211> 1170
<212> DNA
<213> Artificial

<220>
<223> Gt_ADH codon optimized

<220>
<221> CDS
<222> (1)..(1170)

<400> 30
atg cag aac ttc acc ttt cgt aat ccg acg aaa ctg atc ttc ggt cgc 48
Met Gln Asn Phe Thr Phe Arg Asn Pro Thr Lys Leu Ile Phe Gly Arg
1               5                   10                  15

ggc caa atc gag caa ctg aag gaa gaa gtg cct aag tat ggt aag aaa 96
Gly Gln Ile Glu Gln Leu Lys Glu Glu Val Pro Lys Tyr Gly Lys Lys
            20                  25                  30

gtt ctg ttg gtc tat ggt ggc ggc agc att aaa cgc aat ggc ctg tac 144
Val Leu Leu Val Tyr Gly Gly Gly Ser Ile Lys Arg Asn Gly Leu Tyr
        35                  40                  45

gac gag gtg atg agc ctg ctg acg gat att ggt gca gaa gtc gtg gag 192
Asp Glu Val Met Ser Leu Leu Thr Asp Ile Gly Ala Glu Val Val Glu
    50                  55                  60

ctg cca ggt gtt gag ccg aac ccg cgt ctg agc acc gtg aag aaa ggt 240
Leu Pro Gly Val Glu Pro Asn Pro Arg Leu Ser Thr Val Lys Lys Gly
65                  70                  75                  80

gtg gac atc tgt cgt cgt gag ggt atc gaa ttt ctg ctg gcg gtt ggt 288
Val Asp Ile Cys Arg Arg Glu Gly Ile Glu Phe Leu Leu Ala Val Gly
                85                  90                  95

ggc ggt agc gtc att gac tgc acg aag gca atc gcg gct ggt gcc aaa 336
Gly Gly Ser Val Ile Asp Cys Thr Lys Ala Ile Ala Ala Gly Ala Lys
            100                 105                 110

ttt gat ggc gac ccg tgg gaa ttc atc acc aaa aag gcg acc gtt acg 384
Phe Asp Gly Asp Pro Trp Glu Phe Ile Thr Lys Lys Ala Thr Val Thr
        115                 120                 125

gag gct ctg ccg ttt ggc acc gtc ttg acc ctg gcc gca acc ggt agc 432
Glu Ala Leu Pro Phe Gly Thr Val Leu Thr Leu Ala Ala Thr Gly Ser
    130                 135                 140

gag atg aat gcg cga tcg ggc agc gtt att act aac tgg gag act aaa 480
Glu Met Asn Ala Arg Ser Gly Ser Val Ile Thr Asn Trp Glu Thr Lys
145                 150                 155                 160

gag aaa tac ggt tgg ggt tct ccg gtg acg ttc ccg cag ttc tcc att 528
Glu Lys Tyr Gly Trp Gly Ser Pro Val Thr Phe Pro Gln Phe Ser Ile
                165                 170                 175

ctg gac cca acc tat acg atg acg gtt cct aaa gac cac acc gtc tat 576
Leu Asp Pro Thr Tyr Thr Met Thr Val Pro Lys Asp His Thr Val Tyr
            180                 185                 190

ggt atc gtg gat atg atg agc cac gtt ttt gaa caa tac ttc cac cac 624
Gly Ile Val Asp Met Met Ser His Val Phe Glu Gln Tyr Phe His His
        195                 200                 205

acc ccg aac acc ccg ctg cag gat cgt atg tgc gaa gca gta ctg aaa 672
Thr Pro Asn Thr Pro Leu Gln Asp Arg Met Cys Glu Ala Val Leu Lys
    210                 215                 220

acg gtt atc gaa gcg gca ccg aaa ctg gtc gat gat ctg gag aat tac 720
Thr Val Ile Glu Ala Ala Pro Lys Leu Val Asp Asp Leu Glu Asn Tyr
225                 230                 235                 240

gaa ctg cgt gaa acc att atg tat tcg ggc act atc gca ctg aac ggt 768
Glu Leu Arg Glu Thr Ile Met Tyr Ser Gly Thr Ile Ala Leu Asn Gly
                245                 250                 255

ttc ctg cag atg ggc gtt cgc ggt gat tgg gcg acc cat gac atc gag 816
Phe Leu Gln Met Gly Val Arg Gly Asp Trp Ala Thr His Asp Ile Glu
            260                 265                 270

cat gca gtc tct gct gtg tac gat atc ccg cac gcc ggt ggc ctg gcc 864
His Ala Val Ser Ala Val Tyr Asp Ile Pro His Ala Gly Gly Leu Ala
        275                 280                 285

att ttg ttc ccg aat tgg atg aag cat gtc ctg gat gag aac gtt agc 912
Ile Leu Phe Pro Asn Trp Met Lys His Val Leu Asp Glu Asn Val Ser
    290                 295                 300

cgc ttt gcg caa ctg gcg gtg cgt gtg ttt gac gtc gac ccg acg ggt 960
Arg Phe Ala Gln Leu Ala Val Arg Val Phe Asp Val Asp Pro Thr Gly
305                 310                 315                 320

aag acc gag cgt gat gtg gcc ctg gaa ggt att gag cgt ctg cgt gcg 1008
Lys Thr Glu Arg Asp Val Ala Leu Glu Gly Ile Glu Arg Leu Arg Ala
                325                 330                 335

ttt tgg agc agc ctg ggc gct ccg tcc cgc ctg gca gat tac ggt att 1056
Phe Trp Ser Ser Leu Gly Ala Pro Ser Arg Leu Ala Asp Tyr Gly Ile
            340                 345                 350

ggc gaa gaa aat ctg gag ctg atg gcc gac aag gcg atg gcg ttt ggt 1104
Gly Glu Glu Asn Leu Glu Leu Met Ala Asp Lys Ala Met Ala Phe Gly
        355                 360                 365

gag ttc ggc cgt ttc aag acc ttg aac cgc gac gac gtt ttg gcg att 1152
Glu Phe Gly Arg Phe Lys Thr Leu Asn Arg Asp Asp Val Leu Ala Ile
    370                 375                 380

ttg cgt gcg agc ctg taa 1170
Leu Arg Ala Ser Leu
385

<210>  31
<211>  389
<212>  PRT
<213>  Artificial

<220>
<223>  Synthetic Construct

<400>  31

Met Gln Asn Phe Thr Phe Arg Asn Pro Thr Lys Leu Ile Phe Gly Arg
1               5                   10                  15

Gly Gln Ile Glu Gln Leu Lys Glu Glu Val Pro Lys Tyr Gly Lys Lys
            20                  25                  30

Val Leu Leu Val Tyr Gly Gly Gly Ser Ile Lys Arg Asn Gly Leu Tyr
        35                  40                  45

Asp Glu Val Met Ser Leu Leu Thr Asp Ile Gly Ala Glu Val Val Glu
    50                  55                  60

Leu Pro Gly Val Glu Pro Asn Pro Arg Leu Ser Thr Val Lys Lys Gly
65                  70                  75                  80

Val Asp Ile Cys Arg Arg Glu Gly Ile Glu Phe Leu Leu Ala Val Gly
                85                  90                  95

Gly Gly Ser Val Ile Asp Cys Thr Lys Ala Ile Ala Ala Gly Ala Lys
            100                 105                 110

Phe Asp Gly Asp Pro Trp Glu Phe Ile Thr Lys Lys Ala Thr Val Thr
        115                 120                 125

Glu Ala Leu Pro Phe Gly Thr Val Leu Thr Leu Ala Ala Thr Gly Ser
    130                 135                 140

Glu Met Asn Ala Arg Ser Gly Ser Val Ile Thr Asn Trp Glu Thr Lys
145                 150                 155                 160

Glu Lys Tyr Gly Trp Gly Ser Pro Val Thr Phe Pro Gln Phe Ser Ile
                165                 170                 175

Leu Asp Pro Thr Tyr Thr Met Thr Val Pro Lys Asp His Thr Val Tyr
            180                 185                 190

Gly Ile Val Asp Met Met Ser His Val Phe Glu Gln Tyr Phe His His
        195                 200                 205

Thr Pro Asn Thr Pro Leu Gln Asp Arg Met Cys Glu Ala Val Leu Lys
    210                 215                 220

Thr Val Ile Glu Ala Ala Pro Lys Leu Val Asp Asp Leu Glu Asn Tyr
225                 230                 235                 240

Glu Leu Arg Glu Thr Ile Met Tyr Ser Gly Thr Ile Ala Leu Asn Gly
                245                 250                 255

Phe Leu Gln Met Gly Val Arg Gly Asp Trp Ala Thr His Asp Ile Glu
            260                 265                 270

His Ala Val Ser Ala Val Tyr Asp Ile Pro His Ala Gly Gly Leu Ala
        275                 280                 285

Ile Leu Phe Pro Asn Trp Met Lys His Val Leu Asp Glu Asn Val Ser
    290                 295                 300

Arg Phe Ala Gln Leu Ala Val Arg Val Phe Asp Val Asp Pro Thr Gly
305                 310                 315                 320

Lys Thr Glu Arg Asp Val Ala Leu Glu Gly Ile Glu Arg Leu Arg Ala
                325                 330                 335

Phe Trp Ser Ser Leu Gly Ala Pro Ser Arg Leu Ala Asp Tyr Gly Ile
            340                 345                 350

Gly Glu Glu Asn Leu Glu Leu Met Ala Asp Lys Ala Met Ala Phe Gly
        355                 360                 365

Glu Phe Gly Arg Phe Lys Thr Leu Asn Arg Asp Asp Val Leu Ala Ile
    370                 375                 380

Leu Arg Ala Ser Leu
385

<210>  32
<211>  407
<212>  PRT
<213>  Geobacillus thermodentrificans

<400>  32

Met Gly Ser Ser His His His His His His Ser Ser Gly Leu Val Pro
1               5                   10                  15

Arg Gly Ser His Met Gln Asn Phe Thr Phe Arg Asn Pro Thr Lys Leu
            20                  25                  30

Ile Phe Gly Arg Gly Gln Ile Glu Gln Leu Lys Glu Glu Val Pro Lys
        35                  40                  45

Tyr Gly Lys Lys Val Leu Leu Val Tyr Gly Gly Gly Ser Ile Lys Arg
    50                  55                  60

Asn Gly Leu Tyr Asp Glu Val Met Ser Leu Leu Thr Asp Ile Gly Ala
65                  70                  75                  80

Glu Val Val Glu Leu Pro Gly Val Glu Pro Asn Pro Arg Leu Ser Thr
                85                  90                  95

Val Lys Lys Gly Val Asp Ile Cys Arg Arg Glu Gly Ile Glu Phe Leu
            100                 105                 110

Leu Ala Val Gly Gly Gly Ser Val Ile Asp Cys Thr Lys Ala Ile Ala
        115                 120                 125

Ala Gly Ala Lys Phe Asp Gly Asp Pro Trp Glu Phe Ile Thr Lys Lys
    130                 135                 140

Ala Thr Val Thr Glu Ala Leu Pro Phe Gly Thr Val Leu Thr Leu Ala
145                 150                 155                 160

Ala Thr Gly Ser Glu Met Asn Ala Gly Ser Val Ile Thr Asn Trp Glu
                165                 170                 175

Thr Lys Glu Lys Tyr Gly Trp Gly Ser Pro Val Thr Phe Pro Gln Phe
            180                 185                 190

Ser Ile Leu Asp Pro Thr Tyr Thr Met Thr Val Pro Lys Asp His Thr
        195                 200                 205

Val Tyr Gly Ile Val Asp Met Met Ser His Val Phe Glu Gln Tyr Phe
    210                 215                 220

His His Thr Pro Asn Thr Pro Leu Gln Asp Arg Met Cys Glu Ala Val
225                 230                 235                 240

Leu Lys Thr Val Ile Glu Ala Ala Pro Lys Leu Val Asp Asp Leu Glu
                245                 250                 255

Asn Tyr Glu Leu Arg Glu Thr Ile Met Tyr Ser Gly Thr Ile Ala Leu
            260                 265                 270

Asn Gly Phe Leu Gln Met Gly Val Arg Gly Asp Trp Ala Thr His Asp
        275                 280                 285

Ile Glu His Ala Val Ser Ala Val Tyr Asp Ile Pro His Ala Gly Gly
    290                 295                 300

Leu Ala Ile Leu Phe Pro Asn Trp Met Lys His Val Leu Asp Glu Asn

305                 310                 315                 320

Val Ser Arg Phe Ala Gln Leu Ala Val Arg Val Phe Asp Val Asp Pro
                325                 330                 335

Thr Gly Lys Thr Glu Arg Asp Val Ala Leu Glu Gly Ile Glu Arg Leu
            340                 345                 350

Arg Ala Phe Trp Ser Ser Leu Gly Ala Pro Ser Arg Leu Ala Asp Tyr
        355                 360                 365

Gly Ile Gly Glu Glu Asn Leu Glu Leu Met Ala Asp Lys Ala Met Ala
    370                 375                 380

Phe Gly Glu Phe Gly Arg Phe Lys Thr Leu Asn Arg Asp Asp Val Leu
385                 390                 395                 400

Ala Ile Leu Arg Ala Ser Leu
                405

<210>  33
<211>  969
<212>  DNA
<213>  Archaeoglobus fulgidus

<220>
<221>  CDS
<222>  (1)..(969)

<400>  33
atg gag act ctt att ttg act cag gag gaa gta gaa tcc ctg ata tcg       48
Met Glu Thr Leu Ile Leu Thr Gln Glu Glu Val Glu Ser Leu Ile Ser
1               5                   10                  15

atg gac gag gcg atg aat gct gtt gag gag gcg ttc aga ctt tac gct       96
Met Asp Glu Ala Met Asn Ala Val Glu Glu Ala Phe Arg Leu Tyr Ala
            20                  25                  30

ctt ggt aaa gct caa atg ccg cca aag gtg tat ctt gag ttc gaa aaa      144
Leu Gly Lys Ala Gln Met Pro Pro Lys Val Tyr Leu Glu Phe Glu Lys
        35                  40                  45

ggc gat tta agg gct atg ccg gcg cat tta atg ggg tac gca gga ttg      192
Gly Asp Leu Arg Ala Met Pro Ala His Leu Met Gly Tyr Ala Gly Leu
    50                  55                  60

aag tgg gtg aac tcc cat ccc gga aat ccg gat aaa ggc ctg cca aca      240
Lys Trp Val Asn Ser His Pro Gly Asn Pro Asp Lys Gly Leu Pro Thr
65                  70                  75                  80

gtc atg gcg ttg atg ata ctg aac agc cct gag acg ggg ttt ccc ttg      288
Val Met Ala Leu Met Ile Leu Asn Ser Pro Glu Thr Gly Phe Pro Leu
                85                  90                  95

gct gtg atg gac gca acc tac acg acc tct ctg aga acc ggt gct gct      336
Ala Val Met Asp Ala Thr Tyr Thr Thr Ser Leu Arg Thr Gly Ala Ala
            100                 105                 110

gga gga ata gcg gcg aag tat ctg gca agg aag aac agc agc gtt ttt 384
Gly Gly Ile Ala Ala Lys Tyr Leu Ala Arg Lys Asn Ser Ser Val Phe
        115                 120                 125

gga ttt atc ggc tgc gga act cag gcg tat ttc cag ctc gaa gct ctg 432
Gly Phe Ile Gly Cys Gly Thr Gln Ala Tyr Phe Gln Leu Glu Ala Leu
    130                 135                 140

agg aga gtg ttt gat ata gga gaa gtc aag gcc tac gat gtt agg gaa 480
Arg Arg Val Phe Asp Ile Gly Glu Val Lys Ala Tyr Asp Val Arg Glu
145                 150                 155                 160

aag gca gcg aaa aag ttc gtt agc tat tgc gaa gat agg gga atc tca 528
Lys Ala Ala Lys Lys Phe Val Ser Tyr Cys Glu Asp Arg Gly Ile Ser
                165                 170                 175

gct tcc gtg cag cct gct gag gag gcg agc aga tgc gat gtc ctt gtc 576
Ala Ser Val Gln Pro Ala Glu Glu Ala Ser Arg Cys Asp Val Leu Val
            180                 185                 190

acc aca act ccg tca cga aaa cca gtt gtt aag gct gag tgg gtt gag 624
Thr Thr Thr Pro Ser Arg Lys Pro Val Val Lys Ala Glu Trp Val Glu
        195                 200                 205

gag ggg acg cac ata aac gca atc ggt gcg gac ggg cct gga aag cag 672
Glu Gly Thr His Ile Asn Ala Ile Gly Ala Asp Gly Pro Gly Lys Gln
    210                 215                 220

gaa ctg gat gta gaa ata ctt aaa aag gct aaa att gtt gtc gat gac 720
Glu Leu Asp Val Glu Ile Leu Lys Lys Ala Lys Ile Val Val Asp Asp
225                 230                 235                 240

ctt gaa cag gcc aag cac ggc ggg gag att aat gtg gcg gtt tca aaa 768
Leu Glu Gln Ala Lys His Gly Gly Glu Ile Asn Val Ala Val Ser Lys
                245                 250                 255

ggc gtc ata ggt gtt gaa gat gtt cac gca acc atc ggg gag gtt ata 816
Gly Val Ile Gly Val Glu Asp Val His Ala Thr Ile Gly Glu Val Ile
            260                 265                 270

gcc ggt ctg aag gat gga agg gag agc gat gag gag att aca atc ttc 864
Ala Gly Leu Lys Asp Gly Arg Glu Ser Asp Glu Glu Ile Thr Ile Phe
        275                 280                 285

gac tcc aca ggc ttg gcg att cag gac gtg gct gtt gca aaa gtc gtt 912
Asp Ser Thr Gly Leu Ala Ile Gln Asp Val Ala Val Ala Lys Val Val
    290                 295                 300

tat gag aac gcc ctt agc aag aac gtg ggg agt aaa ata aag ttt ttc 960
Tyr Glu Asn Ala Leu Ser Lys Asn Val Gly Ser Lys Ile Lys Phe Phe
305                 310                 315                 320

agg ata tga 969
Arg Ile

<210> 34
<211> 322
<212> PRT
<213> Archaeoglobus fulgidus

<400> 34

Met Glu Thr Leu Ile Leu Thr Gln Glu Glu Val Glu Ser Leu Ile Ser
1               5                   10                  15

Met Asp Glu Ala Met Asn Ala Val Glu Glu Ala Phe Arg Leu Tyr Ala
            20                  25                  30

Leu Gly Lys Ala Gln Met Pro Pro Lys Val Tyr Leu Glu Phe Glu Lys
        35                  40                  45

Gly Asp Leu Arg Ala Met Pro Ala His Leu Met Gly Tyr Ala Gly Leu
    50                  55                  60

Lys Trp Val Asn Ser His Pro Gly Asn Pro Asp Lys Gly Leu Pro Thr
65                  70                  75                  80

Val Met Ala Leu Met Ile Leu Asn Ser Pro Glu Thr Gly Phe Pro Leu
                85                  90                  95

Ala Val Met Asp Ala Thr Tyr Thr Thr Ser Leu Arg Thr Gly Ala Ala
            100                 105                 110

Gly Gly Ile Ala Ala Lys Tyr Leu Ala Arg Lys Asn Ser Ser Val Phe
        115                 120                 125

Gly Phe Ile Gly Cys Gly Thr Gln Ala Tyr Phe Gln Leu Glu Ala Leu
    130                 135                 140

Arg Arg Val Phe Asp Ile Gly Glu Val Lys Ala Tyr Asp Val Arg Glu
145                 150                 155                 160

Lys Ala Ala Lys Lys Phe Val Ser Tyr Cys Glu Asp Arg Gly Ile Ser
                165                 170                 175

Ala Ser Val Gln Pro Ala Glu Glu Ala Ser Arg Cys Asp Val Leu Val
            180                 185                 190

Thr Thr Thr Pro Ser Arg Lys Pro Val Val Lys Ala Glu Trp Val Glu
        195                 200                 205

Glu Gly Thr His Ile Asn Ala Ile Gly Ala Asp Gly Pro Gly Lys Gln
    210                 215                 220

Glu Leu Asp Val Glu Ile Leu Lys Lys Ala Lys Ile Val Val Asp Asp
225                 230                 235                 240

Leu Glu Gln Ala Lys His Gly Gly Glu Ile Asn Val Ala Val Ser Lys
                245                 250                 255

Gly Val Ile Gly Val Glu Asp Val His Ala Thr Ile Gly Glu Val Ile

260                 265                 270

Ala Gly Leu Lys Asp Gly Arg Glu Ser Asp Glu Glu Ile Thr Ile Phe
        275                 280                 285

Asp Ser Thr Gly Leu Ala Ile Gln Asp Val Ala Val Ala Lys Val Val
    290                 295                 300

Tyr Glu Asn Ala Leu Ser Lys Asn Val Gly Ser Lys Ile Lys Phe Phe
305                 310                 315                 320

Arg Ile

<210>  35
<211>  342
<212>  PRT
<213>  Archaeoglobus fulgidus

<400>  35

Met Gly Ser Ser His His His His His His Ser Ser Gly Leu Val Pro
1               5                   10                  15

Arg Gly Ser His Met Glu Thr Leu Ile Leu Thr Gln Glu Glu Val Glu
            20                  25                  30

Ser Leu Ile Ser Met Asp Glu Ala Met Asn Ala Val Glu Glu Ala Phe
        35                  40                  45

Arg Leu Tyr Ala Leu Gly Lys Ala Gln Met Pro Pro Lys Val Tyr Leu
    50                  55                  60

Glu Phe Glu Lys Gly Asp Leu Arg Ala Met Pro Ala His Leu Met Gly
65                  70                  75                  80

Tyr Ala Gly Leu Lys Trp Val Asn Ser His Pro Gly Asn Pro Asp Lys
                85                  90                  95

Gly Leu Pro Thr Val Met Ala Leu Met Ile Leu Asn Ser Pro Glu Thr
            100                 105                 110

Gly Phe Pro Leu Ala Val Met Asp Ala Thr Tyr Thr Thr Ser Leu Arg
        115                 120                 125

Thr Gly Ala Ala Gly Gly Ile Ala Ala Lys Tyr Leu Ala Arg Lys Asn
    130                 135                 140

Ser Ser Val Phe Gly Phe Ile Gly Cys Gly Thr Gln Ala Tyr Phe Gln
145                 150                 155                 160

Leu Glu Ala Leu Arg Arg Val Phe Asp Ile Gly Glu Val Lys Ala Tyr
                165                 170                 175

Asp Val Arg Glu Lys Ala Ala Lys Lys Phe Val Ser Tyr Cys Glu Asp
            180                 185                 190

Arg Gly Ile Ser Ala Ser Val Gln Pro Ala Glu Glu Ala Ser Arg Cys
        195                 200                 205

Asp Val Leu Val Thr Thr Thr Pro Ser Arg Lys Pro Val Val Lys Ala
    210                 215                 220

Glu Trp Val Glu Glu Gly Thr His Ile Asn Ala Ile Gly Ala Asp Gly
225                 230                 235                 240

Pro Gly Lys Gln Glu Leu Asp Val Glu Ile Leu Lys Lys Ala Lys Ile
                245                 250                 255

Val Val Asp Asp Leu Glu Gln Ala Lys His Gly Gly Glu Ile Asn Val
            260                 265                 270

Ala Val Ser Lys Gly Val Ile Gly Val Glu Asp Val His Ala Thr Ile
        275                 280                 285

Gly Glu Val Ile Ala Gly Leu Lys Asp Gly Arg Glu Ser Asp Glu Glu
    290                 295                 300

Ile Thr Ile Phe Asp Ser Thr Gly Leu Ala Ile Gln Asp Val Ala Val
305                 310                 315                 320

Ala Lys Val Val Tyr Glu Asn Ala Leu Ser Lys Asn Val Gly Ser Lys
                325                 330                 335

Ile Lys Phe Phe Arg Ile
            340

<210>  36
<211>  36
<212>  DNA
<213>  Artificial

<220>
<223>  PCR Forward Primer

<400>  36
cgcgcggcag ccatatggag actcttattt tgactc                                36

<210>  37
<211>  42
<212>  DNA
<213>  Artificial

<220>
<223>  PCR Rev Primer

<400>  37
gtgcggccgc aagcttttat atcctgaaaa actttatttt ac                          42


<210>  38
<211>  1110
<212>  DNA
<213>  Thermus thermophilus


<220>
<221>  CDS
<222>  (1)..(1110)

<400>  38
atg gtg atc ggc gtg ccg aag gag atc aag acc ttg gag aac cgg gtg       48
Met Val Ile Gly Val Pro Lys Glu Ile Lys Thr Leu Glu Asn Arg Val
1               5                   10                  15

gcc ctc acg ccc ggc ggg gtg gag agc ctg gtc agg cgc ggc cac acc       96
Ala Leu Thr Pro Gly Gly Val Glu Ser Leu Val Arg Arg Gly His Thr
            20                  25                  30

gtg ctg gtg gag cgg ggg gcc ggg gag ggc tcg ggg ctt tcc gac gcg      144
Val Leu Val Glu Arg Gly Ala Gly Glu Gly Ser Gly Leu Ser Asp Ala
        35                  40                  45

gag tac gcc cgg gcc ggg gcc gag ctc gtg ggc cgg gag gag gcc tgg      192
Glu Tyr Ala Arg Ala Gly Ala Glu Leu Val Gly Arg Glu Glu Ala Trp
    50                  55                  60

ggt gcg gag atg gtg gtg aag gtg aag gag ccc cta ccc gag gag tac      240
Gly Ala Glu Met Val Val Lys Val Lys Glu Pro Leu Pro Glu Glu Tyr
65                  70                  75                  80

ggc ttc ctg cgg gag ggc ctc atc ctc ttc acc tac ctt cac ctg gcc      288
Gly Phe Leu Arg Glu Gly Leu Ile Leu Phe Thr Tyr Leu His Leu Ala
                85                  90                  95

gcg gac cgc ggc ctc acc gag gcc atg ctc cgt agc ggg gtc acg ggc      336
Ala Asp Arg Gly Leu Thr Glu Ala Met Leu Arg Ser Gly Val Thr Gly
            100                 105                 110

atc gcc tac gag acc gtc cag ctt ccc gac ggc acc ctc ccc ctc ctc      384
Ile Ala Tyr Glu Thr Val Gln Leu Pro Asp Gly Thr Leu Pro Leu Leu
        115                 120                 125

gtc ccc atg agc gag gtg gcg ggg cgg atg gcc ccc cag gtg ggg gcc      432
Val Pro Met Ser Glu Val Ala Gly Arg Met Ala Pro Gln Val Gly Ala
    130                 135                 140

cag ttc ctg gag aag ccc aag ggg ggc cgg ggg gtc ctc ctc ggg ggg      480
Gln Phe Leu Glu Lys Pro Lys Gly Gly Arg Gly Val Leu Leu Gly Gly
145                 150                 155                 160

gtg ccg ggg gtg gcc ccg gcc agc gtg gtg atc ctc ggg ggc ggg acc      528
Val Pro Gly Val Ala Pro Ala Ser Val Val Ile Leu Gly Gly Gly Thr
                165                 170                 175

gtg ggc acc aac gcg gcc aag atc gcc ctg ggg atg ggg gcc cag gtg      576
Val Gly Thr Asn Ala Ala Lys Ile Ala Leu Gly Met Gly Ala Gln Val

180                 185                 190

acc atc ctg gac gtg aac cac aag cgc ctc cag tat ctg gac gac gtc       624
Thr Ile Leu Asp Val Asn His Lys Arg Leu Gln Tyr Leu Asp Asp Val
        195                 200                 205

ttc ggc ggg cgg gtg atc acc ctc acc gcc acc gag gcc aac atc aaa       672
Phe Gly Gly Arg Val Ile Thr Leu Thr Ala Thr Glu Ala Asn Ile Lys
    210                 215                 220

aag agc gtc cag cac gcg gac ctc ctc atc ggg gcc gtc ctc gtc ccc       720
Lys Ser Val Gln His Ala Asp Leu Leu Ile Gly Ala Val Leu Val Pro
225                 230                 235                 240

ggg gcc aag gcc ccc aag ctc gtc acc cgg gac atg ctc tct ctg atg       768
Gly Ala Lys Ala Pro Lys Leu Val Thr Arg Asp Met Leu Ser Leu Met
                245                 250                 255

aag gag gga gcg gtg atc gtg gac gtg gcc gtg gac cag ggg ggg tgc       816
Lys Glu Gly Ala Val Ile Val Asp Val Ala Val Asp Gln Gly Gly Cys
            260                 265                 270

gtg gag acc atc cgg ccc acc acc cac gcc gag ccc acc tac gtg gtg       864
Val Glu Thr Ile Arg Pro Thr Thr His Ala Glu Pro Thr Tyr Val Val
        275                 280                 285

gac ggg gtg gtc cac tac ggg gtg gcc aac atg ccc ggg gcg gtg ccc       912
Asp Gly Val Val His Tyr Gly Val Ala Asn Met Pro Gly Ala Val Pro
    290                 295                 300

agg acc agc acc ttc gcc ctc acc aac cag acc ctg ccc tac gtg ttg       960
Arg Thr Ser Thr Phe Ala Leu Thr Asn Gln Thr Leu Pro Tyr Val Leu
305                 310                 315                 320

aag ctc gcg gag aag ggg ctg gac gcc ctt ctg gag gac gcg gcc ctt      1008
Lys Leu Ala Glu Lys Gly Leu Asp Ala Leu Leu Glu Asp Ala Ala Leu
                325                 330                 335

ctc aag ggg ctc aac acc cac aaa ggc cgc ctc acc cac ccc ggg gtg      1056
Leu Lys Gly Leu Asn Thr His Lys Gly Arg Leu Thr His Pro Gly Val
            340                 345                 350

gcc gag gcc ttc ggc ctg ccc tac acg cct ccc gag gag gcc ttg agg      1104
Ala Glu Ala Phe Gly Leu Pro Tyr Thr Pro Pro Glu Glu Ala Leu Arg
        355                 360                 365

ggg tga                                                              1110
Gly

<210>  39
<211>  369
<212>  PRT
<213>  Thermus thermophilus

<400>  39

Met Val Ile Gly Val Pro Lys Glu Ile Lys Thr Leu Glu Asn Arg Val
1               5                   10                  15

Ala Leu Thr Pro Gly Gly Val Glu Ser Leu Val Arg Arg Gly His Thr
            20                  25                  30

Val Leu Val Glu Arg Gly Ala Gly Glu Gly Ser Gly Leu Ser Asp Ala
        35                  40                  45

Glu Tyr Ala Arg Ala Gly Ala Glu Leu Val Gly Arg Glu Glu Ala Trp
    50                  55                  60

Gly Ala Glu Met Val Val Lys Val Lys Glu Pro Leu Pro Glu Glu Tyr
65                  70                  75                  80

Gly Phe Leu Arg Glu Gly Leu Ile Leu Phe Thr Tyr Leu His Leu Ala
                85                  90                  95

Ala Asp Arg Gly Leu Thr Glu Ala Met Leu Arg Ser Gly Val Thr Gly
            100                 105                 110

Ile Ala Tyr Glu Thr Val Gln Leu Pro Asp Gly Thr Leu Pro Leu Leu
        115                 120                 125

Val Pro Met Ser Glu Val Ala Gly Arg Met Ala Pro Gln Val Gly Ala
    130                 135                 140

Gln Phe Leu Glu Lys Pro Lys Gly Gly Arg Gly Val Leu Leu Gly Gly
145                 150                 155                 160

Val Pro Gly Val Ala Pro Ala Ser Val Val Ile Leu Gly Gly Gly Thr
                165                 170                 175

Val Gly Thr Asn Ala Ala Lys Ile Ala Leu Gly Met Gly Ala Gln Val
            180                 185                 190

Thr Ile Leu Asp Val Asn His Lys Arg Leu Gln Tyr Leu Asp Asp Val
        195                 200                 205

Phe Gly Gly Arg Val Ile Thr Leu Thr Ala Thr Glu Ala Asn Ile Lys
    210                 215                 220

Lys Ser Val Gln His Ala Asp Leu Leu Ile Gly Ala Val Leu Val Pro
225                 230                 235                 240

Gly Ala Lys Ala Pro Lys Leu Val Thr Arg Asp Met Leu Ser Leu Met
                245                 250                 255

Lys Glu Gly Ala Val Ile Val Asp Val Ala Val Asp Gln Gly Gly Cys
            260                 265                 270

Val Glu Thr Ile Arg Pro Thr Thr His Ala Glu Pro Thr Tyr Val Val
        275                 280                 285

Asp Gly Val Val His Tyr Gly Val Ala Asn Met Pro Gly Ala Val Pro
    290                 295                 300

Arg Thr Ser Thr Phe Ala Leu Thr Asn Gln Thr Leu Pro Tyr Val Leu
305                 310                 315                 320

Lys Leu Ala Glu Lys Gly Leu Asp Ala Leu Leu Glu Asp Ala Ala Leu
                325                 330                 335

Leu Lys Gly Leu Asn Thr His Lys Gly Arg Leu Thr His Pro Gly Val
            340                 345                 350

Ala Glu Ala Phe Gly Leu Pro Tyr Thr Pro Pro Glu Glu Ala Leu Arg
        355                 360                 365

Gly

<210>  40
<211>  389
<212>  PRT
<213>  Thermus thermophilus

<400>  40

Met Gly Ser Ser His His His His His His Ser Ser Gly Leu Val Pro
1               5                   10                  15

Arg Gly Ser His Met Val Ile Gly Val Pro Lys Glu Ile Lys Thr Leu
            20                  25                  30

Glu Asn Arg Val Ala Leu Thr Pro Gly Gly Val Glu Ser Leu Val Arg
        35                  40                  45

Arg Gly His Thr Val Leu Val Glu Arg Gly Ala Gly Glu Gly Ser Gly
    50                  55                  60

Leu Ser Asp Ala Glu Tyr Ala Arg Ala Gly Ala Glu Leu Val Gly Arg
65                  70                  75                  80

Glu Glu Ala Trp Gly Ala Glu Met Val Val Lys Val Lys Glu Pro Leu
                85                  90                  95

Pro Glu Glu Tyr Gly Phe Leu Arg Glu Gly Leu Ile Leu Phe Thr Tyr
            100                 105                 110

Leu His Leu Ala Ala Asp Arg Gly Leu Thr Glu Ala Met Leu Arg Ser
        115                 120                 125

Gly Val Thr Gly Ile Ala Tyr Glu Thr Val Gln Leu Pro Asp Gly Thr
    130                 135                 140

Leu Pro Leu Leu Val Pro Met Ser Glu Val Ala Gly Arg Met Ala Pro
145                 150                 155                 160

Gln Val Gly Ala Gln Phe Leu Glu Lys Pro Lys Gly Gly Arg Gly Val
                165                 170                 175

Leu Leu Gly Gly Val Pro Gly Val Ala Pro Ala Ser Val Val Ile Leu
            180                 185                 190

Gly Gly Gly Thr Val Gly Thr Asn Ala Ala Lys Ile Ala Leu Gly Met
        195                 200                 205

Gly Ala Gln Val Thr Ile Leu Asp Val Asn His Lys Arg Leu Gln Tyr
    210                 215                 220

Leu Asp Asp Val Phe Gly Gly Arg Val Ile Thr Leu Thr Ala Thr Glu
225                 230                 235                 240

Ala Asn Ile Lys Lys Ser Val Gln His Ala Asp Leu Leu Ile Gly Ala
                245                 250                 255

Val Leu Val Pro Gly Ala Lys Ala Pro Lys Leu Val Thr Arg Asp Met
            260                 265                 270

Leu Ser Leu Met Lys Glu Gly Ala Val Ile Val Asp Val Ala Val Asp
        275                 280                 285

Gln Gly Gly Cys Val Glu Thr Ile Arg Pro Thr Thr His Ala Glu Pro
    290                 295                 300

Thr Tyr Val Val Asp Gly Val Val His Tyr Gly Val Ala Asn Met Pro
305                 310                 315                 320

Gly Ala Val Pro Arg Thr Ser Thr Phe Ala Leu Thr Asn Gln Thr Leu
                325                 330                 335

Pro Tyr Val Leu Lys Leu Ala Glu Lys Gly Leu Asp Ala Leu Leu Glu
            340                 345                 350

Asp Ala Ala Leu Leu Lys Gly Leu Asn Thr His Lys Gly Arg Leu Thr
        355                 360                 365

His Pro Gly Val Ala Glu Ala Phe Gly Leu Pro Tyr Thr Pro Pro Glu
    370                 375                 380

Glu Ala Leu Arg Gly
385

<210> 41
<211> 30
<212> DNA
<213> Artificial

<220>
<223> PCR Forward Primer

<400> 41
cgcgcggcag ccatatggtg atcggcgtgc 30


<210> 42
<211> 35
<212> DNA
<213> Artificial

<220>
<223> PCR Reverse Primer

<400> 42
gtgcggccgc aagcttttac cccctcaagg cctcc 35


<210> 43
<211> 1125
<212> DNA
<213> Vibrio proteolyticus


<220>
<221> CDS
<222> (1)..(1125)

<400> 43
atg atc att ggc gta cct aag gaa atc aaa aac cac gaa tac cgc gtc 48
Met Ile Ile Gly Val Pro Lys Glu Ile Lys Asn His Glu Tyr Arg Val
1               5                   10                  15

ggt atg att cca gca agt gtc cgt gag cta atc tca cat ggt cac caa 96
Gly Met Ile Pro Ala Ser Val Arg Glu Leu Ile Ser His Gly His Gln
            20                  25                  30

gtt ttt gtt gaa acc aat gcc ggc gca ggc atc ggt ttt tca gac gat 144
Val Phe Val Glu Thr Asn Ala Gly Ala Gly Ile Gly Phe Ser Asp Asp
        35                  40                  45

gat tac atc gct gta ggc gca tcc att ctt cct act gct gct gag gtt 192
Asp Tyr Ile Ala Val Gly Ala Ser Ile Leu Pro Thr Ala Ala Glu Val
    50                  55                  60

ttc gct caa gca gat atg att gtt aag gtc aaa gaa cct caa gct gtc 240
Phe Ala Gln Ala Asp Met Ile Val Lys Val Lys Glu Pro Gln Ala Val
65                  70                  75                  80

gaa cga gct atg ctc aaa gaa ggg caa att tta ttt act tat ttg cac 288
Glu Arg Ala Met Leu Lys Glu Gly Gln Ile Leu Phe Thr Tyr Leu His
                85                  90                  95

ctg gca cca gat ttt cca caa act gaa gac ctg atc aag agc aaa gct 336
Leu Ala Pro Asp Phe Pro Gln Thr Glu Asp Leu Ile Lys Ser Lys Ala
            100                 105                 110

gtc tgc ata gcc tat gag act gta aca gat aat atg ggt cgc ttg cca 384

Val Cys Ile Ala Tyr Glu Thr Val Thr Asp Asn Met Gly Arg Leu Pro
        115                 120                 125

cta tta gca cca atg tct gaa gtt gcg ggt cgt atg tct atc cag gca      432
Leu Leu Ala Pro Met Ser Glu Val Ala Gly Arg Met Ser Ile Gln Ala
    130                 135                 140

ggc gca caa act ctc gag aaa tct cac ggc ggc cgt ggc ctg ctg ctg      480
Gly Ala Gln Thr Leu Glu Lys Ser His Gly Gly Arg Gly Leu Leu Leu
145                 150                 155                 160

ggt ggc gta cca ggt gtt gaa cca gcg aaa gtt gtg atc gtc ggc ggc      528
Gly Gly Val Pro Gly Val Glu Pro Ala Lys Val Val Ile Val Gly Gly
                165                 170                 175

ggc gtc gtg ggt gct aac gca gct cgt atg gca gtt ggc atg cgc gcc      576
Gly Val Val Gly Ala Asn Ala Ala Arg Met Ala Val Gly Met Arg Ala
            180                 185                 190

gac gtt act atc tta gac cgt aac atc gat aca ctg cgt aag ctg gat      624
Asp Val Thr Ile Leu Asp Arg Asn Ile Asp Thr Leu Arg Lys Leu Asp
        195                 200                 205

gaa gag ttt cag ggc cgt gcc aaa gtg gtt tac tca act gaa gat gcg      672
Glu Glu Phe Gln Gly Arg Ala Lys Val Val Tyr Ser Thr Glu Asp Ala
    210                 215                 220

att gaa aaa cac gtt ctg gcg gcc gat ctg gtt atc ggt gca gtt ctg      720
Ile Glu Lys His Val Leu Ala Ala Asp Leu Val Ile Gly Ala Val Leu
225                 230                 235                 240

atc cca ggt gcc gca gct cct aaa ctg gtg aca aaa gag cac atc gct      768
Ile Pro Gly Ala Ala Ala Pro Lys Leu Val Thr Lys Glu His Ile Ala
                245                 250                 255

aaa atg aaa cca ggc gcg gct gtt gtc gac gtt gct atc gac cag ggc      816
Lys Met Lys Pro Gly Ala Ala Val Val Asp Val Ala Ile Asp Gln Gly
            260                 265                 270

ggt tgt ttt gaa act tct cac gcg aca acg cac gca gat cca act tac      864
Gly Cys Phe Glu Thr Ser His Ala Thr Thr His Ala Asp Pro Thr Tyr
        275                 280                 285

atc gtt gat gat gtg gtt cac tac tgt gta gca aac atg cca ggg gct      912
Ile Val Asp Asp Val Val His Tyr Cys Val Ala Asn Met Pro Gly Ala
    290                 295                 300

gtt gcc cgc act tcc act ttt gca ctg aac aac gca acc ctg cca tat      960
Val Ala Arg Thr Ser Thr Phe Ala Leu Asn Asn Ala Thr Leu Pro Tyr
305                 310                 315                 320

atc gtt aaa ctg gca aac aaa gga tac cgt gaa gct ctg ctg gct gac     1008
Ile Val Lys Leu Ala Asn Lys Gly Tyr Arg Glu Ala Leu Leu Ala Asp
                325                 330                 335

cac ggc ttc cta gaa ggc ctg aat gtt att cac ggt aaa gtg acg tgc     1056
His Gly Phe Leu Glu Gly Leu Asn Val Ile His Gly Lys Val Thr Cys
            340                 345                 350

aaa gaa gtc gca gaa gcg ttt aat ctg gaa tac gta cag ccg gaa acc     1104
Lys Glu Val Ala Glu Ala Phe Asn Leu Glu Tyr Val Gln Pro Glu Thr
        355                 360                 365

gcg atc gcc atg ttc aac taa                                         1125
Ala Ile Ala Met Phe Asn

    370

<210>  44
<211>  374
<212>  PRT
<213>  Vibrio proteolyticus

<400>  44

Met Ile Ile Gly Val Pro Lys Glu Ile Lys Asn His Glu Tyr Arg Val
1               5                   10                  15

Gly Met Ile Pro Ala Ser Val Arg Glu Leu Ile Ser His Gly His Gln
            20                  25                  30

Val Phe Val Glu Thr Asn Ala Gly Ala Gly Ile Gly Phe Ser Asp Asp
        35                  40                  45

Asp Tyr Ile Ala Val Gly Ala Ser Ile Leu Pro Thr Ala Ala Glu Val
    50                  55                  60

Phe Ala Gln Ala Asp Met Ile Val Lys Val Lys Glu Pro Gln Ala Val
65                  70                  75                  80

Glu Arg Ala Met Leu Lys Glu Gly Gln Ile Leu Phe Thr Tyr Leu His
                85                  90                  95

Leu Ala Pro Asp Phe Pro Gln Thr Glu Asp Leu Ile Lys Ser Lys Ala
            100                 105                 110

Val Cys Ile Ala Tyr Glu Thr Val Thr Asp Asn Met Gly Arg Leu Pro
        115                 120                 125

Leu Leu Ala Pro Met Ser Glu Val Ala Gly Arg Met Ser Ile Gln Ala
    130                 135                 140

Gly Ala Gln Thr Leu Glu Lys Ser His Gly Gly Arg Gly Leu Leu Leu
145                 150                 155                 160

Gly Gly Val Pro Gly Val Glu Pro Ala Lys Val Val Ile Val Gly Gly
                165                 170                 175

Gly Val Val Gly Ala Asn Ala Ala Arg Met Ala Val Gly Met Arg Ala
            180                 185                 190

Asp Val Thr Ile Leu Asp Arg Asn Ile Asp Thr Leu Arg Lys Leu Asp
        195                 200                 205

Glu Glu Phe Gln Gly Arg Ala Lys Val Val Tyr Ser Thr Glu Asp Ala
    210                 215                 220

Ile Glu Lys His Val Leu Ala Ala Asp Leu Val Ile Gly Ala Val Leu
225                 230                 235                 240

Ile Pro Gly Ala Ala Ala Pro Lys Leu Val Thr Lys Glu His Ile Ala
                245                 250                 255

Lys Met Lys Pro Gly Ala Ala Val Val Asp Val Ala Ile Asp Gln Gly
            260                 265                 270

Gly Cys Phe Glu Thr Ser His Ala Thr Thr His Ala Asp Pro Thr Tyr
        275                 280                 285

Ile Val Asp Asp Val Val His Tyr Cys Val Ala Asn Met Pro Gly Ala
    290                 295                 300

Val Ala Arg Thr Ser Thr Phe Ala Leu Asn Asn Ala Thr Leu Pro Tyr
305                 310                 315                 320

Ile Val Lys Leu Ala Asn Lys Gly Tyr Arg Glu Ala Leu Leu Ala Asp
                325                 330                 335

His Gly Phe Leu Glu Gly Leu Asn Val Ile His Gly Lys Val Thr Cys
            340                 345                 350

Lys Glu Val Ala Glu Ala Phe Asn Leu Glu Tyr Val Gln Pro Glu Thr
        355                 360                 365

Ala Ile Ala Met Phe Asn
    370

<210>  45
<211>  394
<212>  PRT
<213>  Vibrio proteolyticus

<400>  45

Met Gly Ser Ser His His His His His His Ser Ser Gly Leu Val Pro
1               5                   10                  15

Arg Gly Ser His Met Ile Ile Gly Val Pro Lys Glu Ile Lys Asn His
            20                  25                  30

Glu Tyr Arg Val Gly Met Ile Pro Ala Ser Val Arg Glu Leu Ile Ser
        35                  40                  45

His Gly His Gln Val Phe Val Glu Thr Asn Ala Gly Ala Gly Ile Gly
    50                  55                  60

Phe Ser Asp Asp Asp Tyr Ile Ala Val Gly Ala Ser Ile Leu Pro Thr

65                  70                  75                  80

Ala Ala Glu Val Phe Ala Gln Ala Asp Met Ile Val Lys Val Lys Glu
                85                  90                  95

Pro Gln Ala Val Glu Arg Ala Met Leu Lys Glu Gly Gln Ile Leu Phe
            100                 105                 110

Thr Tyr Leu His Leu Ala Pro Asp Phe Pro Gln Thr Glu Asp Leu Ile
        115                 120                 125

Lys Ser Lys Ala Val Cys Ile Ala Tyr Glu Thr Val Thr Asp Asn Met
    130                 135                 140

Gly Arg Leu Pro Leu Leu Ala Pro Met Ser Glu Val Ala Gly Arg Met
145                 150                 155                 160

Ser Ile Gln Ala Gly Ala Gln Thr Leu Glu Lys Ser His Gly Gly Arg
                165                 170                 175

Gly Leu Leu Leu Gly Gly Val Pro Gly Val Glu Pro Ala Lys Val Val
            180                 185                 190

Ile Val Gly Gly Gly Val Val Gly Ala Asn Ala Ala Arg Met Ala Val
        195                 200                 205

Gly Met Arg Ala Asp Val Thr Ile Leu Asp Arg Asn Ile Asp Thr Leu
    210                 215                 220

Arg Lys Leu Asp Glu Glu Phe Gln Gly Arg Ala Lys Val Val Tyr Ser
225                 230                 235                 240

Thr Glu Asp Ala Ile Glu Lys His Val Leu Ala Ala Asp Leu Val Ile
                245                 250                 255

Gly Ala Val Leu Ile Pro Gly Ala Ala Ala Pro Lys Leu Val Thr Lys
            260                 265                 270

Glu His Ile Ala Lys Met Lys Pro Gly Ala Ala Val Val Asp Val Ala
        275                 280                 285

Ile Asp Gln Gly Gly Cys Phe Glu Thr Ser His Ala Thr Thr His Ala
    290                 295                 300

Asp Pro Thr Tyr Ile Val Asp Asp Val Val His Tyr Cys Val Ala Asn
305                 310                 315                 320

Met Pro Gly Ala Val Ala Arg Thr Ser Thr Phe Ala Leu Asn Asn Ala
                325                 330                 335

Thr Leu Pro Tyr Ile Val Lys Leu Ala Asn Lys Gly Tyr Arg Glu Ala
            340                 345                 350

Leu Leu Ala Asp His Gly Phe Leu Glu Gly Leu Asn Val Ile His Gly
        355                 360                 365

Lys Val Thr Cys Lys Glu Val Ala Glu Ala Phe Asn Leu Glu Tyr Val
    370                 375                 380

Gln Pro Glu Thr Ala Ile Ala Met Phe Asn
385                 390

<210>  46
<211>  31
<212>  DNA
<213>  Artificial

<220>
<223>  PCR Forward primer

<400>  46
cgcgcggcag ccatatgatc attggcgtac c                                    31

<210>  47
<211>  32
<212>  DNA
<213>  Artificial

<220>
<223>  PCR Reverse Primer

<400>  47
gtgcggccgc aagcttttag ttgaacatgg cg                                   32

<210>  48
<211>  1275
<212>  DNA
<213>  Bacillus subtilis

<220>
<221>  CDS
<222>  (1)..(1275)

<400>  48
atg tca gca aag caa gtc tcg aaa gat gaa gaa aaa gaa gct ctt aac       48
Met Ser Ala Lys Gln Val Ser Lys Asp Glu Glu Lys Glu Ala Leu Asn
1               5                   10                  15

tta ttt ctg tct acc caa aca atc att aag gaa gcc ctt cgg aag ctg       96
Leu Phe Leu Ser Thr Gln Thr Ile Ile Lys Glu Ala Leu Arg Lys Leu
            20                  25                  30

ggt tat ccg gga gat atg tat gaa ctc atg aaa gag ccg cag aga atg      144
Gly Tyr Pro Gly Asp Met Tyr Glu Leu Met Lys Glu Pro Gln Arg Met
        35                  40                  45

ctc act gtc cgc att ccg gtc aaa atg gac aat ggg agc gtc aaa gtg 192
Leu Thr Val Arg Ile Pro Val Lys Met Asp Asn Gly Ser Val Lys Val
    50                  55                  60

ttc aca ggc tac cgg tca cag cac aat gat gct gtc ggt ccg aca aag 240
Phe Thr Gly Tyr Arg Ser Gln His Asn Asp Ala Val Gly Pro Thr Lys
65                  70                  75                  80

ggg ggc gtt cgc ttc cat cca gaa gtt aat gaa gag gaa gta aag gca 288
Gly Gly Val Arg Phe His Pro Glu Val Asn Glu Glu Glu Val Lys Ala
                85                  90                  95

tta tcc att tgg atg acg ctc aaa tgc ggg att gcc aat ctt cct tac 336
Leu Ser Ile Trp Met Thr Leu Lys Cys Gly Ile Ala Asn Leu Pro Tyr
            100                 105                 110

ggc ggc ggg aag ggc ggt att att tgt gat ccg cgg aca atg tca ttt 384
Gly Gly Gly Lys Gly Gly Ile Ile Cys Asp Pro Arg Thr Met Ser Phe
        115                 120                 125

gga gaa ctg gaa agg ctg agc agg ggg tat gtc cgt gcc atc agc cag 432
Gly Glu Leu Glu Arg Leu Ser Arg Gly Tyr Val Arg Ala Ile Ser Gln
    130                 135                 140

atc gtc ggt ccg aca aag gat att cca gct ccc gat gtg tac acc aat 480
Ile Val Gly Pro Thr Lys Asp Ile Pro Ala Pro Asp Val Tyr Thr Asn
145                 150                 155                 160

tcg cag att atg gcg tgg atg atg gat gag tac agc cgg ctg cgg gaa 528
Ser Gln Ile Met Ala Trp Met Met Asp Glu Tyr Ser Arg Leu Arg Glu
                165                 170                 175

ttc gat tct ccg ggc ttt att aca ggt aaa ccg ctt gtt ttg gga gga 576
Phe Asp Ser Pro Gly Phe Ile Thr Gly Lys Pro Leu Val Leu Gly Gly
            180                 185                 190

tcg caa gga cgg gaa aca gcg acg gca cag ggc gtc acg att tgt att 624
Ser Gln Gly Arg Glu Thr Ala Thr Ala Gln Gly Val Thr Ile Cys Ile
        195                 200                 205

gaa gag gcg gtg aag aaa aaa ggg atc aag ctg caa aac gcg cgc atc 672
Glu Glu Ala Val Lys Lys Lys Gly Ile Lys Leu Gln Asn Ala Arg Ile
    210                 215                 220

atc ata cag ggc ttt gga aac gcg ggt agc ttc ctg gcc aaa ttc atg 720
Ile Ile Gln Gly Phe Gly Asn Ala Gly Ser Phe Leu Ala Lys Phe Met
225                 230                 235                 240

cac gat gcg ggc gcg aag gtg atc ggg att tct gat gcc aat ggc ggg 768
His Asp Ala Gly Ala Lys Val Ile Gly Ile Ser Asp Ala Asn Gly Gly
                245                 250                 255

ctc tac aac cca gac ggc ctt gat atc cct tat ttg ctc gat aaa cgg 816
Leu Tyr Asn Pro Asp Gly Leu Asp Ile Pro Tyr Leu Leu Asp Lys Arg
            260                 265                 270

gac agc ttt ggt atg gtc acc aat tta ttt act gac gtc atc aca aat 864
Asp Ser Phe Gly Met Val Thr Asn Leu Phe Thr Asp Val Ile Thr Asn
        275                 280                 285

gag gag ctg ctt gaa aag gat tgc gat att tta gtg cct gcc gcg atc 912
Glu Glu Leu Leu Glu Lys Asp Cys Asp Ile Leu Val Pro Ala Ala Ile
    290                 295                 300

tcc aat caa atc aca gcc aaa aac gca cat aac att cag gcg tca atc 960

Ser Asn Gln Ile Thr Ala Lys Asn Ala His Asn Ile Gln Ala Ser Ile
305                 310                 315                 320

gtc gtt gaa gcg gcg aac ggc ccg aca acc att gat gcc act aag atc      1008
Val Val Glu Ala Ala Asn Gly Pro Thr Thr Ile Asp Ala Thr Lys Ile
                325                 330                 335

ctg aat gaa aga ggc gtg ctg ctt gtg ccg gat atc cta gcg agt gcc      1056
Leu Asn Glu Arg Gly Val Leu Leu Val Pro Asp Ile Leu Ala Ser Ala
            340                 345                 350

ggc ggc gtc acg gtt tct tat ttt gaa tgg gtg caa aac aac caa gga      1104
Gly Gly Val Thr Val Ser Tyr Phe Glu Trp Val Gln Asn Asn Gln Gly
        355                 360                 365

tat tat tgg tcg gaa gaa gag gtt gca gaa aaa ctg aga agc gtc atg      1152
Tyr Tyr Trp Ser Glu Glu Glu Val Ala Glu Lys Leu Arg Ser Val Met
    370                 375                 380

gtc agc tcg ttc gaa aca att tat caa aca gcg gca aca cat aaa gtg      1200
Val Ser Ser Phe Glu Thr Ile Tyr Gln Thr Ala Ala Thr His Lys Val
385                 390                 395                 400

gat atg cgt ttg gcg gct tac atg acg ggc atc aga aaa tcg gca gaa      1248
Asp Met Arg Leu Ala Ala Tyr Met Thr Gly Ile Arg Lys Ser Ala Glu
                405                 410                 415

gca tcg cgt ttc cgc gga tgg gtc taa                                  1275
Ala Ser Arg Phe Arg Gly Trp Val
            420


<210>  49
<211>  424
<212>  PRT
<213>  Bacillus subtilis

<400>  49

Met Ser Ala Lys Gln Val Ser Lys Asp Glu Glu Lys Glu Ala Leu Asn
1               5                   10                  15


Leu Phe Leu Ser Thr Gln Thr Ile Ile Lys Glu Ala Leu Arg Lys Leu
            20                  25                  30


Gly Tyr Pro Gly Asp Met Tyr Glu Leu Met Lys Glu Pro Gln Arg Met
        35                  40                  45


Leu Thr Val Arg Ile Pro Val Lys Met Asp Asn Gly Ser Val Lys Val
    50                  55                  60


Phe Thr Gly Tyr Arg Ser Gln His Asn Asp Ala Val Gly Pro Thr Lys
65                  70                  75                  80


Gly Gly Val Arg Phe His Pro Glu Val Asn Glu Glu Glu Val Lys Ala
                85                  90                  95


Leu Ser Ile Trp Met Thr Leu Lys Cys Gly Ile Ala Asn Leu Pro Tyr
            100                 105                 110

Gly Gly Gly Lys Gly Gly Ile Ile Cys Asp Pro Arg Thr Met Ser Phe
        115                 120                 125

Gly Glu Leu Glu Arg Leu Ser Arg Gly Tyr Val Arg Ala Ile Ser Gln
    130                 135                 140

Ile Val Gly Pro Thr Lys Asp Ile Pro Ala Pro Asp Val Tyr Thr Asn
145                 150                 155                 160

Ser Gln Ile Met Ala Trp Met Met Asp Glu Tyr Ser Arg Leu Arg Glu
                165                 170                 175

Phe Asp Ser Pro Gly Phe Ile Thr Gly Lys Pro Leu Val Leu Gly Gly
            180                 185                 190

Ser Gln Gly Arg Glu Thr Ala Thr Ala Gln Gly Val Thr Ile Cys Ile
        195                 200                 205

Glu Glu Ala Val Lys Lys Lys Gly Ile Lys Leu Gln Asn Ala Arg Ile
    210                 215                 220

Ile Ile Gln Gly Phe Gly Asn Ala Gly Ser Phe Leu Ala Lys Phe Met
225                 230                 235                 240

His Asp Ala Gly Ala Lys Val Ile Gly Ile Ser Asp Ala Asn Gly Gly
                245                 250                 255

Leu Tyr Asn Pro Asp Gly Leu Asp Ile Pro Tyr Leu Leu Asp Lys Arg
            260                 265                 270

Asp Ser Phe Gly Met Val Thr Asn Leu Phe Thr Asp Val Ile Thr Asn
        275                 280                 285

Glu Glu Leu Leu Glu Lys Asp Cys Asp Ile Leu Val Pro Ala Ala Ile
    290                 295                 300

Ser Asn Gln Ile Thr Ala Lys Asn Ala His Asn Ile Gln Ala Ser Ile
305                 310                 315                 320

Val Val Glu Ala Ala Asn Gly Pro Thr Thr Ile Asp Ala Thr Lys Ile
                325                 330                 335

Leu Asn Glu Arg Gly Val Leu Leu Val Pro Asp Ile Leu Ala Ser Ala
            340                 345                 350

Gly Gly Val Thr Val Ser Tyr Phe Glu Trp Val Gln Asn Asn Gln Gly
        355                 360                 365

Tyr Tyr Trp Ser Glu Glu Glu Val Ala Glu Lys Leu Arg Ser Val Met
    370                 375                 380

Val Ser Ser Phe Glu Thr Ile Tyr Gln Thr Ala Ala Thr His Lys Val
385                 390                 395                 400

Asp Met Arg Leu Ala Ala Tyr Met Thr Gly Ile Arg Lys Ser Ala Glu
                405                 410                 415

Ala Ser Arg Phe Arg Gly Trp Val
            420

<210>  50
<211>  1266
<212>  DNA
<213>  Pyrobaculum islandicum

<220>
<221>  CDS
<222>  (1)..(1266)

<400>  50
atg gag agg aca ggg ttt tta gag tat gtg ctt aac tat gta aaa aaa        48
Met Glu Arg Thr Gly Phe Leu Glu Tyr Val Leu Asn Tyr Val Lys Lys
1               5                   10                  15

ggc gta gaa tta ggc gga ttt ccc gaa gat ttc tat aaa atc ttg tct        96
Gly Val Glu Leu Gly Gly Phe Pro Glu Asp Phe Tyr Lys Ile Leu Ser
            20                  25                  30

agg cct agg cgt gtt ttg att gtt aat att cct gtg agg ttg gat ggt       144
Arg Pro Arg Arg Val Leu Ile Val Asn Ile Pro Val Arg Leu Asp Gly
        35                  40                  45

ggt ggt ttt gag gtg ttt gag ggt tat cgt gtg cag cat tgt gat gtt       192
Gly Gly Phe Glu Val Phe Glu Gly Tyr Arg Val Gln His Cys Asp Val
    50                  55                  60

ctc ggc ccg tat aag ggt ggg gtt cgt ttt cat cct gag gtt act ctt       240
Leu Gly Pro Tyr Lys Gly Gly Val Arg Phe His Pro Glu Val Thr Leu
65                  70                  75                  80

gcc gac gac gtc gcc ttg gct att tta atg acg ttg aaa aat agc cta       288
Ala Asp Asp Val Ala Leu Ala Ile Leu Met Thr Leu Lys Asn Ser Leu
                85                  90                  95

gct ggc ttg ccg tat ggc ggc gct aaa ggc gca gta cgt gta gac cct       336
Ala Gly Leu Pro Tyr Gly Gly Ala Lys Gly Ala Val Arg Val Asp Pro
            100                 105                 110

aag aaa ctc tcg cag aga gaa ctt gaa gag ctc tct aga gga tat gcc       384
Lys Lys Leu Ser Gln Arg Glu Leu Glu Glu Leu Ser Arg Gly Tyr Ala
        115                 120                 125

aga gcc ata gcc ccc ctc ata ggc gac gta gtc gac atc cca gcg cct       432
Arg Ala Ile Ala Pro Leu Ile Gly Asp Val Val Asp Ile Pro Ala Pro
    130                 135                 140

gac gtt ggg aca aac gcc caa atc atg gcg tgg atg gta gac gaa tat       480

Asp Val Gly Thr Asn Ala Gln Ile Met Ala Trp Met Val Asp Glu Tyr
145                 150                 155                 160

tca aaa ata aag ggc tac aac gtg ccc ggg gta ttc acc tca aaa cca      528
Ser Lys Ile Lys Gly Tyr Asn Val Pro Gly Val Phe Thr Ser Lys Pro
                165                 170                 175

cca gag cta tgg gga aac cca gtg aga gaa tac gcc aca ggc ttt gga      576
Pro Glu Leu Trp Gly Asn Pro Val Arg Glu Tyr Ala Thr Gly Phe Gly
            180                 185                 190

gta gca gta gcg aca aga gaa atg gca aaa aaa cta tgg ggc gga ata      624
Val Ala Val Ala Thr Arg Glu Met Ala Lys Lys Leu Trp Gly Gly Ile
        195                 200                 205

gaa gga aaa aca gta gcg ata cag ggt atg ggt aat gtg ggg agg tgg      672
Glu Gly Lys Thr Val Ala Ile Gln Gly Met Gly Asn Val Gly Arg Trp
    210                 215                 220

aca gcg tat tgg cta gaa aaa atg ggc gct aag gtg ata gct gtg tct      720
Thr Ala Tyr Trp Leu Glu Lys Met Gly Ala Lys Val Ile Ala Val Ser
225                 230                 235                 240

gat ata aat ggc gta gct tat aga aag gag gga ctt aat gtt gaa ttg      768
Asp Ile Asn Gly Val Ala Tyr Arg Lys Glu Gly Leu Asn Val Glu Leu
                245                 250                 255

ata caa aaa aac aag gga ctt aca ggg cct gct ctc gta gaa ctg ttt      816
Ile Gln Lys Asn Lys Gly Leu Thr Gly Pro Ala Leu Val Glu Leu Phe
            260                 265                 270

acg aca aaa gac aat gcc gaa ttt gtt aaa aac cca gat gcc ata ttt      864
Thr Thr Lys Asp Asn Ala Glu Phe Val Lys Asn Pro Asp Ala Ile Phe
        275                 280                 285

aaa cta gac gtc gac att ttc gtc ccc gcc gct att gag aat gtc att      912
Lys Leu Asp Val Asp Ile Phe Val Pro Ala Ala Ile Glu Asn Val Ile
    290                 295                 300

agg ggt gat aat gct ggg ctt gtg aag gct agg ctt gtt gtt gag ggt      960
Arg Gly Asp Asn Ala Gly Leu Val Lys Ala Arg Leu Val Val Glu Gly
305                 310                 315                 320

gct aat ggc cct act act cct gag gct gag agg att ttg tat gag agg     1008
Ala Asn Gly Pro Thr Thr Pro Glu Ala Glu Arg Ile Leu Tyr Glu Arg
                325                 330                 335

ggg gtt gtc gta gtt cct gat atc ctc gcc aac gcc ggc ggc gtg att     1056
Gly Val Val Val Val Pro Asp Ile Leu Ala Asn Ala Gly Gly Val Ile
            340                 345                 350

atg tcg tat tta gag tgg gtg gag aat ctc cag tgg tat atc tgg gac     1104
Met Ser Tyr Leu Glu Trp Val Glu Asn Leu Gln Trp Tyr Ile Trp Asp
        355                 360                 365

gag gag gag act aga aag aga ctt gaa aac ata atg gta aac aac gtc     1152
Glu Glu Glu Thr Arg Lys Arg Leu Glu Asn Ile Met Val Asn Asn Val
    370                 375                 380

gag aga gta tac aag aga tgg cag aga gaa aaa gga tgg aca atg aga     1200
Glu Arg Val Tyr Lys Arg Trp Gln Arg Glu Lys Gly Trp Thr Met Arg
385                 390                 395                 400

gac gca gca ata gta acc gca tta gaa aga ata tac aac gcc atg aaa     1248
Asp Ala Ala Ile Val Thr Ala Leu Glu Arg Ile Tyr Asn Ala Met Lys

405                 410                 415

ata aga ggg tgg atc taa                                         1266
Ile Arg Gly Trp Ile
            420

<210>  51
<211>  421
<212>  PRT
<213>  Pyrobaculum islandicum

<400>  51

Met Glu Arg Thr Gly Phe Leu Glu Tyr Val Leu Asn Tyr Val Lys Lys
1               5                   10                  15

Gly Val Glu Leu Gly Gly Phe Pro Glu Asp Phe Tyr Lys Ile Leu Ser
            20                  25                  30

Arg Pro Arg Arg Val Leu Ile Val Asn Ile Pro Val Arg Leu Asp Gly
        35                  40                  45

Gly Gly Phe Glu Val Phe Glu Gly Tyr Arg Val Gln His Cys Asp Val
    50                  55                  60

Leu Gly Pro Tyr Lys Gly Gly Val Arg Phe His Pro Glu Val Thr Leu
65                  70                  75                  80

Ala Asp Asp Val Ala Leu Ala Ile Leu Met Thr Leu Lys Asn Ser Leu
                85                  90                  95

Ala Gly Leu Pro Tyr Gly Gly Ala Lys Gly Ala Val Arg Val Asp Pro
            100                 105                 110

Lys Lys Leu Ser Gln Arg Glu Leu Glu Glu Leu Ser Arg Gly Tyr Ala
        115                 120                 125

Arg Ala Ile Ala Pro Leu Ile Gly Asp Val Val Asp Ile Pro Ala Pro
    130                 135                 140

Asp Val Gly Thr Asn Ala Gln Ile Met Ala Trp Met Val Asp Glu Tyr
145                 150                 155                 160

Ser Lys Ile Lys Gly Tyr Asn Val Pro Gly Val Phe Thr Ser Lys Pro
                165                 170                 175

Pro Glu Leu Trp Gly Asn Pro Val Arg Glu Tyr Ala Thr Gly Phe Gly
            180                 185                 190

Val Ala Val Ala Thr Arg Glu Met Ala Lys Lys Leu Trp Gly Gly Ile
        195                 200                 205

Glu Gly Lys Thr Val Ala Ile Gln Gly Met Gly Asn Val Gly Arg Trp
    210                 215                 220

Thr Ala Tyr Trp Leu Glu Lys Met Gly Ala Lys Val Ile Ala Val Ser
225                 230                 235                 240

Asp Ile Asn Gly Val Ala Tyr Arg Lys Glu Gly Leu Asn Val Glu Leu
                245                 250                 255

Ile Gln Lys Asn Lys Gly Leu Thr Gly Pro Ala Leu Val Glu Leu Phe
            260                 265                 270

Thr Thr Lys Asp Asn Ala Glu Phe Val Lys Asn Pro Asp Ala Ile Phe
        275                 280                 285

Lys Leu Asp Val Asp Ile Phe Val Pro Ala Ala Ile Glu Asn Val Ile
    290                 295                 300

Arg Gly Asp Asn Ala Gly Leu Val Lys Ala Arg Leu Val Val Glu Gly
305                 310                 315                 320

Ala Asn Gly Pro Thr Thr Pro Glu Ala Glu Arg Ile Leu Tyr Glu Arg
                325                 330                 335

Gly Val Val Val Val Pro Asp Ile Leu Ala Asn Ala Gly Gly Val Ile
            340                 345                 350

Met Ser Tyr Leu Glu Trp Val Glu Asn Leu Gln Trp Tyr Ile Trp Asp
        355                 360                 365

Glu Glu Glu Thr Arg Lys Arg Leu Glu Asn Ile Met Val Asn Asn Val
    370                 375                 380

Glu Arg Val Tyr Lys Arg Trp Gln Arg Glu Lys Gly Trp Thr Met Arg
385                 390                 395                 400

Asp Ala Ala Ile Val Thr Ala Leu Glu Arg Ile Tyr Asn Ala Met Lys
                405                 410                 415

Ile Arg Gly Trp Ile
            420

<210>  52
<211>  1353
<212>  DNA
<213>  Clostridium symbiosum

<220>
<221>  CDS

<222>  (1)..(1353)

<400>  52
atg agc aag tat gtt gac aga gta att gcg gaa gtc gag aag aaa tac      48
Met Ser Lys Tyr Val Asp Arg Val Ile Ala Glu Val Glu Lys Lys Tyr
1               5                   10                  15

gca gac gag ccg gag ttc gta caa aca gta gaa gag gtt tta tct tct      96
Ala Asp Glu Pro Glu Phe Val Gln Thr Val Glu Glu Val Leu Ser Ser
            20                  25                  30

tta ggt ccg gtt gtt gat gca cat ccg gag tat gag gaa gta gct ctt      144
Leu Gly Pro Val Val Asp Ala His Pro Glu Tyr Glu Glu Val Ala Leu
        35                  40                  45

ctc gag aga atg gta att ccg gag aga gtt atc gag ttc cgt gtt ccg      192
Leu Glu Arg Met Val Ile Pro Glu Arg Val Ile Glu Phe Arg Val Pro
    50                  55                  60

tgg gag gat gac aac ggc aag gtt cat gta aac aca ggc tac cgc gtt      240
Trp Glu Asp Asp Asn Gly Lys Val His Val Asn Thr Gly Tyr Arg Val
65                  70                  75                  80

cag ttc aac ggc gcc att gga ccg tac aag ggc ggc tta cgt ttt gct      288
Gln Phe Asn Gly Ala Ile Gly Pro Tyr Lys Gly Gly Leu Arg Phe Ala
                85                  90                  95

ccg tct gta aac ctt tcc atc atg aaa ttc tta ggc ttt gag cag gca      336
Pro Ser Val Asn Leu Ser Ile Met Lys Phe Leu Gly Phe Glu Gln Ala
            100                 105                 110

ttc aaa gac agc ctt aca aca ctt cct atg ggt ggt gcc aaa ggt ggt      384
Phe Lys Asp Ser Leu Thr Thr Leu Pro Met Gly Gly Ala Lys Gly Gly
        115                 120                 125

tcc gac ttc gat ccg aac ggt aag agc gac aga gaa gtt atg aga ttc      432
Ser Asp Phe Asp Pro Asn Gly Lys Ser Asp Arg Glu Val Met Arg Phe
    130                 135                 140

tgc cag gct ttc atg aca gaa tta tac cgt cac atc gga ccg gac att      480
Cys Gln Ala Phe Met Thr Glu Leu Tyr Arg His Ile Gly Pro Asp Ile
145                 150                 155                 160

gat gtt cct gca ggt gac ctt ggt gta ggc gct cgt gaa atc ggt tat      528
Asp Val Pro Ala Gly Asp Leu Gly Val Gly Ala Arg Glu Ile Gly Tyr
                165                 170                 175

atg tac gga cag tat cgt aaa att gtc ggc gga ttc tac aac ggc gtt      576
Met Tyr Gly Gln Tyr Arg Lys Ile Val Gly Gly Phe Tyr Asn Gly Val
            180                 185                 190

ctg aca ggt aag gca aga tcc ttc ggt ggt tcc tta gtt cgt ccg gaa      624
Leu Thr Gly Lys Ala Arg Ser Phe Gly Gly Ser Leu Val Arg Pro Glu
        195                 200                 205

gct aca gga tac ggc tcc gta tac tat gta gaa gca gtt atg aaa cat      672
Ala Thr Gly Tyr Gly Ser Val Tyr Tyr Val Glu Ala Val Met Lys His
    210                 215                 220

gag aac gat acc ctg gtt ggc aag aca gtt gca tta gca ggt ttt ggt      720
Glu Asn Asp Thr Leu Val Gly Lys Thr Val Ala Leu Ala Gly Phe Gly
225                 230                 235                 240

aac gta gca tgg ggc gct gct aag aag ctt gct gaa tta ggc gct aaa      768
Asn Val Ala Trp Gly Ala Ala Lys Lys Leu Ala Glu Leu Gly Ala Lys

245                 250                 255

gca gtt aca ctt tcc gga cca gac gga tac atc tac gat ccg gaa ggc      816
Ala Val Thr Leu Ser Gly Pro Asp Gly Tyr Ile Tyr Asp Pro Glu Gly
        260                 265                 270

atc aca aca gaa gag aag atc aac tac atg ctt gag atg aga gca tcc      864
Ile Thr Thr Glu Glu Lys Ile Asn Tyr Met Leu Glu Met Arg Ala Ser
        275                 280                 285

gga cgt aac aaa gtt cag gat tat gca gac aag ttc ggc gta cag ttc      912
Gly Arg Asn Lys Val Gln Asp Tyr Ala Asp Lys Phe Gly Val Gln Phe
    290                 295                 300

ttc ccg ggt gag aaa cca tgg ggc cag aag gtt gac atc atc atg cca      960
Phe Pro Gly Glu Lys Pro Trp Gly Gln Lys Val Asp Ile Ile Met Pro
305                 310                 315                 320

tgc gct act cag aac gat gtt gat tta gag cag gct aag aag atc gtt     1008
Cys Ala Thr Gln Asn Asp Val Asp Leu Glu Gln Ala Lys Lys Ile Val
                325                 330                 335

gct aac aac gtt aaa tac tac atc gaa gtt gct aac atg cca act acc     1056
Ala Asn Asn Val Lys Tyr Tyr Ile Glu Val Ala Asn Met Pro Thr Thr
            340                 345                 350

aac gaa gct ctc aga ttc tta atg cag cag cca aac atg gta gta gct     1104
Asn Glu Ala Leu Arg Phe Leu Met Gln Gln Pro Asn Met Val Val Ala
        355                 360                 365

cct tcc aag gct gta aac gca ggc ggc gtt ctt gta tcc ggc ttt gag     1152
Pro Ser Lys Ala Val Asn Ala Gly Gly Val Leu Val Ser Gly Phe Glu
    370                 375                 380

atg agc cag aac agc gag aga ctt tcc tgg aca gca gag gaa gtt gac     1200
Met Ser Gln Asn Ser Glu Arg Leu Ser Trp Thr Ala Glu Glu Val Asp
385                 390                 395                 400

tcc aaa ctt cat cag gtt atg aca gat atc cat gac ggt tcc gct gca     1248
Ser Lys Leu His Gln Val Met Thr Asp Ile His Asp Gly Ser Ala Ala
                405                 410                 415

gca gca gag cgt tac ggc tta ggc tac aat ctg gtt gcc ggc gct aac     1296
Ala Ala Glu Arg Tyr Gly Leu Gly Tyr Asn Leu Val Ala Gly Ala Asn
            420                 425                 430

atc gtt gga ttc cag aag att gct gat gct atg atg gct cag ggc att     1344
Ile Val Gly Phe Gln Lys Ile Ala Asp Ala Met Met Ala Gln Gly Ile
        435                 440                 445

gct tgg taa                                                         1353
Ala Trp
    450


<210>  53
<211>  450
<212>  PRT
<213>  Clostridium symbiosum

<400>  53

Met Ser Lys Tyr Val Asp Arg Val Ile Ala Glu Val Glu Lys Lys Tyr
1               5                   10                  15

Ala Asp Glu Pro Glu Phe Val Gln Thr Val Glu Glu Val Leu Ser Ser
                20                  25                  30

Leu Gly Pro Val Val Asp Ala His Pro Glu Tyr Glu Glu Val Ala Leu
        35                  40                  45

Leu Glu Arg Met Val Ile Pro Glu Arg Val Ile Glu Phe Arg Val Pro
    50                  55                  60

Trp Glu Asp Asp Asn Gly Lys Val His Val Asn Thr Gly Tyr Arg Val
65                  70                  75                  80

Gln Phe Asn Gly Ala Ile Gly Pro Tyr Lys Gly Gly Leu Arg Phe Ala
                85                  90                  95

Pro Ser Val Asn Leu Ser Ile Met Lys Phe Leu Gly Phe Glu Gln Ala
            100                 105                 110

Phe Lys Asp Ser Leu Thr Thr Leu Pro Met Gly Gly Ala Lys Gly Gly
        115                 120                 125

Ser Asp Phe Asp Pro Asn Gly Lys Ser Asp Arg Glu Val Met Arg Phe
    130                 135                 140

Cys Gln Ala Phe Met Thr Glu Leu Tyr Arg His Ile Gly Pro Asp Ile
145                 150                 155                 160

Asp Val Pro Ala Gly Asp Leu Gly Val Gly Ala Arg Glu Ile Gly Tyr
                165                 170                 175

Met Tyr Gly Gln Tyr Arg Lys Ile Val Gly Gly Phe Tyr Asn Gly Val
            180                 185                 190

Leu Thr Gly Lys Ala Arg Ser Phe Gly Gly Ser Leu Val Arg Pro Glu
        195                 200                 205

Ala Thr Gly Tyr Gly Ser Val Tyr Tyr Val Glu Ala Val Met Lys His
    210                 215                 220

Glu Asn Asp Thr Leu Val Gly Lys Thr Val Ala Leu Ala Gly Phe Gly
225                 230                 235                 240

Asn Val Ala Trp Gly Ala Ala Lys Lys Leu Ala Glu Leu Gly Ala Lys
                245                 250                 255

Ala Val Thr Leu Ser Gly Pro Asp Gly Tyr Ile Tyr Asp Pro Glu Gly
            260                 265                 270

Ile Thr Thr Glu Glu Lys Ile Asn Tyr Met Leu Glu Met Arg Ala Ser
        275                 280                 285

Gly Arg Asn Lys Val Gln Asp Tyr Ala Asp Lys Phe Gly Val Gln Phe
    290                 295                 300

Phe Pro Gly Glu Lys Pro Trp Gly Gln Lys Val Asp Ile Ile Met Pro
305                 310                 315                 320

Cys Ala Thr Gln Asn Asp Val Asp Leu Glu Gln Ala Lys Lys Ile Val
                325                 330                 335

Ala Asn Asn Val Lys Tyr Tyr Ile Glu Val Ala Asn Met Pro Thr Thr
            340                 345                 350

Asn Glu Ala Leu Arg Phe Leu Met Gln Gln Pro Asn Met Val Val Ala
        355                 360                 365

Pro Ser Lys Ala Val Asn Ala Gly Gly Val Leu Val Ser Gly Phe Glu
    370                 375                 380

Met Ser Gln Asn Ser Glu Arg Leu Ser Trp Thr Ala Glu Glu Val Asp
385                 390                 395                 400

Ser Lys Leu His Gln Val Met Thr Asp Ile His Asp Gly Ser Ala Ala
                405                 410                 415

Ala Ala Glu Arg Tyr Gly Leu Gly Tyr Asn Leu Val Ala Gly Ala Asn
            420                 425                 430

Ile Val Gly Phe Gln Lys Ile Ala Asp Ala Met Met Ala Gln Gly Ile
        435                 440                 445

Ala Trp
    450

<210> 54
<211> 1257
<212> DNA
<213> Thermococcus profundus

<220>
<221> CDS
<222> (1)..(1257)

<400> 54
atg gtc gag att gac ccg ttt gag atg gcc gtt aag cag ctt gag agg 48
Met Val Glu Ile Asp Pro Phe Glu Met Ala Val Lys Gln Leu Glu Arg
1               5                   10                  15

gct gct cag tac atg gac ata agc gaa gag gcc ctc gag tgg ctt aag 96
Ala Ala Gln Tyr Met Asp Ile Ser Glu Glu Ala Leu Glu Trp Leu Lys

20                  25                  30

aag ccc atg agg atc gtc gaa gtt agc gtt ccg ata gag atg gac gac      144
Lys Pro Met Arg Ile Val Glu Val Ser Val Pro Ile Glu Met Asp Asp
        35                  40                  45

ggt tct gtt aag gtt ttc acc ggt ttc cgc gtt cag cac aac tgg gct      192
Gly Ser Val Lys Val Phe Thr Gly Phe Arg Val Gln His Asn Trp Ala
    50                  55                  60

cgc ggc ccg acc aag ggt ggt ata agg tgg cac ccg gcc gag acc ctc      240
Arg Gly Pro Thr Lys Gly Gly Ile Arg Trp His Pro Ala Glu Thr Leu
65                  70                  75                  80

agc acc gtt aag gcc ctc gct acc tgg atg acc tgg aag gtt gca gtt      288
Ser Thr Val Lys Ala Leu Ala Thr Trp Met Thr Trp Lys Val Ala Val
                85                  90                  95

gtc gac ctc ccc tac ggt gga ggt aag ggt ggc atc atc gtc aac ccg      336
Val Asp Leu Pro Tyr Gly Gly Gly Lys Gly Gly Ile Ile Val Asn Pro
            100                 105                 110

aag gag ctc tcc gag agg gag cag gag agg ctc gct cgc gct tac ata      384
Lys Glu Leu Ser Glu Arg Glu Gln Glu Arg Leu Ala Arg Ala Tyr Ile
        115                 120                 125

agg gcc gtt tat gac gtc atc ggt ccg tgg acc gac att ccg gct ccc      432
Arg Ala Val Tyr Asp Val Ile Gly Pro Trp Thr Asp Ile Pro Ala Pro
    130                 135                 140

gac gtc tac acc aac ccg aag atc atg ggc tgg atg atg gac gag tat      480
Asp Val Tyr Thr Asn Pro Lys Ile Met Gly Trp Met Met Asp Glu Tyr
145                 150                 155                 160

gaa aca ata atg agg agg aag ggc ccg gcc ttc ggt gtc atc act gga      528
Glu Thr Ile Met Arg Arg Lys Gly Pro Ala Phe Gly Val Ile Thr Gly
                165                 170                 175

aag ccg ctc agc att ggc ggt tca ctc ggt agg ggt aca gca aca gcc      576
Lys Pro Leu Ser Ile Gly Gly Ser Leu Gly Arg Gly Thr Ala Thr Ala
            180                 185                 190

cag ggt gca ata ttc acc atc agg gag gcc gcc aag gcc ctc ggc atc      624
Gln Gly Ala Ile Phe Thr Ile Arg Glu Ala Ala Lys Ala Leu Gly Ile
        195                 200                 205

gac ctc aag ggc aag aag atc gcc gtc cag ggc tat ggt aac gcc ggc      672
Asp Leu Lys Gly Lys Lys Ile Ala Val Gln Gly Tyr Gly Asn Ala Gly
    210                 215                 220

tac tac acc gcc aag ctc gcc aag gag cag ctc ggc atg acc gtc gtc      720
Tyr Tyr Thr Ala Lys Leu Ala Lys Glu Gln Leu Gly Met Thr Val Val
225                 230                 235                 240

gcc gtc agc gac agc cgc ggc ggc atc tac aac ccg gac ggc ctc gac      768
Ala Val Ser Asp Ser Arg Gly Gly Ile Tyr Asn Pro Asp Gly Leu Asp
                245                 250                 255

ccg gac gag gtt ctc aag tgg aag agg gag cac ggc tca gtt aag gac      816
Pro Asp Glu Val Leu Lys Trp Lys Arg Glu His Gly Ser Val Lys Asp
            260                 265                 270

ttc cca gga gcg acc aac atc acc aac gag gag ctc ctc gag ctc gag      864
Phe Pro Gly Ala Thr Asn Ile Thr Asn Glu Glu Leu Leu Glu Leu Glu
        275                 280                 285

gta gac gtc ctc gct ccg gca gct atc gaa gag gtc atc acc gag aag 912
Val Asp Val Leu Ala Pro Ala Ala Ile Glu Glu Val Ile Thr Glu Lys
    290                 295                 300

aac gcc gac aac atc aag gcc aag atc gtc gcc gag gtt gcc aac ggt 960
Asn Ala Asp Asn Ile Lys Ala Lys Ile Val Ala Glu Val Ala Asn Gly
305                 310                 315                 320

cca gtt acg cca gag gca gac gac atc ctc cgc gag aag ggc atc ctc 1008
Pro Val Thr Pro Glu Ala Asp Asp Ile Leu Arg Glu Lys Gly Ile Leu
                325                 330                 335

cag att ccg gac ttc ctg tgc aac gcc ggt ggc gtc acc gtc agc tac 1056
Gln Ile Pro Asp Phe Leu Cys Asn Ala Gly Gly Val Thr Val Ser Tyr
            340                 345                 350

ttc gag tgg gtc cag aac ata aac ggc tac tac tgg acc gag gag gag 1104
Phe Glu Trp Val Gln Asn Ile Asn Gly Tyr Tyr Trp Thr Glu Glu Glu
        355                 360                 365

gtc cgc gag aag ctc gac aag aag atg acc aag gcc ttc tgg gag gtc 1152
Val Arg Glu Lys Leu Asp Lys Lys Met Thr Lys Ala Phe Trp Glu Val
    370                 375                 380

tac aac acc cac aag gac aag aac atc cac atg agg gac gcc gcc tac 1200
Tyr Asn Thr His Lys Asp Lys Asn Ile His Met Arg Asp Ala Ala Tyr
385                 390                 395                 400

gtc gtc gcg gtc agc agg gtt tac cag gcc atg aag gac cgc gga tgg 1248
Val Val Ala Val Ser Arg Val Tyr Gln Ala Met Lys Asp Arg Gly Trp
                405                 410                 415

gtg aag aag 1257
Val Lys Lys

<210> 55
<211> 419
<212> PRT
<213> Thermococcus profundus

<400> 55

Met Val Glu Ile Asp Pro Phe Glu Met Ala Val Lys Gln Leu Glu Arg
1               5                   10                  15

Ala Ala Gln Tyr Met Asp Ile Ser Glu Glu Ala Leu Glu Trp Leu Lys
            20                  25                  30

Lys Pro Met Arg Ile Val Glu Val Ser Val Pro Ile Glu Met Asp Asp
        35                  40                  45

Gly Ser Val Lys Val Phe Thr Gly Phe Arg Val Gln His Asn Trp Ala
    50                  55                  60

Arg Gly Pro Thr Lys Gly Gly Ile Arg Trp His Pro Ala Glu Thr Leu
65                  70                  75                  80

Ser Thr Val Lys Ala Leu Ala Thr Trp Met Thr Trp Lys Val Ala Val
                85                  90                  95

Val Asp Leu Pro Tyr Gly Gly Gly Lys Gly Gly Ile Ile Val Asn Pro
            100                 105                 110

Lys Glu Leu Ser Glu Arg Glu Gln Glu Arg Leu Ala Arg Ala Tyr Ile
        115                 120                 125

Arg Ala Val Tyr Asp Val Ile Gly Pro Trp Thr Asp Ile Pro Ala Pro
    130                 135                 140

Asp Val Tyr Thr Asn Pro Lys Ile Met Gly Trp Met Met Asp Glu Tyr
145                 150                 155                 160

Glu Thr Ile Met Arg Arg Lys Gly Pro Ala Phe Gly Val Ile Thr Gly
                165                 170                 175

Lys Pro Leu Ser Ile Gly Gly Ser Leu Gly Arg Gly Thr Ala Thr Ala
            180                 185                 190

Gln Gly Ala Ile Phe Thr Ile Arg Glu Ala Ala Lys Ala Leu Gly Ile
        195                 200                 205

Asp Leu Lys Gly Lys Lys Ile Ala Val Gln Gly Tyr Gly Asn Ala Gly
    210                 215                 220

Tyr Tyr Thr Ala Lys Leu Ala Lys Glu Gln Leu Gly Met Thr Val Val
225                 230                 235                 240

Ala Val Ser Asp Ser Arg Gly Gly Ile Tyr Asn Pro Asp Gly Leu Asp
                245                 250                 255

Pro Asp Glu Val Leu Lys Trp Lys Arg Glu His Gly Ser Val Lys Asp
            260                 265                 270

Phe Pro Gly Ala Thr Asn Ile Thr Asn Glu Glu Leu Leu Glu Leu Glu
        275                 280                 285

Val Asp Val Leu Ala Pro Ala Ala Ile Glu Glu Val Ile Thr Glu Lys
    290                 295                 300

Asn Ala Asp Asn Ile Lys Ala Lys Ile Val Ala Glu Val Ala Asn Gly
305                 310                 315                 320

Pro Val Thr Pro Glu Ala Asp Asp Ile Leu Arg Glu Lys Gly Ile Leu
                325                 330                 335

Gln Ile Pro Asp Phe Leu Cys Asn Ala Gly Gly Val Thr Val Ser Tyr

340                 345                 350

Phe Glu Trp Val Gln Asn Ile Asn Gly Tyr Tyr Trp Thr Glu Glu Glu
        355                 360                 365

Val Arg Glu Lys Leu Asp Lys Lys Met Thr Lys Ala Phe Trp Glu Val
    370                 375                 380

Tyr Asn Thr His Lys Asp Lys Asn Ile His Met Arg Asp Ala Ala Tyr
385                 390                 395                 400

Val Val Ala Val Ser Arg Val Tyr Gln Ala Met Lys Asp Arg Gly Trp
                405                 410                 415

Val Lys Lys

<210>  56
<211>  1380
<212>  DNA
<213>  Escherichia coli

<220>
<221>  CDS
<222>  (1)..(1380)

<400>  56
atg aac agg tta cct tcg agc gca tcg gct tta gcg tgc agc gcc cac        48
Met Asn Arg Leu Pro Ser Ser Ala Ser Ala Leu Ala Cys Ser Ala His
1               5                   10                  15

gcc ctg aat ctc att gag aag cga acg ctg gat cat gag gag atg aaa        96
Ala Leu Asn Leu Ile Glu Lys Arg Thr Leu Asp His Glu Glu Met Lys
            20                  25                  30

gca ctt aac cga gag gtg att gaa tac ttc aaa gag cat gtc aat ccg       144
Ala Leu Asn Arg Glu Val Ile Glu Tyr Phe Lys Glu His Val Asn Pro
        35                  40                  45

ggg ttt tta gag tat cgc aaa tct gtt acc gcc ggc ggg gat tac gga       192
Gly Phe Leu Glu Tyr Arg Lys Ser Val Thr Ala Gly Gly Asp Tyr Gly
    50                  55                  60

gcc gta gag tgg caa gcg gga agt tta aat acg ctt gtc gac acc cag       240
Ala Val Glu Trp Gln Ala Gly Ser Leu Asn Thr Leu Val Asp Thr Gln
65                  70                  75                  80

ggc cag gag ttt atc gac tgc ctg gga ggt ttt gga att ttc aac gtg       288
Gly Gln Glu Phe Ile Asp Cys Leu Gly Gly Phe Gly Ile Phe Asn Val
                85                  90                  95

ggg cac cgt aat cca gtt gtg gtt tcc gcc gta cag aat caa ctt gcg       336
Gly His Arg Asn Pro Val Val Val Ser Ala Val Gln Asn Gln Leu Ala
            100                 105                 110

aaa caa ccg ctg cac agc cag gag ctg ctc gat ccg tta cgg gcg atg       384
Lys Gln Pro Leu His Ser Gln Glu Leu Leu Asp Pro Leu Arg Ala Met
        115                 120                 125

ttg gcg aaa acc ctt gct gcg cta acg ccc ggt aaa ctg aaa tac agc     432
Leu Ala Lys Thr Leu Ala Ala Leu Thr Pro Gly Lys Leu Lys Tyr Ser
    130                 135                 140

ttc ttc tgt aat agc ggc acc gag tcc gtt gaa gca gcg ctg aag ctg     480
Phe Phe Cys Asn Ser Gly Thr Glu Ser Val Glu Ala Ala Leu Lys Leu
145                 150                 155                 160

gcg aaa gct tac cag tca ccg cgc ggc aag ttt act ttt att gcc acc     528
Ala Lys Ala Tyr Gln Ser Pro Arg Gly Lys Phe Thr Phe Ile Ala Thr
                165                 170                 175

agc ggc gcg ttc cac ggt aaa tca ctt ggc gcg ctg tcg gcc acg gcg     576
Ser Gly Ala Phe His Gly Lys Ser Leu Gly Ala Leu Ser Ala Thr Ala
            180                 185                 190

aaa tcg acc ttc cgc aaa ccg ttt atg ccg tta ctg ccg ggc ttc cgt     624
Lys Ser Thr Phe Arg Lys Pro Phe Met Pro Leu Leu Pro Gly Phe Arg
        195                 200                 205

cat gtg ccg ttt ggc aat atc gaa gcc atg cgc acg gct ctt aac gag     672
His Val Pro Phe Gly Asn Ile Glu Ala Met Arg Thr Ala Leu Asn Glu
    210                 215                 220

tgc aaa aaa acc ggt gat gat gtg gct gcg gtg atc ctc gaa ccg att     720
Cys Lys Lys Thr Gly Asp Asp Val Ala Ala Val Ile Leu Glu Pro Ile
225                 230                 235                 240

cag ggt gaa ggt ggc gta att ctg ccg ccg ccg ggc tat ctc acc gcc     768
Gln Gly Glu Gly Gly Val Ile Leu Pro Pro Pro Gly Tyr Leu Thr Ala
                245                 250                 255

gta cgt aag cta tgc gat gag ttc ggc gca ctg atg atc ctc gat gaa     816
Val Arg Lys Leu Cys Asp Glu Phe Gly Ala Leu Met Ile Leu Asp Glu
            260                 265                 270

gta caa acg ggc atg ggg cgc acg ggc aag atg ttc gcc tgc gag cat     864
Val Gln Thr Gly Met Gly Arg Thr Gly Lys Met Phe Ala Cys Glu His
        275                 280                 285

gag aac gta cag ccg gat atc ctc tgc ctt gcc aaa gcg ctc ggc ggc     912
Glu Asn Val Gln Pro Asp Ile Leu Cys Leu Ala Lys Ala Leu Gly Gly
    290                 295                 300

ggc gtg atg ccg att ggc gcg acc atc gcc act gaa gag gtg ttt tca     960
Gly Val Met Pro Ile Gly Ala Thr Ile Ala Thr Glu Glu Val Phe Ser
305                 310                 315                 320

gtt ctg ttc gac aac cca ttc ctg cat acc acc acc ttt ggc ggc aac     1008
Val Leu Phe Asp Asn Pro Phe Leu His Thr Thr Thr Phe Gly Gly Asn
                325                 330                 335

ccg ctg gcc tgt gcg gcg gcg ctg gcg acc atc aat gtg ttg ctg gag     1056
Pro Leu Ala Cys Ala Ala Ala Leu Ala Thr Ile Asn Val Leu Leu Glu
            340                 345                 350

cag aac tta ccg gct cag gct gag caa aaa ggc gat atg ttg ctg gac     1104
Gln Asn Leu Pro Ala Gln Ala Glu Gln Lys Gly Asp Met Leu Leu Asp
        355                 360                 365

ggt ttc cgt caa ctg gcg cgg gaa tat ccc gat ctg gta cag gaa gcg     1152
Gly Phe Arg Gln Leu Ala Arg Glu Tyr Pro Asp Leu Val Gln Glu Ala
    370                 375                 380

cgt ggt aaa ggg atg ttg atg gcg att gag ttt gtt gat aac gaa atc 1200
Arg Gly Lys Gly Met Leu Met Ala Ile Glu Phe Val Asp Asn Glu Ile
385                 390                 395                 400

ggc tat aac ttt gcc agc gag atg ttc cgc cag cgc gta ctg gtg gcc 1248
Gly Tyr Asn Phe Ala Ser Glu Met Phe Arg Gln Arg Val Leu Val Ala
                405                 410                 415

gga acg ctc aat aac gcc aaa acg atc cgc att gaa ccg cca ctg aca 1296
Gly Thr Leu Asn Asn Ala Lys Thr Ile Arg Ile Glu Pro Pro Leu Thr
            420                 425                 430

ctg acc att gaa cag tgt gaa ctg gtg atc aaa gcg gcg cgt aag gcg 1344
Leu Thr Ile Glu Gln Cys Glu Leu Val Ile Lys Ala Ala Arg Lys Ala
        435                 440                 445

ctg gcg gcc atg cga gta agt gtc gaa gaa gcg taa 1380
Leu Ala Ala Met Arg Val Ser Val Glu Glu Ala
    450                 455

<210> 57
<211> 459
<212> PRT
<213> Escherichia coli

<400> 57

Met Asn Arg Leu Pro Ser Ser Ala Ser Ala Leu Ala Cys Ser Ala His
1               5                   10                  15

Ala Leu Asn Leu Ile Glu Lys Arg Thr Leu Asp His Glu Glu Met Lys
            20                  25                  30

Ala Leu Asn Arg Glu Val Ile Glu Tyr Phe Lys Glu His Val Asn Pro
        35                  40                  45

Gly Phe Leu Glu Tyr Arg Lys Ser Val Thr Ala Gly Gly Asp Tyr Gly
    50                  55                  60

Ala Val Glu Trp Gln Ala Gly Ser Leu Asn Thr Leu Val Asp Thr Gln
65                  70                  75                  80

Gly Gln Glu Phe Ile Asp Cys Leu Gly Gly Phe Gly Ile Phe Asn Val
                85                  90                  95

Gly His Arg Asn Pro Val Val Val Ser Ala Val Gln Asn Gln Leu Ala
            100                 105                 110

Lys Gln Pro Leu His Ser Gln Glu Leu Leu Asp Pro Leu Arg Ala Met
        115                 120                 125

Leu Ala Lys Thr Leu Ala Ala Leu Thr Pro Gly Lys Leu Lys Tyr Ser
    130                 135                 140

Phe Phe Cys Asn Ser Gly Thr Glu Ser Val Glu Ala Ala Leu Lys Leu

145                 150                 155                 160

Ala Lys Ala Tyr Gln Ser Pro Arg Gly Lys Phe Thr Phe Ile Ala Thr
                165                 170                 175

Ser Gly Ala Phe His Gly Lys Ser Leu Gly Ala Leu Ser Ala Thr Ala
            180                 185                 190

Lys Ser Thr Phe Arg Lys Pro Phe Met Pro Leu Leu Pro Gly Phe Arg
        195                 200                 205

His Val Pro Phe Gly Asn Ile Glu Ala Met Arg Thr Ala Leu Asn Glu
    210                 215                 220

Cys Lys Lys Thr Gly Asp Asp Val Ala Ala Val Ile Leu Glu Pro Ile
225                 230                 235                 240

Gln Gly Glu Gly Gly Val Ile Leu Pro Pro Pro Gly Tyr Leu Thr Ala
                245                 250                 255

Val Arg Lys Leu Cys Asp Glu Phe Gly Ala Leu Met Ile Leu Asp Glu
            260                 265                 270

Val Gln Thr Gly Met Gly Arg Thr Gly Lys Met Phe Ala Cys Glu His
        275                 280                 285

Glu Asn Val Gln Pro Asp Ile Leu Cys Leu Ala Lys Ala Leu Gly Gly
    290                 295                 300

Gly Val Met Pro Ile Gly Ala Thr Ile Ala Thr Glu Glu Val Phe Ser
305                 310                 315                 320

Val Leu Phe Asp Asn Pro Phe Leu His Thr Thr Thr Phe Gly Gly Asn
                325                 330                 335

Pro Leu Ala Cys Ala Ala Ala Leu Ala Thr Ile Asn Val Leu Leu Glu
            340                 345                 350

Gln Asn Leu Pro Ala Gln Ala Glu Gln Lys Gly Asp Met Leu Leu Asp
        355                 360                 365

Gly Phe Arg Gln Leu Ala Arg Glu Tyr Pro Asp Leu Val Gln Glu Ala
    370                 375                 380

Arg Gly Lys Gly Met Leu Met Ala Ile Glu Phe Val Asp Asn Glu Ile
385                 390                 395                 400

Gly Tyr Asn Phe Ala Ser Glu Met Phe Arg Gln Arg Val Leu Val Ala
                405                 410                 415

Gly Thr Leu Asn Asn Ala Lys Thr Ile Arg Ile Glu Pro Pro Leu Thr
            420                 425                 430

Leu Thr Ile Glu Gln Cys Glu Leu Val Ile Lys Ala Ala Arg Lys Ala
        435                 440                 445

Leu Ala Ala Met Arg Val Ser Val Glu Glu Ala
    450                 455

<210>  58
<211>  34
<212>  DNA
<213>  artificial

<220>
<223>  PCR primer

<400>  58
gcgcgcatat gaacaggtta ccttcgagcg catc                                   34

<210>  59
<211>  35
<212>  DNA
<213>  Artificial

<220>
<223>  PCR primer

<400>  59
cgcgcgctcg agttacgctt cttcgacact tactc                                  35

<210>  60
<211>  1323
<212>  DNA
<213>  Achromobacter denitrificans

<220>
<221>  CDS
<222>  (1)..(1323)

<400>  60
atg agc gct gcc aaa ctg ccc gac ctg tcc cac ctc tgg atg ccc ttt        48
Met Ser Ala Ala Lys Leu Pro Asp Leu Ser His Leu Trp Met Pro Phe
1               5                   10                  15

acc gcc aac cgg cag ttc aag gcg aac ccc cgc ctg ctg gcc tcg gcc        96
Thr Ala Asn Arg Gln Phe Lys Ala Asn Pro Arg Leu Leu Ala Ser Ala
            20                  25                  30

aag ggc atg tac tac acg tct ttc gac ggc cgc cag atc ctg gac ggc       144
Lys Gly Met Tyr Tyr Thr Ser Phe Asp Gly Arg Gln Ile Leu Asp Gly
        35                  40                  45

acg gcc ggc ctg tgg tgc gtg aac gcc ggc cac tgc cgc gaa gaa atc       192
Thr Ala Gly Leu Trp Cys Val Asn Ala Gly His Cys Arg Glu Glu Ile
    50                  55                  60

gtc tcc gcc atc gcc agc cag gcc ggc gtc atg gac tac gcg ccg ggg     240
Val Ser Ala Ile Ala Ser Gln Ala Gly Val Met Asp Tyr Ala Pro Gly
65                  70                  75                  80

ttc cag ctc ggc cac ccg ctg gcc ttc gag gcc gcc acc gcc gtg gcc     288
Phe Gln Leu Gly His Pro Leu Ala Phe Glu Ala Ala Thr Ala Val Ala
                85                  90                  95

ggc ctg atg ccg cag ggc ctg gac cgc gtg ttc ttc acc aat tcg ggc     336
Gly Leu Met Pro Gln Gly Leu Asp Arg Val Phe Phe Thr Asn Ser Gly
            100                 105                 110

tcc gaa tcg gtg gac acc gcg ctg aag atc gcc ctg gcc tac cac cgc     384
Ser Glu Ser Val Asp Thr Ala Leu Lys Ile Ala Leu Ala Tyr His Arg
        115                 120                 125

gcg cgc ggc gag gcg cag cgc acc cgc ctc atc ggg cgc gag cgc ggc     432
Ala Arg Gly Glu Ala Gln Arg Thr Arg Leu Ile Gly Arg Glu Arg Gly
    130                 135                 140

tac cac ggc gtg ggc ttc ggc ggc att tcc gtg ggc ggc atc tcg ccc     480
Tyr His Gly Val Gly Phe Gly Gly Ile Ser Val Gly Gly Ile Ser Pro
145                 150                 155                 160

aac cgc aag acc ttc tcc ggc gcg ctg ctg ccg gcc gtg gac cac ctg     528
Asn Arg Lys Thr Phe Ser Gly Ala Leu Leu Pro Ala Val Asp His Leu
                165                 170                 175

ccg cac acc cac agc ctg gaa cac aac gcc ttc acg cgc ggc cag ccc     576
Pro His Thr His Ser Leu Glu His Asn Ala Phe Thr Arg Gly Gln Pro
            180                 185                 190

gag tgg ggc gcg cac ctg gcc gac gag ttg gaa cgc atc atc gcc ctg     624
Glu Trp Gly Ala His Leu Ala Asp Glu Leu Glu Arg Ile Ile Ala Leu
        195                 200                 205

cac gac gcc tcc acc atc gcg gcc gtg atc gtc gag ccc atg gcc ggc     672
His Asp Ala Ser Thr Ile Ala Ala Val Ile Val Glu Pro Met Ala Gly
    210                 215                 220

tcc acc ggc gtg ctc gtc ccg ccc aag ggc tat ctc gaa aaa ctg cgc     720
Ser Thr Gly Val Leu Val Pro Pro Lys Gly Tyr Leu Glu Lys Leu Arg
225                 230                 235                 240

gaa atc acc gcc cgc cac ggc att ctg ctg atc ttc gac gaa gtc atc     768
Glu Ile Thr Ala Arg His Gly Ile Leu Leu Ile Phe Asp Glu Val Ile
                245                 250                 255

acc gcg tac ggc cgc ctg ggc gag gcc acc gcc gcg gcc tat ttc ggc     816
Thr Ala Tyr Gly Arg Leu Gly Glu Ala Thr Ala Ala Ala Tyr Phe Gly
            260                 265                 270

gta acg ccc gac ctc atc acc atg gcc aag ggc gtg agc aac gcc gcc     864
Val Thr Pro Asp Leu Ile Thr Met Ala Lys Gly Val Ser Asn Ala Ala
        275                 280                 285

gtt ccg gcc ggc gcc gtc gcg gtg cgc cgc gaa gtg cat gac gcc atc     912
Val Pro Ala Gly Ala Val Ala Val Arg Arg Glu Val His Asp Ala Ile
    290                 295                 300

gtc aac gga ccg caa ggc ggc atc gag ttc ttc cac ggc tac acc tac     960
Val Asn Gly Pro Gln Gly Gly Ile Glu Phe Phe His Gly Tyr Thr Tyr
305                 310                 315                 320

tcg gcc cac ccg ctg gcc gcc gcc gcc gtg ctc gcc acg ctg gac atc     1008

Ser Ala His Pro Leu Ala Ala Ala Ala Val Leu Ala Thr Leu Asp Ile
                325                 330                 335

tac cgc cgc gaa gac ctg ttc gcc cgc gcc cgc aag ctg tcg gcc gcg      1056
Tyr Arg Arg Glu Asp Leu Phe Ala Arg Ala Arg Lys Leu Ser Ala Ala
            340                 345                 350

ttc gag gaa gcc gcc cac agc ctc aag ggc gcg ccg cac gtc atc gac      1104
Phe Glu Glu Ala Ala His Ser Leu Lys Gly Ala Pro His Val Ile Asp
        355                 360                 365

gtg cgc aac atc ggc ctg gtg gcc ggc atc gag ctg tcg ccg cgc gaa      1152
Val Arg Asn Ile Gly Leu Val Ala Gly Ile Glu Leu Ser Pro Arg Glu
    370                 375                 380

ggc gcc ccg ggc gcg cgc gcc gcc gaa gcc ttc cag aaa tgc ttc gac      1200
Gly Ala Pro Gly Ala Arg Ala Ala Glu Ala Phe Gln Lys Cys Phe Asp
385                 390                 395                 400

acc ggc ctc atg gtg cgc tac acg ggc gac atc ctc gcg gtg tcg cct      1248
Thr Gly Leu Met Val Arg Tyr Thr Gly Asp Ile Leu Ala Val Ser Pro
                405                 410                 415

ccg ctc atc gtc gac gaa aac cag atc ggc cag atc ttc gag ggc atc      1296
Pro Leu Ile Val Asp Glu Asn Gln Ile Gly Gln Ile Phe Glu Gly Ile
            420                 425                 430

ggc aag gtg ctc aag gaa gtg gct tag                                  1323
Gly Lys Val Leu Lys Glu Val Ala
        435                 440


<210>  61
<211>  440
<212>  PRT
<213>  Achromobacter denitrificans

<400>  61

Met Ser Ala Ala Lys Leu Pro Asp Leu Ser His Leu Trp Met Pro Phe
1               5                   10                  15


Thr Ala Asn Arg Gln Phe Lys Ala Asn Pro Arg Leu Leu Ala Ser Ala
            20                  25                  30


Lys Gly Met Tyr Tyr Thr Ser Phe Asp Gly Arg Gln Ile Leu Asp Gly
        35                  40                  45


Thr Ala Gly Leu Trp Cys Val Asn Ala Gly His Cys Arg Glu Glu Ile
    50                  55                  60


Val Ser Ala Ile Ala Ser Gln Ala Gly Val Met Asp Tyr Ala Pro Gly
65                  70                  75                  80


Phe Gln Leu Gly His Pro Leu Ala Phe Glu Ala Ala Thr Ala Val Ala
                85                  90                  95


Gly Leu Met Pro Gln Gly Leu Asp Arg Val Phe Phe Thr Asn Ser Gly
            100                 105                 110

Ser Glu Ser Val Asp Thr Ala Leu Lys Ile Ala Leu Ala Tyr His Arg
        115                 120                 125

Ala Arg Gly Glu Ala Gln Arg Thr Arg Leu Ile Gly Arg Glu Arg Gly
    130                 135                 140

Tyr His Gly Val Gly Phe Gly Gly Ile Ser Val Gly Gly Ile Ser Pro
145                 150                 155                 160

Asn Arg Lys Thr Phe Ser Gly Ala Leu Leu Pro Ala Val Asp His Leu
                165                 170                 175

Pro His Thr His Ser Leu Glu His Asn Ala Phe Thr Arg Gly Gln Pro
            180                 185                 190

Glu Trp Gly Ala His Leu Ala Asp Glu Leu Glu Arg Ile Ile Ala Leu
        195                 200                 205

His Asp Ala Ser Thr Ile Ala Ala Val Ile Val Glu Pro Met Ala Gly
    210                 215                 220

Ser Thr Gly Val Leu Val Pro Pro Lys Gly Tyr Leu Glu Lys Leu Arg
225                 230                 235                 240

Glu Ile Thr Ala Arg His Gly Ile Leu Leu Ile Phe Asp Glu Val Ile
                245                 250                 255

Thr Ala Tyr Gly Arg Leu Gly Glu Ala Thr Ala Ala Ala Tyr Phe Gly
            260                 265                 270

Val Thr Pro Asp Leu Ile Thr Met Ala Lys Gly Val Ser Asn Ala Ala
        275                 280                 285

Val Pro Ala Gly Ala Val Ala Val Arg Arg Glu Val His Asp Ala Ile
    290                 295                 300

Val Asn Gly Pro Gln Gly Gly Ile Glu Phe Phe His Gly Tyr Thr Tyr
305                 310                 315                 320

Ser Ala His Pro Leu Ala Ala Ala Ala Val Leu Ala Thr Leu Asp Ile
                325                 330                 335

Tyr Arg Arg Glu Asp Leu Phe Ala Arg Ala Arg Lys Leu Ser Ala Ala
            340                 345                 350

Phe Glu Glu Ala Ala His Ser Leu Lys Gly Ala Pro His Val Ile Asp
        355                 360                 365

Val Arg Asn Ile Gly Leu Val Ala Gly Ile Glu Leu Ser Pro Arg Glu
    370                 375                 380

Gly Ala Pro Gly Ala Arg Ala Ala Glu Ala Phe Gln Lys Cys Phe Asp
385                 390                 395                 400

Thr Gly Leu Met Val Arg Tyr Thr Gly Asp Ile Leu Ala Val Ser Pro
                405                 410                 415

Pro Leu Ile Val Asp Glu Asn Gln Ile Gly Gln Ile Phe Glu Gly Ile
            420                 425                 430

Gly Lys Val Leu Lys Glu Val Ala
        435                 440

<210>  62
<211>  439
<212>  PRT
<213>  Homo sapiens

<400>  62

<210>  63
<211>  301
<212>  PRT
<213>  Homo sapiens

<400>  63

Met Asn Thr Gly Val Glu Ala Gly Glu Thr Ala Cys Lys Leu Ala Arg
1               5                   10                  15

Lys Trp Gly Tyr Thr Val Lys Gly Ile Gln Lys Tyr Lys Ala Lys Ile
            20                  25                  30

Val Phe Ala Ala Gly Asn Phe Trp Gly Arg Thr Leu Ser Ala Ile Ser
        35                  40                  45

Ser Ser Thr Asp Pro Thr Ser Tyr Asp Gly Phe Gly Pro Phe Met Pro
    50                  55                  60

Gly Phe Asp Ile Ile Pro Tyr Asn Asp Leu Pro Ala Leu Glu Arg Ala
65                  70                  75                  80

Leu Gln Asp Pro Asn Val Ala Ala Phe Met Val Glu Pro Ile Gln Gly
                85                  90                  95

Glu Ala Gly Val Val Val Pro Asp Pro Gly Tyr Leu Met Gly Val Arg
            100                 105                 110

Glu Leu Cys Thr Arg His Gln Val Leu Phe Ile Ala Asp Glu Ile Gln
        115                 120                 125

Thr Gly Leu Ala Arg Thr Gly Arg Trp Leu Ala Val Asp Tyr Glu Asn
    130                 135                 140

Val Arg Pro Asp Ile Val Leu Leu Gly Lys Ala Leu Ser Gly Gly Leu
145                 150                 155                 160

Tyr Pro Val Ser Ala Val Leu Cys Asp Asp Asp Ile Met Leu Thr Ile
                165                 170                 175

Lys Pro Gly Glu His Gly Ser Thr Tyr Gly Gly Asn Pro Leu Gly Cys
            180                 185                 190

Arg Val Ala Ile Ala Ala Leu Glu Val Leu Glu Glu Glu Asn Leu Ala
        195                 200                 205

Glu Asn Ala Asp Lys Leu Gly Ile Ile Leu Arg Asn Glu Leu Met Lys
    210                 215                 220

Leu Pro Ser Asp Val Val Thr Ala Val Arg Gly Lys Gly Leu Leu Asn
225                 230                 235                 240

Ala Ile Val Ile Lys Glu Thr Lys Asp Trp Asp Ala Trp Lys Val Cys
                245                 250                 255

Leu Arg Leu Arg Asp Asn Gly Leu Leu Ala Lys Pro Thr His Gly Asp
            260                 265                 270

Ile Ile Arg Phe Ala Pro Pro Leu Val Ile Lys Glu Asp Glu Leu Arg
        275                 280                 285

Glu Ser Ile Glu Ile Ile Asn Lys Thr Ile Leu Ser Phe
    290                 295                 300

<210>  64
<211>  414
<212>  PRT
<213>  Plasmodium yoelii

<400>  64

Met Glu Phe Val Lys Asp Leu Lys Thr Pro Glu Asp Tyr Ile Asn Asn
1               5                   10                  15

Glu Leu Lys Tyr Gly Ala His Asn Tyr Asp Pro Ile Pro Val Val Leu
            20                  25                  30

Lys Arg Ala Lys Gly Val Phe Val Tyr Asp Val Asn Asp Lys Arg Tyr
        35                  40                  45

Tyr Asp Phe Leu Ser Ala Tyr Ser Ser Val Asn Gln Gly His Cys His
    50                  55                  60

Pro Asn Ile Leu Asn Ala Met Ile Asn Gln Ala Lys Asn Leu Thr Ile
65                  70                  75                  80

Cys Ser Arg Ala Phe Phe Ser Val Pro Leu Gly Ile Cys Glu Arg Tyr
                85                  90                  95

Leu Thr Asn Leu Leu Gly Tyr Asp Lys Val Leu Met Met Asn Thr Gly
            100                 105                 110

Ala Glu Ala Asn Glu Thr Ala Tyr Lys Leu Cys Arg Lys Trp Gly Tyr
        115                 120                 125

Glu Val Lys Lys Ile Pro Glu Asn Met Ala Lys Ile Val Val Cys Lys
    130                 135                 140

Asn Asn Phe Ser Gly Arg Thr Leu Gly Cys Ile Ser Ala Ser Thr Thr
145                 150                 155                 160

Lys Lys Cys Thr Ser Asn Phe Gly Pro Phe Ala Pro Gln Phe Ser Lys
                165                 170                 175

Val Pro Tyr Asp Asp Leu Glu Ala Leu Glu Glu Glu Leu Lys Asp Pro
            180                 185                 190

Asn Val Cys Ala Phe Ile Val Glu Pro Ile Gln Gly Glu Ala Gly Val
        195                 200                 205

Ile Val Pro Ser Asp Asn Tyr Leu Gln Gly Val Tyr Asp Ile Cys Lys
    210                 215                 220

Lys Tyr Asn Val Leu Phe Val Ala Asp Glu Val Gln Thr Gly Leu Gly
225                 230                 235                 240

Arg Thr Gly Lys Leu Leu Cys Val His His Tyr Asn Val Lys Pro Asp
                245                 250                 255

Val Ile Leu Leu Gly Lys Ala Leu Ser Gly Gly His Tyr Pro Ile Ser
            260                 265                 270

Ala Val Leu Ala Asn Asp Asp Ile Met Leu Val Ile Lys Pro Gly Glu
        275                 280                 285

His Gly Ser Thr Tyr Gly Gly Asn Pro Leu Ala Ala Ser Ile Cys Val
    290                 295                 300

Glu Ala Leu Asn Val Leu Ile Asn Glu Lys Leu Cys Glu Asn Ala Glu
305                 310                 315                 320

Lys Leu Gly Gly Pro Phe Leu Glu Asn Leu Lys Arg Glu Leu Lys Asp
                325                 330                 335

Ser Lys Ile Val Arg Asp Val Arg Gly Lys Gly Leu Leu Cys Ala Ile
            340                 345                 350

Glu Phe Lys Asn Glu Leu Val Asn Val Leu Asp Ile Cys Leu Lys Leu
        355                 360                 365

Lys Glu Asn Gly Leu Ile Thr Arg Asp Val His Asp Lys Thr Ile Arg
    370                 375                 380

Leu Thr Pro Pro Leu Cys Ile Thr Lys Glu Gln Leu Asp Glu Cys Thr
385                 390                 395                 400

Glu Ile Ile Val Lys Thr Val Lys Phe Phe Asp Glu Arg Phe
                405                 410

<210>  65
<211>  414
<212>  PRT
<213>  Plasmodium falciparum

<400>  65

Met Asp Phe Val Lys Glu Leu Lys Ser Ser Gln Asp Tyr Met Asn Asn
1               5                   10                  15

Glu Leu Thr Tyr Gly Ala His Asn Tyr Asp Pro Ile Pro Val Val Leu
            20                  25                  30

Lys Arg Gly Lys Gly Val Phe Val Tyr Asp Ile Glu Asp Arg Arg Tyr
        35                  40                  45

Tyr Asp Phe Leu Ser Ala Tyr Ser Ser Val Asn Gln Gly His Cys His
    50                  55                  60

Pro Asp Ile Leu Asn Ala Met Ile Asn Gln Ala Lys Lys Leu Thr Ile
65                  70                  75                  80

Cys Ser Arg Ala Phe Phe Ser Asp Ser Leu Gly Val Cys Glu Arg Tyr
                85                  90                  95

Leu Thr Asn Leu Phe Gly Tyr Asp Lys Val Leu Met Met Asn Thr Gly
            100                 105                 110

Ala Glu Ala Ser Glu Thr Ala Tyr Lys Leu Cys Arg Lys Trp Gly Tyr
        115                 120                 125

Glu Val Lys Lys Ile Pro Glu Asn Ser Ala Lys Ile Ile Val Cys Asn
    130                 135                 140

Asn Asn Phe Ser Gly Arg Thr Leu Gly Cys Val Ser Ala Ser Thr Asp
145                 150                 155                 160

Lys Lys Cys Lys Asn Asn Phe Gly Pro Phe Val Pro Asn Phe Leu Lys
                165                 170                 175

Val Pro Tyr Asp Asp Leu Glu Ala Leu Glu Lys Glu Leu Gln Asp Pro
            180                 185                 190

Asn Val Cys Ala Phe Ile Val Glu Pro Val Gln Gly Glu Ala Gly Val

195                 200                 205

Ile Val Pro Ser Asp Ser Tyr Phe Pro Gly Val Ala Ser Leu Cys Lys
    210                 215                 220

Lys Tyr Asn Val Leu Phe Val Ala Asp Glu Val Gln Thr Gly Leu Gly
225                 230                 235                 240

Arg Thr Gly Lys Leu Leu Cys Thr His His Tyr Gly Val Lys Pro Asp
                245                 250                 255

Val Ile Leu Leu Gly Lys Ala Leu Ser Gly Gly His Tyr Pro Ile Ser
            260                 265                 270

Ala Ile Leu Ala Asn Asp Asp Val Met Leu Val Leu Lys Pro Gly Glu
        275                 280                 285

His Gly Ser Thr Tyr Gly Gly Asn Pro Leu Ala Ala Ala Ile Cys Val
    290                 295                 300

Glu Ala Leu Lys Val Leu Ile Asn Glu Lys Leu Cys Glu Asn Ala Asp
305                 310                 315                 320

Lys Leu Gly Ala Pro Phe Leu Gln Asn Leu Lys Glu Gln Leu Lys Asp
                325                 330                 335

Ser Lys Val Val Arg Glu Val Arg Gly Lys Gly Leu Leu Cys Ala Ile
            340                 345                 350

Glu Phe Lys Asn Asp Leu Val Asn Val Trp Asp Ile Cys Leu Lys Phe
        355                 360                 365

Lys Glu Asn Gly Leu Ile Thr Arg Ser Val His Asp Lys Thr Val Arg
    370                 375                 380

Leu Thr Pro Pro Leu Cys Ile Thr Lys Glu Gln Leu Asp Glu Cys Thr
385                 390                 395                 400

Glu Ile Ile Val Lys Thr Val Lys Phe Phe Asp Asp Asn Leu
                405                 410

<210>  66
<211>  396
<212>  PRT
<213>  Bacillus anthracis

<400>  66

Met Ile Gln Thr Lys Asp Ile Ile Glu Leu Thr Asp Thr Tyr Gly Ala
1               5                   10                  15

Asn Asn Tyr His Pro Leu Pro Ile Val Ile Ser Lys Ala Glu Gly Val
                20                  25                  30

Trp Val Glu Asp Pro Glu Gly Asn Arg Tyr Met Asp Leu Leu Ser Ala
        35                  40                  45

Tyr Ser Ala Val Asn Gln Gly His Arg His Pro Lys Ile Ile Asn Ala
    50                  55                  60

Leu Ile Asp Gln Ala Asn Arg Val Thr Leu Thr Ser Arg Ala Phe His
65                  70                  75                  80

Ser Asp Gln Leu Gly Pro Trp Tyr Glu Lys Val Ala Lys Leu Thr Asn
                85                  90                  95

Lys Glu Met Val Leu Pro Met Asn Thr Gly Ala Glu Ala Val Glu Thr
            100                 105                 110

Ala Ile Lys Thr Ala Arg Arg Trp Ala Tyr Asp Val Lys Lys Val Glu
        115                 120                 125

Ala Asn Arg Ala Glu Ile Ile Val Cys Glu Asp Asn Phe His Gly Arg
    130                 135                 140

Thr Met Gly Ala Val Ser Met Ser Ser Asn Glu Glu Tyr Lys Arg Gly
145                 150                 155                 160

Phe Gly Pro Met Leu Pro Gly Ile Ile Val Ile Pro Tyr Gly Asp Leu
                165                 170                 175

Glu Ala Leu Lys Ala Ala Ile Thr Pro Asn Thr Ala Ala Phe Ile Leu
            180                 185                 190

Glu Pro Ile Gln Gly Glu Ala Gly Ile Asn Ile Pro Pro Ala Gly Phe
        195                 200                 205

Leu Lys Glu Ala Leu Glu Val Cys Lys Lys Glu Asn Val Leu Phe Val
    210                 215                 220

Ala Asp Glu Ile Gln Thr Gly Leu Gly Arg Thr Gly Lys Val Phe Ala
225                 230                 235                 240

Cys Asp Trp Asp Asn Val Thr Pro Asp Met Tyr Ile Leu Gly Lys Ala
                245                 250                 255

Leu Gly Gly Gly Val Phe Pro Ile Ser Cys Ala Ala Ala Asn Arg Asp
            260                 265                 270

Ile Leu Gly Val Phe Glu Pro Gly Ser His Gly Ser Thr Phe Gly Gly
        275                 280                 285

Asn Pro Leu Ala Cys Ala Val Ser Ile Ala Ala Leu Glu Val Leu Glu
    290                 295                 300

Glu Glu Lys Leu Thr Glu Arg Ser Leu Gln Leu Gly Glu Lys Leu Val
305                 310                 315                 320

Gly Gln Leu Lys Glu Ile Asp Asn Pro Met Ile Thr Glu Val Arg Gly
                325                 330                 335

Lys Gly Leu Phe Ile Gly Ile Glu Leu Asn Glu Pro Ala Arg Pro Tyr
            340                 345                 350

Cys Glu Gln Leu Lys Ala Ala Gly Leu Leu Cys Lys Glu Thr His Glu
        355                 360                 365

Asn Val Ile Arg Ile Ala Pro Pro Leu Val Ile Ser Glu Glu Asp Leu
    370                 375                 380

Glu Trp Ala Phe Gln Lys Ile Lys Ala Val Leu Ser
385                 390                 395

<210>  67
<211>  1374
<212>  DNA
<213>  Streptomyces clavuligerus

<220>
<221>  CDS
<222>  (1)..(1374)

<400>  67
atg ggc gaa gca gca cgc cac ccc gac ggc gat ttc tcg gac gtg gga      48
Met Gly Glu Ala Ala Arg His Pro Asp Gly Asp Phe Ser Asp Val Gly
1               5                   10                  15

aac ctc cac gct cag gac gtg cac cag gca ctt gag cag cat atg ctc      96
Asn Leu His Ala Gln Asp Val His Gln Ala Leu Glu Gln His Met Leu
            20                  25                  30

gtc gac ggg tac gac ctc gtt ctc gac ctc gac gcc agc tcc ggc gtc      144
Val Asp Gly Tyr Asp Leu Val Leu Asp Leu Asp Ala Ser Ser Gly Val
        35                  40                  45

tgg ctc gtc gac gcc gtc acc cag aag cgg tat ctc gac ctc ttt tcc      192
Trp Leu Val Asp Ala Val Thr Gln Lys Arg Tyr Leu Asp Leu Phe Ser
    50                  55                  60

ttc ttt gcc tcg gcg ccg ctc gga atc aac ccg ccc agc att gtc gag      240
Phe Phe Ala Ser Ala Pro Leu Gly Ile Asn Pro Pro Ser Ile Val Glu
65                  70                  75                  80

gac ccg gca ttc atg cgg gag ctg gcc gtg gcc gcg gtc aac aag ccg      288
Asp Pro Ala Phe Met Arg Glu Leu Ala Val Ala Ala Val Asn Lys Pro

85                  90                  95

tcg aac ccc gat ctt tat tcg gtg ccg tac gcc cgt ttc gtc aag acc     336
Ser Asn Pro Asp Leu Tyr Ser Val Pro Tyr Ala Arg Phe Val Lys Thr
            100                 105                 110

ttc gcc cgg gtc ctc ggc gac ccc cgg ctg ccg cgg ctg ttc ttc gtg     384
Phe Ala Arg Val Leu Gly Asp Pro Arg Leu Pro Arg Leu Phe Phe Val
        115                 120                 125

gac ggc ggg gcg ctg gcc gtg gag aac gcg ctc aag gcg gcc ctc gac     432
Asp Gly Gly Ala Leu Ala Val Glu Asn Ala Leu Lys Ala Ala Leu Asp
    130                 135                 140

tgg aag gcc cag aag ctg ggc ctc gcc gag ccg gac acc gac cgg ctc     480
Trp Lys Ala Gln Lys Leu Gly Leu Ala Glu Pro Asp Thr Asp Arg Leu
145                 150                 155                 160

cag gtg ctg cat ctg gag cgc tcg ttc cac ggc cgc agc ggc tac acc     528
Gln Val Leu His Leu Glu Arg Ser Phe His Gly Arg Ser Gly Tyr Thr
                165                 170                 175

atg tcg ctg acg aac acc gag ccg tcc aag acc gcc cgc ttc ccc aag     576
Met Ser Leu Thr Asn Thr Glu Pro Ser Lys Thr Ala Arg Phe Pro Lys
            180                 185                 190

ttc ggc tgg cca cgg atc tcg tcc ccc gcc ctc cag cac ccg ccg gcc     624
Phe Gly Trp Pro Arg Ile Ser Ser Pro Ala Leu Gln His Pro Pro Ala
        195                 200                 205

gag cac acc ggc gcc aac cag gag gcc gag cga cgg gcg ctg gag gcc     672
Glu His Thr Gly Ala Asn Gln Glu Ala Glu Arg Arg Ala Leu Glu Ala
    210                 215                 220

gcc cgg gag gcg ttc gca gcg gcg gac ggc atg atc gcc tgc ttc atc     720
Ala Arg Glu Ala Phe Ala Ala Ala Asp Gly Met Ile Ala Cys Phe Ile
225                 230                 235                 240

gcg gag ccc atc cag ggc gag ggc ggc gac aac cac ctc agc gcg gag     768
Ala Glu Pro Ile Gln Gly Glu Gly Gly Asp Asn His Leu Ser Ala Glu
                245                 250                 255

ttc ctc cag gcc atg cag cgg ctc tgc cac gag aac gac gcc ctg ttc     816
Phe Leu Gln Ala Met Gln Arg Leu Cys His Glu Asn Asp Ala Leu Phe
            260                 265                 270

gtc ctg gac gag gtg cag agc ggc tgc ggc atc acc ggt acc gcc tgg     864
Val Leu Asp Glu Val Gln Ser Gly Cys Gly Ile Thr Gly Thr Ala Trp
        275                 280                 285

gcc tac cag cag ctc ggc ctc cag ccc gac ctg gtg gcc ttc ggc aag     912
Ala Tyr Gln Gln Leu Gly Leu Gln Pro Asp Leu Val Ala Phe Gly Lys
    290                 295                 300

aag acc cag gtc tgc ggg gtg atg ggc ggc ggc cgg atc gac gag gtc     960
Lys Thr Gln Val Cys Gly Val Met Gly Gly Gly Arg Ile Asp Glu Val
305                 310                 315                 320

ccc gag aac gtc ttc gcc gtc tcc tcc cgg atc agc tcc acc tgg ggc     1008
Pro Glu Asn Val Phe Ala Val Ser Ser Arg Ile Ser Ser Thr Trp Gly
                325                 330                 335

ggc aac ctc gcc gac atg gtc cgc gcc acc cgg ctg ctg gag acg atc     1056
Gly Asn Leu Ala Asp Met Val Arg Ala Thr Arg Leu Leu Glu Thr Ile
            340                 345                 350

gag cgc acc cag gtc ttc gac acc gtc gtc cag cgc ggc aag tac ttc 1104
Glu Arg Thr Gln Val Phe Asp Thr Val Val Gln Arg Gly Lys Tyr Phe
        355                 360                 365

cgg gac ggc ctg gag gac ctg gcc gcc cgc cac ccc tcc gtc gtg acc 1152
Arg Asp Gly Leu Glu Asp Leu Ala Ala Arg His Pro Ser Val Val Thr
    370                 375                 380

aac gcc cgc ggc cgg ggc ctg atg tgc gcg gtc gac ctg ccg gac acc 1200
Asn Ala Arg Gly Arg Gly Leu Met Cys Ala Val Asp Leu Pro Asp Thr
385                 390                 395                 400

cgg acc cgc aat gag gtg ctg cgg ctc atg tac acg gag cac cag gtc 1248
Arg Thr Arg Asn Glu Val Leu Arg Leu Met Tyr Thr Glu His Gln Val
                405                 410                 415

atc gcc ctg ccc tgc ggc ggg cgc agc ctc cgg ttc cgc ccc gcg ctg 1296
Ile Ala Leu Pro Cys Gly Gly Arg Ser Leu Arg Phe Arg Pro Ala Leu
            420                 425                 430

acg atc gcc gag cac gag atc gac cag gcc ctt cag gcg ctg gcg agc 1344
Thr Ile Ala Glu His Glu Ile Asp Gln Ala Leu Gln Ala Leu Ala Ser
        435                 440                 445

agt gtc acg gcg gtc gcc gag agc gtc tga 1374
Ser Val Thr Ala Val Ala Glu Ser Val
    450                 455

<210> 68
<211> 457
<212> PRT
<213> Streptomyces clavuligerus

<400> 68

Met Gly Glu Ala Ala Arg His Pro Asp Gly Asp Phe Ser Asp Val Gly
1               5                   10                  15

Asn Leu His Ala Gln Asp Val His Gln Ala Leu Glu Gln His Met Leu
            20                  25                  30

Val Asp Gly Tyr Asp Leu Val Leu Asp Leu Asp Ala Ser Ser Gly Val
        35                  40                  45

Trp Leu Val Asp Ala Val Thr Gln Lys Arg Tyr Leu Asp Leu Phe Ser
    50                  55                  60

Phe Phe Ala Ser Ala Pro Leu Gly Ile Asn Pro Pro Ser Ile Val Glu
65                  70                  75                  80

Asp Pro Ala Phe Met Arg Glu Leu Ala Val Ala Ala Val Asn Lys Pro
                85                  90                  95

Ser Asn Pro Asp Leu Tyr Ser Val Pro Tyr Ala Arg Phe Val Lys Thr
            100                 105                 110

Phe Ala Arg Val Leu Gly Asp Pro Arg Leu Pro Arg Leu Phe Phe Val
        115                 120                 125

Asp Gly Gly Ala Leu Ala Val Glu Asn Ala Leu Lys Ala Ala Leu Asp
    130                 135                 140

Trp Lys Ala Gln Lys Leu Gly Leu Ala Glu Pro Asp Thr Asp Arg Leu
145                 150                 155                 160

Gln Val Leu His Leu Glu Arg Ser Phe His Gly Arg Ser Gly Tyr Thr
                165                 170                 175

Met Ser Leu Thr Asn Thr Glu Pro Ser Lys Thr Ala Arg Phe Pro Lys
            180                 185                 190

Phe Gly Trp Pro Arg Ile Ser Ser Pro Ala Leu Gln His Pro Pro Ala
        195                 200                 205

Glu His Thr Gly Ala Asn Gln Glu Ala Glu Arg Arg Ala Leu Glu Ala
    210                 215                 220

Ala Arg Glu Ala Phe Ala Ala Ala Asp Gly Met Ile Ala Cys Phe Ile
225                 230                 235                 240

Ala Glu Pro Ile Gln Gly Glu Gly Gly Asp Asn His Leu Ser Ala Glu
                245                 250                 255

Phe Leu Gln Ala Met Gln Arg Leu Cys His Glu Asn Asp Ala Leu Phe
            260                 265                 270

Val Leu Asp Glu Val Gln Ser Gly Cys Gly Ile Thr Gly Thr Ala Trp
        275                 280                 285

Ala Tyr Gln Gln Leu Gly Leu Gln Pro Asp Leu Val Ala Phe Gly Lys
    290                 295                 300

Lys Thr Gln Val Cys Gly Val Met Gly Gly Gly Arg Ile Asp Glu Val
305                 310                 315                 320

Pro Glu Asn Val Phe Ala Val Ser Ser Arg Ile Ser Ser Thr Trp Gly
                325                 330                 335

Gly Asn Leu Ala Asp Met Val Arg Ala Thr Arg Leu Leu Glu Thr Ile
            340                 345                 350

Glu Arg Thr Gln Val Phe Asp Thr Val Val Gln Arg Gly Lys Tyr Phe
        355                 360                 365

Arg Asp Gly Leu Glu Asp Leu Ala Ala Arg His Pro Ser Val Val Thr

370                 375                 380

Asn Ala Arg Gly Arg Gly Leu Met Cys Ala Val Asp Leu Pro Asp Thr
385                 390                 395                 400

Arg Thr Arg Asn Glu Val Leu Arg Leu Met Tyr Thr Glu His Gln Val
                405                 410                 415

Ile Ala Leu Pro Cys Gly Gly Arg Ser Leu Arg Phe Arg Pro Ala Leu
            420                 425                 430

Thr Ile Ala Glu His Glu Ile Asp Gln Ala Leu Gln Ala Leu Ala Ser
        435                 440                 445

Ser Val Thr Ala Val Ala Glu Ser Val
    450                 455

<210>  69
<211>  1350
<212>  DNA
<213>  Mycobacterium tuberculosis

<220>
<221>  CDS
<222>  (1)..(1350)

<400>  69
atg gcc gcc gtc gtg aag tcc gtc gct ctt gcc ggt aga cca aca aca      48
Met Ala Ala Val Val Lys Ser Val Ala Leu Ala Gly Arg Pro Thr Thr
1               5                   10                  15

cca gac cgg gtt cat gag gtg cta ggg cgc agc atg ctg gtc gac ggt      96
Pro Asp Arg Val His Glu Val Leu Gly Arg Ser Met Leu Val Asp Gly
            20                  25                  30

ctg gac ata gtg ctc gat ctg acc cgt tcg ggc ggt tca tat ctg gtc      144
Leu Asp Ile Val Leu Asp Leu Thr Arg Ser Gly Gly Ser Tyr Leu Val
        35                  40                  45

gac gct ata acg ggt cgg cgc tac ctg gac atg ttc aca ttc gtt gcc      192
Asp Ala Ile Thr Gly Arg Arg Tyr Leu Asp Met Phe Thr Phe Val Ala
    50                  55                  60

tcc tcg gca ctg ggt atg aat ccc ccg gcg ctg gtg gac gac cgg gag      240
Ser Ser Ala Leu Gly Met Asn Pro Pro Ala Leu Val Asp Asp Arg Glu
65                  70                  75                  80

ttc cat gcc gaa ctc atg cag gcc gcg ctg aac aag ccc agc aat tcc      288
Phe His Ala Glu Leu Met Gln Ala Ala Leu Asn Lys Pro Ser Asn Ser
                85                  90                  95

gac gtg tac tcg gtg gcg atg gcc cgc ttc gtc gag acc ttc gcc cgt      336
Asp Val Tyr Ser Val Ala Met Ala Arg Phe Val Glu Thr Phe Ala Arg
            100                 105                 110

gtc ttg ggc gac ccg gcg ctg ccg cat ctg ttc ttc gtc gaa ggg ggc      384
Val Leu Gly Asp Pro Ala Leu Pro His Leu Phe Phe Val Glu Gly Gly
        115                 120                 125

gcc ctg gcg gtg gag aac gcg ctc aaa gcc gcg ttc gac tgg aag agt     432
Ala Leu Ala Val Glu Asn Ala Leu Lys Ala Ala Phe Asp Trp Lys Ser
    130                 135                 140

cgg cac aac caa gcc cat ggg atc gac ccg gcg ctg ggc act caa gtg     480
Arg His Asn Gln Ala His Gly Ile Asp Pro Ala Leu Gly Thr Gln Val
145                 150                 155                 160

ctc cac ctg cgc ggg gca ttt cac ggc cgc agc ggc tat acc ctg tcg     528
Leu His Leu Arg Gly Ala Phe His Gly Arg Ser Gly Tyr Thr Leu Ser
                165                 170                 175

ctg acc aac acc aag ccg acc att acc gcc cgg ttc ccg aaa ttc gac     576
Leu Thr Asn Thr Lys Pro Thr Ile Thr Ala Arg Phe Pro Lys Phe Asp
            180                 185                 190

tgg cca cgc atc gat gcg ccg tac atg cgg ccc ggc ctg gat gag ccc     624
Trp Pro Arg Ile Asp Ala Pro Tyr Met Arg Pro Gly Leu Asp Glu Pro
        195                 200                 205

gct atg gcc gcg cta gag gcc gag gcg cta cgc cag gcc cgc gcg gca     672
Ala Met Ala Ala Leu Glu Ala Glu Ala Leu Arg Gln Ala Arg Ala Ala
    210                 215                 220

ttc gag acc cgc ccg cac gac atc gcg tgt ttt gtg gcc gaa ccc atc     720
Phe Glu Thr Arg Pro His Asp Ile Ala Cys Phe Val Ala Glu Pro Ile
225                 230                 235                 240

cag ggc gag ggt ggc gac cgc cat ttc cgc ccg gag ttt ttc gct gcg     768
Gln Gly Glu Gly Gly Asp Arg His Phe Arg Pro Glu Phe Phe Ala Ala
                245                 250                 255

atg cgc gag ctg tgc gac gag ttt gat gca ctg ctg atc ttc gac gaa     816
Met Arg Glu Leu Cys Asp Glu Phe Asp Ala Leu Leu Ile Phe Asp Glu
            260                 265                 270

gta cag acc ggc tgc ggg ttg acc gga acc gcc tgg gca tac cag cag     864
Val Gln Thr Gly Cys Gly Leu Thr Gly Thr Ala Trp Ala Tyr Gln Gln
        275                 280                 285

ttg gat gtc gca ccc gac atc gtg gcg ttc ggc aag aag acg cag gta     912
Leu Asp Val Ala Pro Asp Ile Val Ala Phe Gly Lys Lys Thr Gln Val
    290                 295                 300

tgc gga gtg atg gcc ggt cgg cgg gtg gac gag gtc gcc gac aat gtg     960
Cys Gly Val Met Ala Gly Arg Arg Val Asp Glu Val Ala Asp Asn Val
305                 310                 315                 320

ttc gcg gtc cca tca cgg ctc aac tcg aca tgg ggt ggc aat ctt acc    1008
Phe Ala Val Pro Ser Arg Leu Asn Ser Thr Trp Gly Gly Asn Leu Thr
                325                 330                 335

gac atg gtg cgc gcc cgc cgc atc ttg gag gtc atc gaa gcc gag ggc    1056
Asp Met Val Arg Ala Arg Arg Ile Leu Glu Val Ile Glu Ala Glu Gly
            340                 345                 350

ctg ttc gag cgg gcg gtg cag cac ggt aag tat ctg cgc gcc cgg ctt    1104
Leu Phe Glu Arg Ala Val Gln His Gly Lys Tyr Leu Arg Ala Arg Leu
        355                 360                 365

gac gaa ctc gcc gcg gac ttc ccg gca gtg gtt ctc gat ccg cgc ggc    1152
Asp Glu Leu Ala Ala Asp Phe Pro Ala Val Val Leu Asp Pro Arg Gly
    370                 375                 380

cgc ggg ctg atg tgt gcg ttt agc ctg ccg acc acc gcc gac cgt gac      1200
Arg Gly Leu Met Cys Ala Phe Ser Leu Pro Thr Thr Ala Asp Arg Asp
385                 390                 395                 400

gag ttg atc cgc cag ctg tgg caa cgt gcg gtg att gtg ttg ccg gcc      1248
Glu Leu Ile Arg Gln Leu Trp Gln Arg Ala Val Ile Val Leu Pro Ala
                405                 410                 415

ggt gca gac acc gtg cga ttc cgt cca ccg ctg acg gtt tca acc gcc      1296
Gly Ala Asp Thr Val Arg Phe Arg Pro Pro Leu Thr Val Ser Thr Ala
            420                 425                 430

gag atc gac gcc gcg ata gcc gcg gtg cgc agc gcg tta ccg gtg gtg      1344
Glu Ile Asp Ala Ala Ile Ala Ala Val Arg Ser Ala Leu Pro Val Val
        435                 440                 445

acg taa                                                              1350
Thr

<210>  70
<211>  449
<212>  PRT
<213>  Mycobacterium tuberculosis

<400>  70

Met Ala Ala Val Val Lys Ser Val Ala Leu Ala Gly Arg Pro Thr Thr
1               5                   10                  15

Pro Asp Arg Val His Glu Val Leu Gly Arg Ser Met Leu Val Asp Gly
            20                  25                  30

Leu Asp Ile Val Leu Asp Leu Thr Arg Ser Gly Gly Ser Tyr Leu Val
        35                  40                  45

Asp Ala Ile Thr Gly Arg Arg Tyr Leu Asp Met Phe Thr Phe Val Ala
    50                  55                  60

Ser Ser Ala Leu Gly Met Asn Pro Pro Ala Leu Val Asp Asp Arg Glu
65                  70                  75                  80

Phe His Ala Glu Leu Met Gln Ala Ala Leu Asn Lys Pro Ser Asn Ser
                85                  90                  95

Asp Val Tyr Ser Val Ala Met Ala Arg Phe Val Glu Thr Phe Ala Arg
            100                 105                 110

Val Leu Gly Asp Pro Ala Leu Pro His Leu Phe Phe Val Glu Gly Gly
        115                 120                 125

Ala Leu Ala Val Glu Asn Ala Leu Lys Ala Ala Phe Asp Trp Lys Ser
    130                 135                 140

Arg His Asn Gln Ala His Gly Ile Asp Pro Ala Leu Gly Thr Gln Val

145                 150                 155                 160

Leu His Leu Arg Gly Ala Phe His Gly Arg Ser Gly Tyr Thr Leu Ser
                165                 170                 175

Leu Thr Asn Thr Lys Pro Thr Ile Thr Ala Arg Phe Pro Lys Phe Asp
            180                 185                 190

Trp Pro Arg Ile Asp Ala Pro Tyr Met Arg Pro Gly Leu Asp Glu Pro
        195                 200                 205

Ala Met Ala Ala Leu Glu Ala Glu Ala Leu Arg Gln Ala Arg Ala Ala
    210                 215                 220

Phe Glu Thr Arg Pro His Asp Ile Ala Cys Phe Val Ala Glu Pro Ile
225                 230                 235                 240

Gln Gly Glu Gly Gly Asp Arg His Phe Arg Pro Glu Phe Phe Ala Ala
                245                 250                 255

Met Arg Glu Leu Cys Asp Glu Phe Asp Ala Leu Leu Ile Phe Asp Glu
            260                 265                 270

Val Gln Thr Gly Cys Gly Leu Thr Gly Thr Ala Trp Ala Tyr Gln Gln
        275                 280                 285

Leu Asp Val Ala Pro Asp Ile Val Ala Phe Gly Lys Lys Thr Gln Val
    290                 295                 300

Cys Gly Val Met Ala Gly Arg Arg Val Asp Glu Val Ala Asp Asn Val
305                 310                 315                 320

Phe Ala Val Pro Ser Arg Leu Asn Ser Thr Trp Gly Gly Asn Leu Thr
                325                 330                 335

Asp Met Val Arg Ala Arg Arg Ile Leu Glu Val Ile Glu Ala Glu Gly
            340                 345                 350

Leu Phe Glu Arg Ala Val Gln His Gly Lys Tyr Leu Arg Ala Arg Leu
        355                 360                 365

Asp Glu Leu Ala Ala Asp Phe Pro Ala Val Val Leu Asp Pro Arg Gly
    370                 375                 380

Arg Gly Leu Met Cys Ala Phe Ser Leu Pro Thr Thr Ala Asp Arg Asp
385                 390                 395                 400

Glu Leu Ile Arg Gln Leu Trp Gln Arg Ala Val Ile Val Leu Pro Ala
                405                 410                 415

Gly Ala Asp Thr Val Arg Phe Arg Pro Pro Leu Thr Val Ser Thr Ala
            420                 425                 430

Glu Ile Asp Ala Ala Ile Ala Ala Val Arg Ser Ala Leu Pro Val Val
        435                 440                 445

Thr

## Description

The present invention relates to a novel enzymatically catalyzed method for the production of aliphatic primary amines, which method comprises the enzymatic oxidation of a primary aliphatic alcohol catalyzed by an alcohol dehydrogenase, amination of the resulting oxocompound catalyzed by a transaminase and optionally coupled with an enzymatically catalyzed simultaneous regeneration of the cofactor and amine donor; recombinant expression systems and microorganisms procuring the required enzyme activities; and bioreactors for performing such methods; as well as the use of the obtained amines in organic synthesis, in particular for preparing organic polymers.

### Background of the invention

Aliphatic primary amines are among the most important intermediates in the chemical industry. Of special industrial importance is their production by a metal-catalyzed reaction of ammonia with alcohols. Usually this reaction affords harsh conditions (elevated temperatures, high pressure), produces undesired by-products like secondary and tertiary amines (see Kirk-Othmer Encyclopedia of Chemical Technology, 5th ed, Wiley-Interscience, Hoboke, New Jersey, 2004, Vol. 2, page 544) and is not applicable to starting materials of limited thermal stability. Therefore there is still a demand for mild and selective procedures for the direct conversion of alcohols with ammonia into primary amines.

It has been demonstrated that mildness and selectivity are the domains of biotechnology. Therefore, we evaluated biocatalytic routes to primary amines starting from the corresponding alcohols. As in nature amino groups are introduced and exchanged via transaminase-catalyzed steps (see:J. M. Berg, J.L. Tymoczko, L. Stryer, Biochemistry, 7th ed., Freeman and company, Houndsmills, England, 2012, p. 704), these enzymes were considered to play a major role in the intended overall process.

Aminotransferases are pyridoxal phosphate (PLP)-containing enzymes that transfer an amino group from an amine donor, which can be an amino acid, to a keto compound or an aldehyde compound. The reaction occurs via formation of a pyridoxamine intermediate in which the amino group is carried by the modified PLP-containing cofactor. Aminotransferase reactions that aim to produce amines thus are dependent on the addition of an external amino acid or other organic amine that serves as the amine donor. Furthermore, the aldehydes and ketones (oxo-compounds) which act as amine acceptors are often chemically unstable and difficult to handle. The biocatalytic production of amines from aldehydes thus is hampered by problems related to the requirement for an organic amine donor and the instability and reactivity of the amine acceptors.

### Summary of the invention

This problem was surprisingly solved by providing an enzymatically catalyzed method as defined in the attached claims. In this method, problems caused by reactivity of the keto or aldehyde compound are prevented by using an alcohol as the starting compound, which is converted to a primary amine without addition of reducing substrates or an organic amine donor. The substrates to be added are the alcohol and ammonia. Three enzymes are added: an alcohol dehydrogenase that converts the alcohol to the aldehyde, producing also a reduced nicotinamide cofactor. An aminotransferase is added to convert the aldehyde to a primary amine, using an amino acid as amine donor, producing also a ketoacid. An amino acid dehydrogenase converts the ketoacid formed by the aminotransferase back to the amino acid, using ammonia and the reduced nicotinamide cofactor formed by the alcohol dehydrogenase.

The three-enzyme catalytic cycle thus requires no external source of reducing equivalents because the reduced cofactor is formed during alcohol oxidation. Only catalytic amounts of the redox nicotinamide cofactor and of the compound that transfers the amine group are needed. Furthermore, the cycle requires no external amino acid, since the amino acid is formed by a reaction of the ketoacid with ammonia, using the reduced cofactor.

### Description of the figures:

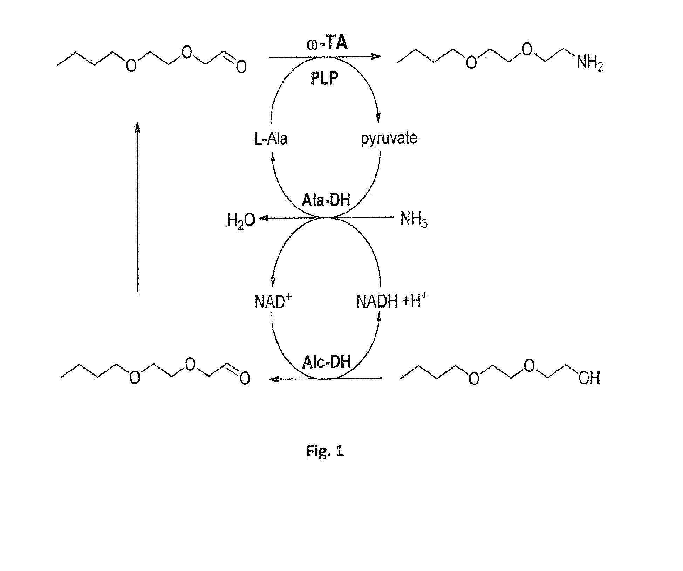

**Figure 1** illustrates the synthetic scheme of a biocatalytic process of the invention, exemplified by the conversion of the model substrate butyl diglycol (BDG) to BDG amine via BDG aldehyde and catalyzed by the enzymes alcohol dehydrogenase (AlcDH) and omega transaminase (w-TA) under consumption of cofactor NAD⁺ and L-alanine as amino donor. For cofactor and alanine regeneration the reaction is coupled with the reaction catalyzed by the regenerating enzyme alanine dehydrogenase (AlaDH) in the presence of an ammonia source which catalyzes the simultaneous regeneration of L-alanine from pyruvate and NAD⁺ from NADH. PLP = pyridoxal-5'-phosphate.

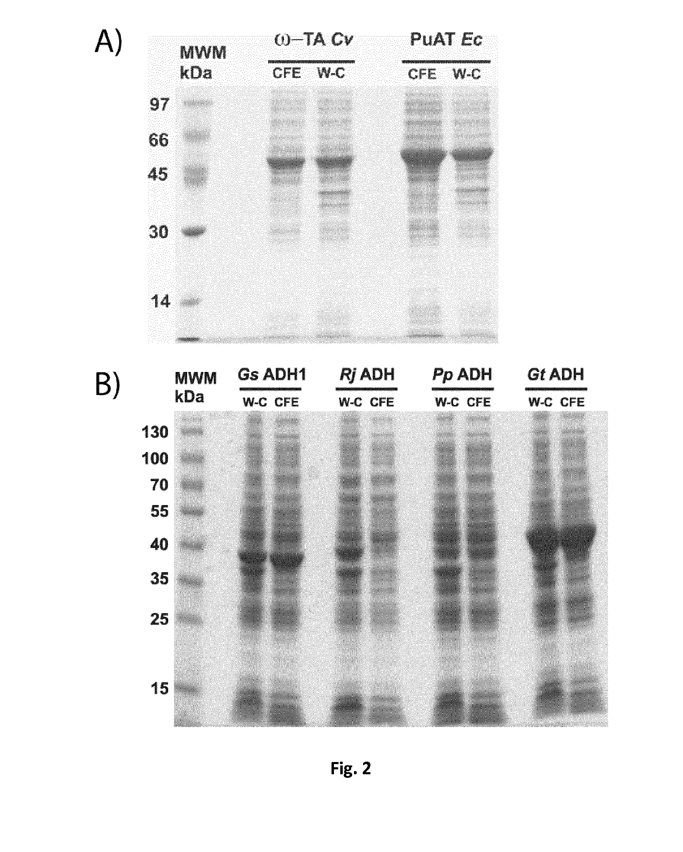

**Figure 2** illustrates the successful expression of A) transaminases Cv-w-TA, PuAT, and EcAT and B) alcohol dehydrogenases. The SDS gel shows protein bands of each enzyme contained in CFE (cell-free extract) or W-C (whole-cells)

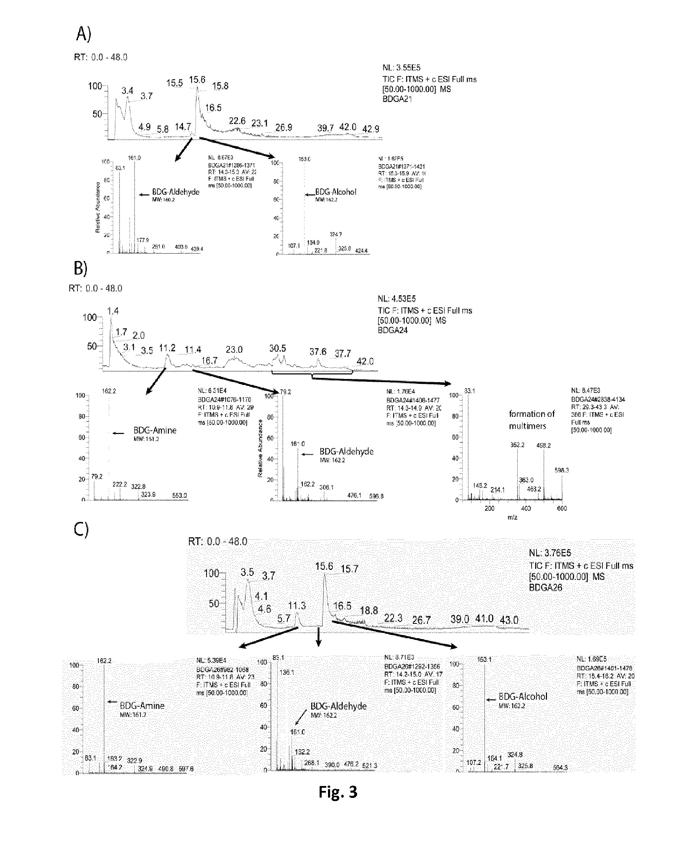

**Figure 3A)** illustrates the LC-MS analysis of the conversion of BDG-Alcohol to BDG-Aldehyde in a 24 h biocatalytic reaction using 1.5 mg commercial horse liver ADH (HL ADH, Sigma-Aldrich), 10 mM BDG-Alcohol 10 mM NAD⁺, 20 mM bicine-buffer (pH 8.8) in 1ml total volume at 30°C. Figure 3 B) illustrates the LC-MS analysis of the conversion of BDG-Aldehyde to BDG-Amine in a 24h biocatalysis using Cv w-TA (0.75 mg), 10 mM BDG-Aldehyde, 10 mM L-alanine, 20 mM bicine-buffer (pH 8.8) in 1ml total volume at 30°C. Figure 3 C) illustrates the LC-MS analysis of a 24h biocatalysis using Cv w-TA (0.75 mg) and commercial horse liver alcohol dehydrogenase (ADH) (1.5 mg, HL ADH, Sigma-Aldrich) in 1 ml reaction volume to convert BDG-Alcohol (10 mM) into BDG-Amine using NAD⁺ (10 mM) and L-alanine (10 mM) as cosubstrates.

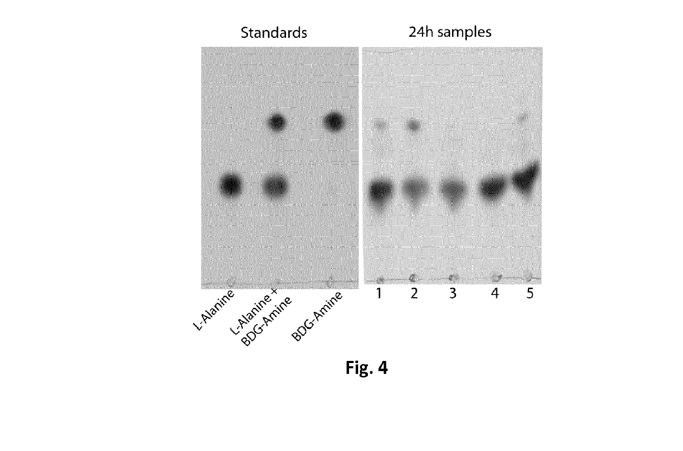

**Figure 4** illustrates a thin layer chromatography (TLC) of different biocatalytic conversions of BDG-Alcohol to BDG-Amine by combination of Cv w-TA and different ADHs, stained with ninhydrine to selectively detect amines. 1: Cv w-TA and commercial equine ADH; 2: Cv w-TA and *Geobacillus stearothermophilus* ADH (*E. coli* cell free extract); 3: Cv w-TA and *Geobacillus thermodenitrificans* NG80-2 ADH (*E. coli* cell free extract); 4: Control reaction without Cv w-TA and with commercial equine ADH; 5: Control reaction of BDG-Aldehyde with Cv w-TA.

### Detailed description of the invention

### 1. Specific embodiments

The present invention relates to the following particular embodiments:
1. A biocatalytic method of preparing an aliphatic primary mono- or polyamine, i.p. diamine, which method comprises:
   a) the enzymatic oxidation the corresponding aliphatic primary mono- or polyalcohol, i.p. dialcohol of said amine in the presence of the cofactor NAD(P)⁺ and of an NAD(P)⁺ dependent alcohol dehydrogenase (AlcDH) (E.C.1.1.1.x in particular 1.1.1.1 or 1.1.1.2) to form the corresponding oxo compound of said alcohol, and wherein the cofactor NAD(P)⁺ is reduced to form NAD(P)H;
   b) the enzymatic conversion of said oxo compound in the presence of b1) a sacrificial alpha-amino carboxylic acid of the formula III

      Z-CH(NH₂)COOH (III)

      wherein
      Z is the side chain of a natural or artificial, in particular natural, alpha-amino acid, in particular alanine or glutamine; and
   b2) a transaminase (E.C.2.6.1.x) and optionally pyridoxal phosphate to form the desired aliphatic amine while converting the sacrificial alpha-amino carboxylic acid to the corresponding alpha-keto carboxylic acid of the formula IV

      Z-C(=O)COOH (IV)

      wherein Z is as defined above.
      Non-limiting examples of primary amines or corresponding alcohols are given below, i.e. for example amines of formula I and alcohols of formula II.
   In particular, the cofactor of the redox reaction is NAD⁺/NADH.
2. The method of embodiment 1, which further comprises a coupled process of cofactor regeneration for regenerating the consumed cofactor NAD(P)⁺, in particular NAD⁺.
3. The method of embodiment 2, wherein said coupled cofactor regenerating process also comprises the regeneration of the consumed sacrificial alpha-amino carboxylic acid in particular alanine or glutamate. Thus, if cofactor regeneration and regeneration of sacrificial alpha-amino carboxylic acid are coupled to the amination process merely sub-stoichiometric, or catalytic, amounts of cofactor and carboxylic acid are required. In an embodiment, wherein regeneration of said sacrificial alpha-amino carboxylic acid shall not take place, preferentially said acid is applied in at least stoichiometric amounts, or even at a molar excess of said acid may be of advantage.
4. The method of embodiment 3, wherein said cofactor regenerating process encompasses the enzymatic reduction (i.e. reductive amination) of said alpha-keto carboxylic acid of the formula IV, in particular pyruvate or alpha ketoglutarate, to regenerate the alpha-amino carboxylic acid of the formula III, in particular alanine or glutamate, and the simultaneous oxidation of NAD(P)H to regenerate the consumed cofactor NAD(P)⁺ in the presence of an NAD(P)⁺ dependent alpha amino acid dehydrogenase (AADH) (E.C. 1.4.1.x) and a source of ammonia, like ammonia gas and/or or ammonia salts (like ammonium halogenides, i.p. ammonium chloride) as for example in the form of an optionally buffered solution containing ammonia and/or an ammonium salt and optionally ammonia gas
5. The method of one of the preceding embodiments, wherein aliphatic alcohol is a mono-or polyvalent aliphatic alcohol carrying one or more "terminal" hydroxy groups, and wherein said terminal hydroxy groups of said aliphatic alcohol are either partly or fully aminated by said method.
6. A biocatalytic method of preparing a primary amine of the general formula I or la

   RNH₂ (I)

   R'(NH₂)ₘ (Ia)

   wherein
   m is an integer of at least 2, like 2, 3, 4, or 5; in particular 2;
   R is selected from straight chain or branched hydrocarbyl, straight chain or branched hydroxyhydrocarbyl or straight chain or branched alkoxyhydrocarbyl or a polyhydrocarbylether group;
   R' is selected from straight chain or branched hydrocarbylene, and a polyhydrocarbylene ether group;
   which method comprises
   a) the enzymatic oxidation the corresponding alcohol of the general formula II or IIa

      ROH (II)

      R'(OH)ₘ (IIa)

      wherein
      m, R' and R is as defined above
      in the presence of the cofactor NAD(P)⁺ and of an NAD(P)⁺ dependent alcohol dehydrogenase (AlcDH) (E.C.1.1.1.x, in particular 1.1.1.1 or 1.1.1.2) to form the corresponding oxo compound of said alcohol,
      and wherein the cofactor NAD(P)⁺ is reduced to form NAD(P)H;
   b) the enzymatic conversion of said oxo compound in the presence of b1) a sacrificial alpha-amino carboxylic acid of the formula III

      Z-CH(NH₂)COOH (III)

      wherein
      Z is the side chain of a natural or artificial, in particular natural, alpha-amino acid
      and
   b2) a transaminase (E.C.2.6.1.x) and optionally pyridoxal phosphate to form the desired amine of the above formula I while converting the sacrificial alpha-amino carboxylic acid to the corresponding alpha-keto carboxylic acid of the formula IV

      Z-C(=O)COOH (IV)

      wherein
      Z is the residue of a natural or artificial alpha-amino acid.
      In particular, the cofactor of the redox reaction is NAD⁺/NADH.
      In particular R is a polyhydrocarbylether group
      (i.e. for example "alkyl-O-(-alkylene-O-)ₙ-") and
      R' is a polyhydrocarbyleneether group
      (i.e. for example "-(-alkylene-O-)ₙ-").
      In particular mixtures, like statistical mixtures of alcohols of formula I or alcohols of formula Ia, are used as substrates; in particular of substrates wherein R is a polyhydrocarbylether group and R' is a polyhydrocarbyleneether group.
7. The method of embodiment 6, which further comprises a coupled process for regenerating the consumed cofactor NAD(P)⁺, in particular NAD⁺.
8. The method of embodiment 7, wherein said cofactor regenerating process also comprises a regeneration step of the consumed sacrificial alpha-amino carboxylic acid.
9. The method of embodiment 8, wherein said cofactor regenerating step encompasses the enzymatic reductive amination of said alpha-keto carboxylic acid of the formula IV, in particular pyruvate or alpha ketoglutarate, to regenerate the alpha-amino carboxylic acid of the formula III, in particular alanine or glutamate, and the simultaneous oxidation of NAD(P)H, in particular NADH to regenerate the consumed cofactor NAD(P)⁺, in particular NAD⁺, in the presence of an NAD(P)⁺ in particular NAD⁺, dependent alpha amino acid dehydrogenase (AADH) (E.C. 1.4.1.x) and a source of ammonia, as for example in the form of a buffered solution containing ammonia and/or an ammonium salt.
10. The method of one of the preceding embodiments, which is performed in an aqueous reaction medium, optionally in the presence of at least one organic water-miscible or water immiscible co-solvent.
11. The method of one of the preceding embodiments wherein the individual reactions are performed consecutively or simultaneously in the same reaction mixture.
12. The method of embodiment 1 or 6, wherein said transaminase is an omega transaminase (w-TA) (E.C.2.6.1.18), an L-ornithine aminotransferase (EC 2.6.1.13) or a L-lysine-6-transaminase (2.6.1.36).
13. The method of embodiment 4 or 9, wherein said alpha amino acid dehydrogenase (AADH) is selected from:
   a) an alanine dehydrogenase (AlaDH) (E.C. 1.4.1.1) or
   b) a glutamate dehydrogenase (GluDH) (E.C. 1.4.1.2, 1.4.1.3 or 1.4.1.4, in particular 1.4.1.2).
14. The method of one of the preceding embodiments wherein the enzymes are selected as follows:
   a) alcohol dehydrogenase (E.C1.1.1.1) having an amino acid sequence according to SEQ ID NO 11, 16, 21, 27, 31 or an enzyme having a sequence having sequence identity of at least 60%, as for example at least 70, 80, 85, 90, 92, 95, 96, 97, 98 or 99 % sequence identity, to SEQ ID NO: 11, 16, 21, 27, 31 and still retaining the intended enzyme activity (or function), i.e. being applicable as alcohol dehydrogenase;
   b) amino group transferring enzymes, selected from omega transaminase (E.C.2.1.6.18) (i.e. a transaminase capable of aldehyde amination) having an amino acid sequence according to SEQ ID NO:3, 6, 57 or 61; L-ornithine aminotransferases (EC 2.6.1.13) having an amino acid sequence according to SEQ ID NO:62, 63, 64, 65 or 66; or L-lysine-6-transaminases (2.6.1.36) having an amino acid sequence according to SEQ ID NO: 68 or 70; .or an enzyme having a sequence having sequence identity of at least 60%, as for example at least 70, 80, 85, 90, 92, 95, 96, 97, 98 or 99 % sequence identity, to SEQ ID NO:3, 6, 57, 61, 62, 63, 64, 65, 66, 68 or 70; and still retaining the intended enzyme activity (or function), i.e. being applicable as amino group transferring enzyme;
   c) alanine dehydrogenase (E.C1.4.1.1) having an amino acid sequence according to SEQ ID NO:34, 39 or 44 or an enzyme having a sequence having sequence identity of at least 60%, as for example at least 70, 80, 85, 90, 92, 95, 96, 97, 98 or 99 % sequence identity, to SEQ ID NO: 34, 39 or 44; and still retaining the intended enzyme activity (or function), i.e. being applicable as alanine dehydrogenase; or a glutamate dehydrogenase (GluDH) (E.C. 1.4.1.2) having an amino acid sequence according to SEQ ID NO:49, 51, 53 or 55 or an enzyme having a sequence having sequence identity of at least 60%. as for example at least 70, 80, 85, 90, 92, 95, 96, 97, 98 or 99 % sequence identity, to SEQ ID NO: 49, 51, 53 or 55 and still retaining the intended enzyme activity (or function), i.e. being applicable as glutamate dehydrogenase.
15. The method of one of the preceding embodiments wherein the process is performed in the presence of the set of required enzymes in isolated form, one or more recombinant microorganisms expressing the required set of enzymes or cell homogenates or cell lysates obtained therefrom.
16. The method of one of the preceding embodiments wherein the alcohol substrates are selected from:
   a) monovalent alcohols of the formulae:
      AO-(-C₂-C₆-alkylene-O-)ₙ-C₂-C₆-alkylene-OH or
      AO-(-C₂-C₄-alkylene-O-)ₙ-C₂-C₄-alkylene-OH
      wherein
      A is C₁-C₂₂ alkyl, C₁-C₆-alkyl or C₁-C₄-alkyl; or AO is replaced by H; and
      n is 0 or an integer from 1 to 100, 1 to 50, 1 to 20 or 1 to 10, like 1, 2, 3 , 4 or 5, and
      wherein said alkyl and alkylene groups may be straight chain or branched;
   b) bivalent alcohols of the formulae:
      HO-C₁-C₁₂-alkylene-OH, or
      HO-(-C₂-C₆-alkylene-O-)ₙ-C₂-C₆-alkylene-OH like
      HO-C₁-C₆-alkylene-OH, or
      HO-(-C₂-C₄-alkylene-O-)ₙ-C₂-C₄-alkylene-OH
      wherein n is an integer from 1 to 100, 1 to 50, 1 to 20 or 1 to 10, like 1, 2, 3, 4 or 5, and said alkyl and alkylene groups may be straight chain or branched; or
      mixtures of such compounds;
   c) trivalent alkohols 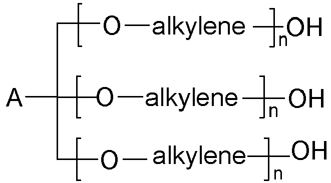 wherein
      alkylene is a straight chain or branched C₂-C₆-alkylene or C₂-C₆-alkylene group A is H or C₁-C₁₂-alkyl or C₁-C₆-alkyl and parameters n are same or different and integers from 1 to 50, 1 to 20 or 1 to 10, like 1, 2, 3, 4 or 5; or mixtures of such compounds.
17. The method of one of the preceding embodiments further comprising isolating the produced amine from the reaction medium.
18. The use of an amine, in particular polyetheramines, as prepared by a method of anyone of the preceding embodiments in the preparation of organic polymers.
19. A recombinant constitutive or non-constitutive expression vector carrying under the control of at least one regulatory element the coding sequence of at least one of the following enzymes:
   a) alcohol dehydrogenase (E.C1.1.1.1) having an amino acid sequence according to SEQ ID NO 11, 16, 21, 27, 31 or an enzyme having a sequence having sequence identity of at least 60%, as for example at least 70, 80, 85, 90, 92, 95, 96, 97, 98 or 99 % sequence identity, to SEQ ID NO: 11, 16, 21, 27, 31 and still retaining the intended enzyme activity (or function), i.e. being applicable as alcohol dehydrogenase
   b) amino group transferring enzymes, selected from omega transaminase (E.C.2.1.6.18) (i.e. a transaminase capable of aldehyde amination) having an amino acid sequence according to SEQ ID NO:3, 6, 57 or 61; L-ornithine aminotransferases (EC 2.6.1.13) having an amino acid sequence according to SEQ ID NO:62, 63, 64, 65 or 66; or L-lysine-6-transaminases (2.6.1.36) having an amino acid sequence according to SEQ ID NO: 68 or 70; .or an enzyme having a sequence having sequence identity of at least 60%, as for example at least 70, 80, 85, 90, 92, 95, 96, 97, 98 or 99 % sequence identity, to SEQ ID NO:3, 6, 57, 61, 62, 63, 64, 65, 66, 68 or 70; and still retaining the intended enzyme activity (or function), i.e. being applicable as amino group transferring enzyme;
   c) alanine dehydrogenase (E.C1.4.1.1) having an amino acid sequence according to SEQ ID NO:34, 39 or 44 or an enzyme having a sequence having sequence identity of at least 60%, as for example at least 70, 80, 85, 90, 92, 95, 96, 97, 98 or 99 % sequence identity, to SEQ ID NO: 34, 39 or 44; and still retaining the intended enzyme activity (or function), i.e. being applicable as alanine dehydrogenase or a glutamate dehydrogenase (E.C. 1.4.1.2) having an amino acid sequence according to SEQ ID NO: 49, 51, 53 or 55or an enzyme having a sequence having sequence identity of at least 60%, as for example at least 70, 80, 85, 90, 92, 95, 96, 97, 98 or 99 % sequence identity, to SEQ ID NO: 49, 51, 53 or 55 and still retaining the intended enzyme activity (or function), i.e. being applicable as glutamate dehydrogenase.
20. A recombinant microorganism functionally expressing at least one enzyme as defined in the embodiments 1 to 14.
21. The recombinant microorganism of embodiment 20, carrying at least one expression vector according to embodiment 18 and functionally expressing said encoded enzymes.
22. The use of a recombinant expression vector of embodiment 19 or of a recombinant microorganism of embodiment 20 or 21 in a method as embodimented in anyone of the embodiments 1 to 17.
23. A bioreactor for performing a biocatalytic method of anyone of the embodiments 1 to 16 containing in a reaction medium at least one recombinant microorganism of anyone of the embodiments 19 and 20, or at least one enzyme as defined in anyone of the embodiments 1 to 13 in free od immobilized form.

In case of a terminal hydroxy group to be converted to an amino group) linked to an asymmetric carbon atom the reaction may or may not be performed stereospecifically.

### 2. Explanation of particular terms

Unless otherwise stated the following meanings shall apply:
The term "biocatalytic" or "enzymatically catalysed" method refers to any method performed in the presence of catalytic activity of an enzyme as defined herein, i.e. in the presence of isolated pure or crude enzyme or entire microbial cells containing or expressing such enzyme activity.

The term "stereospecific" means that one out of several stereoisomers or enantiomers is formed by the enzyme in high enantiomeric excess or purity, of at least 90 %ee, preferably at least 95 %ee, in particular at least 98 %ee, or at least 99 %ee. the ee% value is calculated according to the following formula
ee% = [X_{A}-X_{B}]/[X_{A}+X_{B}]*100,
wherein X_{A} and X_{B} refer to the molar fraction of enantiomer A or B, respectively.

The term "substantially pure" is meant to describe a molecule, which is homogeneous by one or more purity or homogeneity characteristics used by those of skill in the art.

A "substantially pure" protein or enzyme means that the desired purified protein is essentially free from contaminating cellular components, as evidenced by a single band following polyacrylamide-sodium dodecyl sulfate gel electrophoresis (SDS-PAGE). A "substantially pure" enzyme or protein will show constant and reproducible characteristics within standard experimental deviations for parameters such as the following: molecular mass, chromatographic migration, amino acid composition, amino acid sequence, blocked or unblocked N-terminus, HPLC elution profile, biological activity, and other such parameters. For example, a "substantially pure" protein or enzyme means that the desired purified protein is essentially free from contaminating cellular components, as evidenced by a single band following polyacrylamide-sodium dodecyl sulfate gel electrophoresis (SDS-PAGE).The term, however, is not meant to exclude artificial or synthetic mixtures of said enzyme or protein with other compounds. In addition, the term is not meant to exclude fusion proteins optionally isolated from a recombinant host, or fusions proteins with functional residues like histidin archer groups.

"Sacrificial" alpha-amino carboxylic acid is understood as compound which is consumed during the course of the catalytic main reaction (here in particular formation of aliphatic primary amine), but also encompasses the situation, where the consumed carboxylic acid is regenerated.

"Natural alpha amino acid residues" are derived from Ala, Arg, Asn, Asp, Cys, Gin, Glu, Gly, His, Ile, Leu, Lys, Met, Phe, Ser, Thr, Trp, Tyr, Val, Pro.

Within chemical compounds as defined herein and in the attached claims the following meanings shall apply:

**Hydrocarbyl** can be interpreted widely and comprises straight-chain and branched hydrocarbon radicals, linked to one functional group, like OH or NH₂, In particular these functional groups are terminal groups. These residues are essentially composed of carbon and hydrogen atoms, which may optionally additionally comprise heteroatoms, for example O, N, NH, S, within their chain thereof, and may have a chain length of 1 to 50, 2 to 30 or 3 to 15 consecutive carbon atoms and optionally heteroatoms .

Hydrocarbyl especially represents residues and especially straight-chain or branched alkyl, alkenyl or alkinyl, optionally interrupted by one or more, for example 1, 2, 3, 4, 5, 6, 7, 8, 9 or 10 heteroatom groups such as -O- or-NH-, in particular -O-.

In particular, hydrocarbyl residues may comprise repetitive, as for example 1 to 100, 1 to 50, 1 to 20, 2 to 15 or 3 to 10 or 4 to 7, oxyalkylene or oxyalkenylen, i.p. oxyalkylene, units as defined below. These type of residues are also called **"polyhydrocarbylether**" residues.

"Hydroxyl-substituted hydrocarbyl" or "hydroxyhydrocarbyl" represents especially the hydroxyl-substituted analogs of the alkyl or alkenyl radicals defined herein.

"Alkoxyl-substituted hydrocarbyl" or "alkoxyhydrocarbyl" represents especially the alkoxy-substituted analogs of the alkyl or alkenyl radicals defined herein.

**Hydrocarbylene** designate the analogues of the above hydrocarbyl residuess but now linked to at least 2, like 2,or 3, i.p. 2 functional groups, like OH or NH₂. In particular these functional groups are terminal groups.

**Polyhydrocarbyleneether** residues designate the analogues of the above polyhydrocarbylether residues but now linked to at least 2, like 2,or 3, i.p. 2 functional groups, like OH or NH₂. In particular said functional groups are terminal groups.

**Alkyl as well as alkyl fragments of residues derived there from, as for example alkoxy,** represent saturated, linear or branched hydrocarbon chains having 1 to 4, 1 to 6, 1 to 8, 1 to 10, 1 to 22 or 1 to 30 carbon atoms, like

**C₁-C₆-Alkyl** like **C₁-C₄-Alkyl,** as for example methyl, ethyl, propyl, 1-methylethyl, butyl, 1-methyl-propyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl und 1-ethyl-2-methylpropyl.

**C₁-C₆-Alkoxy,** like **C₁-C₄-alkoxy,** as for example methoxy, ethoxy, n-propoxy, 1-methylethoxy, butoxy, 1-methylpropoxy, 2-methylpropoxy or 1,1-dimethylethoxy; as well as e.g. pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy or 1-ethyl-2-methylpropoxy;

**Hydroxy-C₁-C₁₀-alkyl, e.g. hydroxy-C₁-C₆-alkyl, or hydroxy-C₁-C₄-alkyl, or hydroxy-C₄-C₁₀-alkyl:** e.g. hydroxymethyl, 1- or 2-hydroxyethyl, 1-, 2- or 3-hydroxypropyl, 1-hydroxymethylethyl, 1-, 2-, 3- or 4-hydroxybutyl, 1-hydroxymethylpropyl and 2-hydroxymethylpropyl; or other hydroxy-substituted analogs of the above alkyl residues, such as in particular the meanings of R given in Table 4, e.g. 6-hydroxy-6-methylhept-1-yl.

**Alkenyl:** one-fold or multiple, in particular one-fold non-saturated, straight-chain or branched hydrocarbon residues having 2 to 4, 2 to 6, 2 to 8, or 2 to 10 carbon atoms and a double bond in any position, like C2-C6-alkenyl, as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl.

**Alkenyloxy:** oxygen-linked analogs of the above alkenyl groups, like corresponding C₂-C₆-alkenyloxy groups.

**Alkylene:** staight chain or branched hydrocarbon bridges having up to 7 carbon atoms, as for example C₁-C₇-alkylen groups like -CH₂-, -(CH₂)₂-, -(CH₂)₃-,-(CH₂)₄-, - (CH₂)₂-CH(CH₃)-, -CH₂-CH(CH₃)-CH₂- , (CH₂)₄-, -(CH₂)₅-, -(CH₂)₆, -(CH₂)₇-, -CH(CH₃)-CH₂-CH₂-CH(CH₃)- or -CH(CH₃)-CH₂-CH₂-CH₂-CH(CH₃)- or C₁-C₄-alkylen groups selected from -CH₂-, -(CH₂)₂-, -(CH₂)₃-,-(CH₂)₄-, -(CH₂)₂-CH(CH₃)-, -CH₂-CH(CH₃)-CH₂.

**Alkenylene:** represent the one-fold or multiply, in particular, one-fold non-saturated, analogues of the above alkylen groups, having 2 to 8 carbon atoms, in particular C₂-C₇-alkenylenes or C₂-C₄-alkenylenes, like -CH=CH-, -CH=CH-CH₂-, - CH₂-CH=CH-, -CH=CH-CH₂-CH₂-, -CH₂-CH=CH-CH₂-, -CH₂-CH₂-CH=CH-, -CH(CH₃)-CH=CH-, -CH₂-C(CH₃)=CH-.

**Oxyalkylene** and **Oxyalkenylene** represent the oxygen terminated analogues of the above alkylene or alkenylene groups of the formulae -O-alkylene-, -alkylene-O-, -O-alkenylene- or -alkenylene-O-;

**Optional substituents** may be selected from - COOH, -COO-alkyl, -OH, -SH, - CN, amino, -NO₂, alkyl, or alkenyl, alkyl or alkenyl being as defined above.

### 3. Further embodiments of the invention

### 3.1 Proteins according to the invention

The present invention is not limited to the specifically mentioned enzymes/proteins, but also extends to functional equivalents thereof.

"Functional equivalents" or "analogs" or "functional mutations" of the concretely disclosed enzymes are, within the scope of the present invention, various polypeptides thereof, which moreover possess the desired biological function or activity, e.g. enzyme activity.

For example, "functional equivalents" means enzymes, which, in a test used for enzymatic activity, display at least a 1 to 10%, or at least 20%, or at least 50%, or at least 75%, or at least 90% higher or lower activity of an enzyme, as defined herein.

"Functional equivalents", according to the invention, also means in particular mutants, which, in at least one sequence position of the amino acid sequences stated above, have an amino acid that is different from that concretely stated, but nevertheless possess one of the aforementioned biological activities. "Functional equivalents" thus comprise the mutants obtainable by one or more, like 1 to 20, 1 to 15, 1 to 10 or 1 to 5, amino acid additions, substitutions, deletions and/or inversions, where the stated changes can occur in any sequence position, provided they lead to a mutant with the profile of properties according to the invention. Functional equivalence is in particular also provided if the reactivity patterns coincide qualitatively between the mutant and the unchanged polypeptide, i.e. if for example the same substrates are converted at a different rate. Examples of suitable amino acid substitutions are shown in the following table:

| **Original residue** | **Examples of substitution** |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met; Leu ; Tyr |
| Ser | Thr |
| Thr | Ser |
| Trp | Tyr |
| Tyr | Trp ; Phe |
| Val | Ile; Leu |

"Functional equivalents" in the above sense are also "precursors" of the polypeptides described, as well as "functional derivatives" and "salts" of the polypeptides.

"Precursors" are in that case natural or synthetic precursors of the polypeptides with or without the desired biological activity.

The expression "salts" means salts of carboxyl groups as well as salts of acid addition of amino groups of the protein molecules according to the invention. Salts of carboxyl groups can be produced in a known way and comprise inorganic salts, for example sodium, calcium, ammonium, iron and zinc salts, and salts with organic bases, for example amines, such as triethanolamine, arginine, lysine, piperidine and the like. Salts of acid addition, for example salts with inorganic acids, such as hydrochloric acid or sulfuric acid and salts with organic acids, such as acetic acid and oxalic acid, are also covered by the invention.

"Functional derivatives" of polypeptides according to the invention can also be produced on functional amino acid side groups or at their N-terminal or C-terminal end using known techniques. Such derivatives comprise for example aliphatic esters of carboxylic acid groups, amides of carboxylic acid groups, obtainable by reaction with ammonia or with a primary or secondary amine; N-acyl derivatives of free amino groups, produced by reaction with acyl groups; or O-acyl derivatives of free hydroxy groups, produced by reaction with acyl groups.

"Functional equivalents" naturally also comprise polypeptides that can be obtained from other organisms, as well as naturally occurring variants. For example, areas of homologous sequence regions can be established by sequence comparison, and equivalent enzymes can be determined on the basis of the concrete parameters of the invention.

"Functional equivalents" also comprise fragments, preferably individual domains or sequence motifs, of the polypeptides according to the invention, which for example display the desired biological function.

"Functional equivalents" are, moreover, fusion proteins, which have one of the polypeptide sequences stated above or functional equivalents derived there from and at least one further, functionally different, heterologous sequence in functional N-terminal or C-terminal association (i.e. without substantial mutual functional impairment of the fusion protein parts). Non-limiting examples of these heterologous sequences are e.g. signal peptides, histidine anchors or enzymes.

"Functional equivalents" that are also included according to the invention are homologues of the concretely disclosed proteins. These possess percent identity values as stated above. Said values refer to the identity with the concretely disclosed amino acid sequences, and may be calculated according to the algorithm of Pearson and Lipman, Proc. Natl. Acad, Sci. (USA) 85(8), 1988, 2444-2448.

The percent identity values may also be calculated from BLAST alignments, algorithm blastp (protein-protein BLAST) or by applying the Clustal setting as given below. Typical percent identity values are for example in the range of 50 % or more, as for example at least 60, 70, 80, 85, 90, 91, 92, 93, 94, 95, 96, 97, 98 or 99 %.

A percentage identity of a homologous polypeptide according to the invention means in particular the percentage identity of the amino acid residues relative to the total length of one of the amino acid sequences concretely described herein.

In the case of a possible protein glycosylation, "functional equivalents" according to the invention comprise proteins of the type designated above in deglycosylated or glycosylated form as well as modified forms that can be obtained by altering the glycosylation pattern.

Such functional equivalents or homologues of the proteins or polypeptides according to the invention can be produced by mutagenesis, e.g. by point mutation, lengthening or shortening of the protein.

Such functional equivalents or homologues of the proteins according to the invention can be identified by screening combinatorial databases of mutants, for example shortening mutants. For example, a variegated database of protein variants can be produced by combinatorial mutagenesis at the nucleic acid level, e.g. by enzymatic ligation of a mixture of synthetic oligonucleotides. There are a great many methods that can be used for the production of databases of potential homologues from a degenerated oligonucleotide sequence. Chemical synthesis of a degenerated gene sequence can be carried out in an automatic DNA synthesizer, and the synthetic gene can then be ligated in a suitable expression vector. The use of a degenerated genome makes it possible to supply all sequences in a mixture, which code for the desired set of potential protein sequences. Methods of synthesis of degenerated oligonucleotides are known to a person skilled in the art (e.g. Narang, S.A. (1983) Tetrahedron 39:3; Itakura et al. (1984) Annu. Rev. Biochem. 53:323; Itakura et al. (1984) Science 198:1056; Ike et al. (1983) Nucleic Acids Res. 11:477).

In the prior art, several techniques are known for the screening of gene products of combinatorial databases, which were produced by point mutations or shortening, and for the screening of cDNA libraries for gene products with a selected property. These techniques can be adapted for the rapid screening of the gene banks that were produced by combinatorial mutagenesis of homologues according to the invention. The techniques most frequently used for the screening of large gene banks, which are based on a high-throughput analysis, comprise cloning of the gene bank in expression vectors that can be replicated, transformation of the suitable cells with the resultant vector database and expression of the combinatorial genes in conditions in which detection of the desired activity facilitates isolation of the vector that codes for the gene whose product was detected. Recursive Ensemble Mutagenesis (REM), a technique that increases the frequency of functional mutants in the databases, can be used in combination with the screening tests, in order to identify homologues (Arkin and Yourvan (1992) PNAS 89:7811-7815; Delgrave et al. (1993) Protein Engineering 6(3):327-331).

### 3.2 Coding nucleic acid sequences

The invention also relates to nucleic acid sequences that code for enzymes/proteins as defined herein.

The present invention also relates to nucleic acids with a certain degree of "identity" to the sequences specifically disclosed herein. "Identity" between two nucleic acids means identity of the nucleotides, in each case over the entire length of the nucleic acid.

For example the identity may be calculated by means of the Vector NTI Suite 7.1 program of the company Informax (USA) employing the Clustal Method (Higgins DG, Sharp PM. Fast and sensitive multiple sequence alignments on a microcomputer. Comput Appl. Biosci. 1989 Apr; 5(2):151-1) with the following settings:
Multiple alignment parameters:

| | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighing | 0 |

Pairwise alignment parameter:

| | |
|---|---|
| FAST algorithm | on |
| K-tuplesize | 1 |
| Gap penalty | 3 |
| Window size | 5 |
| Number of best diagonals | 5 |

Alternatively the identity may be determined according to Chenna, Ramu, Sugawara, Hideaki, Koike,Tadashi, Lopez, Rodrigo, Gibson, Toby J, Higgins, Desmond G, Thompson, Julie D. Multiple sequence alignment with the Clustal series of programs. (2003) Nucleic Acids Res 31 (13):3497-500, the web page: http://www.ebi.ac.uk/Tools/clustalw/index.html# and the following settings

| | |
|---|---|
| DNA Gap Open Penalty | 15.0 |
| DNA Gap Extension Penalty | 6.66 |
| DNA Matrix | Identity |
| Protein Gap Open Penalty | 10.0 |
| Protein Gap Extension Penalty | 0.2 |
| Protein matrix | Gonnet |
| Protein/DNA ENDGAP | -1 |
| Protein/DNA GAPDIST | 4 |

All the nucleic acid sequences mentioned herein (single-stranded and double-stranded DNA and RNA sequences, for example cDNA and mRNA) can be produced in a known way by chemical synthesis from the nucleotide building blocks, e.g. by fragment condensation of individual overlapping, complementary nucleic acid building blocks of the double helix. Chemical synthesis of oligonucleotides can, for example, be performed in a known way, by the phosphoamidite method (Voet, Voet, 2nd edition, Wiley Press, New York, pages 896-897). The accumulation of synthetic oligonucleotides and filling of gaps by means of the Klenow fragment of DNA polymerase and ligation reactions as well as general cloning techniques are described in Sambrook et al. (1989), see below.

The invention also relates to nucleic acid sequences (single-stranded and double-stranded DNA and RNA sequences, e.g. cDNA and mRNA), coding for one of the above polypeptides and their functional equivalents, which can be obtained for example using artificial nucleotide analogs.

The invention relates both to isolated nucleic acid molecules, which code for polypeptides or proteins according to the invention or biologically active segments thereof, and to nucleic acid fragments, which can be used for example as hybridization probes or primers for identifying or amplifying coding nucleic acids according to the invention.

The nucleic acid molecules according to the invention can in addition contain non-translated sequences from the 3' and/or 5' end of the coding genetic region.

The invention further relates to the nucleic acid molecules that are complementary to the concretely described nucleotide sequences or a segment thereof.

The nucleotide sequences according to the invention make possible the production of probes and primers that can be used for the identification and/or cloning of homologous sequences in other cellular types and organisms. Such probes or primers generally comprise a nucleotide sequence region which hybridizes under "stringent" conditions (see below) on at least about 12, preferably at least about 25, for example about 40, 50 or 75 successive nucleotides of a sense strand of a nucleic acid sequence according to the invention or of a corresponding antisense strand.

An "isolated" nucleic acid molecule is separated from other nucleic acid molecules that are present in the natural source of the nucleic acid and can moreover be substantially free from other cellular material or culture medium, if it is being produced by recombinant techniques, or can be free from chemical precursors or other chemicals, if it is being synthesized chemically.

A nucleic acid molecule according to the invention can be isolated by means of standard techniques of molecular biology and the sequence information supplied according to the invention. For example, cDNA can be isolated from a suitable cDNA library, using one of the concretely disclosed complete sequences or a segment thereof as hybridization probe and standard hybridization techniques (as described for example in Sambrook, J., Fritsch, E.F. and Maniatis, T. Molecular Cloning: A Laboratory Manual. 2nd edition, Cold Spring Harbor Laboratory, Cold Spring Harbor Laboratory Press, Cold Spring Harbor, NY, 1989). In addition, a nucleic acid molecule comprising one of the disclosed sequences or a segment thereof, can be isolated by the polymerase chain reaction, using the oligonucleotide primers that were constructed on the basis of this sequence. The nucleic acid amplified in this way can be cloned in a suitable vector and can be characterized by DNA sequencing. The oligonucleotides according to the invention can also be produced by standard methods of synthesis, e.g. using an automatic DNA synthesizer.

Nucleic acid sequences according to the invention or derivatives thereof, homologues or parts of these sequences, can for example be isolated by usual hybridization techniques or the PCR technique from other bacteria, e.g. via genomic or cDNA libraries. These DNA sequences hybridize in standard conditions with the sequences according to the invention.

"Hybridize" means the ability of a polynucleotide or oligonucleotide to bind to an almost complementary sequence in standard conditions, whereas nonspecific binding does not occur between non-complementary partners in these conditions. For this, the sequences can be 90-100% complementary. The property of complementary sequences of being able to bind specifically to one another is utilized for example in Northern Blotting or Southern Blotting or in primer binding in PCR or RT-PCR.

Short oligonucleotides of the conserved regions are used advantageously for hybridization. However, it is also possible to use longer fragments of the nucleic acids according to the invention or the complete sequences for the hybridization. These standard conditions vary depending on the nucleic acid used (oligonucleotide, longer fragment or complete sequence) or depending on which type of nucleic acid - DNA or RNA - is used for hybridization. For example, the melting temperatures for DNA:DNA hybrids are approx. 10°C lower than those of DNA: RNA hybrids of the same length.

For example, depending on the particular nucleic acid, standard conditions mean temperatures between 42 and 58°C in an aqueous buffer solution with a concentration between 0.1 to 5 x SSC (1 X SSC = 0.15 M NaCl, 15 mM sodium citrate, pH 7.2) or additionally in the presence of 50% formamide, for example 42°C in 5 x SSC, 50% formamide. Advantageously, the hybridization conditions for DNA:DNA hybrids are 0.1 x SSC and temperatures between about 20°C to 45°C, preferably between about 30°C to 45°C. For DNA:RNA hybrids the hybridization conditions are advantageously 0.1 x SSC and temperatures between about 30°C to 55°C, preferably between about 45°C to 55°C. These stated temperatures for hybridization are examples of calculated melting temperature values for a nucleic acid with a length of approx. 100 nucleotides and a G + C content of 50% in the absence of formamide. The experimental conditions for DNA hybridization are described in relevant genetics textbooks, for example Sambrook et al., 1989, and can be calculated using formulae that are known by a person skilled in the art, for example depending on the length of the nucleic acids, the type of hybrids or the G + C content. A person skilled in the art can obtain further information on hybridization from the following textbooks: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

"Hybridization" can in particular be carried out under stringent conditions. Such hybridization conditions are for example described in Sambrook, J., Fritsch, E.F., Maniatis, T., in: Molecular Cloning (A Laboratory Manual), 2nd edition, Cold Spring Harbor Laboratory Press, 1989, pages 9.31-9.57 or in Current Protocols in Molecular Biology, John Wiley & Sons, N.Y. (1989), 6.3.1-6.3.6.

"Stringent" hybridization conditions mean in particular: Incubation at 42°C overnight in a solution consisting of 50% formamide, 5 x SSC (750 mM NaCl, 75 mM tri-sodium citrate), 50 mM sodium phosphate (pH 7.6), 5x Denhardt Solution, 10% dextran sulfate and 20 g/ml denatured, sheared salmon sperm DNA, followed by washing of the filters with 0.1x SSC at 65°C.

The invention also relates to derivatives of the concretely disclosed or derivable nucleic acid sequences.

Thus, further nucleic acid sequences according to the invention can be derived from the sequences specifically disclosed herein and can differ from it by addition, substitution, insertion or deletion of individual or several nucleotides, and furthermore code for polypeptides with the desired profile of properties.

The invention also encompasses nucleic acid sequences that comprise so-called silent mutations or have been altered, in comparison with a concretely stated sequence, according to the codon usage of a special original or host organism, as well as naturally occurring variants, e.g. splicing variants or allelic variants, thereof.

It also relates to sequences that can be obtained by conservative nucleotide substitutions (i.e. the amino acid in question is replaced by an amino acid of the same charge, size, polarity and/or solubility).

The invention also relates to the molecules derived from the concretely disclosed nucleic acids by sequence polymorphisms. These genetic polymorphisms can exist between individuals within a population owing to natural variation. These natural variations usually produce a variance of 1 to 5% in the nucleotide sequence of a gene.

Derivatives of nucleic acid sequences according to the invention mean for example allelic variants, having at least 60% homology at the level of the derived amino acid, preferably at least 80% homology, quite especially preferably at least 90% homology over the entire sequence range (regarding homology at the amino acid level, reference should be made to the details given above for the polypeptides). Advantageously, the homologies can be higher over partial regions of the sequences.

Furthermore, derivatives are also to be understood to be homologues of the nucleic acid sequences according to the invention, for example animal, plant, fungal or bacterial homologues , shortened sequences, single-stranded DNA or RNA of the coding and noncoding DNA sequence. For example, homologues have, at the DNA level, a homology of at least 40%, preferably of at least 60%, especially preferably of at least 70%, quite especially preferably of at least 80% over the entire DNA region given in a sequence specifically disclosed herein.

Moreover, derivatives are to be understood to be, for example, fusions with promoters. The promoters that are added to the stated nucleotide sequences can be modified by at least one nucleotide exchange, at least one insertion, inversion and/or deletion, though without impairing the functionality or efficacy of the promoters. Moreover, the efficacy of the promoters can be increased by altering their sequence or can be exchanged completely with more effective promoters even of organisms of a different genus.

### 3.3 Preparation of functional mutants

The skilled reader is also aware of methods of generating functional mutants of particular sequences disclosed herein.

Depending on the technique applied, a skilled reader may generate arbitrary or directed mutations in genes or non-coding nucleic acid regions (which, for example, may be of importance for regulating gene expression) and, afterwards, may generate suitable gene libraries. The molecular biological method required therefore all well known in the art, and, for example, described by Sambrook and Russell, Molecular Cloning. 3. Edition, Cold Spring Harbor Laboratory Press 2001.

Methods of modifying genes and consequently of modifying the encoded proteins are well known to the skilled reader, as for example
- site-specific mutagenesis wherein single or multiple nucleotides of a gene are specifically replaced (Trower MK (Ed.) 1996; In vitro mutagenesis protocols. Humana Press, New Jersey),
- saturation mutagenesis, wherein at any position of a gene the codon of any amino acid may be exchanged or added (Kegler-Ebo DM, Docktor CM, DiMaio D (1994) Nucleic Acids Res 22:1593; Barettino D, Feigenbutz M, Valcárel R, Stunnenberg HG (1994) Nucleic Acids Res 22:541; Barik S (1995) Mol Biotechnol 3:1),
- error-prone polymerase chain reaction( PCR), wherein nucleotide sequences are mutated via the action of an incorrectly functioning DNA-polymerase (Eckert KA, Kunkel TA (1990) Nucleic Acids Res 18:3739);
- SeSaM method (Sequence Saturation Method), wherein preferred substitutions are avoided by the polymerase (Schenk et al., Biospektrum, Vol. 3, 2006, 277-279)
- Passaging of genes in mutator-strains, showing an increased occurrence of mutations of nucleotide sequences, for example in view of a defective DNA-repair mechanism (Greener A, Callahan M, Jerpseth B (1996) An efficient random mutagenesis technique using an E.coli mutator strain. In: Trower MK (Hrsg.) In vitro mutagenesis protocols. Humana Press, New Jersey), or
- DNA-Shuffling, wherein a pool of closely related genes is formed and digested and wherein the fragments are used as templates for a PCR reaction, and wherein full-length mosaic genes are formed (Stemmer WPC (1994) Nature 370:389; Stemmer WPC (1994) Proc Natl Acad Sci USA 91:10747).

By applying the so-called directed evolution technique (see for example Reetz MT und Jaeger K-E (1999), Topics Curr Chem 200:31; Zhao H, Moore JC, Volkov AA, Arnold FH (1999), Methods for optimizing industrial enzymes by directed evolution, In: Demain AL, Davies JE (Hrsg.) Manual of industrial microbiology and biotechnology. American Society for Microbiology) a skilled reader will be able to specifically prepare functional mutants in large scale. In a first step libraries of a specific protein are generated, for example, by applying any one of the above mentioned methods. Afterwards said libraries are expressed, for example by applying bacteria or phage display systems.

Those genes expressing functional mutants showing the desired feature profile may be selected and subjected to further mutation. The steps of mutation and selection or screening may be repeated iteratively until one of the obtained mutants shows the desired feature profile.

By the iterative approach a limited number of mutations, as for example 1 to 5 mutations, may be performed and their influence on the enzyme feature at issue may be evaluated and further improved mutants may be selected stepwise. Said selected mutant may then be subjected to a further mutation in substantially the same may. The number of single mutants to be evaluated may be reduced significantly in this way.

The teaching of the present invention provide important information as regards structure and sequence of the enzyme/ protein at issue, based on which it should be possible to generate further enzymes/proteins with the desired modified feature profile. In particular, so-called hot spots, i.e. sequence regions may be defined, which potentially may be suited for further mutation in order to modify or generate a desired feature of the enzyme/protein.

### 3.4 Constructs used according to the invention

The invention also relates to expression constructs, containing, under the genetic control of regulatory nucleic acid sequences, a nucleic acid sequence coding for a polypeptide or fusion protein according to the invention; as well as vectors comprising at least one of these expression constructs.

"Expression unit" means, according to the invention, a nucleic acid with expression activity, which comprises a promoter as defined herein and, after functional association with a nucleic acid that is to be expressed or a gene, regulates the expression, i.e. the transcription and the translation of this nucleic acid or of this gene. In this context, therefore, it is also called a "regulatory nucleic acid sequence". In addition to the promoter, other regulatory elements may be present, e.g. enhancers.

"Expression cassette" or "expression construct" means, according to the invention, an expression unit, which is functionally associated with the nucleic acid that is to be expressed or the gene that is to be expressed. In contrast to an expression unit, an expression cassette thus comprises not only nucleic acid sequences which regulate transcription and translation, but also the nucleic acid sequences which should be expressed as protein as a result of the transcription and translation.

The terms "expression" or "overexpression" describe, in the context of the invention, the production or increase of intracellular activity of one or more enzymes in a microorganism, which are encoded by the corresponding DNA. For this, it is possible for example to insert a gene in an organism, replace an existing gene by another gene, increase the number of copies of the gene or genes, use a strong promoter or use a gene that codes for a corresponding enzyme with a high activity, and optionally these measures can be combined.

Preferably such constructs according to the invention comprise a promoter 5'-upstream from the respective coding sequence, and a terminator sequence 3'-downstream, and optionally further usual regulatory elements, in each case functionally associated with the coding sequence.

A "promoter", a "nucleic acid with promoter activity" or a "promoter sequence" mean, according to the invention, a nucleic acid which, functionally associated with a nucleic acid that is to be transcribed, regulates the transcription of this nucleic acid.

"Functional" or "operative" association means, in this context, for example the sequential arrangement of one of the nucleic acids with promoter activity and of a nucleic acid sequence that is to be transcribed and optionally further regulatory elements, for example nucleic acid sequences that enable the transcription of nucleic acids, and for example a terminator, in such a way that each of the regulatory elements can fulfill its function in the transcription of the nucleic acid sequence. This does not necessarily require a direct association in the chemical sense. Genetic control sequences, such as enhancer sequences, can also exert their function on the target sequence from more remote positions or even from other DNA molecules. Arrangements are preferred in which the nucleic acid sequence that is to be transcribed is positioned behind (i.e. at the 3' end) the promoter sequence, so that the two sequences are bound covalently to one another. The distance between the promoter sequence and the nucleic acid sequence that is to be expressed transgenically can be less than 200 bp (base pairs), or less than 100 bp or less than 50 bp.

Apart from promoters and terminators, examples of other regulatory elements that may be mentioned are targeting sequences, enhancers, polyadenylation signals, selectable markers, amplification signals, replication origins and the like. Suitable regulatory sequences are described for example in Goeddel, Gene Expression Technology: Methods in Enzymology 185, Academic Press, San Diego, CA (1990).

Nucleic acid constructs according to the invention comprise in particular sequences selected from those, specifically mentioned herein or derivatives and homologues thereof, as well as the nucleic acid sequences that can be derived from amino acid sequences specifically mentioned herein which are advantageously associated operatively or functionally with one or more regulating signal for controlling, e.g. increasing, gene expression.

In addition to these regulatory sequences, the natural regulation of these sequences can still be present in front of the actual structural genes and optionally can have been altered genetically, so that natural regulation is switched off and the expression of the genes has been increased. The nucleic acid construct can also be of a simpler design, i.e. without any additional regulatory signals being inserted in front of the coding sequence and without removing the natural promoter with its regulation. Instead, the natural regulatory sequence is silenced so that regulation no longer takes place and gene expression is increased.

A preferred nucleic acid construct advantageously also contains one or more of the aforementioned enhancer sequences, functionally associated with the promoter, which permit increased expression of the nucleic acid sequence. Additional advantageous sequences, such as other regulatory elements or terminators, can also be inserted at the 3' end of the DNA sequences. One or more copies of the nucleic acids according to the invention can be contained in the construct. The construct can also contain other markers, such as antibiotic resistances or auxotrophy-complementing genes, optionally for selection on the construct.

Examples of suitable regulatory sequences are contained in promoters such as cos-, tac-, trp-, tet-, trp-tet-, Ipp-, lac-, Ipp-lac-, lacl^{q-,} T7-, T5-, T3-, gal-, trc-, ara-, rhaP (rhaP_{BAD})SP6-, lambda-P_{R}- or in the lambda-P_{L} promoter, which find application advantageously in Gram-negative bacteria. Other advantageous regulatory sequences are contained for example in the Gram-positive promoters ace, amy and SP02, in the yeast or fungal promoters ADC1, MFalpha, AC, P-60, CYC1, GAPDH, TEF, rp28, ADH. Artificial promoters can also be used for regulation.

For expression, the nucleic acid construct is inserted in a host organism advantageously in a vector, for example a plasmid or a phage, which permits optimum expression of the genes in the host. In addition to plasmids and phages, vectors are also to be understood as meaning all other vectors known to a person skilled in the art, e.g. viruses, such as SV40, CMV, baculovirus and adenovirus, transposons, IS elements, phasmids, cosmids, and linear or circular DNA. These vectors can be replicated autonomously in the host organism or can be replicated chromosomally. These vectors represent a further embodiment of the invention.

Suitable plasmids are, for example in *E. coli,* pLG338, pACYC184, pBR322, pUC18, pUC19, pKC30, pRep4, pHS1, pKK223-3, pDHE19.2, pHS2, pPLc236, pMBL24, pLG200, pUR290, pIN-III¹¹³-B1, λgt11 or pBdCl; in nocardioform actinomycetes pJAM2; in *Streptomyces* plJ101, pIJ364, pIJ702 or pIJ361; in bacillus pUB110, pC194 or pBD214; in *Corynebacterium* pSA77 or pAJ667; in fungi pALS1, pIL2 or pBB116; in yeasts 2alphaM, pAG-1, YEp6, YEp13 or pEMBLYe23 or in plants pLGV23, pGHlac⁺, pBIN19, pAK2004 or pDH51. The aforementioned plasmids represent a small selection of the possible plasmids. Other plasmids are well known to a person skilled in the art and will be found for example in the book Cloning Vectors (Eds. Pouwels P.H. et al. Elsevier, Amsterdam-New York-Oxford, 1985, ISBN 0 444 904018). Further suitable plasmids are also mentioned in the experimental part.

In a further embodiment of the vector, the vector containing the nucleic acid construct according to the invention or the nucleic acid according to the invention can be inserted advantageously in the form of a linear DNA in the microorganisms and integrated into the genome of the host organism through heterologous or homologous recombination. This linear DNA can comprise a linearized vector such as plasmid or just the nucleic acid construct or the nucleic acid according to the invention.

For optimum expression of heterologous genes in organisms, it is advantageous to alter the nucleic acid sequences in accordance with the specific codon usage employed in the organism. The codon usage can easily be determined on the basis of computer evaluations of other, known genes of the organism in question.

The production of an expression cassette according to the invention is based on fusion of a suitable promoter with a suitable coding nucleotide sequence and a terminator signal or polyadenylation signal. Common recombination and cloning techniques are used for this, as described for example in T. Maniatis, E.F. Fritsch and J. Sambrook, Molecular Cloning: A Laboratory Manual, Cold Spring Harbor Laboratory, Cold Spring Harbor, NY (1989) as well as in T.J. Silhavy, M.L. Berman and L.W. Enquist, Experiments with Gene Fusions, Cold Spring Harbor Laboratory, Cold Spring Harbor, NY (1984) and in Ausubel, F.M. et al., Current Protocols in Molecular Biology, Greene Publishing Assoc. and Wiley Interscience (1987).

The recombinant nucleic acid construct or gene construct is inserted advantageously in a host-specific vector for expression in a suitable host organism, to permit optimum expression of the genes in the host. Vectors are well known to a person skilled in the art and will be found for example in "Cloning Vectors" (Pouwels P.H. et al., Publ. Elsevier, Amsterdam-New York-Oxford, 1985).

### 3.5 Hosts that can be used according to the invention

Depending on the context, the term "microorganism" means the starting microorganism (wild-type) or a genetically modified microorganism according to the invention, or both.

The term "wild-type" means, according to the invention, the corresponding starting microorganism, and need not necessarily correspond to a naturally occurring organism.

By means of the vectors according to the invention, recombinant microorganisms can be produced, which have been transformed for example with at least one vector according to the invention and can be used for the fermentative production according to the invention.

Advantageously, the recombinant constructs according to the invention, described above, are inserted in a suitable host system and expressed. Preferably, common cloning and transfection methods that are familiar to a person skilled in the art are used, for example co-precipitation, protoplast fusion, electroporation, retroviral transfection and the like, in order to secure expression of the stated nucleic acids in the respective expression system. Suitable systems are described for example in Current Protocols in Molecular Biology, F. Ausubel et al., Publ. Wiley Interscience, New York 1997, or Sambrook et al. Molecular Cloning: A Laboratory Manual. 2nd edition, Cold Spring Harbor Laboratory, Cold Spring Harbor Laboratory Press, Cold Spring Harbor, NY, 1989.

The host organism or host organisms according to the invention preferably contain at least one of the nucleic acid sequences, nucleic acid constructs or vectors described in this invention, which code for an enzyme activity according to the above definition.

### 3.6 Enzymatic production of products of the invention

The reaction may be run in the substrate, if liquid, as reaction medium, containing 0,1 mM to 1 M of water, or in excess of water (1 M to 55,5 M).

The reaction mixture may be homogenous or non-homogenous and may contain two or more liquid phases or liquid and solid phases.

An organic co-solvent may be added in a suitable proportion of from 0,1 to 80 vol.-%. Optionally the substrate may be pre-dissolved in the organic co-solvent and then be added to the aqueous medium. The reaction mixture may be agitated or shaken at a suitable intensity, at a suitable temperature in the range of 10 to 80, 10 to 50, or 25 to 40 like 25 to 30 °C for a suitable period of time. After sufficient reaction time, as for example 1 to 120 h or 10 to 24 h the products may be extracted.

An aliquot of at least one alcohol dehydrogenase and transaminase enzyme as defined above is provided in dissolved or dispersed, for example immobilized form, in a proper protein concentration for performing the biotransformations, as for example in the range of 1 mg/l to 10 g/l or 10 to 300 or 75 to 125 µg/ml.

The medium may further contain the sacrificial alpha amino acid (for example in a concentration in the range of about 0,01 to 10 mM). A source of ammonia, as for example an ammonium salt, like ammonium chloride, may be present (in equimolar amount or in molar excess to the substrate.) which may serve as a buffer itself.

Ideally, only ammonia, an ammonia salt and a sacrificial amino acid are added to the enzymes the cofactor(s) and the substrate(s).

However, the reaction may also be run in a buffered system. Thus, the reaction can also be performed in buffered reaction medium containing a suitable buffer, as for example Tris-HCl buffer, MOPS, HIPES , or HEPES buffer (for example 0,01 to 0,1 M) in a pH within the range of 6 to 11, 7 to 10, or 7.5 to 9.5, containing the substrate in a suitable concentration (about 0,1 to 100 mM) and the cofactor (NADH) (in a proper concentration range, like 0,1 to 100 mM).

For cofactor-recycling an aliquot of required enzyme (i.p. of the alpha amino acid dehydrogenase), may be added to the reaction mixture, in dissolved or dispersed, as for example immobilized form and at a suitable proportion (for example in range of 1 to 50 or 5 to 15 or 10 U/ml) together with the co-substrate (like glucose) also in proper concentration (like 0.1 to 100 mM) to drive the recycling reaction, which is preferably coupled to the principal redox reaction.

### 3.7 Fermentative production of products of the invention

The invention also relates to methods for the fermentative production of compounds of primary amines, in particular of formula (I).

The recombinant microorganisms as used according to the invention can be cultivated continuously or discontinuously in the batch process or in the fed batch or repeated fed batch process. A review of known methods of cultivation will be found in the textbook by Chmiel (Bioprozesstechnik 1. Einführung in die Bioverfahrenstechnik (Gustav Fischer Verlag, Stuttgart, 1991)) or in the textbook by Storhas (Bioreaktoren und periphere Einrichtungen (Vieweg Verlag, Braunschweig/Wiesbaden, 1994)).

The culture medium that is to be used must satisfy the requirements of the particular strains in an appropriate manner. Descriptions of culture media for various microorganisms are given in the handbook "Manual of Methods for General Bacteriology" of the American Society for Bacteriology (Washington D. C., USA, 1981).

These media that can be used according to the invention generally comprise one or more sources of carbon, sources of nitrogen, inorganic salts, vitamins and/or trace elements.

Preferred sources of carbon are sugars, such as mono-, di- or polysaccharides. Very good sources of carbon are for example glucose, fructose, mannose, galactose, ribose, sorbose, ribulose, lactose, maltose, sucrose, raffinose, starch or cellulose. Sugars can also be added to the media via complex compounds, such as molasses, or other by-products from sugar refining. It may also be advantageous to add mixtures of various sources of carbon. Other possible sources of carbon are oils and fats such as soybean oil, sunflower oil, peanut oil and coconut oil, fatty acids such as palmitic acid, stearic acid or linoleic acid, alcohols such as glycerol, methanol or ethanol and organic acids such as acetic acid or lactic acid.

Sources of nitrogen are usually organic or inorganic nitrogen compounds or materials containing these compounds. Examples of sources of nitrogen include ammonia gas or ammonium salts, such as ammonium sulfate, ammonium chloride, ammonium phosphate, ammonium carbonate or ammonium nitrate, nitrates, urea, amino acids or complex sources of nitrogen, such as corn-steep liquor, soybean flour, soybean protein, yeast extract, meat extract and others. The sources of nitrogen can be used separately or as a mixture.

Inorganic salt compounds that may be present in the media comprise the chloride, phosphate or sulfate salts of calcium, magnesium, sodium, cobalt, molybdenum, potassium, manganese, zinc, copper and iron.

Inorganic sulfur-containing compounds, for example sulfates, sulfites, dithionites, tetrathionates, thiosulfates, sulfides, but also organic sulfur compounds, such as mercaptans and thiols, can be used as sources of sulfur.

Phosphoric acid, potassium dihydrogenphosphate or dipotassium hydrogenphosphate or the corresponding sodium-containing salts can be used as sources of phosphorus.

Chelating agents can be added to the medium, in order to keep the metal ions in solution. Especially suitable chelating agents comprise dihydroxyphenols, such as catechol or protocatechuate, or organic acids, such as citric acid.

The fermentation media used according to the invention may also contain other growth factors, such as vitamins or growth promoters, which include for example biotin, riboflavin, thiamine, folic acid, nicotinic acid, pantothenate and pyridoxine. Growth factors and salts often come from complex components of the media, such as yeast extract, molasses, corn-steep liquor and the like. In addition, suitable precursors can be added to the culture medium. The precise composition of the compounds in the medium is strongly dependent on the particular experiment and must be decided individually for each specific case. Information on media optimization can be found in the textbook "Applied Microbiol. Physiology, A Practical Approach" (Publ. P.M. Rhodes, P.F. Stanbury, IRL Press (1997) p. 53-73, ISBN 0 19 963577 3). Growing media can also be obtained from commercial suppliers, such as Standard 1 (Merck) or BHI (Brain heart infusion, DIFCO) etc.

All components of the medium are sterilized, either by heating (20 min at 2.0 bar and 121 °C) or by sterile filtration. The components can be sterilized either together, or if necessary separately. All the components of the medium can be present at the start of growing, or optionally can be added continuously or by batch feed.

The temperature of the culture is normally between 15°C and 45°C, preferably 25°C to 40°C and can be kept constant or can be varied during the experiment. The pH value of the medium should be in the range from 5 to 8.5, preferably around 7.0. The pH value for growing can be controlled during growing by adding basic compounds such as sodium hydroxide, potassium hydroxide, ammonia or ammonia water or acid compounds such as phosphoric acid or sulfuric acid. Antifoaming agents, e.g. fatty acid polyglycol esters, can be used for controlling foaming. To maintain the stability of plasmids, suitable substances with selective action, e.g. antibiotics, can be added to the medium. Oxygen or oxygen-containing gas mixtures, e.g. the ambient air, are fed into the culture in order to maintain aerobic conditions. The temperature of the culture is normally from 20°C to 45°C. Culture is continued until a maximum of the desired product has formed. This is normally achieved within 10 hours to 160 hours.

The cells can be disrupted optionally by high-frequency ultrasound, by high pressure, e.g. in a French pressure cell, by osmolysis, by the action of detergents, lytic enzymes or organic solvents, by means of homogenizers or by a combination of several of the methods listed.

The enzyme activities required for performing the intended biotransformation (with or without regeneration of cofactor and/ or sacrificial amino acid) may be expressed, i.e. provided by one singe or two or more, in particular two or three recombinant microorganisms.

Vectors and methods adapted to the preparation of recombinant microorganisms carrying and expressing one or more, or even all enzyme activities required for performing the method of the invention are known in the art e.g. for one gene: pET-system, Novagen or pBAD-system, Invitrogen; Plasmid-system for two genes e.g. pACYC-Duet1, Novagen)

For example, to amplify an aminotransferase gene, a forward which contains an Ndel restriction site and a reverse primer which contains an Xhol site are used in a PCR reaction to amplify the complete coding sequence. The amplified product is isolated and cloned using the Ndel and Xhol sites of the pET28b+ plasmid.

After transformation into *E*. *coli* strain BL21(DE3), transformed cells are grown in a rich medium such as LB (Luria broth), TB (Terrific broth) or TBS medium (Terrific Broth Sorbitol. Protein expression is induced and growth of the cells is continued, after which cells are harvested by centrifugation and cell-free extract is prepared by sonication. Such a cell-free extract serves as a source of enzyme for biocatalytic reactions.

### 3.8 Enzyme immobilization

If an enzyme as sued according to the invention is, immobilised, it is attached to an inert carrier. Suitable carrier materials are known in the art and are, e.g., disclosed in EP-A-1149849, EP-A-1 069 183 and DE-OS 100193773 as well as the literature references cited therein (all of which are specifically enclosed with regard to carrier materials). Examples for suitable carrier materials are clays, clay minerals such as kaolinite, diatomeceous erth, perlite, silica, alumina, sodium carbonate, calcium carbonate, cellulose powder, anion exchanger materials, synthetic polymers, such as polystyrene, acrylic resins, phenol formaldehyde resins, polyurethanes and polyolefines, such as polyethylene and polypropylene. For preparing carrier-bound enzymes the carrier materials usually are used in the form of fine powders, wherein porous forms are preferred. The particle size of the carrier material usually does not exceed 5 mm, in particular 2 mm. In case the at least one enzyme is present in a whole-cell-preparation, said whole-cell-preparation may be present in a free or immobilised form. Suitable carrier materials are e.g. Ca-alginate or Carrageenan. Enzymes as well as cells may directly be linked by glutaraldehyde. A wide range of immobilisation methods is known in the art (e.g. J. Lalonde and A. Margolin "Immobilization of Enzymes" in K. Drauz und H. Waldmann, Enzyme Catalysis in Organic Synthesis 2002, Vol.III, 991-1032, Wiley-VCH, Weinheim).

### 3.9 Product isolation

The methodology of the present invention can further include a step of recovering compounds as produced according to the invention,. The term "recovering" includes extracting, harvesting, isolating or purifying the compound from culture or reaction media. Recovering the compound can be performed according to any conventional isolation or purification methodology known in the art including, but not limited to, treatment with a conventional resin (e.g., anion or cation exchange resin, non-ionic absorption resin, etc.), treatment with a conventional adsorbant (e.g., activated charcoal, silicic acid, silica gel, cellulose, alumina, etc.), alteration of pH, solvent extraction (e.g., with a conventional solvent such as an alcohol, ethyl acetate, hexane and the like), distillation, dialysis, filtration, concentration, crystallization, recrystallization, pH adjustment, lyophilization and the like.

### 4. Non-limiting examples of primary amines

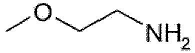

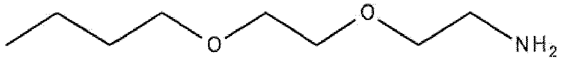

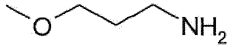

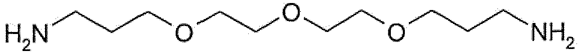

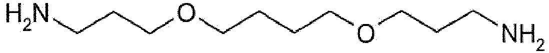

and the corresponding mono amines of the above diamines.

Examples of polyhydrocarbylene ether mono- and diamines are: 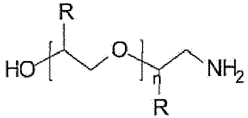 with R = H or methyl and n an integer from 1 to 100, 1 to 50, 1 to 20 or 1 to 10; 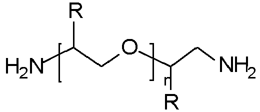 with R = H or methyl and n an integer from 1 to 100, 1 to 50, 1 to 20 or 1 to 10 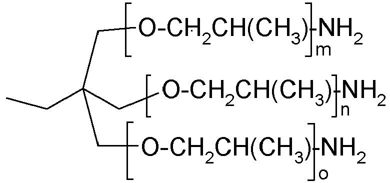 with m, n and o same or different and being an integer from 1 to 50, 1 to 20 or 1 to 10 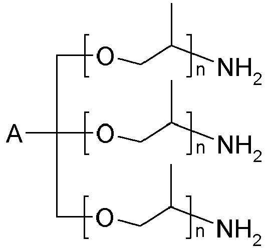 with A being H or methyl, and n being an integer from 1 to 50, 1 to 20 or 1 to 10;

### Experimental Part

Unless otherwise stated the following experiments have been performed by applying standard equipment, methods, chemicals, and biochemicals as used in genetic engineering, fermentative production of chemical compounds by cultivation of microorganisms and in the analysis and isolation of products. See also Sambrook et al, and Chmiel et al as cited herein above.

### Materials and methods

### a) General Methods

Protein concentrations were determined using the Uptima BC assay protein purification kit (Interchim, Montlucon, France) with bovine serum albumin as standard. SDS-PAGE was done in a Mini-Sub Cell GT (BioRad, Munich). Proteins were stained with SimpIyBlue™ Safe Stain (Invitrogen, Carlsbad, CA, USA).

Thin layer chromatography was performed using TLC plates and a 3:1:1 mixture of 1-butanol, acetic acid and water. The plates were stained by application of ninhydrine staining solution (0.2 g Ninhydrine, 0.5 ml acetic acid,100 ml 1-butanol, 4.5 ml water) and heat.

LC-MS-analysis was performed on a LCQ Fleet system (Thermo-Fisher, Landsmeer, The Netherlands) coupled to a Finnigan Surveyor HPLC-system (Thermo-Fisher, Landsmeer, The Netherlands). The separation was performed on a Alltima HP C18-column (3 pm, 100 mm length, ID 2.1 mm) using following gradient of 0.1% formic acid in water (eluent A) an 0.07% formic acid in acetonitrile (eluent B): 0-2 min, 100% A; 2-32 min gradient to 20% A and 80% B; 32-37 min constant 20% A and 80% B; 37-38 min gradient back to 100% A; 38-48 min constant 100% A. Detection was performed using the MS with electron spray ionisation and detection in positive mode.

All the enzymes were purified by one step using immobilized metal affinity chromatography (IMAC, HisTrap HP column 5 ml, GE Healthcare) using an Äkta-purifier (GE Healthcare, Munich, Germany). First, the cells expressing the desired protein were obtained by centrifugation and disrupted by sonication at 4 °C followed by centrifugation for 45 min at 15,000 rpm to obtain cell-free extracts (CFEs). The CFE was loaded on the column followed by washing, then the enzyme was eluted at a flow rate of 1 ml/min with 15 column volumes of a linear gradient of 0-0.5 M imidazole in a buffer containing 20 mM Tris-HCl, pH 8.0, 0.5 M NaCl, and 0.01% (vol/vol) β-mercaptoethanol. In the last step the purified solution was desalted against a buffer consisting of 25 mM Tris-HCl, pH 8.0 containing 10% glycerol. Aliquotes were stored a -20 °C.

Determination of AT activity using OPA-HPLC: To test the enzyme activity, an aminotransferase (AT) assay cocktail was prepared, consisting of 20 mM BDG-Amine, 10 mM pyruvate (or 2-oxoglutarate) and 50 µM PLP in 50 mM MOPS, pH 7.6 (or 50 mM HEPES, pH 8.3). Reactions were started by addition of purified enzyme or CFE and incubated at 30 °C. The conversion was monitored by taking samples at different times. To 50 µl sample, 50 µl 2 M HCl was added to quench the reaction, and the mixture was kept on ice for 5 min. Then, 45 µl 2 M NaOH was added to neutralize the pH and 50 µl of water for dilution. Immediately prior to injection, 1 µl of sample was mixed with 2 µl ortho-phthalaldehyde (OPA) solution and 5 µl of 0.4 M NaB03, pH 10.4, in an HPLC autosampler (Hill et al., 1979, Anal. Chem. 51:1338-1341). The OPA solution was prepared by first dissolving 15 mg OPA in 50 µl absolute ethanol, which then was added to a mixture of 4.42 ml of 0.4 M NaB03, pH 10.4, 15 µl of 30% (wt/vol) Brij 35 and 11 µl of β-mercaptoethanol (99%).

Quantitative analyses of alanine or glutamate produced from BDG-Amine were performed with a C18 OPA Adsorbosphere column connected to a Jasco HPLC system after pre-derivatization of the sample with OPA. Separation was carried out by HPLC using 20 mM sodium acetate, pH 5.5, containing 5% (vol/vol) tetrahydrofuran (THF) as eluent A and acetonitrile as eluent B, with a flow rate of 1 ml/min. Detection was done with a fluorescence detector, using excitation at 350 nm and measuring emission at 450 nm. Eluent A and eluent B were used with a gradient program as follows: 0-5 min, 100:0; 5-12 min, from 100:0 to 80:20; 12-16 min 80:20; 16-24 min from 80:20 to 40:60; 24-28 min 40:60; from 28-30 min 40:60 to 100:0; from 30-35 min re-equilibration at 100:0. Retention times for derivatized L-α-alanine and L-α-glutamate were 7.7 and 2.3 min, respectively. One unit is defined as the amount of enzyme that catalyzes the formation of one µmol of L-α-alanine or L-α-glutamate min⁻¹ at concentrations of 20 mM BDG-Amine and 10 mM pyruvate or 2-oxoglutarate.

### b) Materials

All chemicals were purchased from Fluka-Sigma-Aldrich (Munich, Germany) or Acros Organics (Geel, Belgium). BDG-Aldehyde and BDG-Amine were provided by BASF.

### c) Enzymes

### (1) Transaminases (TA)

- *Vibrio fluvialis* w-TA (AEA39183.1)
- *Chromobacterium violaceum* w-TA (NP_901695)
- *Achromobacter denitrificans* w-TA (AAP92672.1)
- *E. coli* putrescine w-TA (NP_417544)

### Vibrio fluvialis ω-TA

This TA is well characterized (Shin and Kim (2000) J Org Chem 67:2848-2853) having 50 kDa MW, a homodimeric structure and an empty crystal structure is available (3NUI). The enzyme is widely used in biocatalysis. It has a specific activity of 1.2 U/mg (with α-methylbenzylamine), an optimum temperature of 37 °C and an optimum pH of 9. Amino donor substrates are aromatic and aliphatic amines and α-amino acids. Amino acceptor substrates are pyruvate, glyxolate, aliphatic aldehydes (C₃-C₉).

### Chromobacterium violaceum w-TA

This TA has an MW of 45 KDa (Kaulmann et al. (2007) Enzyme Microb Tech 41:628-637), a specific activity of 1.2 U/mg (with α-methylbenzylamine), an optimum temperature of 37 °C and an optimum pH of 7. Amino donor substrates are aromatic and aliphatic amines and α-amino acids. Amino acceptor substrates are pyruvate, glyxolate, aliphatic aldehydes (C₃-C₉).

### Achromobacter denitrificans w-TA

This TA has an MW of 47 KDa (Yun et al. (2004) Appl Environ Microbiol 70:2529-2534), a specific activity of 10.8 U/mg (with α-methylbenzylamine), an optimum temperature of 37 °C and an optimum pH of 9. Amino donor substrates are short-chain aliphatic β-amino acids, aromatic and aliphatic secondary amines. Amino acceptor substrates are pyruvate, glyxolate, aliphatic aldehydes (C₃-C₄).

### E. coli putrescine TA

This is a well studied TA (Samsonova et al. (2003) BMC Microbiol 3:2-2) from *E. coli* with an MW of 50 kDa, a specific activity of 11.7 U/mg (with putrescine), an optimum temperature of 20-80 °C and an optimum pH of 9. Amino donor substrates are aliphatic primary amines and α-amino acids. Amino acceptor substrates are α-ketoglutarate, α-ketobutyrate, pyruvate.

### (2) Alanine dehydrogenases (AlaDH)

### AlaDH from Archeoglobus fulgidus (DSM 4304)

The enzyme is thermostable (Vₘₐₓ at 82 °C), and shows still very high activity at 25 °C; it is 100 % active for more than 3 months at 25 °C; It tolerates high salt concentrations (Smith, M. Mayhew, H. Robinson, A. Heroux, D. Charlton, M. Holden, D. Gallagher, Acta. Crystallogr. D. 2003, 59, 2328-2331; D. Gallagher, H. Monbouquette, I. Schroder, H. Robinson, M. Holden, N. Smith, J Mol Biol 2004, 342, 119-130).

### AlaDH from Thermus thermophilus HB8

The reported activity is similar to B. *subtilis* AlaDH but thermostable; the crystal structure available (Z. Vali, F. Kilar, S. Lakatos, S. A. Venyaminov, P. Závodszky, Biochimica et Biophysica Acta (BBA) - Enzymology 1980, 615, 34-47).

### AlaDH from Vibrio proteolyticus (DSM 30189)

For this enzyme very high activity and stable are reported; it tolerates very high salt concentrations (S. Kato, T. Ohshima, A. Galkin, L. Kulakova, T. Yoshimura, N. Esaki, Journal Of Molecular Catalysis B-Enzymatic 2003, 23, 373-378).

### (3) Alcohol dehydrogeases (AlcDH)

### Rhodococcus jostii RHA1

Holder et al., (2011) PLoS Genet 7:e1002219.

### Geobacillus stearothermophilus (ATCC 12976) Acc. No BAA14411

The enzyme is described by Robinson et al. (1994, Biochim Biophys Acta 218:432-434).

### Geobacillus stearothermophilus (DSM 2334) Acc. No CAA80989

The enzyme is 90 % similar to ATCC 12976 ADH (Robinson et al. (1994) Biochim Biophys Acta 218:432-434).

### Geobacillus thermodenitrificans NG80-2, NAD-dept ADH2 (gi: AB068223.1)

Optimum temperature 60 °C; optimum pH 8.0; 50 % activity retained after 14 h at 60 °C; (Liu et al. (2009) Microbiology 155:2078-2085).

### Pseudomonas putida K23-1. No gene described. N-term. Identical to Alc-DH from P. putida BIRD-1 or TJI-51). ID ADR59556.1, CP002290.

Zn-containing long-chain n-alkanol DH, optimum temperature 50 °C; optimum pH 9.5 (forward reaction); Substrates: 1-octanol (4.6 U/mg), 1-nonanol (4.4 U/mg), 1-decanol (6.7 U/mg), 1-undecanol (4.0 U/mg)) (Nagashima et al. (1996) J Ferment Bioeng 82:328-333).

### d) Cloning of genes

Routine manipulation of DNA, PCR and construction of recombinant plasmids were performed as described in (Sambrook J & Russell DW (2001) Molecular Cloning: a Laboratory Manual, 3rd edn. Cold Spring. Harbor Laboratory Press, Cold Spring Harbor, NY).

### Example 1: Cloning, Expression of transaminases and testing for activity

The genes for w-TA from *C*. *violaceum* DSM30191 (Cv w-TA) and putrescine TA from *E. coli* (*Ec* PuAT) were amplified by PCR and cloned in an pET-based expression vector with *N*-terminal His-tag (Ndel/Xhol). The primers that were used are listed in the Table A.

Synthetic genes for w-TA from *V. fluvialis* JS17 (*Vf* ω-TA) (DNA2.0 Inc) were also cloned in pET-28b+ with *N*-terminal His-tag (Ndel/Xhol).

A very good expression in *E. coli* C41 (DE3) for all proteins (mostly soluble) was observed. (see Figure 2: SDS Gel for *Cv* ω-TA and *Ec* PuAT expression)

The *Cv* ω-TA, *Vf* ω-TA and PuAT were produced at 1.5 I scale; all enzymes were purified to homogeneity with yields of 200 mg/l, 160 mg/l and 80 mg/l, respectively.

Initial (deamination) tests were performed with the amine target substrate (BDG-amine) and pyruvate. Additionally, two ω-TAs (TA95 and 96) were purchased from Sigma and tested.

### Reaction conditions:

- 20 mM BDG-amine
- 10 mM pyruvate/ α-ketoglutarate^{#}
- 50 mM MOPS (pH 7.6) or 50 mM HEPES (pH 8.3)
- enz: 35-200 µg
- volume: 0.5 ml
- temperature: 30°C

### Results:

| **enzyme** | **time (h)** | **conversion (%)** | **specific activity (mU/mg)** |
|---|---|---|---|
| *Cv* ω-TA | 16 | 27 | 252 |
| *Vf* ω-TA | 16 | 25 | 441 |
| PuAT | 16 | 16 | 7 |
| ω-TA 96 | 16 | 8.5 | nt |
| ω-TA 95 | 16 | < 0.4 | nt |
| nt = not tested | | | |

### Example 2: Cloning, Expression of alcohol dehydrogeanses and testing for activity

The genes for alcohol dehydrogenases from *Geobacillus stearothermophilus* DSM 297 (*Gs ADH*), *Rhodococcus jostii* RHA1 (*Rj* ADH) were amplified by PCR and cloned in an pET-based expression vector with *N*-terminal His-tag (restriction sites: Ndel/Xhol and Ndel/Hindlll, respectively). The primers that were used are listed in the Table A

Synthetic genes for *Geobacillus thermodenitrificans* NG80-2 (*Gt* ADH) and *Pseudomonas putida* BIRD-1 (*Pp* ADH) (DNA2.0 Inc) were also cloned in pET-28b+ with *N*-terminal His-tag (Ndel/Hindlll).

A good expression in *E. coli* C41 (DE3) for all ADHs (mostly soluble except maybe for the one from *R. jostii*) was observed. (see Figure 2b: SDS Gel for overexpression of *Gs*, *Rj*, *Pp* and *Gt* ADHs.

The *Gs, Rj, Gt and Pp* ADH were purified using the His-tag for metal affinity chromatography. The initial activity of the oxidation of BDG-Alcohol to BDG-Aldehyde was monitored spectrophotometrically using the absorption of the produced NADH at 340 nm. The background activity of the enzymes, producing NADH without BDG-Alcohol present was substracted from the observed activities.

Additionally the commercially horse liver ADH (HL ADH) purchased from Sigma-Aldrich was tested.

Reaction conditions:
- 10 mM BDG-Alcohol
- 50 mM Bicine-buffer (pH 8.8)
- 10 mM NAD⁺
- Enzyme concentration 0.1-0.8 mg/ml
- Reaction volume: 0.5 ml

### Results:

| | specific activity (U/mg) |
|---|---|
| *Gs* ADH | **0.039** |
| *Gt* ADH | < 0.001 |
| *Rj* ADH | < 0.001 |
| *Pp* ADH | 0.012 |
| HL ADH | 0.003 |

In initial experiments with HL ADH, LC-MS analysis was used to identify that BDG-Aldehyde was formed (Figure 3 A).

### Example 3: Alcohol to amine conversion

The following amination reaction was further investigated: 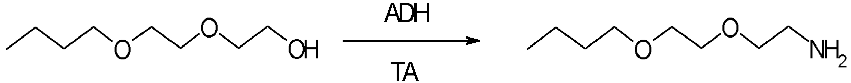 Cv w-TA was chosen for further study.
a) The conversion of BDG-alcohol to BDG-aldehyde by Alc-DH was confirmed by LC-MS (Figure 3A, see Example 2). The activities of different ADH were tested and Gs ADH was identified to be the most active ADH tested.
b) The conversion of BDG-aldehyde to BDG-amine by ω-TAs was confirmed by LC-MS (Figure 3 B) and thin layer chromatography (Figure 4, lane 5). It could also be observed that BDG-Aldehyde can react with the formed BDG-Amine to form multimers. Therefore, the accumulation of BDG-Aldehyde should be avoided. This can be achieved by using higher transaminase activities and less ADH activity.

### Reaction conditions:

- ~10 mM BDG-Aldehyde (very low solubility, used an emulsion)
- 10 mM L-alanine
- 20 mM bicine-buffer (pH 8.8)
- *Cv* ω-TA (0.75 mg)
- Total volume: 1 ml

c) The conversion of BDG-alcohol to BDG-amine by ADH + ω-TAs was confirmed by LC-MS (Figure 3 C) and thin layer chromatography (Figure 4, lanes 1-3). Only very little or no BDG-aldehyde was accumulating in the reaction mixture since the used HL ADH was less active compared to the ω-TAs.

### Reaction conditions

- 10 mM BDG-Alcohol
- 10 mM L-alanine
- 10 mM NHD⁺
- 50 mM bicine-buffer (pH 8.8)
- 1.5 mg ADH
- *Cv* w-TA (0.75 mg)
- Total volume: 1 ml

**Table A: List of SEQ ID Nos**

| Assignment of SEQ ID NOs: | | | |
|---|---|---|---|
| SEQ ID NO: | Description | Organism | Type |
| 1 | Vf-ωTA | *Vibrio fluvialis* | NS |
| 2 | Vf-ωTA; codon optimized | *Vibrio fluvialis* | NS |
| 3 | Vf-ωTA | *Vibrio fluvialis* | AS |
| 4 | Vf-ωTA; His₆-tag | *Vibrio fluvialis* | AS |
| 5 | Cv-ωTA | *Chromobacterium violaceum* | NS |
| 6 | Cv-ωTA | *Chromobacterium violaceum* | AS |
| 7 | Cv-ωTA; His₆-tag | *Chromobacterium violaceum* | AS |
| 8 | Cv-ωTA-Fw; PCR Primer | GCGCGCATATGCAGAAGCAACGTACGACCAGCCAAT | NS |
| 9 | Cv-ωTA-Rv; PCR Primer | GATATTGTACTCGAGCTAAGCCAGCCCGCGCCTT C | NS |
| 10 | Rj-ADH | *Rhodococcus jostii* | NS |
| 11 | Rj-ADH | *Rhodococcus jostii* | AS |
| 12 | Rj-ADH; His₆-tag | *Rhodococcus jostii.* | AS |
| 13 | Rj-ADH-Fw; PCR Primer | GCGCGCATATGAAGACCAAAGCAGCGGTTCTG | NS |
| 14 | Rj-ADH-Rv; PCR Primer | GAGCTAAGCTTCTACTGCTGGTGGACGATCACG | NS |
| 15 | Gs-ADH1 | *Geobacillus stearothermophilus* | NS |
| 16 | Gs-ADH1 | *Geobacillus stearothermophilus* | AS |
| 17 | Gs-ADH1; His₆-tag | *Geobacillus stearothermophilus* | AS |
| 18 | Gs-ADH1-Fw; PCR-Primer | GCTTGACCATATGAAAGCTGCAGTTGTGGAAC | NS |
| 19 | Gs-ADH1-Rv; PCR Primer | GGATCGCTCGAGTTAATCTACTTTTAACACGAC | NS |
| 20 | Gs-ADH2 | *Geobacillus stearothermophilus* | NS |
| 21 | Gs-ADH2 | *Geobacillus stearothermophilus* | AS |
| 22 | Gs-ADH2; His₆-tag | *Geobacillus stearothermophilus* | AS |
| 23 | Gs-ADH2-Fw; PCR Primer | GCTTGACCATATGAAAGCAGCAGTAGTTAACG | NS |
| 24 | Gs-ADH2-Rv; PCR Primer | GGATCGCTCGAGTCAATCCTCCTTCAATTTTAGTACG | NS |
| 25 | Pp-ADH | *Pseudomonas putida* | NS |
| 26 | Pp-ADH; codon optimized | *Pseudomonas putida* | NS |
| 27 | Pp-ADH | *Pseudomonas putida* | AS |
| 28 | Pp-ADH; His₆-tag | *Pseudomonas putida* | AS |
| 29 | Gt-ADH | *Geobacillus thermodenitrificans* | NS |
| 30 | Gt-ADH; codon optimized | *Geobacillus thermodenitrificans* | NS |
| 31 | Gt-ADH | *Geobacillus thermodenitrificans* | AS |
| 32 | Gt-ADH; His₆-tag | *Geobacillus thermodenitrificans* | AS |
| 33 | Af-AlaDH | *Archaeoglobus fulgidus* | NS |
| 34 | Af-AlaDH | *Archaeoglobus fulgidus* | AS |
| 35 | Af-AlaDH; His₆-tag | *Archaeoglobus fulgidus* | AS |
| 36 | Af-AADH-Fw; PCR Primer | CGCGCGGCAGCCATATGGAGACTCTTATTTTGACTC | NS |
| 37 | Af-ADDH-Rv; PCR Primer | GTGCGGCCGCAAGCTTTTATATCCTGAAAAACTTTATT TTAC | NS |
| 38 | Tt-AlaDH | *Thermus thermophilus* | NS |
| 39 | Tt-AlaDH | *Thermus thermophilus* | AS |
| 40 | Tt-AlaDH; His₆-tag | *Thermus thermophilus* | AS |
| 41 | Tt-AADH-Fw; PCR Primer | CGCGCGGCAGCCATATGGTGATCGGCGTGC | NS |
| 42 | Tt-AADH-Rv; PCR Primer | GTGCGGCCGCAAGCTTTTACCCCCTCAAGGCCTCC | NS |
| 43 | Vp-AlaDH | *Vibrio proteolyticus* | NS |
| 44 | Vp-AlaDH | *Vibrio proteolyticus* | AS |
| 45 | Vp-AlaDH; His₆-tag | *Vibrio proteolyticus* | AS |
| 46 | Vp-AADH-Fw; PCR Primer | CGCGCGGCAGCCATATGATCATTGGCGTACC | NS |
| 47 | Vp-AADH-Rv; PCR Primer | GTGCGGCCGCAAGCTTTTAGTTGAACATGGCG | NS |
| 48 | Bs-GluDH | *Bacillus subtilis* | NS |
| 49 | Bs-GluDH | *Bacillus subtilis* | AS |
| 50 | Pi-GluDH | *Pyrobaculum islandicum* | NS |
| 51 | Pi-GluDH | *Bacillus subtilis* | AS |
| 52 | Cs-GluDH | *Clostridium symbiosum* | NS |
| 53 | Cs-GluDH | *Clostridium symbiosum* | AS |
| 54 | Tp-GluDH | *Thermococcus profundus* | NS |
| 55 | Tp-GluDH | *Thermococcus profundus* | AS |
| 56 | PuAT | *E. coli* K12 | NS |
| 57 | PuAT | *E. coli* K12 | AS |
| 58 | Ec PuAT Fw, PCR Primer | GCGCGCATATGAACAGGTTACCTTCGAGCGCATC | NS |
| 59 | Ec PuAT Rv: PCR Primer | CGCGCGCTCGAGTTACGCTTCTTCGACACTTACTC | NS |
| 60 | Ad-omega-TA | *Achromobacter denitrificans Y2k-2.* | NS |
| 61 | Ad-omega-TA | *Achromobacter denitrificans Y2k-2.* | AS |
| 62 | L-Ornithine AT Isoform 1 | *Homo sapiens* | AS |
| 63 | L-Ornithine AT Isoform 2 | *Homo sapiens* | AS |
| 64 | Ornithine aminotransferase PY00104 | *Plasmodium yoelii* | AS |
| 65 | *Ornithine delta-aminotransferase* | *Plasmodium falciparum* | AS |
| 66 | Ornithine-oxo acid transaminase RocD | *Bacillus anthracis* | AS |
| 67 | L-Lysine 6-transaminase | *Streptomyces clavuligerus ATCC 27064* | NS |
| 68 | L-Lysine 6-transaminase | *Streptomyces clavuligerus ATCC 27064* | AS |
| 69 | L-Lysine 6-transaminase | *Mycobacterium tuberculosis H37Rv* | NS |
| 70 | L-Lysine 6-transaminase | *Mycobacterium tuberculosis H37Rv* | AS |

| | | | |
|---|---|---|---|
| AS = Amino acid sequence NS = Nucleotide sequence | | | |

Documents as cited herein are incorporated by reference

### Annex 1- Sequences of cloned genes

### Transaminases

### Omega transaminase (Vf-ωTA) from Vibrio fluvialis strain JS17

The synthetic gene and codon optimization was ordered at DNA2.0.
GenBank protein ID sequence: AEA39183.1
Uniprot protein ID sequence: F2XBU9

Protein sequence with *N*-terminal His₆-tag and linker (first 20 AAs colored in red):

MGSSHHHHHHSSGLVPRGSHMNKPQSWEARAETYSLYGFTDMPSLHQRGTVVVTH
GEGPYIVDVNGRRYLDANSGLWNMVAGFDHKGLIDAAKAQYERFPGYHAFFGRMSD
QTVMLSEKLVEVSPFDSGRVFYTNSGSEANDTMVKMLWFLHAAEGKPQKRKILTRW
NAYHGVTAVSASMTGKPYNSVFGLPLPGFVHLTCPHYWRYGEEGETEEQFVARLAR
ELEETIQREGADTIAGFFAEPVMGAGGVIPPAKGYFQAILPILRKYDIPVISDEVICGFGR
TGNTWGCVTYDFTPDAIISSKNLTAGFFPMGAVILGPELSKRLETAIEAIEEFPHGFTAS
GHPVGCAIALKAIDVVMNEGLAENVRRLAPRFEERLKHIAERPNIGEYRGIGFMWALE
AVKDKASKTPFDGNLSVSERIANTCTDLGLICRPLGQSVVLCPPFILTEAQMDEMFDKL
EKALDKVFAEVA

GenBank: HQ418483.1 >gi|327207065|gb|HQ418483.1| *Vibrio fluvialis* strain JS17 codon optimized transaminase gene

ATGAATAAACCGCAAAGCTGGGAAGCGCGTGCGGAAACGTACAGCTTGTACGGC
TTTACCGATATGCCGAGCCTGCACCAGCGCGGTACCGTTGTCGTCACGCATGGT
GAGGGCCCGTATATTGTCGATGTGAATGGCCGTCGTTACTTGGATGCGAATTCTG
GTCTGTGGAACATGGTTGCTGGTTTTGATCACAAGGGCCTGATTGACGCGGCGAA
GGCCCAGTATGAGCGTTTTCCGGGTTATCACGCGTTCTTCGGCCGTATGAGCGAC
CAGACCGTCATGTTGAGCGAAAAACTGGTCGAGGTTTCGCCGTTCGACAGCGGT
CGCGTTTTCTATACCAACAGCGGTAGCGAGGCGAACGATACCATGGTTAAGATGC
TGTGGTTCCTGCATGCCGCGGAAGGTAAACCTCAGAAGCGCAAGATTTTGACGCG
CTGGAACGCGTATCACGGTGTCACGGCGGTGAGCGCGAGCATGACGGGTAAACC
GTACAATTCCGTGTTTGGTCTGCCTCTGCCGGGTTTTGTTCACCTGACGTGCCCG
CACTACTGGCGCTACGGCGAAGAGGGTGAGACTGAAGAGCAATTTGTCGCCCGT
CTGGCGCGTGAACTGGAAGAAACCATCCAGCGCGAAGGCGCGGACACTATCGCC

GGTTTCTTTGCCGAGCCGGTTATGGGTGCTGGTGGCGTGATCCCGCCAGCGAAA
GGTTATTTCCAAGCAATCCTGCCGATTCTGCGTAAGTACGACATTCCGGTGATTAG
CGATGAGGTGATCTGTGGCTTCGGTCGTACCGGTAATACCTGGGGTTGTGTGACC
TACGATTTCACCCCAGACGCAATCATCTCTAGCAAAAACCTGACTGCAGGTTTCTT
TCCAATGGGCGCTGTCATCCTGGGTCCGGAGCTGAGCAAGCGTCTGGAAACGGC
CATCGAGGCCATTGAAGAGTTCCCGCACGGTTTCACCGCGAGCGGCCATCCGGT
GGGCTGCGCGATTGCGCTGAAAGCGATTGACGTGGTTATGAACGAAGGTCTGGC
AGAAAACGTCCGTCGTTTGGCACCGCGCTTTGAAGAGCGCCTGAAACATATTGCA
GAGCGTCCGAACATCGGTGAGTACCGTGGCATTGGTTTTATGTGGGCGCTGGAA
GCAGTGAAGGACAAAGCTTCCAAGACCCCGTTCGACGGCAATCTGAGCGTGTCT
GAGCGCATTGCGAATACGTGCACCGATCTGGGCCTGATCTGCCGTCCGTTGGGC
CAATCCGTTGTTCTGTGTCCGCCGTTTATCCTGACCGAGGCACAGATGGACGAGA
TGTTTGATAAACTGGAGAAAGCACTGGACAAGGTGTTCGCTGAGGTTGCCTAA

>gi|327207065|gb|HQ418483.1| *Vibrio fluvialis* strain JS17 transaminase gene, original sequence

ATGAACAAACCGCAAAGCTGGGAAGCCCGGGCCGAGACCTATTCGCTCTATGGTT
TCACCGACATGCCTTCGCTGCATCAGCGCGGCACGGTCGTCGTGACCCATGGCG
AGGGACCCTATATCGTCGATGTGAATGGCCGGCGTTATCTGGACGCCAACTCGG
GCCTGTGGAACATGGTCGCGGGCTTTGACCACAAGGGGCTGATCGACGCCGCCA
AGGCCCAATACGAGCGTTTTCCCGGTTATCACGCCTTTTTCGGCCGCATGTCCGA
TCAGACGGTAATGCTGTCGGAAAAGCTGGTCGAGGTGTCGCCCTTTGATTCGGG
CCGGGTGTTCTATACAAACTCGGGGTCCGAGGCGAATGACACCATGGTCAAGAT
GCTATGGTTCCTGCATGCAGCCGAGGGCAAACCGCAAAAGCGCAAGATCCTGAC
CCGCTGGAACGCCTATCACGGCGTGACCGCCGTTTCGGCCAGCATGACCGGCAA
GCCCTATAATTCGGTCTTTGGCCTGCCGCTGCCGGGCTTTGTGCATCTGACCTGC
CCGCATTACTGGCGCTATGGCGAAGAGGGCGAAACCGAAGAGCAGTTCGTCGCC
CGCCTCGCCCGCGAGCTGGAGGAAACGATCCAGCGCGAGGGCGCCGACACCAT
CGCCGGTTTCTTTGCCGAACCGGTGATGGGCGCGGGCGGCGTGATTCCCCCGG
CCAAGGGGTATTTCCAGGCGATCCTGCCAATCCTGCGCAAATATGACATCCCGGT
CATCTCGGACGAGGTGATCTGCGGTTTCGGACGCACCGGTAACACCTGGGGCTG
CGTGACCTATGACTTTACACCCGATGCAATCATCTCGTCCAAGAATCTTACAGCGG
GCTTTTTCCCCATGGGGGCGGTGATCCTTGGCCCGGAACTTTCCAAACGGCTGG
AAACCGCAATCGAGGCGATCGAGGAATTCCCCCATGGCTTTACCGCCTCGGGCC
ATCCGGTCGGCTGTGCTATTGCGCTGAAAGCAATCGACGTGGTGATGAATGAAGG

GCTGGCTGAGAACGTCCGCCGCCTTGCCCCCCGTTTCGAGGAAAGGCTGAAACA
TATCGCCGAGCGCCCGAACATCGGTGAATATCGCGGCATCGGCTTCATGTGGGC
GCTGGAGGCTGTCAAGGACAAGGCAAGCAAGACGCCGTTCGACGGCAACCTGTC
GGTCAGCGAGCGTATCGCCAATACCTGCACCGATCTGGGGCTGATTTGCCGGCC
GCTTGGTCAGTCCGTCGTCCTTTGTCCGCCCTTTATCCTGACCGAGGCGCAGATG
GATGAGATGTTCGATAAACTCGAAAAAGCCCTTGATAAGGTCTTTGCCGAGGTTG
CCTGA

The gene was then cloned in pET-28b(+) plasmid (Novagen) using Ndel and Xhol restriction sites. A *N*-terminal His₆-tag is present.

**Omega transaminase (Cv-ωTA) from *Chromobacterium violaceum* ATCC 12472 DSM 30191**. The strain was ordered from the DSMZ strain collection, genomic DNA was extracted.
Uniprot protein ID sequence: Q7NWG4
NCBI reference sequence: NC_005085.1

Protein sequence with *N*-terminal His₆-tag and linker (first 20 AAs colored in red):

MGSSHHHHHHSSGLVPRGSHMQKQRTTSQWRELDAAHHLHPFTDTASLNQAGARV
MTRGEGVYLWDSEGNKIIDGMAGLWCVNVGYGRKDFAEAARRQMEELPFYNTFFKT
THPAVVELSSLLAEVTPAGFDRVFYTNSGSESVDTMIRMVRRYWDVQGKPEKKTLIG
RWNGYHGSTIGGASLGGMKYMHEQGDLPIPGMAHIEQPWWYKHGKDMTPDEFGVV
AARWLEEKILEIGADKVAAFVGEPIQGAGGVIVPPATYWPEIERICRKYDVLLVADEVIC
GFGRTGEWFGHQHFGFQPDLFTAAKGLSSGYLPIGAVFVGKRVAEGLIAGGDFNHGF
TYSGHPVCAAVAHANVAALRDEGIVQRVKDDIGPYMQKRWRETFSRFEHVDDVRGV
GMVQAFTLVKNKAKRELFPDFGEIGTLCRDIFFRNNLIMRACGDHIVSAPPLVMTRAEV
DEMLAVAERCLEEFEQTLKARGLA

>gi|34495455:2206636-2208015 *Chromobacterium violaceum* ATCC 12472 DSM30191 transaminase gene

ATGCAGAAGCAACGTACGACCAGCCAATGGCGCGAACTGGATGCCGCCCATCAC
CTGCATCCGTTCACCGATACCGCATCGCTGAACCAGGCGGGCGCGCGTGATG
ACGCGCGGAGAGGGCGTCTACCTGTGGGATTCGGAAGGCAACAAGATCATCGAC
GGCATGGCCGGACTGTGGTGCGTGAACGTCGGCTACGGCCGCAAGGACTTTGCC
GAAGCGGCGCGCCGGCAGATGGAAGAGCTGCCGTTCTACAACACCTTCTTCAAG

ACCACCCATCCGGCGGTGGTCGAGCTGTCCAGCCTGCTGGCTGAAGTGACGCCG
GCCGGTTTCGACCGCGTGTTCTATACCAATTCCGGTTCCGAATCGGTGGACACCA
TGATCCGCATGGTGCGCCGCTACTGGGACGTGCAGGGCAAGCCGGAGAAGAAG
ACGCTGATCGGCCGCTGGAACGGCTATCACGGCTCCACCATCGGCGGCGCCAG
CCTGGGCGGCATGAAGTACATGCACGAGCAGGGCGACTTGCCGATTCCGGGCAT
GGCCCACATCGAGCAGCCTTGGTGGTACAAGCACGGCAAGGACATGACGCCGGA
CGAGTTCGGCGTGGTGGCCGCGCTGGCTGGAAGAGAAGATTCTGGAAATCGG
CGCCGACAAGGTGGCCGCCTTCGTCGGCGAACCCATCCAGGGCGCCGGCGGCG
TGATCGTCCCGCCGGCCACCTACTGGCCGGAAATCGAGCGCATTTGCCGCAAGT
ACGACGTGCTGCTGGTGGCCGACGAAGTGATCTGCGGCTTCGGGCGTACCGGC
GAATGGTTCGGCCATCAGCATTTCGGCTTCCAGCCCGACCTGTTCACCGCCGCCA
AGGGCCTGTCCTCCGGCTATCTGCCGATAGGCGCGGTCTTTGTCGGCAAGCGCG
TGGCCGAAGGCCTGATCGCCGGCGGCGACTTCAACCACGGCTTCACCTACTCCG
GCCACCCGGTCTGCGCCGCCGTCGCCCACGCCAACGTGGCGGCGCTGCGCGAC
GAGGGCATCGTCCAGCGCGTCAAGGACGACATCGGCCCGTACATGCAAAAGCGC
TGGCGTGAAACCTTCAGCCGTTTCGAGCATGTGGACGACGTGCGCGGCGTCGGC
ATGGTGCAGGCGTTCACCCTGGTGAAGAACAAGGCGAAGCGCGAGCTGTTCCCC
GATTTCGGCGAGATCGGCACGCTGTGCCGCGACATCTTCTTCCGCAACAACCTGA
TCATGCGGGCATGCGGCGACCACATCGTGTCGGCGCCGCCGCTGGTGATGACG
CGGGCGGAAGTGGACGAGATGCTGGCGGTGGCGGAACGCTGTCTGGAGGAATT
CGAGCAGACGCTGAAGGCGCGCGGGCTGGCTTAG

Following primers were used to amplify the gene and to clone it into the pET-28b(+)-expression vector from Novagen including an *N*-terminal His₆-tag.
Cv_ωTA_Fw: 5' - GCGCGCATATGCAGAAGCAACGTACGACCAGCCAAT - 3'
Cv_ωTA_Rv: 5' - GATATTGTACTCGAGCTAAGCCAGCCCGCGCGCCTTC - 3'
Ndel and Xhol restriction sites were used to digest vector and the amplified gene.

**Putrescine aminotransferase (PuAT) from *E*. *coli* K12**. The strain was available in our laboratory collection, genomic DNA was extracted.
Uniprot protein ID sequence: P42588
NCBI reference sequence: NP_417544
GenBank protein ID: AAC76108.3

Protein sequence with *N*-terminal His₆-tag and linker (first 20 AAs colored in red):

MGSSHHHHHHSSGLVPRGSHMNRLPSSASALACSAHALNLIEKRTLDHEEMKALNRE
VIEYFKEHVNPGFLEYRKSVTAGGDYGAVEWQAGSLNTLVDTQGQEFIDCLGGFGIF
NVGHRNPVVVSAVQNQLAKQPLHSQELLDPLRAMLAKTLAALTPGKLKYSFFCNSGT
ESVEAALKLAKAYQSPRGKFTFIATSGAFHGKSLGALSATAKSTFRKPFMPLLPGFRH
VPFGNIEAMRTALNECKKTGDDVAAVILEPIQGEGGVILPPPGYLTAVRKLCDEFGALM
ILDEVQTGMGRTGKMFACEHENVQPDILCLAKALGGGVMPIGATIATEEVFSVLFDNP
FLHTTTFGGNPLACAAALATINVLLEQNLPAQAEQKGDMLLDGFRQLAREYPDLVQEA
RGKGMLMAIEFVDNEIGYNFASEMFRQRVLVAGTLNNAKTIRIEPPLTLTIEQCELVIKA
ARKALAAMRVSVEEA

>gi|49175990:3217516-3218895 *Escherichia coli* str. K-12 substr. MG1655 chromosome, putrescine aminotransferase gene

ATGAACAGGTTACCTTCGAGCGCATCGGCTTTAGCGTGCAGCGCCCACGCCCTG
AATCTCATTGAGAAGCGAACGCTGGATCATGAGGAGATGAAAGCACTTAACCGAG
AGGTGATTGAATACTTCAAAGAGCATGTCAATCCGGGGTTTTTAGAGTATCGCAAA
TCTGTTACCGCCGGCGGGGATTACGGAGCCGTAGAGTGGCAAGCGGGAAGTTTA
AATACGCTTGTCGACACCCAGGGCCAGGAGTTTATCGACTGCCTGGGAGGTTTTG
GAATTTTCAACGTGGGGCACCGTAATCCAGTTGTGGTTTCCGCCGTACAGAATCA
ACTTGCGAAACAACCGCTGCACAGCCAGGAGCTGCTCGATCCGTTACGGGCGAT
GTTGGCGAAAACCCTTGCTGCGCTAACGCCCGGTAAACTGAAATACAGCTTCTTC
TGTAATAGCGGCACCGAGTCCGTTGAAGCAGCGCTGAAGCTGGCGAAAGCTTAC
CAGTCACCGCGCGGCAAGTTTACTTTTATTGCCACCAGCGGCGCGTTCCACGGTA
AATCACTTGGCGCGCTGTCGGCCACGGCGAAATCGACCTTCCGCAAACCGTTTAT
GCCGTTACTGCCGGGCTTCCGTCATGTGCCGTTTGGCAATATCGAAGCCATGCGC
ACGGCTCTTAACGAGTGCAAAAAAACCGGTGATGATGTGGCTGCGGTGATCCTCG
AACCGATTCAGGGTGAAGGTGGCGTAATTCTGCCGCCGCCGGGCTATCTCACCG
CCGTACGTAAGCTATGCGATGAGTTCGGCGCACTGATGATCCTCGATGAAGTACA
AACGGGCATGGGGCGCACGGGCAAGATGTTCGCCTGCGAGCATGAGAACGTACA
GCCGGATATCCTCTGCCTTGCCAAAGCGCTCGGCGGCGGCGTGATGCCGATTGG
CGCGACCATCGCCACTGAAGAGGTGTTTTCAGTTCTGTTCGACAACCCATTCCTG
CATACCACCACCTTTGGCGGCAACCCGCTGGCCTGTGCGGCGGCGCTGGCGAC
CATCAATGTGTTGCTGGAGCAGAACTTACCGGCTCAGGCTGAGCAAAAAGGCGAT
ATGTTGCTGGACGGTTTCCGTCAACTGGCGCGGGAATATCCCGATCTGGTACAGG
AAGCGCGTGGTAAAGGGATGTTGATGGCGATTGAGTTTGTTGATAACGAAATCGG
CTATAACTTTGCCAGCGAGATGTTCCGCCAGCGCGTACTGGTGGCCGGAACGCT
CAATAACGCCAAAACGATCCGCATTGAACCGCCACTGACACTGACCATTGAACAG
TGTGAACTGGTGATCAAAGCGGCGCGTAAGGCGCTGGCGGCCATGCGAGTAAGT
GTCGAAGAAGCGTAA

The gene was then cloned in pET-28b(+) plasmid (Novagen) using Ndel and Xhol restriction sites. A *N*-terminal His₆-tag is present.

**Primers used for PCR amplification** of patA gene from genomic DNA isolated from *E. coli* K12.
Ec PuAT Fw: 5'-GCGCGCATATGAACAGGTTACCTTCGAGCGCATC-3'
Ec PuAT Rv: 5'-CGCGCG**CTCGAG**TTACGCTTCTTCGACACTTACTC -3'

**Omega transaminase (*Ad*-ω-TA) from *Achromobacter denitrificans* Y2k-2.**
GenBank protein ID sequence: AAP92672.1
Uniprot protein ID sequence: Q7WWK8

Protein sequence with *N*-terminal His₆-tag and linker (first 20 AAs underlined):

MGSSHHHHHHSSGLVPRGSHMSAAKLPDLSHLWMPFTANRQFKANPRLLASAKGM
YYTSFDGRQILDGTAGLWCVNAGHCREEIVSAIASQAGVMDYAPGFQLGHPLAFEAA
TAVAGLMPQGLDRVFFTNSGSESVDTALKIALAYHRARGEAQRTRLIGRERGYHGVG
FGGISVGGISPNRKTFSGALLPAVDHLPHTHSLEHNAFTRGQPEWGAHLADELERIIAL
HDASTIAAVIVEPMAGSTGVLVPPKGYLEKLREITARHGILLIFDEVITAYGRLGEATAAA
YFGVTPDLITMAKGVSNAAVPAGAVAVRREVHDAIVNGPQGGIEFFHGYTYSAHPLAA
AAVLATLDIYRREDLFARARKLSAAFEEAAHSLKGAPHVIDVRNIGLVAGIELSPREGAP
GARAAEAFQKCFDTGLMVRYTGDILAVSPPLIVDENQIGQIFEGIGKVLKEVA

>gi|33086797|gb|AY330220.1| *Achromobacter denitrificans* omega-amino acid:pyruvate transaminase (aptA) complete gene sequence

ATGAGCGCTGCCAAACTGCCCGACCTGTCCCACCTCTGGATGCCCTTTACCGCCA
ACCGGCAGTTCAAGGCGAACCCCCGCCTGCTGGCCTCGGCCAAGGGCATGTACT
ACACGTCTTTCGACGGCCGCCAGATCCTGGACGGCACGGCCGGCCTGTGGTGC
GTGAACGCCGGCCACTGCCGCGAAGAAATCGTCTCCGCCATCGCCAGCCAGGCC
GGCGTCATGGACTACGCGCCGGGGTTCCAGCTCGGCCACCCGCTGGCCTTCGA
GGCCGCCACCGCCGTGGCCGGCCTGATGCCGCAGGGCCTGGACCGCGTGTTCT
TCACCAATTCGGGCTCCGAATCGGTGGACACCGCGCTGAAGATCGCCCTGGCCT
ACCACCGCGCGCGCGGCGAGGCGCAGCGCACCCGCCTCATCGGGCGCGAGCG
CGGCTACCACGGCGTGGGCTTCGGCGGCATTTCCGTGGGCGGCATCTCGCCCAA
CCGCAAGACCTTCTCCGGCGCGCTGCTGCCGGCCGTGGACCACCTGCCGCACA
CCCACAGCCTGGAACACAACGCCTTCACGCGCGGCCAGCCCGAGTGGGGCGCG
CACCTGGCCGACGAGTTGGAACGCATCATCGCCCTGCACGACGCCTCCACCATC
GCGGCCGTGATCGTCGAGCCCATGGCCGGCTCCACCGGCGTGCTCGTCCCGCC
CAAGGGCTATCTCGAAAAACTGCGCGAAATCACCGCCCGCCACGGCATTCTGCT
GATCTTCGACGAAGTCATCACCGCGTACGGCCGCCTGGGCGAGGCCACCGCCG
CGGCCTATTTCGGCGTAACGCCCGACCTCATCACCATGGCCAAGGGCGTGAGCA
ACGCCGCCGTTCCGGCCGGCGCCGTCGCGGTGCGCCGCGAAGTGCATGACGCC
ATCGTCAACGGACCGCAAGGCGGCATCGAGTTCTTCCACGGCTACACCTACTCG
GCCCACCCGCTGGCCGCCGCCGCCGTGCTCGCCACGCTGGACATCTACCGCCG
CGAAGACCTGTTCGCCCGCGCCCGCAAGCTGTCGGCCGCGTTCGAGGAAGCCG
CCCACAGCCTCAAGGGCGCGCCGCACGTCATCGACGTGCGCAACATCGGCCTG
GTGGCCGGCATCGAGCTGTCGCCGCGCGAAGGCGCCCCGGGCGCGCGCGCCG

CCGAAGCCTTCCAGAAATGCTTCGACACCGGCCTCATGGTGCGCTACACGGGCG
ACATCCTCGCGGTGTCGCCTCCGCTCATCGTCGACGAAAACCAGATCGGCCAGA
TCTTCGAGGGCATCGGCAAGGTGCTCAAGGAAGTGGCTTAG

**L-Ornithine Aminotransferases** (EC 2.6.1.13)

Enzymes that catalyzes transamination of ornithine with 2-oxoglutarate forming L-glutamate 5-semialdehyde and L-glutamate. Transamination takes place at the gamma carbon atom (C₅ atom) forming an aldehyde functionality at that position.

### a) Human recombinant ornithine aminotransferase

Pdb: 1OAT
Uniprot: P04181

Protein sequence:
Isoform 1
>sp|P04181|OAT_HUMAN Ornithine aminotransferase, mitochondrial OS=Homo sapiens GN=OAT PE=1 SV=1

### Isoform 2:

>sp|P04181-2|OAT_HUMAN Isoform 2 of Ornithine aminotransferase, mitochondrial OS=Homo sapiens GN=OAT

MNTGVEAGETACKLARKWGYTVKGIQKYKAKIVFAAGNFWGRTLSAISSSTDPTSYDGFG
PFMPGFDIIPYNDLPALERALQDPNVAAFMVEPIQGEAGVVVPDPGYLMGVRELCTRHQV
LFIADEIQTGLARTGRWLAVDYENVRPDIVLLGKALSGGLYPVSAVLCDDDIMLTIKPGE
HGSTYGGNPLGCRVAIAALEVLEEENLAENADKLGIILRNELMKLPSDVVTAVRGKGLLN
AIVIKETKDWDAWKVCLRLRDNGLLAKPTHGDIIRFAPPLVIKEDELRESIEIINKTILS
F

### b) Ornithine aminotransferase PY00104 from Plasmodium Yoelii

Pdb: 1Z7D

UniProtKB: Q7RT90

Genebank: AABL01000033

### Protein sequence:

>sp|Q7RT90|OAT_PLAYO Ornithine aminotransferase OS=Plasmodium yoelii yoelii GN=OAT PE=1 SV=1

MEFVKDLKTPEDYINNELKYGAHNYDPIPVVLKRAKGVFVYDVNDKRYYDFLSAYSSVNQ
GHCHPNILNAMINQAKNLTICSRAFFSVPLGICERYLTNLLGYDKVLMMNTGAEANETAY
KLCRKWGYEVKKIPENMAKIVVCKNNFSGRTLGCISASTTKKCTSNFGPFAPQFSKVPYD
DLEALEEELKDPNVCAFIVEPIQGEAGVIVPSDNYLQGVYDICKKYNVLFVADEVQTGLG
RTGKLLCVHHYNVKPDVILLGKALSGGHYPISAVLANDDIMLVIKPGEHGSTYGGNPLAA
SICVEALNVLINEKLCENAEKLGGPFLENLKRELKDSKIVRDVRGKGLLCAIEFKNELVN
VLDICLKLKENGLITRDVHDKTIRLTPPLCITKEQLDECTEIIVKTVKFFDERF

### c) Plasmodium falciparum ornithine delta-aminotransferase

Pdb: 3NTJ; 3LG0

Uniprot: Q6LFH8

**Genebank:AF249587**

### Proteinsequence:

>sp|Q6LFH8|OAT_PLAF7 Ornithine aminotransferase OS=Plasmodium falciparum (isolate 3D7) GN=OAT PE=1 SV=1

MDFVKELKSSQDYMNNELTYGAHNYDPIPVVLKRGKGVFVYDIEDRRYYDFLSAYSSVNQ
GHCHPDILNAMINQAKKLTICSRAFFSDSLGVCERYLTNLFGYDKVLMMNTGAEASETAY
KLCRKWGYEVKKIPENSAKIIVCNNNFSGRTLGCVSASTDKKCKNNFGPFVPNFLKVPYD
DLEALEKELQDPNVCAFIVEPVQGEAGVIVPSDSYFPGVASLCKKYNVLFVADEVQTGLG
RTGKLLCTHHYGVKPDVILLGKALSGGHYPISAILANDDVMLVLKPGEHGSTYGGNPLAA
AICVEALKVLINEKLCENADKLGAPFLQNLKEQLKDSKVVREVRGKGLLCAIEFKNDLVN
VWDICLKFKENGLITRSVHDKTVRLTPPLCITKEQLDECTEIIVKTVKFFDDNL

### d) Ornithine-oxo acid transaminase RocD from Bacillus anthracis

**Pdb: 3RUY**

**Uniprot: Q81TV3**

**Genebank: AE016879**

### Proteinsequence:

>sp|Q81TV3|OAT_BACAN Ornithine aminotransferase OS=Bacillus anthracis GN=rocD PE=1 SV=1

MIQTKDIIELTDTYGANNYHPLPIVISKAEGVWVEDPEGNRYMDLLSAYSAVNQGHRHPK
IINALIDQANRVTLTSRAFHSDQLGPWYEKVAKLTNKEMVLPMNTGAEAVETAIKTARRW
AYDVKKVEANRAEIIVCEDNFHGRTMGAVSMSSNEEYKRGFGPMLPGIIVIPYGDLEALK
AAITPNTAAFILEPIQGEAGINIPPAGFLKEALEVCKKENVLFVADEIQTGLGRTGKVFA

CDWDNVTPDMYILGKALGGGVFPISCAAANRDILGVFEPGSHGSTFGGNPLACAVSIAAL
EVLEEEKLTERSLQLGEKLVGQLKEIDNPMITEVRGKGLFIGIELNEPARPYCEQLKAAG
LLCKETHENVIRIAPPLVISEEDLEWAFQKIKAVLS

### L-Lysine 6-transaminase (EC 2.6.1.36)

Enzymes that catalyzes transamination of L-lysine with 2-oxoglutarate forming 2-aminoadipate 6-semialdehyde and L-glutamate. Transamination takes place at the epsilon carbon atom (C₆ atom) forming an aldehyde functionality at this position.

### a) L-Lysine 6-transaminase from Streptomyces clavuligerus ATCC 27064

Protein ID sequence: AAU93525.1

NCBI reference sequence: AY742798

MGEAARHPDGDFSDVGNLHAQDVHQALEQHMLVDGYDLVLDLDASSGVWLVDAVT
QKRYLDLFSFFASAPLGINPPSIVEDPAFMRELAVAAVNKPSNPDLYSVPYARFVKTFA
RVLGDPRLPRLFFVDGGALAVENALKAALDWKAQKLGLAEPDTDRLQVLHLERSFHG
RSGYTMSLTNTEPSKTARFPKFGWPRISSPALQHPPAEHTGANQEAERRALEAAREA
FAAADGMIACFIAEPIQGEGGDNHLSAEFLQAMQRLCHENDALFVLDEVQSGCGITGT
AWAYQQLGLQPDLVAFGKKTQVCGVMGGGRIDEVPENVFAVSSRISSTWGGNLADM
VRATRLLETIERTQVFDTVVQRGKYFRDGLEDLAARHPSVVTNARGRGLMCAVDLPD
TRTRNEVLRLMYTEHQVIALPCGGRSLRFRPALTIAEHEIDQALQALASSVTAVAESV

>gi|53771851|gb|AY742798.1| *Streptomyces clavuligerus* L-lysine-epsilon aminotransferase (lat) gene, complete cds

ATGGGCGAAGCAGCACGCCACCCCGACGGCGATTTCTCGGACGTGGAAACCTC
CACGCTCAGGACGTGCACCAGGCACTTGAGCAGCATATGCTCGTCGACGGGTAC
GACCTCGTTCTCGACCTCGACGCCAGCTCCGGCGTCTGGCTCGTCGACGCCGTC
ACCCAGAAGCGGTATCTCGACCTCTTTTCCTTCTTTGCCTCGGCGCCGCTCGGAA
TCAACCCGCCCAGCATTGTCGAGGACCCGGCATTCATGCGGGAGCTGGCCGTGG
CCGCGGTCAACAAGCCGTCGAACCCCGATCTTTATTCGGTGCCGTACGCCCGTTT
CGTCAAGACCTTCGCCCGGGTCCTCGGCGACCCCCGGCTGCCGCGGCTGTTCTT
CGTGGACGGCGGGGCGCTGGCCGTGGAGAACGCGCTCAAGGCGGCCCTCGACT
GGAAGGCCCAGAAGCTGGGCCTCGCCGAGCCGGACACCGACCGGCTCCAGGTG
CTGCATCTGGAGCGCTCGTTCCACGGCCGCAGCGGCTACACCATGTCGCTGACG
AACACCGAGCCGTCCAAGACCGCCCGCTTCCCCAAGTTCGGCTGGCCACGGATC
TCGTCCCCCGCCCTCCAGCACCCGCCGGCCGAGCACACCGGCGCCAACCAGGA
GGCCGAGCGACGGGCGCTGGAGGCCGCCCGGGAGGCGTTCGCAGCGGCGGAC
GGCATGATCGCCTGCTTCATCGCGGAGCCCATCCAGGGCGAGGGCGGCGACAA
CCACCTCAGCGCGGAGTTCCTCCAGGCCATGCAGCGGCTCTGCCACGAGAACGA
CGCCCTGTTCGTCCTGGACGAGGTGCAGAGCGGCTGCGGCATCACCGGTACCG
CCTGGGCCTACCAGCAGCTCGGCCTCCAGCCCGACCTGGTGGCCTTCGGCAAGA
AGACCCAGGTCTGCGGGGTGATGGGCGGCGGCCGGATCGACGAGGTCCCCGAG
AACGTCTTCGCCGTCTCCTCCCGGATCAGCTCCACCTGGGGCGGCAACCTCGCC
GACATGGTCCGCGCCACCCGGCTGCTGGAGACGATCGAGCGCACCCAGGTCTTC
GACACCGTCGTCCAGCGCGGCAAGTACTTCCGGGACGGCCTGGAGGACCTGGC

CGCCCGCCACCCCTCCGTCGTGACCAACGCCCGCGGCCGGGGCCTGATGTGCG
CGGTCGACCTGCCGGACACCCGGACCCGCAATGAGGTGCTGCGGCTCATGTACA
CGGAGCACCAGGTCATCGCCCTGCCCTGCGGCGGGCGCAGCCTCCGGTTCCGC
CCCGCGCTGACGATCGCCGAGCACGAGATCGACCAGGCCCTTCAGGCGCTGGC
GAGCAGTGTCACGGCGGTCGCCGAGAGCGTCTGA

### b) L-Lysine 6-transaminase from Mycobacterium tuberculosis H37Rv

Uniprot protein ID sequence: P63509
NCBI reference sequence: NP_217807.1

PDB: 2CIN, 2CJD, 2CJG and 2CJH

MAAVVKSVALAGRPTTPDRVHEVLGRSMLVDGLDIVLDLTRSGGSYLVDAITGRRYLD
MFTFVASSALGMNPPALVDDREFHAELMQAALNKPSNSDVYSVAMARFVETFARVLG
DPALPHLFFVEGGALAVENALKAAFDWKSRHNQAHGIDPALGTQVLHLRGAFHGRSG
YTLSLTNTKPTITARFPKFDWPRIDAPYMRPGLDEPAMAALEAEALRQARAAFETRPH
DIACFVAEPIQGEGGDRHFRPEFFAAMRELCDEFDALLIFDEVQTGCGLTGTAWAYQ
QLDVAPDIVAFGKKTQVCGVMAGRRVDEVADNVFAVPSRLNSTWGGNLTDMVRARR
ILEVIEAEGLFERAVQHGKYLRARLDELAADFPAVVLDPRGRGLMCAFSLPTTADRDE
LIRQLWQRAVIVLPAGADTVRFRPPLTVSTAEIDAAIAAVRSALPVVT

>gi|38490319:202782-204131 Mycobacterium tuberculosis H37Rv complete lat gene

ATGGCCGCCGTCGTGAAGTCCGTCGCTCTTGCCGGTAGACCAACAACACCAGAC
CGGGTTCATGAGGTGCTAGGGCGCAGCATGCTGGTCGACGGTCTGGACATAGTG
CTCGATCTGACCCGTTCGGGCGGTTCATATCTGGTCGACGCTATAACGGGTCGG
CGCTACCTGGACATGTTCACATTCGTTGCCTCCTCGGCACTGGGTATGAATCCCC
CGGCGCTGGTGGACGACCGGGAGTTCCATGCCGAACTCATGCAGGCCGCGCTG
AACAAGCCCAGCAATTCCGACGTGTACTCGGTGGCGATGGCCCGCTTCGTCGAG
ACCTTCGCCCGTGTCTTGGGCGACCCGGCGCTGCCGCATCTGTTCTTCGTCGAA
GGGGGCGCCCTGGCGGTGGAGAACGCGCTCAAAGCCGCGTTCGACTGGAAGAG
TCGGCACAACCAAGCCCATGGGATCGACCCGGCGCTGGGCACTCAAGTGCTCCA
CCTGCGCGGGGCATTTCACGGCCGCAGCGGCTATACCCTGTCGCTGACCAACAC
CAAGCCGACCATTACCGCCCGGTTCCCGAAATTCGACTGGCCACGCATCGATGC
GCCGTACATGCGGCCCGGCCTGGATGAGCCCGCTATGGCCGCGCTAGAGGCCG
AGGCGCTACGCCAGGCCCGCGCGGCATTCGAGACCCGCCCGCACGACATCGCG
TGTTTTGTGGCCGAACCCATCCAGGGCGAGGGTGGCGACCGCCATTTCCGCCCG
GAGTTTTTCGCTGCGATGCGCGAGCTGTGCGACGAGTTTGATGCACTGCTGATCT
TCGACGAAGTACAGACCGGCTGCGGGTTGACCGGAACCGCCTGGGCATACCAGC
AGTTGGATGTCGCACCCGACATCGTGGCGTTCGGCAAGAAGACGCAGGTATGCG
GAGTGATGGCCGGTCGGCGGGTGGACGAGGTCGCCGACAATGTGTTCGCGGTC
CCATCACGGCTCAACTCGACATGGGGTGGCAATCTTACCGACATGGTGCGCGCC
CGCCGCATCTTGGAGGTCATCGAAGCCGAGGGCCTGTTCGAGCGGGCGGTGCA
GCACGGTAAGTATCTGCGCGCCCGGCTTGACGAACTCGCCGCGGACTTCCCGGC
AGTGGTTCTCGATCCGCGCGGCCGCGGGCTGATGTGTGCGTTTAGCCTGCCGAC
CACCGCCGACCGTGACGAGTTGATCCGCCAGCTGTGGCAACGTGCGGTGATTGT
GTTGCCGGCCGGTGCAGACACCGTGCGATTCCGTCCACCGCTGACGGTTTCAAC
CGCCGAGATCGACGCCGCGATAGCCGCGGTGCGCAGCGCGTTACCGGTGGTGA
CGTAA

### Alcohol Dehydrogenases

**Alcohol dehydrogenase (Rj-ADH) from *Rhodococcus jostii* RHA1**. The microorganism was available in our laboratory collection, genomic DNA was extracted for gene amplification.
>ENA|ABG94302|ABG94302.1 *Rhodococcus jostii* RHA1 alcohol dehydrogenase : Location:1..1113 
ATGAAGACCAAAGCAGCGGTTCTGTTCGAAACGCACAAGCCGTTCGAAATCGTGG
AACTCGAGCTCGACGGTCCGGGCCCTGGCGAAGTGCTCATCAAGTATGTCGCGG
CAGGCCTGTGCCACTCGGACCTGCACCTCACCGACGGCGATCTGCCGCCGCGTT
TCCCGATCGTCGGCGGGCACGAGGGTTCCGGCATCATCGAGGAAGTCGGCCCC
GGTGTCACCAAGGTGAAGCCCGGCGACCACGTGGTCTGCAGCTTCATCCCGAAC
TGCGGCACGTGTCGGTACTGTGCCACCGGCCGCCAGAACCTGTGTGACATGGGC
GCCACCATCCTCGAGGGGTCGATGCCCGACGGCTCGTTCCGGTTCCACTCGGGC
AAGCAGGATTTCGGTGCCATGTGCATGCTCGGTACGTTCGCCGAGCGCGCCACC
ATCTCGCAGCACTCGGTGGTGAAGGTGGACGACTGGCTCCCCCTGGAGACCGCC
GTCCTCGTCGGCTGCGGTGTGCCGTCCGGTTGGGGCACCGCGGTGTACGCCGG
CGGAGTTCGCGCCGGTGACACGGTCGTCATCTACGGCATCGGCGGTCTCGGCAT
CAACGCCGTGCAGGGCGCGGTCAGCTCCGGCGCCAAGTACGTCGTGGTGGTGG
ATCCGGTGGCGATGAAGCGGGAGGCCGCGCTGAAGTTCGGCGCCACCCACGCG
TTCGCGGACGCCGCGACCGCGGCGGAGAAGGTCAACGAACTGACCTGGGGTCA
GGGCGCCGATCAGGCCCTCATCCTGGTGGGCACCGTCGACGAAGAGGTGGTGC
AGAACGCGACCGCCGTCATCGGCAAGGGCGGCACCGTCGTCATCACCGGCCTC
GCCGACCCCGCCAAGCTCACCGTGCACATCTCGGGAACCGACCTGACCCTGAAC
CAGAAGACGATCAAGGGTTCGCTGTTCGGTTCGGCCAACCCGCAGTACGACATC
GTCCGCCTGCTGCGCCTGTACGACGCGGGTCAGCTCAAGCTCGACGAGCTGATC
ACCACCAAGTACACGCTCGAGCAGGTGAACGAGGGTTACCAGGATCTGCGGGAC
GGAAAGAACCTGCGTGGCGTGATCGTCCACCAGCAGTAG
 >gi|110819018|gb|ABG94302.1| alcohol dehydrogenase [Rhodococcus jostii RHA1]

Protein sequence with N-terminal His₆-tag and linker (first 20 AAs underlined):

MGSSHHHHHHSSGLVPRGSHMKTKAAVLFETHKPFEIVELELDGPGPGEVLIKYVAA
GLCHSDLHLTDGDLPPRFPIVGGHEGSGIIEEVGPGVTKVKPGDHVVCSFIPNCGTCR

YCATGRQNLCDMGATILEGSMPDGSFRFHSGKQDFGAMCMLGTFAERATISQHSVV
KVDDWLPLETAVLVGCGVPSGWGTAVYAGGVRAGDTVVIYGIGGLGINAVQGAVSS
GAKYVVVVDPVAMKREAALKFGATHAFADAATAAEKVNELTWGQGADQALILVGTVD
EEVVQNATAVIGKGGTVVITGLADPAKLTVHISGTDLTLNQKTIKGSLFGSANPQYDIVR
LLRLYDAGQLKLDELITTKYTLEQVNEGYQDLRDGKNLRGVIVHQQ

Following primers were used to amplify the gene and to clone it into the pET-28b(+)-expression vector from Novagen including an N-terminal His₆-tag.
Rj_ADH_Fw: 5⊐ - GCGCGCATATGAAGACCAAAGCAGCGGTTCTG - 3 ⊐
Rj_ADH_Rv: 5⊐ - GAGCTAAGCTTCTACTGCTGGTGGACGATCACG - 3⊐
Ndel and Hindlll restriction sites were used to digest vector and the amplified gene.

**Alcohol dehydrogenase (Gs-ADH1) from *Geobacillus stearothermophilus* ATCC 12976 DSM 297**. The strain was ordered from the DSMZ strain collection, genomic DNA was extracted.
>gi|216229|dbj|D090421.1|BACADHT Geobacillus stearothermophilus adhT gene for alcohol dehydrogenase

ATGAAAGCTGCAGTTGTGGAACAATTTAAAAAGCCGTTACAAGTGAAAGAAGTGG
AAAAACCTAAGATCTCATACGGGGAAGTATTAGTGCGCATCAAAGCGTGTGGGT
ATGCCATACAGACTTGCATGCCGCACATGGCGACTGGCCTGTAAAGCCTAAACTG
CCTCTCATTCCTGGCCATGAAGGCGTCGGTGTAATTGAAGAAGTAGGTCCTGGGG
TAACACATTTAAAAGTTGGAGATCGCGTAGGTATCCCTTGGCTTTATTCGGCGTGC
GGTCATTGTGACTATTGCTTAAGCGGACAAGAAACATTATGCGAACGTCAACAAAA
CGCTGGCTATTCCGTCGATGGTGGTTATGCTGAATATTGCCGTGCTGCAGCCGAT
TATGTCGTAAAAATTCCTGATAACTTATCGTTTGAAGAAGCCGCTCCAATCTTTTGC
GCTGGTGTAACAACATATAAAGCGCTCAAAGTAACAGGCGCAAAACCAGGTGAAT
GGGTAGCCATTTACGGTATCGGCGGGCTTGGACATGTCGCAGTCCAATACGCAAA
GGCGATGGGGTTAAACGTCGTTGCTGTCGATTTAGGTGATGAAAAACTTGAGCTT
GCTAAACAACTTGGTGCAGATCTTGTCGTCAATCCGAAACATGATGATGCAGCAC
AATGGATAAAAGAAAAAGTGGGCGGTGTGCATGCGACTGTCGTCACAGCTGTTTC
AAAAGCCGCGTTCGAATCAGCCTACAAATCCATTCGTCGCGGTGGTGCTTGCGTA
CTCGTCGGATTACCGCCGGAAGAAATACCTATTCCAATTTTCGATACAGTATTAAA
TGGAGTAAAAATTATTGGTTCTATCGTTGGTACGCGCAAAGACTTACAAGAGGCAC
TTCAATTTGCAGCAGAAGGAAAAGTAAAAACAATTGTCGAAGTGCAACCGCTTGAA

AACATTAACGACGTATTCGATCGTATGTTAAAAGGGCAAATTAACGGCCGCGTCGT
GTTAAAAGTAGATTAA

Protein sequence ID: BAA14441
>gi|1168347|sp|P12311.2 or BAA14411|ADH1_GEOSE RecName: Full=Alcohol dehydrogenase; AltName: Full=ADH-T ATCC 12976

Protein sequence with N-terminal His₆-tag and linker (first 20 AAs underlined):

MGSSHHHHHHSSGLVPRGSHMKAAVVEQFKKPLQVKEVEKPKISYGEVLVRIKACGV
CHTDLHAAHGDWPVKPKLPLIPGHEGVGVIEEVGPGVTHLKVGDRVGIPWLYSACGH
CDYCLSGQETLCERQQNAGYSVDGGYAEYCRAAADYVVKIPDNLSFEEAAPIFCAGV
TTYKALKVTGAKPGEWVAIYGIGGLGHVAVQYAKAMGLNVVAVDLGDEKLELAKQLG
ADLVVNPKHDDAAQWIKEKVGGVHATVVTAVSKAAFESAYKSIRRGGACVLVGLPPE
EIPIPIFDTVLNGVKIIGSIVGTRKDLQEALQFAAEGKVKTIVEVQPLENINDVFDRMLKG
QINGRVVLKVD

Following primers were used to amplify the gene and to clone it into the pET-28b(+)-expression vector from Novagen including an N-terminal His₆-tag.
Gs_ADH1_Fw: 5⊐ - GCTTGACCATATGAAAGCTGCAGTTGTGGAAC - 3⊐
Gs_ADH1_Rv: 5⊐ - GGATCGCTCGAGTTAATCTACTTTTAACACGAC - 3⊐
Ndel and Xhol restriction sites were used to digest vector and the amplified gene.

**Alcohol dehydrogenase (Gs-ADH2) from *Geobacillus stearothermophilus* ATCC 12976 DSM 2334**. The strain was ordered from the DSMZ strain collection, genomic DNA was extracted.
GenBank: P42327
Gene ID EMBL: Z25544
>gi|479043|emb|Z25544.1| *B*. *stearothermophilus* coding gene for alcohol dehydrogenase

ATGAAAGCAGCAGTAGTTAACGAATTTAAAAAGCACTTGAAATTAAAGAGGTGGA
AAGACCCAAACTAGAGGAAGGTGAAGTTTTAGTTAAGATCGAAGCATGCGGTGTG
TGCCATACGGATTTACATGCAGCCCATGGTGACTGGCCGATTAAACCGAAATTGC
CGTTAATTCCGGGGCATGAAGGAGTCGGAATTGTTGTAGAAGTCGCAAAGGGAGT
TAAATCAATAAAAGTGGGAGATCGCGTCGGCATCCCATGGTTATATTCTGCATGTG
GTGAATGTGAATATTGTTTAACTGGTCAAGAAACACTTTGCCCGCATCAATTGAAT

GGCGGATATTCCGTTGATGGCGGATATGCGGAATACTGTAAAGCTCCCGCAGATT
ATGTGGCGAAAATTCCTGATAACTTAGATCCAGTTGAAGTAGCGCCAATTTTATGT
GCAGGCGTAACGACATATAAAGCGTTGAAAGTATCAGGTGCTCGGCCAGGTGAAT
GGGTGGCCATTTACGGTATTGGAGGACTTGGTCATATTGCATTACAATACGCCAA
AGCGATGGGACTTAATGTGGTAGCTGTTGATATCAGTGATGAAAAATCAAAATTAG
CCAAAGATTTAGGTGCAGATATTGCAATCAACGGTTTAAAAGAAGATCCTGTCAAA
GCGATACATGATCAAGTAGGCGGAGTTCATGCTGCTATTAGTGTAGCGGTGAATA
AAAAAGCATTTGAACAGGCCTATCAGTCAGTGAAACGGGGAGGCACATTAGTTGT
TGTCGGTCTGCCAAATGCTGATCTGCCTATTCCGATCTTCGATACAGTATTAAACG
GCGTTTCCGTTAAAGGTTCCATCGTCGGTACAAGAAAGGACATGCAGGAAGCATT
AGATTTTGCTGCTCGCGGAAAAGTCCGTCCAATTGTTGAAACCGCTGAATTAGAA
GAAATAAATGAAGTATTTGAAAGAATGGAAAAAGGAAAAATCAACGGTCGTATCGT
ACTAAAATTGAAGGAGGATTGA

Protein sequence ID: CAA80989
>gi|1168351|sp|P42327.1 or CAA80989|ADH2_GEOSE RecName: Full=Alcohol dehydrogenase; Short=ADH

Protein sequence with *N*-terminal His₆-tag and linker (first 20 AAs underlined):

MGSSHHHHHHSSGLVPRGSHMKAAVVNEFKKALEIKEVERPKLEEGEVLVKIEACGV
CHTDLHAAHGDWPIKPKLPLIPGHEGVGIVVEVAKGVKSIKVGDRVGIPWLYSACGEC
EYCLTGQETLCPHQLNGGYSVDGGYAEYCKAPADYVAKIPDNLDPVEVAPILCAGVTT
YKALKVSGARPGEWVAIYGIGGLGHIALQYAKAMGLNVVAVDISDEKSKLAKDLGADIA
INGLKEDPVKAIHDQVGGVHAAISVAVNKKAFEQAYQSVKRGGTLVVVGLPNADLPIPI
FDTVLNGVSVKGSIVGTRKDMQEALDFAARGKVRPIVETAELEEINEVFERMEKGKIN
GRIVLKLKED

The following primers were used to amplify the gene and to clone it into the pET-28b(+)-expression vector from Novagen including an *N*-terminal His₆-tag.
Gs_ADH2_Fw: 5⊐ - GCTTGACCATATGAAAGCAGCAGTAGTTAACG - 3⊐
Gs_ADH2_Rv: 5⊐ - GGATCGCTCGAGTCAATCCTCCTTCAATTTTAGTACG - 3⊐
Ndel and Xhol restriction sites were used to digest vector and the amplified gene.

**Alcohol dehydrogenase (Pp-ADH) from *Pseudomonas putida* strain BIRD-1. The**
synthetic gene and codon optimization was ordered at DNA2.0.
GenBank protein ID sequence: ADR59556.1
Uniprot protein ID sequence: E4RG63

Protein sequence with *N*-terminal His₆-tag and linker (first 20 AAs underlined):

MGSSHHHHHHSSGLVPRGSHMKAAVVAPGRRVDVVEKTLRPLEHGEALLKMQCCG
VCHTDLHVKNGDFGDKTGVVLGHEGIGVVQEVGPGVTSLKPGDRASVAWFYQGCG
HCEYCNSGNETLCRDVKNSGYTVDGGMAEACIVKADYSVKVPDGLDSAAASSITCAG
VTTYKAVKISNVRPGQWIAIYGLGGLGNLALQYAKNVFNAKVIAIDVNEEQLRFASEMG
ADLVVNPLNEDAAKFIQAKTGGAHAAVVTAVAKGAFNSAVDALRAGGRLVAVGLPSE
SMDLNIPRLVLDGIEVVGSLVGTRQDLQEAFQFAAEGKVVPKVTLRPIEHINQIFDEML
EGKIKGRMVIQFEG

Gene sequence:
>gi|386009639:c2l52220-2151210 *Pseudomonas putida* BIRD-1 codon optimized alcohol dehydrogenase gene

ATGAAAGCAGCGGTGGTCGCACCTGGTCGTCGTGTGGACGTAGTCGAGAAAACC
CTGCGTCCGCTGGAACATGGTGAGGCGCTGCTGAAGATGCAATGTTGTGGTGTC
TGCCACACGGATTTGCACGTGAAGAACGGCGACTTTGGTGATAAGACCGGTGTTG
TCCTGGGCCATGAAGGCATCGGCGTGGTTCAAGAGGTCGGTCCGGGTGTTACCA
GCTTGAAACCGGGCGACCGTGCAAGCGTCGCGTGGTTCTATCAGGGCTGCGGCC
ACTGTGAGTATTGCAACTCGGGTAATGAAACTTTGTGCCGCGATGTGAAAAACAG
CGGTTACACGGTTGACGGTGGCATGGCCGAAGCGTGCATTGTCAAGGCGGACTA
CAGCGTTAAAGTGCCAGATGGTCTGGACAGCGCAGCTGCCTCCAGCATTACCTGT
GCGGGCGTGACCACCTACAAAGCGGTGAAGATCAGCAATGTTCGTCGATCGCCG
GGTCAGTGGATTGCTATTTACGGTCTGGGTGGTCTGGGCAACCTGGCACTGCAAT
ATGCGAAGAATGTGTTCAATGCGAAGGTTATCGCGATTGATGTCAATGAAGAGCA
GTTGCGTTTCGCCAGCGAAATGGGTGCGGATCTGGTTGTTAACCCGCTGAACGA
GGACGCCGCAAAGTTTATCCAGGCAAAAACGGGTGGCGCGCATGCCGCGGTAGT
GACCGCGGTTGCGAAAGGCGCATTCAATTCCGCCGTGGACGCCTTGCGCGCTGG
CGGTCGCCTGGTTGCAGTTGGCCTGCCGAGCGAGTCTATGGATCTGAATATCCC
GCGTCTGGTTCTGGACGGTATTGAGGTCGTCGGCAGCCTGGTGGGTACCCGCCA
GGATCTGCAGGAAGCGTTTCAGTTTGCGGCTGAAGGTAAAGTCGTGCCGAAGGTT

ACGCTGCGTCCGATTGAGCACATTAACCAAATCTTTGACGAGATGCTGGAAGGTA
AGATCAAAGGTCGCATGGTGATCCAATTCGAGGGTTAA

NCBI reference sequence: NC_017530.1
>gi|386009639:c2152220-2151210 *Pseudomonas putida* BIRD-1 chromosome, complete genome, original gene sequence

ATGAAAGCTGCTGTCGTTGCACCAGGCCGTCGCGTGGACGTGGTAGAGAAAACC
CTGCGCCCTCTCGAACACGGCGAAGCGCTGCTGAAGATGCAGTGCTGCGGTGTG
TGCCACACCGACCTGCACGTGAAAAATGGCGACTTTGGTGACAAGACCGGTGTA
GTACTGGGCCACGAAGGTATTGGCGTGGTCCAGGAAGTTGGCCCGGGCGTCACC
TCGCTCAAGCCAGGCGACCGTGCCAGCGTTGCCTGGTTCTACCAGGGTTGCGGA
CATTGCGAATATTGCAACAGCGGCAACGAGACCCTGTGCCGCGACGTGAAGAATT
CCGGCTACACCGTGGACGGCGGCATGGCCGAGGCCTGCATCGTCAAGGCCGAC
TACTCGGTTAAAGTGCCCGACGGCCTCGACTCCGCTGCCGCCAGCAGCATCACC
TGCGCCGGCGTCACGACCTACAAGGCAGTGAAAATCTCCAACGTCCGCCCCGGC
CAATGGATCGCCATCTACGGGCTCGGCGGCCTGGGCAACCTGGCCCTGCAATAT
GCCAAGAATGTGTTCAACGCCAAAGTGATCGCCATCGACGTCAACGAAGAGCAAC
TGCGCTTCGCCAGCGAGATGGGCGCGGATCTGGTCGTCAATCCGCTCAACGAAG
ACGCCGCTAAGTTCATCCAGGCCAAGACCGGTGGCGCACATGCTGCCGTCGTTA
CTGCCGTGGCCAAAGGCGCGTTCAATTCTGCCGTCGATGCCTTGCGCGCTGGCG
GGCGACTGGTGGCCGTCGGGCTTCCGTCGGAGTCCATGGACCTGAATATTCCTC
GCCTGGTGCTTGATGGGATCGAAGTGGTGGGCTCGCTGGTAGGTACACGCCAGG
ACCTGCAGGAAGCCTTCCAGTTTGCCGCTGAAGGCAAAGTGGTGCCCAAGGTAA
CGCTGCGACCGATCGAGCATATCAACCAGATCTTTGACGAGATGCTGGAGGGCA
AGATCAAAGGCCGGATGGTGATACAGTTCGAAGGCTGA

The gene was then cloned in pET-28b(+) plasmid (Novagen) using Ndel and Hindlll restriction sites. A *N*-terminal His₆-tag is present.

**Alcohol dehydrogenase (Gt-ADH) from *Geobacillus thermodenitrificans* strain NG80-2**. The synthetic gene and codon optimization was ordered at DNA2.0.
GenBank protein ID sequence: AB068223.1
Uniprot protein ID sequence: A4ISB9

Protein sequence with *N*-terminal His₆-tag and linker (first 20 AAs underlined):

MGSSHHHHHHSSGLVPRGSHMQNFTFRNPTKLIFGRGQIEQLKEEVPKYGKKVLLVY
GGGSIKRNGLYDEVMSLLTDIGAEVVELPGVEPNPRLSTVKKGVDICRREGIEFLLAVG
GGSVIDCTKAIAAGAKFDGDPWEFITKKATVTEALPFGTVLTLAATGSEMNAGSVITN
WETKEKYGWGSPVTFPQFSILDPTYTMTVPKDHTVYGIVDMMSHVFEQYFHHTPNTP
LQDRMCEAVLKTVIEAAPKLVDDLENYELRETIMYSGTIALNGFLQMGVRGDWATHDI
EHAVSAVYDIPHAGGLAILFPNWMKHVLDENVSRFAQLAVRVFDVDPTGKTERDVAL
EGIERLRAFWSSLGAPSRLADYGIGEENLELMADKAMAFGEFGRFKTLNRDDVLAILR
ASL

Gene sequence:
>gi|138893679:c2989987-2988824 *Geobacillus thermodenitrificans* NG80-2 alcohol dehydrogenase codon optimized gene sequence

ATGCAGAACTTCACCTTTCGTAATCCGACGAAACTGATCTTCGGTCGCGGCCAAA
TCGAGCAACTGAAGGAAGAAGTGCCTAAGTATGGTAAGAAAGTTCTGTTGGTCTA
TGGTGGCGGCAGCATTAAACGCAATGGCCTGTACGACGAGGTGATGAGCCTGCT
GACGGATATTGGTGCAGAAGTCGTGGAGCTGCCAGGTGTTGAGCCGAACCCGCG
TCTGAGCACCGTGAAGAAAGGTGTGGACATCTGTCGTCGTGAGGGTATCGAATTT
CTGCTGGCGGTTGGTGGCGGTAGCGTCATTGACTGCACGAAGGCAATCGCGGCT
GGTGCCAAATTTGATGGCGACCCGTGGGAATTCATCACCAAAAAGGCGACCGTTA
CGGAGGCTCTGCCGTTTGGCACCGTCTTGACCCTGGCCGCAACCGGTAGCGAGA
TGAATGCGCGATCGGGCAGCGTTATTACTAACTGGGAGACTAAAGAGAAATACGG
TTGGGGTTCTCCGGTGACGTTCCCGCAGTTCTCCATTCTGGACCCAACCTATACG
ATGACGGTTCCTAAAGACCACACCGTCTATGGTATCGTGGATATGATGAGCCACG
TTTTTGAACAATACTTCCACCACACCCCGAACACCCCGCTGCAGGATCGTATGTG
CGAAGCAGTACTGAAAACGGTTATCGAAGCGGCACCGAAACTGGTCGATGATCTG
GAGAATTACGAACTGCGTGAAACCATTATGTATTCGGGCACTATCGCACTGAACG
GTTTCCTGCAGATGGGCGTTCGCGGTGATTGGGCGACCCATGACATCGAGCATG
CAGTCTCTGCTGTGTACGATATCCCGCACGCCGGTGGCCTGGCCATTTTGTTCCC
GAATTGGATGAAGCATGTCCTGGATGAGAACGTTAGCCGCTTTGCGCAACTGGCG
GTGCGTGTGTTTGACGTCGACCCGACGGGTAAGACCGAGCGTGATGTGGCCCTG
GAAGGTATTGAGCGTCTGCGTGCGTTTTGGAGCAGCCTGGGCGCTCCGTCCCGC
CTGGCAGATTACGGTATTGGCGAAGAAAATCTGGAGCTGATGGCCGACAAGGCG
ATGGCGTTTGGTGAGTTCGGCCGTTTCAAGACCTTGAACCGCGACGACGTTTTGG
CGATTTTGCGTGCGAGCCTGTAA

NCBI reference sequence: NC_009328.1 >gi|138893679:c2989987-2988824 *Geobacillus thermodenitrificans* NG80-2 chromosome, complete genome, original gene sequence

ATGCAAAATTTTACGTTTCGCAATCCGACCAAACTCATTTTTGGAGAGGACAAAT
TGAGCAGCTCAAAGAAGTGCCGAAATATGGCAAAAAGTGCTGCTTGTCTAT
GGGGGCGGCAGCATTAAACGAAACGGACTATATGATGAAGTCATGAGCCTATTGA
CGGACATTGGCGCCGAAGTCGTTGAACTGCCAGGCGTCGAACCGAACCCGCGCC
TTTCGACCGTCAAAAAGGGGTAGACATTTGCAGACGGGAAGGAATTGAATTTTT
GCTTGCCGTTGGCGGCGGCAGTGTGATCGACTGTACGAAAGCGATTGCAGCCGG
CGCGAAGTTTGATGGCGATCCGTGGGAGTTCATTACGAAAAAGCGACTGTCACT
GAGGCGTTGCCGTTTGGGACGGTCCTGACGCTGGCAGCGACCGGCTCGGAAAT
GAACGCCGGATCGGTGATCACCAATTGGGAGACGAAAGAAAAATACGGCTGGGG
CAGCCCGGTGACATTCCCGCAATTTTCGATTTTGGATCCGACGTACACGATGACG
GTGCCGAAAGACCATACCGTTTACGGCATCGTCGATATGATGTCCCATGTGTTTG
AGCAATATTTCCATCATACGCCGAACACGCCGCTGCAAGACCGGATGTGCGAGG
CAGTGTTAAAAACGGTCATTGAGGCGGCGCCAAAATTGGTTGACGACTTAGAGAA
CTACGAGCTGCGCGAGACGATCATGTACTCGGGCACGATCGCCTTAAACGGCTTT
TTGCAAATGGGCGTGCGCGGTGATTGGGCGACGCATGATATCGAGCATGCGGTC
TCTGCTGTATACGATATCCCGCACGCCGGGGGATTGGCCATTTTGTTCCCGAATT
GGATGAAACATGTGCTTGATGAAAATGTCAGCCGTTTCGCCCAACTGGCGGTGCG
CGTCTTTGACGTCGATCCGACGGGCAAAACGGAGCGCGACGTGGCGCTTGAGGG
CATCGAGCGGTTGCGCGCGTTTTGGTCGAGCCTCGGGGCGCCGTCTCGATTGGC
TGATTATGGCATCGGCGAGGAAAATCTCGAGCTGATGGCGGATAAAGCGATGGC
GTTTGGTGAGTTTGGCCGTTTCAAAACGTTAAATCGTGATGATGTGCTCGCCATTT
TGCGTGCGTCTTTATAA

The gene was then cloned in pET-28b(+) plasmid (Novagen) using Ndel and Hindlll restriction sites. A *N*-terminal His₆-tag is present.

### Alanine Dehydrogenases

The following genes of thermostable AlaDHs can be cloned from genomic DNA:
**AlaDH from** *Archaeoglobus fulgidus* **(DSM 4304)**. The strain was ordered from the DSMZ strain collection, genomic DNA was extracted.
GenBank protein ID sequence: AAB89583
Uniprot protein ID sequence: **O28608**

### Crystal structures (PDB): 10MO, 1VLL

Protein sequence with *N*-terminal His₆-tag and linker (first 20 AA underlined):

MGSSHHHHHHSSGLVPRGSHMETLILTQEEVESLISMDEAMNAVEEAFRLYALGKAQ
MPPKVYLEFEKGDLRAMPAHLMGYAGLKWVNSHPGNPDKGLPTVMALMILNSPETG
FPLAVMDATYTTSLRTGAAGGIAAKYLARKNSSVFGFIGCGTQAYFQLEALRRVFDIG
EVKAYDVREKAAKKFVSYCEDRGISASVQPAEEASRCDVLVTTTPSRKPVVKAEWVE
EGTHINAIGADGPGKQELDVEILKKAKIVVDDLEQAKHGGEINVAVSKGVIGVEDVHATI
GEVIAGLKDGRESDEEITIFDSTGLAIQDVAVAKVVYENALSKNVGSKIKFFRI*

>arcB_-_1484888 arcB: alanine dehydrogenase

ATGGAGACTCTTATTTTGACTCAGGAGGAAGTAGAATCCCTGATATCGATGGACGA
GGCGATGAATGCTGTTGAGGAGGC
GTTCAGACTTTACGCTCTTGGTAAAGCTCAAATGCCGCCAAAGGTGTATCTTGAGTT
CGAAAAAGGCGATTTAAGGGCTA
TGCCGGCGCATTTAATGGGGTACGCAGGATTGAAGTGGGTGAACTCCCATCCCGG
AAATCCGGATAAAGGCCTGCCAACA
GTCATGGCGTTGATGATACTGAACAGCCCTGAGACGGGGTTTCCCTTGGCTGTGAT
GGACGCAACCTACACGACCTCTCT
GAGAACCGGTGCTGCTGGAGGAATAGCGGCGAAGTATCTGGCAAGGAAGAACAGC
AGCGTTTTTGGATTTATCGGCTGCG
GAACTCAGGCGTATTTCCAGCTCGAAGCTCTGAGGAGAGTGTTTGATATAGGAGAA
GTCAAGGCCTACGATGTTAGGGAA
AAGGCAGCGAAAAAGTTCGTTAGCTATTGCGAAGATAGGGGAATCTCAGCTTCCGT
GCAGCCTGCTGAGGAGGCGAGCAG
ATGCGATGTCCTTGTCACCACAACTCCGTCACGAAAACCAGTTGTTAAGGCTGAGT
GGGTTGAGGAGGGGACGCACATAA
ACGCAATCGGTGCGGACGGGCCTGGAAAGCAGGAACTGGATGTAGAAATACTTAA
AAAGGCTAAAATTGTTGTCGATGAC
CTTGAACAGGCCAAGCACGGCGGGGAGATTAATGTGGCGGTTTCAAAAGGCGTCA
TAGGTGTTGAAGATGTTCACGCAAC
CATCGGGGAGGTTATAGCCGGTCTGAAGGATGGAAGGGAGAGCGATGAGGAGATT
ACAATCTTCGACTCCACAGGCTTGG
CGATTCAGGACGTGGCTGTTGCAAAAGTCGTTTATGAGAACGCCCTTAGCAAGAAC
GTGGGGAGTAAAATAAAGTTTTTC
AGGATATGA

The following primers were used to amplify the gene and to clone it into the pET28b(+)-expression vector from Novogen including an N-terminal His₆-tag.
Af_AADH_Fw: CGCGCGGCAGCCATATGGAGACTCTTATTTTGACTC
Af_AADH_Rv: GTGCGGCCGCAAGCTTTTATATCCTGAAAAACTTTATTTTAC
Ndel and Hindlll restriction sites were used to digest vector and amplified gene.

**AlaDH from *Thermus thermophilus* (DSM 579)**. Genomic DNA was ordered from the DSMZ strain collection.
GenBank protein ID sequence: BAD70039.1
Uniprot protein ID sequence: Q5SLS7
Crystal structures (PDB): 2EEZ
Protein sequence with *N*-terminal His₆-tag and linker (first 20 AA underlined):

MGSSHHHHHHSSGLVPRGSHMVIGVPKEIKTLENRVALTPGGVESLVRRGHTVLVER
GAGEGSGLSDAEYARAGAELVGREEAWGAEMVVKVKEPLPEEYGFLREGLILFTYLH
LAADRGLTEAMLRSGVTGIAYETVQLPDGTLPLLVPMSEVAGRMAPQVGAQFLEKPK
GGRGVLLGGVPGVAPASVVILGGGTVGTNAAKIALGMGAQVTILDVNHKRLQYLDDV
FGGRVITLTATEANIKKSVQHADLLIGAVLVPGAKAPKLVTRDMLSLMKEGAVIVDVAV
DQGGCVETIRPTTHAEPTYVVDGVVHYGVANMPGAVPRTSTFALTNQTLPYVLKLAE
KGLDALLEDAALLKGLNTHKGRLTHPGVAEAFGLPYTPPEEALRG*
>TTHA0216_-_3168110_(reversed) Thermus thermophilus HB8
ATGGTGATCGGCGTGCCGAAGGAGATCAAGACCTTGGAGAACCGGGTGGCCCTCA
CGCCCGGCGGGGTGGAGAGCCTGGT
CAGGCGCGGCCACACCGTGCTGGTGGAGCGGGGGGCCGGGGAGGGCTCGGGGC
TTTCCGACGCGGAGTACGCCCGGGCCG
GGGCCGAGCTCGTGGGCCGGGAGGAGGCCTGGGGTGCGGAGATGGTGGTGAAG
GTGAAGGAGCCCCTACCCGAGGAGTAC
GGCTTCCTGCGGGAGGGCCTCATCCTCTTCACCTACCTTCACCTGGCCGCGGACC
GCGGCCTCACCGAGGCCATGCTCCG
TAGCGGGGTCACGGGCATCGCCTACGAGACCGTCCAGCTTCCCGACGGCACCCTC
CCCCTCCTCGTCCCCATGAGCGAGG
TGGCGGGGCGGATGGCCCCCCAGGTGGGGGCCCAGTTCCTGGAGAAGCCCAAGG
GGGGCCGGGGGGTCCTCCTCGGGGGG
GTGCCGGGGGTGGCCCCGGCCAGCGTGGTGATCCTCGGGGGCGGGACCGTGGG
CACCAACGCGGCCAAGATCGCCCTGGG

GATGGGGGCCCAGGTGACCATCCTGGACGTGAACCACAAGCGCCTCCAGTATCTG
GACGACGTCTTCGGCGGCGGGTGA
TCACCCTCACCGCCACCGAGGCCAACATCAAAAAGAGCGTCCAGCACGCGGACCT
CCTCATCGGGGCCGTCCTCGTCCCC
GGGGCCAAGGCCCCCAAGCTCGTCACCCGGGACATGCTCTCTCTGATGAAGGAGG
GAGCGGTGATCGTGGACGTGGCCGT
GGACCAGGGGGGGTGCGTGGAGACCATCCGGCCCACCACCCACGCCGAGCCCAC
CTACGTGGTGGACGGGGTGGTCCACT
ACGGGGTGGCCAACATGCCCGGGGCGGTGCCCAGGACCAGCACCTTCGCCCTCA
CCAACCAGACCCTGCCCTACGTGTTG
AAGCTCGCGGAGAAGGGGCTGGACGCCCTTCTGGAGGACGCGGCCCTTCTCAAG
GGGCTCAACACCCACAAAGGCCGCCT
CACCCACCCCGGGGTGGCCGAGGCCTTCGGCCTGCCCTACACGCCTCCCGAGGA
GGCCTTGAGGGGGTGA

The following primers were used to amplify the gene and to clone it in pET28b(+)-expression vector from Novogen including an N-terminal His₆-tag.
Tt_AADH_Fw:CGCGCGGCAGCCATATGGTGATCGGCGTGC
Tt_AADH_Rv:GTGCGGCCGCAAGCTTTTACCCCCTCAAGGCCTCC
Ndel and HindIII restriction sites were used to digest vector and amplified gene.

**AlaDH from *Vibrio proteolyticus* (DSM 30189).**
Genomic DNA was ordered from DSMZ strain collection.
GenBank protein ID sequence: AF070716.1
Uniprot protein ID sequence: 085596

Protein sequence with *N*-terminal His₆-tag and linker (first 20 AA underlined): 
MGSSHHHHHHSSGLVPRGSHMIIGVPKEIKNHEYRVGMIPASVRELISHGHQVFVETN
AGAGIGFSDDDYIAVGASILPTAAEVFAQADMIVKVKEPQAVERAMLKEGQILFTYLHL
APDFPQTEDLIKSKAVCIAYETVTDNMGRLPLLAPMSEVAGRMSIQAGAQTLEKSHGG
RGLLLGGVPGVEPAKVVIVGGGVVGANAARMAVGMRADVTILDRNIDTLRKLDEEFQ
GRAKVVYSTEDAIEKHVLAADLVIGAVLIPGAAAPKLVTKEHIAKMKPGAAVVDVAIDQ
GGCFETSHATTHADPTYIVDDVVHYCVANMPGAVARTSTFALNNATLPYIVKLANKGY
REALLADHGFLEGLNVIHGKVTCKEVAEAFNLEYVQPETAIAMFN*
 >VP_ADH.fasta Vibrio proteolyticus (DSM 30189) 
GATCCTTGGGCGGCTTTTTATAGTTGTCTGCCATTTTTTATTCCACCTTATTACTTCCT
TGCAAAAAATATACTACAAC
TTTTTAATATTCAGTATCAAATTTTACTGACTCACATCTATACTAGACTTTAATTCGACA
GAATTACCCTACACATAGTC
AATACAACCACAATACAATGAGGAGTCAGGATGATCATTGGCGTACCTAAGGAAATCA
AAAACCACGAATACCGCGTCGG
TATGATTCCAGCAAGTGTCCGTGAGCTAATCTCACATGGTCACCAAGTTTTTGTTGAA
ACCAATGCCGGCGCAGGCATCG
GTTTTTCAGACGATGATTACATCGCTGTAGGCGCATCCATTCTTCCTACTGCTGCTGA
GGTTTTCGCTCAAGCAGATATG
ATTGTTAAGGTCAAAGAACCTCAAGCTGTCGAACGAGCTATGCTCAAAGAAGGGCAA
ATTTTATTTACTTATTTGCACCT
GGCACCAGATTTTCCACAAACTGAAGACCTGATCAAGAGCAAAGCTGTCTGCATAGC
CTATGAGACTGTAACAGATAATA
TGGGTCGCTTGCCACTATTAGCACCAATGTCTGAAGTTGCGGGTCGTATGTCTATCC
AGGCAGGCGCACAAACTCTCGAG
AAATCTCACGGCGGCCGTGGCCTGCTGCTGGGTGGCGTACCAGGTGTTGAACCAG
CGAAAGTTGTGATCGTCGGCGGCGG
CGTCGTGGGTGCTAACGCAGCTCGTATGGCAGTTGGCATGCGCGCCGACGTTACTA
TCTTAGACCGTAACATCGATACAC
 
TGCGTAAGCTGGATGAAGAGTTTCAGGGCCGTGCCAAAGTGGTTTACTCAACTGAA
GATGCGATTGAAAAACACGTTCTG
GCGGCCGATCTGGTTATCGGTGCAGTTCTGATCCCAGGTGCCGCAGCTCCTAAACT
GGTGACAAAAGAGCACATCGCTAA
AATGAAACCAGGCGCGGCTGTTGTCGACGTTGCTATCGACCAGGGCGGTTGTTTTG
AAACTTCTCACGCGACAACGCACG
CAGATCCAACTTACATCGTTGATGATGTGGTTCACTACTGTGTAGCAAACATGCCAG
GGGCTGTTGCCCGCACTTCCACT
TTTGCACTGAACAACGCAACCCTGCCATATATCGTTAAACTGGCAAACAAAGGATACC
GTGAAGCTCTGCTGGCTGACCA
CGGCTTCCTAGAAGGCCTGAATGTTATTCACGGTAAAGTGACGTGCAAAGAAGTCG
CAGAAGCGTTTAATCTGGAATACG
TACAGCCGGAAACCGCGATCGCCATGTTCAACTAAGCACTGCTGAACGAATAAAGAA
AGCTCTCACCTTGGTGAGAGCTT
TTTTGTTTATCAGAACCAGCGCTCTAACGCAGTTTATCCAACTGCCGGAACGCCCG
GTTGAGAATCGCAGCCAACTCTT
TATAGCGTGGACGAGATTTGACCGGTTCCAATGCGCAGCCGGTATCAACAATTTTCT
GGTGGATTTCACGATACCAACCA
GCCAACGCTGGAGGTAACTGGGTATTGGCTCGATTCCCCAGCCACCATATACCTTG
TACTGGATC
 Flanking regions of the gene are underlined.

The following primers were used to amplify the gene and to clone it into the pET28b(+)-expression vector from Novogen including an N-terminal His₆-tag.
Vp_AADH_Fw:CGCGCGGCAGCCATATGATCATTGGCGTACC
Vp_AADH_Rv: GTGCGGCCGCAAGCTTTTAGTTGAACATGGCG
Ndel and Hindlll restriction sites were used to digest vector and amplified gene.

### Glutamate dehydrogenases

*Bacillus subtilis* Glutamate DH:
A NAD-dependant glutamate dehydrogenase.

GeneBank protein ID: AB194695
Uniprot protein ID: P39633
PDB-code: 3K92

Protein sequence:

MSAKQVSKDEEKEALNLFLSTQTIIKEALRKLGYPGDMYELMKEPQRMLTVRIPVKMD
NGSVKVFTGYRSQHNDAVGPTKGGVRFHPEVNEEEVKALSIWMTLKCGIANLPYGG
GKGGIICDPRTMSFGELERLSRGYVRAISQIVGPTKDIPAPDVYTNSQIMAWMMDEYS
RLREFDSPGFITGKPLVLGGSQGRETATAQGVTICIEEAVKKKGIKLQNARIIIQGFGNA
GSFLAKFMHDAGAKVIGISDANGGLYNPDGLDIPYLLDKRDSFGMVTNLFTDVITNEEL
LEKDCDILVPAAISNQITAKNAHNIQASIVVEAANGPTTIDATKILNERGVLLVPDILASAG
GVTVSYFEWVQNNQGYYWSEEEVAEKLRSVMVSSFETIYQTAATHKVDMRLAAYMT
GIRKSAEASRFRGWV
MSAKQVSKDEEKEALNLFLSTQTIIKEALRKLGYPGDMYELMKEPQRMLTVRIPVKMD
NGSVKVFTGYRSQHNDAVGPTKGGVRFHPEVNEEEVKALSIWMTLKCGIANLPYGG
GKGGIICDPRTMSFGELERLSRGYVRAISQIVGPTKDIPAPDVYTNSQIMAWMMDEYS
RLREFDSPGFITGKPLVLGGSQGRETATAQGVTICIEEAVKKKGIKLQNARIIIQGFGNA
GSFLAKFMHDAGAKVIGISDANGGLYNPDGLDIPYLLDKRDSFGMVTNLFTDVITNEEL
LEKDCDILVPAAISNQITAKNAHNIQASIVVEAANGPTTIDATKILNERGVLLVPDILASAG
GVTVSYFEWVQNNQGYYWSEEEVAEKLRSVMVSSFETIYQTAATHKVDMRLAAYMT
GIRKSAEASRFRGWV
MSAKQVSKDEEKEALNLFLSTQTIIKEALRKLGYPGDMYELMKEPQRMLTVRIPVKMD
NGSVKVFTGYRSQHNDAVGPTKGGVRFHPEVNEEEVKALSIWMTLKCGIANLPYGG
GKGGIICDPRTMSFGELERLSRGYVRAISQIVGPTKDIPAPDVYTNSQIMAWMMDEYS
RLREFDSPGFITGKPLVLGGSQGRETATAQGVTICIEEAVKKKGIKLQNARIIIQGFGNA
GSFLAKFMHDAGAKVIGISDANGGLYNPDGLDIPYLLDKRDSFGMVTNLFTDVITNEEL
LEKDCDILVPAAISNQITAKNAHNIQASIVVEAANGPTTIDATKILNERGVLLVPDILASAG
GVTVSYFEWVQNNQGYYWSEEEVAEKLRSVMVSSFETIYQTAATHKVDMRLAAYMT
GIRKSAEASRFRGWV

Gene:

ATGTCAGCAAAGCAAGTCTCGAAAGATGAAGAAAAAGAAGCTCTTAACTTATTTCTG
TCTACCCAAACAATCATTAAGGAAGCCCTTCGGAAGCTGGGTTATCCGGAGATAT
GTATGAACTCATGAAAGAGCCGCAGAGAATGCTCACTGTCCGCATTCCGGTCAAAA
TGGACAATGGGAGCGTCAAAGTGTTCACAGGCTACCGGTCACAGCACAATGATGC
TGTCGGTCCGACAAAGGGGGGCGTTCGCTTCCATCCAGAAGTTAATGAAGAGGAA
GTAAAGGCATTATCCATTTGGATGACGCTCAAATGCGGGATTGCCAATCTTCCTTAC
GGCGGCGGGAAGGGCGGTATTATTTGTGATCCGCGGACAATGTCATTTGGAGAAC
TGGAAAGGCTGAGCAGGGGGTATGTCCGTGCCATCAGCCAGATCGTCGGTCCGA
CAAAGGATATTCCAGCTCCCGATGTGTACACCAATTCGCAGATTATGGCGTGGATG
ATGGATGAGTACAGCCGGCTGCGGGAATTCGATTCTCCGGGCTTTATTACAGGTAA
ACCGCTTGTTTTGGGAGGATCGCAAGGACGGAAACAGCGACGGCACAGGGCGT
CACGATTTGTATTGAAGAGGCGGTGAAGAAAAAGGGATCAAGCTGCAAAACGCG
CGCATCATCATACAGGGCTTTGGAAACGCGGGTAGCTTCCTGGCCAAATTCATGCA
CGATGCGGGCGCGAAGGTGATCGGGATTTCTGATGCCAATGGCGGGCTCTACAAC
CCAGACGGCCTTGATATCCCTTATTTGCTCGATAAACGGGACAGCTTTGGTATGGT

CACCAATTTATTTACTGACGTCATCACAAATGAGGAGCTGCTTGAAAAGGATTGCGA
TATTTTAGTGCCTGCCGCGATCTCCAATCAAATCACAGCCAAAAACGCACATAACAT
TCAGGCGTCAATCGTCGTTGAAGCGGCGAACGGCCCGACAACCATTGATGCCACT
AAGATCCTGAATGAAAGAGGCGTGCTGCTTGTGCCGGATATCCTAGCGAGTGCCG
GCGGCGTCACGGTTTCTTATTTTGAATGGGTGCAAAACAACCAAGGATATTATTGGT
CGGAAGAAGAGGTTGCAGAAAAACTGAGAAGCGTCATGGTCAGCTCGTTCGAAAC
AATTTATCAAACAGCGGCAACACATAAAGTGGATATGCGTTTGGCGGCTTACATGAC
GGGCATCAGAAAATCGGCAGAAGCATCGCGTTTCCGCGGATGGGTCTAA

Glutamate DH from *Pyrobaculum islandicum:*
A extremly thermostable NAD-dependant glutamate dehydrogenase

GeneBank protein ID: AB027194
Uniprot protein ID: Q9Y8I4
PDB-code: 1V9L

Protein-Sequence:

MERTGFLEYVLNYVKKGVELGGFPEDFYKILSRPRRVLIVNIPVRLDGGGFEVFEGYR
VQHCDVLGPYKGGVRFHPEVTLADDVALAILMTLKNSLAGLPYGGAKGAVRVDPKKL
SQRELEELSRGYARAIAPLIGDVVDIPAPDVGTNAQIMAWMVDEYSKIKGYNVPGVFTS
KPPELWGNPVREYATGFGVAVATREMAKKLWGGIEGKTVAIQGMGNVGRWTAYWLEK
MGAKVIAVSDINGVAYRKEGLNVELIQKNKGLTGPALVELFTTKDNAEFVKNPDAIFKLD
VDIFVPAAIENVIRGDNAGLVKARLVVEGANGPTTPEAERILYERGVVVVPDILANAGGV
IMSYLEWVENLQWYIWDEEETRKRLENIMVNNVERVYKRWQREKGWTMRDAAIVTAL
ERIYNAMKIRGWI

Gene-Sequence:

ATGGAGAGGACAGGGTTTTAGAGTATGTGCTTAACTATGTAAAAAAGGCGTAGAA
TTAGGCGGATTTCCCGAAGATTTCTATAAAATCTTGTCTAGGCCTAGGCGTGTTTTG
ATTGTTAATATTCCTGTGAGGTTGGATGGTGGTGGTTTTGAGGTGTTTGAGGGTTAT
CGTGTGCAGCATTGTGATGTTCTCGGCCCGTATAAGGGTGGGGTTCGTTTTCATCC
TGAGGTTACTCTTGCCGACGACGTCGCCTTGGCTATTTTAATGACGTTGAAAAATAG
CCTAGCTGGCTTGCCGTATGGCGGCGCTAAAGGCGCAGTACGTGTAGACCCTAAG
AAACTCTCGCAGAGAGAACTTGAAGAGCTCTCTAGAGGATATGCCAGAGCCATAGC
CCCCCTCATAGGCGACGTAGTCGACATCCCAGCGCCTGACGTTGGGACAAACGCC
CAAATCATGGCGTGGATGGTAGACGAATATTCAAAAATAAAGGGCTACAACGTGCC
CGGGGTATTCACCTCAAAACCACCAGAGCTATGGGGAAACCCAGTGAGAGAATAC
GCCACAGGCTTTGGAGTAGCAGTAGCGACAAGAGAAATGGCAAAAAAACTATGGG
GCGGAATAGAAGGAAAAACAGTAGCGATACAGGGTATGGGTAATGTGGGGAGGTG
GACAGCGTATTGGCTAGAAAAAATGGGCGCTAAGGTGATAGCTGTGTCTGATATAAA
TGGCGTAGCTTATAGAAAGGAGGGACTTAATGTTGAATTGATACAAAAAAACAAGG
GACTTACAGGGCCTGCTCTCGTAGAACTGTTTACGACAAAAGACAATGCCGAATTT
GTTAAAAACCCAGATGCCATATTTAAACTAGACGTCGACATTTTCGTCCCCGCCGCT
ATTGAGAATGTCATTAGGGGTGATAATGCTGGGCTTGTGAAGGCTAGGCTTGTTGT
TGAGGGTGCTAATGGCCCTACTACTCCTGAGGCTGAGAGGATTTTGTATGAGAGG
GGGGTTGTCGTAGTTCCTGATATCCTCGCCAACGCCGGCGGCGTGATTATGTCGTA
TTTAGAGTGGGTGGAGAATCTCCAGTGGTATATCTGGGACGAGGAGGAGACTAGA
AAGAGACTTGAAAACATAATGGTAAACAACGTCGAGAGAGTATACAAGAGATGGCA
GAGAGAAAAAGGATGGACAATGAGAGACGCAGCAATAGTAACCGCATTAGAAAGAA
TATACAACGCCATGAAAATAAGAGGGTGGATCTAA

Glutamate Dehydrogenase from *Clostridium symbiosum*

A NAD-dependant glutamate dehydrogenase.

GeneBank protein ID: Z11747
Uniprot protein ID: **P24295**
PDB-code: 1 BGV

Protein sequence:

MSKYVDRVIAEVEKKYADEPEFVQTVEEVLSSLGPVVDAHPEYEEVALLERMVIPERVI
EFRVPWEDDNGKVHVNTGYRVQFNGAIGPYKGGLRFAPSVNLSIMKFLGFEQAFKDS
LTTLPMGGAKGGSDFDPNGKSDREVMRFCQAFMTELYRHIGPDIDVPAGDLGVGARE
IGYMYGQYRKIVGGFYNGVLTGKARSFGGSLVRPEATGYGSVYYVEAVMKHENDTLV
GKTVALAGFGNVAWGAAKKLAELGAKAVTLSGPDGYIYDPEGITTEEKINYMLEMRAS
GRNKVQDYADKFGVQFFPGEKPWGQKVDIIMPCATQNDVDLEQAKKIVANNVKYYIEV
ANMPTTNEALRFLMQQPNMVVAPSKAVNAGGVLVSGFEMSQNSERLSWTAEEVDSK
LHQVMTDIHDGSAAAAERYGLGYNLVAGANIVGFQKIADAMMAQGIAW

Gene-Sequence:

ATGAGCAAGTATGTTGACAGAGTAATTGCGGAAGTCGAGAAGAAATACGCAGACGA
GCCGGAGTTCGTACAAACAGTAGAAGAGGTTTTATCTTCTTTAGGTCCGGTTGTTG
ATGCACATCCGGAGTATGAGGAAGTAGCTCTTCTCGAGAGAATGGTAATTCCGGAG
AGAGTTATCGAGTTCCGTGTTCCGTGGGAGGATGACAACGGCAAGGTTCATGTAA
ACACAGGCTACCGCGTTCAGTTCAACGGCGCCATTGGACCGTACAAGGGCGGCTT
ACGTTTTGCTCCGTCTGTAAACCTTTCCATCATGAAATTCTTAGGCTTTGAGCAGGC
ATTCAAAGACAGCCTTACAACACTTCCTATGGGTGGTGCCAAAGGTGGTTCCGACT
TCGATCCGAACGGTAAGAGCGACAGAGAAGTTATGAGATTCTGCCAGGCTTTCATG
ACAGAATTATACCGTCACATCGGACCGGACATTGATGTTCCTGCAGGTGACCTTGG
TGTAGGCGCTCGTGAAATCGGTTATATGTACGGACAGTATCGTAAAATTGTCGGCG
GATTCTACAACGGCGTTCTGACAGGTAAGGCAAGATCCTTCGGTGGTTCCTTAGTT
CGTCCGGAAGCTACAGGATACGGCTCCGTATACTATGTAGAAGCAGTTATGAAACAT
GAGAACGATACCCTGGTTGGCAAGACAGTTGCATTAGCAGGTTTTGGTAACGTAGC
ATGGGGCGCTGCTAAGAAGCTTGCTGAATTAGGCGCTAAAGCAGTTACACTTTCCG
GACCAGACGGATACATCTACGATCCGGAAGGCATCACAACAGAAGAGAAGATCAA
CTACATGCTTGAGATGAGAGCATCCGGACGTAACAAAGTTCAGGATTATGCAGACA
AGTTCGGCGTACAGTTCTTCCCGGGTGAGAAACCATGGGGCCAGAAGGTTGACAT
CATCATGCCATGCGCTACTCAGAACGATGTTGATTTAGAGCAGGCTAAGAAGATCG
TTGCTAACAACGTTAAATACTACATCGAAGTTGCTAACATGCCAACTACCAACGAAG
CTCTCAGATTCTTAATGCAGCAGCCAAACATGGTAGTAGCTCCTTCCAAGGCTGTA
AACGCAGGCGGCGTTCTTGTATCCGGCTTTGAGATGAGCCAGAACAGCGAGAGAC
TTTCCTGGACAGCAGAGGAAGTTGACTCCAAACTTCATCAGGTTATGACAGATATC
CATGACGGTTCCGCTGCAGCAGCAGAGCGTTACGGCTTAGGCTACAATCTGGTTG
CCGGCGCTAACATCGTTGGATTCCAGAAGATTGCTGATGCTATGATGGCTCAGGGC
ATTGCTTGGTAA

**Glutamate dehydrogenase from *Thermococcus profundus***
A NAD-dependant glutamate dehydrogenase.

GeneBank protein ID: D87814
**Uniprot protein ID: O74024**
**PDB-code: 1 EUZ**

Protein-sequence:

MVEIDPFEMAVKQLERAAQYMDISEEALEWLKKPMRIVEVSVPIEMDDGSVKVFTGFR
VQHNWARGPTKGGIRWHPAETLSTVKALATWMTWKVAVVDLPYGGGKGGIIVNPKEL
SEREQERLARAYIRAVYDVIGPWTDIPAPDVYTNPKIMGWMMDEYETIMRRKGPAFGV
ITGKPLSIGGSLGRGTATAQGAIFTIREAAKALGIDLKGKKIAVQGYGNAGYYTAKLAKE
QLGMTVVAVSDSRGGIYNPDGLDPDEVLKWKREHGSVKDFPGATNITNEELLELEVD
VLAPAAIEEVITEKNADNIKAKIVAEVANGPVTPEADDILREKGILQIPDFLCNAGGVTVS
YFEWVQNINGYYWTEEEVREKLDKKMTKAFWEVYNTHKDKNIHMRDAAYVVAVSRV
YQAMKDRGWVKK

Gene-Sequence:

ATGGTCGAGATTGACCCGTTTGAGATGGCCGTTAAGCAGCTTGAGAGGGCTGCTC
AGTACATGGACATAAGCGAAGAGGCCCTCGAGTGGCTTAAGAAGCCCATGAGGAT
CGTCGAAGTTAGCGTTCCGATAGAGATGGACGACGGTTCTGTTAAGGTTTTCACCG
GTTTCCGCGTTCAGCACAACTGGGCTCGCGGCCCGACCAAGGGTGGTATAAGGT
GGCACCCGGCCGAGACCCTCAGCACCGTTAAGGCCCTCGCTACCTGGATGACCT
GGAAGGTTGCAGTTGTCGACCTCCCCTACGGTGGAGGTAAGGGTGGCATCATCGT
CAACCCGAAGGAGCTCTCCGAGAGGGAGCAGGAGAGGCTCGCTCGCGCTTACAT
AAGGGCCGTTTATGACGTCATCGGTCCGTGGACCGACATTCCGGCTCCCGACGTC
TACACCAACCCGAAGATCATGGGCTGGATGATGGACGAGTATGAAACAATAATGAG
GAGGAAGGGCCCGGCCTTCGGTGTCATCACTGGAAAGCCGCTCAGCATTGGCGG
TTCACTCGGTAGGGGTACAGCAACAGCCCAGGGTGCAATATTCACCATCAGGGAG
GCCGCCAAGGCCCTCGGCATCGACCTCAAGGGCAAGAAGATCGCCGTCCAGGGC
TATGGTAACGCCGGCTACTACACCGCCAAGCTCGCCAAGGAGCAGCTCGGCATGA
CCGTCGTCGCCGTCAGCGACAGCCGCGGCGGCATCTACAACCCGGACGGCCTC
GACCCGGACGAGGTTCTCAAGTGGAAGAGGGAGCACGGCTCAGTTAAGGACTTC
CCAGGAGCGACCAACATCACCAACGAGGAGCTCCTCGAGCTCGAGGTAGACGTC
CTCGCTCCGGCAGCTATCGAAGAGGTCATCACCGAGAAGAACGCCGACAACATCA
AGGCCAAGATCGTCGCCGAGGTTGCCAACGGTCCAGTTACGCCAGAGGCAGACG
ACATCCTCCGCGAGAAGGGCATCCTCCAGATTCCGGACTTCCTGTGCAACGCCGG
TGGCGTCACCGTCAGCTACTTCGAGTGGGTCCAGAACATAAACGGCTACTACTGG
ACCGAGGAGGAGGTCCGCGAGAAGCTCGACAAGAAGATGACCAAGGCCTTCTGG
GAGGTCTACAACACCCACAAGGACAAGAACATCCACATGAGGGACGCCGCCTACG
TCGTCGCGGTCAGCAGGGTTTACCAGGCCATGAAGGACCGCGGATGGGTGAAGA
AG

## Claims

1. A biocatalytic method of preparing an aliphatic primary amine, which method comprises:
a) the enzymatic oxidation the corresponding aliphatic primary alcohol of said amine in the presence of the cofactor NAD(P)⁺ and of an alcohol dehydrogenase (AlcDH) (E.C.1.1.1.x) to form the corresponding oxo compound of said alcohol, and wherein the cofactor NAD(P)⁺ is reduced to form NAD(P)H;
b) the enzymatic conversion of said oxo compound in the presence of
b1) an alpha-amino carboxylic acid of the formula III
Z-CH(NH₂)COOH (III)
wherein
Z is the side chain of a natural or artificial alpha-amino acid; and
b2) a transaminase (E.C.2.6.1.x)
to form the desired aliphatic amine while converting the sacrificial alpha-amino carboxylic acid to the corresponding alpha-keto carboxylic acid of the formula IV
Z-C(=O)COOH (IV)
wherein Z is as defined above.

2. The method of claim 1, which further comprises a coupled process of cofactor regeneration for regenerating the consumed cofactor NAD(P)⁺.

3. The method of claim 2, wherein said coupled cofactor regenerating process also comprises the regeneration of the consumed sacrificial alpha-amino carboxylic acid.

4. The method of claim 3, wherein said cofactor regenerating process encompasses the enzymatic reduction of said alpha-keto carboxylic acid of the formula IV to regenerate the alpha-amino carboxylic acid of the formula III, and the simultaneous oxidation of NAD(P)H to regenerate the consumed cofactor NAD(P)⁺ in the presence of an alpha amino acid dehydrogenase (AADH) (E.C. 1.4.1.x) and a source of ammonia.

5. The method of one of the preceding claims, wherein aliphatic alcohol is a mono-or polyvalent aliphatic alcohol carrying one or more "terminal" hydroxy groups, and wherein said terminal hydroxy groups of said aliphatic alcohol are either partly or fully aminated by said method.

6. A biocatalytic method of preparing a primary amine of the general formula I or la
RNH₂ (I)
R'(NH₂)ₘ (Ia)
wherein
m is an integer of at least 2, like 2, 3, 4, or 5; in particular 2;
R is selected from straight chain or branched hydrocarbyl, straight chain or branched hydroxyhydrocarbyl or straight chain or branched alkoxyhydrocarbyl or a polyhydrocarbylether group;
R' is selected from straight chain or branched hydrocarbylene, a polyhydrocarbyleneether group;
which method comprises
a) the enzymatic oxidation an alcohol of the general formula II or IIa
ROH (II)
R'(OH)ₘ (IIa)
wherein
m, R' and R is as defined above
in the presence of the cofactor NAD(P)⁺ and of an alcohol dehydrogenase (AlcDH) (E.C.1.1.1.x) to form the corresponding oxo compound of said alcohol, and wherein the cofactor NAD(P)⁺ is reduced to form NAD(P)H;
b) the enzymatic conversion of said oxo compound in the presence of
b1) a sacrificial alpha-amino carboxylic acid of the formula III
Z-CH(NH₂)COOH (III)
wherein
Z is the side chain of a natural or artificial alpha-amino acid and
b2) a transaminase (E.C.2.6.1.x)
to form the desired amine of the above formula I while converting the sacrificial alpha-amino carboxylic acid to the corresponding alpha-keto carboxylic acid of the formula IV
Z-C(=O)COOH (IV)
wherein
Z is the residue of a natural or artificial alpha-amino acid.

7. The method of claim 6, which further comprises a coupled process for regenerating the consumed cofactor NAD(P)⁺.

8. The method of claim 7, wherein said cofactor regenerating process also comprises a regeneration step of the consumed sacrificial alpha-amino carboxylic acid.

9. The method of claim 8, wherein said cofactor regenerating step encompasses the enzymatic reduction of said alpha-keto carboxylic acid of the formula IV to regenerate the alpha-amino carboxylic acid of the formula III, and the simultaneous oxidation of NAD(P)H to regenerate the consumed cofactor NAD(P)⁺ in the presence of an alpha amino acid dehydrogenase (AADH) (E.C. 1.4.1.x) and a source of ammonia.

10. The method of one of the preceding claims, which is performed in an aqueous reaction medium, optionally in the presence of at least one organic water-miscible or water immiscible co-solvent.

11. The method of one of the preceding claims wherein the individual reactions are performed consecutively or simultaneously in the same reaction mixture.

12. The method of claim 1 or 6, wherein said transaminase is an omega transaminase (ω-TA) (E.C.2.6.1.18.

13. The method of claim 4 or 9, wherein said alpha amino acid dehydrogenase (AADH) is selected from:
a) an alanine dehydrogenase (AlaDH) (E.C. 1.4.1.1) or
b) a glutamate dehydrogenase (GluDH) (E.C. 1.4.1.2, 1.4.1.3 or 1.4.1.4).

14. The method of one of the preceding claims wherein the enzymes are selected as follows:
a) alcohol dehydrogenase having an amino acid sequence according to SEQ ID NO 11, 16, 21, 27, 31 or an enzyme having a sequence having sequence identity of at least 60% to SEQ ID NO: 11, 16, 21, 27, 31
b) amino group transferring enzymes, selected from omega transaminase (E.C.2.1.6.18) (i.e. a transaminase capable of aldehyde amination) having an amino acid sequence according to SEQ ID NO:3, 6, 57 or 61; L-ornithine aminotransferases (EC 2.6.1.13) having an amino acid sequence according to SEQ ID NO:62, 63, 64, 65 or 66; or L-lysine-6-transaminases (2.6.1.36) having an amino acid sequence according to SEQ ID NO: 68 or 70; .or an enzyme having a sequence having sequence identity of at least 60% sequence identity, to SEQ ID NO:3, 6, 57, 61, 62, 63, 64, 65, 66, 68 or 70; and still retaining the intended enzyme activity (or function), i.e. being applicable as amino group transferring enzyme;
c) alanine dehydrogenase having an amino acid sequence according to SEQ ID NO:34, 39 or 44 or an enzyme having a sequence having sequence identity of at least 60% to SEQ ID NO: 34, 39 or 44; or a glutamate dehydrogenase having an amino acid sequence according to SEQ ID NO: 49, 51, 53 or 55 or an enzyme having a sequence having sequence identity of at least 60% to SEQ ID NO: 49, 51, 53 or 55.

15. The method of one of the preceding claims wherein the process is performed in the presence of the set of required enzymes in isolated form, one or more recombinant microorganisms expressing the required set of enzymes or cell homogenates or cell lysates obtained therefrom.

16. The method of one of the preceding claims wherein the alcohol substrates are selected from:
a) monovalent alcohols of the formulae:
AO-(-C₂-C₆-alkylene-O-)ₙ-C₂-C₆-alkylene-OH
wherein
A is C₁-C₂₂-alkyl, or
AO replaced by H,
n is 0 or an integer from 1 to 100 and
wherein said alkyl and alkylene groups may be straight chain or branched; or
mixtures of such compounds;
b) bivalent alcohols of the formulae:
HO-C₁-C₁₂-alkylene-OH,
HO-(-C₂-C₆-alkylene-O-)ₙ-C₂-C₆-alkylene-OH
wherein n is an integer from 1 to 100 and said alkyl and alkylene groups may be straight chain or branched; or mixtures of such compounds;
c) trivalent alkohols 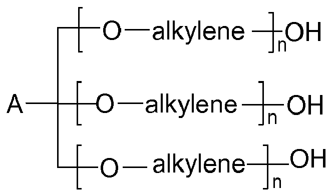 wherein
alkylene is a straight chain or branched C₂-C₆-alkylene group
A is H or C₁-C₁₂-alkyl, and parameters n are same or different and integers from 1 to 50;.or mixtures of such compounds.

17. The method of one of the preceding claims further comprising isolating the produced amine from the reaction medium.

18. The use of an amine, in particular polyetheramines, as prepared by a method of anyone of the preceding claims in the preparation of organic polymers.

19. A recombinant expression vector carrying under the control of at least one regulatory element the coding sequence of at least one of the following enzymes:
a) alcohol dehydrogenase having an amino acid sequence according to SEQ ID NO 11, 16, 21, 27, 31 or an enzyme having a sequence having sequence identity of at least 60% to SEQ ID NO: 11, 16, 21, 27, 31
b) amino group transferring enzymes, selected from omega transaminase (E.C.2.1.6.18) (i.e. a transaminase capable of aldehyde amination) having an amino acid sequence according to SEQ ID NO:3, 6, 57 or 61; L-ornithine aminotransferases (EC 2.6.1.13) having an amino acid sequence according to SEQ ID NO:62, 63, 64, 65 or 66; or L-lysine-6-transaminases (2.6.1.36) having an amino acid sequence according to SEQ ID NO: 68 or 70; .or an enzyme having a sequence having sequence identity of at least 60% sequence identity, to SEQ ID NO:3, 6, 57, 61, 62, 63, 64, 65, 66, 68 or 70; and still retaining the intended enzyme activity (or function), i.e. being applicable as amino group transferring enzyme;
c) alanine dehydrogenase having an amino acid sequence according to SEQ ID NO:34, 39 or 44 or an enzyme having a sequence having sequence identity of at least 60% to SEQ ID NO: 34, 39 or 44; or a glutamate dehydrogenase having an amino acid sequence according to SEQ ID NO: 49, 51, 53 or 55or an enzyme having a sequence having sequence identity of at least 60% to SEQ ID NO: 49, 51, 53 or 55.

20. A recombinant microorganism functionally expressing at least one enzyme as defined in the claims 1 to 14.

21. The recombinant microorganism of claim 20, carrying at least one expression vector according to claim 18 and functionally expressing said encoded enzymes.

22. The use of a recombinant expression vector of claim 19 or of a recombinant microorganism of claim 20 or 21 in a method as claimed in anyone of the claims 1 to 17.

23. A bioreactor for performing a biocatalytic method of anyone of the claims 1 to 16 containing in a reaction medium at least one recombinant microorganism of anyone of the claims 19 and 20, or at least one enzyme as defined in anyone of the claims 1 to 13 in free od immobilized form.
